# EUROPEAN PATENT APPLICATION

(11) **EP 4 810 029 A1**
(43) Date of publication of application: **23.09.2026**
(21) Application number: 24890751.1
(22) Date of filing: 14.11.2024
(51) Int. Cl.: A61K 9/51, A61K 9/127, A61K 47/24, C12N 15/11, A61P 9/00

(54) **PHARMACEUTICAL COMPOSITION COMPRISING NUCLEIC ACID CONSTRUCT AND MEDICAL USE THEREOF**

(30) Priority: 14.11.2023 CN 202311517076; 15.11.2023 CN 202311528241
(71) Applicant: Shanghai Regenelead Therapies Co., Ltd., Shanghai 201206 (CN)
(72) Inventor: LIU, Jun, Shanghai 201206 (CN); TANG, Xiaojiao, Shanghai 201206 (CN); TANG, Qi, Shanghai 201206 (CN); CHENG, Jiewei, Shanghai 201206 (CN); PU, Xiaorong, Shanghai 201206 (CN); NING, Wei, Shanghai 201206 (CN); LIAO, Cheng, Shanghai 201206 (CN)
(74) Representative: Regimbeau
(86) International application number: PCT/CN2024/131953
(87) International publication number: WO 2025/103396

(57) **Abstract**

A pharmaceutical composition comprising a nucleic acid construct and the medical use thereof. Specifically, the present invention relates to a lipid nanoparticle comprising a nucleic acid construct represented by formula I, which can achieve highly efficient delivery of a exogenous target gene, allowing the exogenous target gene to be highly efficiently and rapidly expressed in the body. The present invention has the advantages of no gene integration risk and being easy to scale up to an industrial level, is a more ideal treatment approach than naked plasmids, and can be used as a gene therapy drug for a plurality of diseases.

## Description

### FIELD OF THE INVENTION

The present disclosure relates to the field of pharmaceutical preparations, and specifically relates to a pharmaceutical composition comprising a nucleic acid construct and the medical use thereof.

### BACKGROUND OF THE INVENTION

Gene therapy and genetic vaccination can provide highly specific and personalized treatment and prevention regimens for a variety of diseases including genetic diseases, autoimmune diseases, cancer or tumor-related diseases, inflammatory diseases, and the like.

Both DNA and RNA can be used for gene therapy or genetic vaccination. DNA is stable and easy to manipulate; however, there is a risk of mutagenic events (such as loss of function of a damaged gene) and the like caused by the insertion of DNA fragments into the patient's genome. RNA can avoid undesirable genomic integration, but RNA is susceptible to degradation due to ubiquitous RNases. Therefore, there is a need to improve the stability of RNA to allow its encoded protein products to accumulate *in vivo* to achieve the treatment or prevention of diseases, and maintain the integrity of RNA structure and function during storage and administration. It has been found that naturally occurring eukaryotic mRNA molecules contain stabilizing elements, for example, the untranslated regions (UTRs) at the 5' end and 3' end, and other structural features such as a 5' cap structure or a 3' polyadenylation tail. The 5'UTR and 3'UTR are premature mRNA elements. During mRNA processing, structural features unique to mature mRNA (such as a 5' cap and a 3' polyadenylation tail) are added to the transcribed (premature) mRNA. Regarding the correlation between UTR and mRNA stability, studies have shown that the 3'UTR of α-globin mRNA is an important factor for the stability of α-globin mRNA (Nancy D Rodgers et al., RNA. 2002 Dec;8(12):1526-37; Z Wang et al., Mol Cell Biol. 1999 Jul;19(7):4552-60.).

Peripheral artery disease (PAD) refers to a series of non-coronary systemic syndromes caused by structural and functional abnormalities of arteries supplying the limbs, internal organs, and the brainm which is characterized by stenosis, occlusion, and aneurysmal lesions in the non-coronary blood circulation, involving the aorta and its branch arteries. In PAD, Lower limb ischemic disease is the most common clinically; with the main causes include atherosclerosis obliterans (ASO), diabetic artery obliterans (DAO), and thrombosis angiitis obliterans (TAO). Critical limb ischemia (CLI) belongs to the advanced stage of ASO development. Diabetic foot ulcer (DFU) belongs to a type of DAO. Both of them belong to peripheral vascular diseases, and their pathogenic characteristics are both lower limb pain, ulceration, and necrosis caused by lower limb vascular stenosis or occlusion and insufficient distal blood perfusion. At present, effective measures for the treatment of CLI and DFU are revascularization through surgery or endovascular intervention. However, more than 40% of patients do not meet the requirements for revascularization due to age, complications, and the like, and can only receive conservative drug treatment. Pharmacologicaltreatment can only delay the disease process and cannot cure the disease.

Hepatocyte growth factor (HGF) is a multifunctional mesenchymal-derived growth factor. When it binds to the cell membrane surface receptor c-met, it causes phosphorylation of intracellular tyrosine residues, recruits adaptor proteins, promotes kinase activity, and subsequently activates downstream signaling pathways. HGF is an important regulatory factor in processes such as embryonic development, tissue and organ regeneration, wound healing, and angiogenesis. It can promote the proliferation and migration of endothelial cells and smooth muscle cells. It also promotes the reconstruction of the microvascular network in the ischemic site and inhibits apoptosis. Research results show that intramuscular injection delivery of naked plasmid hHGF-cDNA can effectively promote blood perfusion in the ischemic hindlimbs of rat and rabbit models (Y Taniyama et al. Therapeutic angiogenesis induced by human hepatocyte growth factor gene in rat and rabbit hindlimb ischemia models: preclinical study for treatment of peripheral arterial disease. Gene Ther. 2001 Feb;8(3):181-9). Clinical data shows that intramuscular injection of a naked plasmid in the calf can promote wound healing in patients (S Cui et al. Clinical Safety and Preliminary Efficacy of Plasmid pUDK-HGF Expressing Human Hepatocyte Growth Factor (HGF) in Patients with Critical Limb Ischemia. Eur J Vasc Endovasc Surg. 2015 Oct;50(4):494-501.). However, there are deficiencies in delivering drugs using naked plasmids, such as low *in vivo* transfection efficiency, heavy dosing burden, high risk of DNA integration, and high treatment costs. Moreover, clinical data shows that it does not improve the toe-brachial index, transcutaneous oxygen pressure, and amputation rate. Therefore, there is still large room for improvement in such drugs.

The present disclosure provides such an mRNA, which contains 5'UTRs and 3'UTRs of a novel structure. This reduces early degradation of the mRNA or stabilizes degradation of the mRNA, but without losing or rather enhancing protein translation efficiency. The mRNA has higher stability and can be applied in gene therapy and genetic vaccination. In addition, the present disclosure provides an mRNA capable of expressing human hepatocyte growth factor (hHGF) and a lipid nanoparticle (LNP) delivery system thereof. This can achieve efficient and rapid *in vivo* transformation of exogenous hHGF protein. It has the advantages of no integration risk and easy industrial-scale scale-up. It is an ideal treatment regimen compared to a naked plasmid, and can be used as a gene therapy drug for a variety of diseases such as CLI and DFU.

### SUMMARY OF THE INVENTION

The present disclosure provides a lipid nanoparticle comprising a nucleic acid construct and a lipid phase.

In some embodiments, the lipid phase comprises at least one cationic lipid.

In some embodiments, the cationic lipid of the present disclosure is selected from a tertiary amine lipid.

In some embodiments, the cationic lipid of the present disclosure comprises a tertiary amine, an ester linker, and a branched tail.

In some embodiments, the cationic lipid of the present disclosure comprises a tertiary amine, an ester linker, and at least three branched tails.

In some embodiments, the cationic lipid of the present disclosure comprises a tertiary amine, an ester linker, and three branched tails.

In some embodiments, the cationic lipid of the present disclosure comprises a tertiary amine, an ester linker, and at least four branched tails.

In some embodiments, the branched tail of the present disclosure may be a saturated or unsaturated hydrophobic tail comprising 6-20 carbon atoms.

In some embodiments, the branched tail of the present disclosure may be a saturated hydrophobic tail comprising 6-20 carbon atoms.

In some embodiments, the cationic lipid of the present disclosure comprises a free hydroxy end.

In some embodiments, the cationic lipid of the present disclosure is a compound of Formula A, 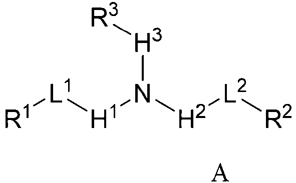
wherein L¹ and L² are each independently selected from -C(O)O-, -OC(O)-, -C(O)-, -OC(O)O-, -O-, -S(O)ₓ-, -S-S-, -C(O)S-, -SC(O)-, -NR^{a}C(O)-, -C(O)NR^{a}-, - NR^{a}C(O)NR^{a}-, -NR^{a}C(O)O-, -OC(O)NR^{a}-, and a bond, and R^{a} is selected from hydrogen, C₁₋₆ alkyl, and C₂₋₆ alkenyl;
H¹ and H² are each independently selected from C₁₋₁₂ heteroalkylene, C₁₋₁₂ alkylene, and C₂₋₁₂ alkenylene;
H³ is selected from C₁₋₂₄ alkylene, C₂₋₂₄ alkenylene, C₃₋₈ cycloalkylene, and C₃₋₈ cycloalkenylene;
R¹ and R² are each independently selected from C₁₋₂₄ alkyl and C₂₋₂₄ alkenyl;
R³ is selected from hydrogen, -CN, -C(O)OR⁴, -OC(O)R⁴, -OR⁵, and -NR⁵C(O)R⁴, wherein R⁴ is selected from C₁₋₆ alkyl and C₂₋₆ alkenyl, and R⁵ is selected from hydrogen, C₁₋₆ alkyl, and C₂₋₆ alkenyl;
x is selected from 0, 1, and 2.

In some embodiments, the cationic lipid of the present disclosure is a compound of Formula A, 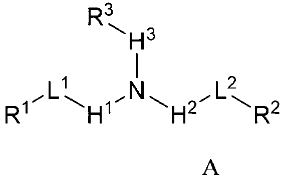
wherein L¹ and L² are each independently selected from -C(O)O-, -OC(O)-, -C(O)-, -OC(O)O-, and -O-;
H¹ and H² are each independently selected from C₁₋₁₂ heteroalkylene, C₁₋₁₂ alkylene, and C₂₋₁₂ alkenylene;
H³ is selected from C₁₋₂₄ alkylene, C₁₋₂₄ heteroalkylene, and C₂₋₂₄ alkenylene;
R¹ and R² are each independently selected from C₁₋₂₄ alkyl and C₂₋₂₄ alkenyl;
R³ is selected from hydrogen, -CN, -C(O)OR⁴, -OC(O)R⁴, -OR⁵, and -NR⁵C(O)R⁴, wherein R⁴ is selected from C₁₋₆ alkyl and C₂₋₆ alkenyl, and R⁵ is selected from hydrogen, C₁₋₆ alkyl, and C₂₋₆ alkenyl.

In some embodiments, the cationic lipid of the present disclosure is a compound of Formula A, 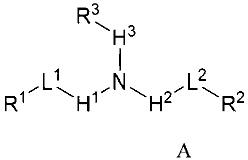
wherein L¹ and L² are each independently selected from -C(O)O- and -OC(O)-;
H¹ and H² are each independently selected from C₁₋₁₂ heteroalkylene, C₁₋₁₂ alkylene, and C₂₋₁₂ alkenylene;
H³ is selected from C₁₋₂₄ alkylene, C₁₋₂₄ heteroalkylene, and C₂₋₂₄ alkenylene;
R¹ and R² are each independently selected from C₁₋₂₄ alkyl and C₂₋₂₄ alkenyl;
R³ is selected from hydrogen, -CN, -C(O)OR⁴, -OC(O)R⁴, -OR⁵, and -NR⁵C(O)R⁴, wherein R⁴ is selected from C₁₋₆ alkyl and C₂₋₆ alkenyl, and R⁵ is selected from hydrogen, C₁₋₆ alkyl, and C₂₋₆ alkenyl.

In some embodiments, the cationic lipid of the present disclosure is a compound of Formula A, 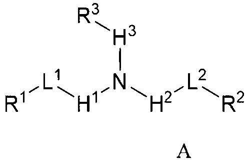
wherein L¹ and L² are each independently selected from -C(O)O- and -OC(O)-;
H¹ and H² are each independently selected from C₁₋₁₂ heteroalkylene, C₁₋₁₂ alkylene, and C₂₋₁₂ alkenylene;
H³ is selected from C₁₋₂₄ alkylene, C₁₋₂₄ heteroalkylene, and C₂₋₂₄ alkenylene;
R¹ and R² are each independently selected from C₁₋₂₄ alkyl and C₂₋₂₄ alkenyl;
R³ is selected from -OR⁵, and R⁵ is selected from hydrogen.

In some embodiments, at least one of H¹ and H² in the compound of Formula A or a salt thereof is a heteroalkylene, and the heteroalkylene comprises at least one heteroatom selected from O, N, and S.

In some embodiments, H¹ in the compound of Formula A or a salt thereof is selected from C₁₋₁₂ heteroalkylene, preferably C₂₋₉ heteroalkylene. In some embodiments, the heteroalkylene is a heteroalkylene comprising at least one oxygen atom. In some embodiments, the heteroalkylene is a heteroalkylene comprising two oxygen atoms. In some embodiments, the heteroalkylene is a heteroalkylene comprising at least one nitrogen atom. In some embodiments, the heteroalkylene is a heteroalkylene comprising at least one oxygen atom.

In some embodiments, at least one of H¹ and H² in the compound of Formula A or a salt thereof is a C₁₋₉ alkylene or a C₂₋₁₀ alkenylene.

In some embodiments, H³ in the compound of Formula A or a salt thereof is selected from C₂₋₂₄ alkenylene; or H³ is selected from C₁₋₂₄ alkylene. In some other embodiments, H³ is selected from C₁₋₂₄ heteroalkylene.

In some embodiments, H³ in the compound of Formula A or a salt thereof is selected from C₁₋₈ alkylene. In some embodiments, H³ in the compound of Formula A or a salt thereof is selected from C₂₋₆ alkylene.

In some embodiments, L¹ and L² in the compound of Formula A or a salt thereof are selected from -C(O)O-.

In some embodiments, L¹ and L² in the compound of Formula A or a salt thereof are selected from -OC(O)-.

In some embodiments, R¹ and R² in the compound of Formula A or a salt thereof are each independently selected from C₂₋₂₄ alkyl (including but not limited to a C₂ alkyl, C₃ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, and C₂₁ alkyl). In some other embodiments, R¹ and R² in the compound of Formula A or a salt thereof are each independently selected from C₄₋₁₈ alkyl.

In some embodiments, R¹ and R² in the compound of Formula A or a salt thereof are each independently selected from branched C₄₋₁₈ alkyl.

In some embodiments, R¹ and R² in the compound of Formula A or a salt thereof are each independently selected from linear C₄₋₁₈ alkyl.

In some embodiments, R¹ in the compound of Formula A or a salt thereof is selected from linear C₄₋₁₈ alkyl, and R² is selected from branched C₄₋₁₈ alkyl.

In some embodiments, R¹ in the compound of Formula A or a salt thereof is selected from branched C₄₋₁₈ alkyl, and R² is selected from branched C₄₋₁₈ alkyl.

In some embodiments, R¹ and R² in the compound of Formula A or a salt thereof are each independently selected from C₂₋₂₄ alkenyl (including but not limited to a C₂ alkenyl, C₃ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, and C₂₁ alkenyl). In some other embodiments, R¹ and R² in the compound of Formula A or a salt thereof are each independently selected from C₄₋₁₈ alkenyl.

In some embodiments, R¹ and R² in the compound of Formula A or a salt thereof are each independently selected from branched C₄₋₁₈ alkenyl.

In some embodiments, R¹ and R² in the compound of Formula A or a salt thereof are each independently selected from linear C₄₋₁₈ alkenyl.

In some embodiments, R¹ in the compound of Formula A or a salt thereof is selected from linear C₄₋₁₈ alkenyl, and R² is selected from branched C₄₋₁₈ alkenyl.

In some embodiments, R¹ in the compound of Formula A or a salt thereof is selected from branched C₄₋₁₈ alkenyl, and R² is selected from branched C₄₋₁₈ alkenyl.

In some embodiments, the cationic lipid of the present disclosure may be selected from N,N-dioleyl-N,N-dimethylammonium chloride (DODAC), N,N-distearyl-N,N-dimethylammonium bromide (DDAB), 1,2-dioleoyltrimethylpropane ammonium chloride (DOTAP) (also known as N-(2,3-dioleoyloxy)propyl)-N,N,N-trimethylammonium chloride and 1,2-dioleoyloxy-3-trimethylaminopropane chloride salt), N-(1-(2,3-dioleoyloxy)propyl)-N,N,N-trimethylammonium chloride (DOTMA), N,N-dimethyl-2,3-dioleoyloxy)propylamine (DODMA), 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N-dimethylaminopropane (γ-DLenDMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazinyl)propane (DLin-MPZ), or 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine, (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)bisdodecan-2-ol (C12-200), 2,2-dilinoleyl-4-(2-dimethylaminoethyl)-[1,3]-dioxolane (DLin-K-C2-DMA), 2,2-dilinoleyl-4-dimethylaminoethyl-[1,3]-dioxolane (DLin-K-DMA), (6Z,9Z,28Z,31Z)-heptatriaconta-6,9,28,31-tetraen-19-yl 4-(dimethylamino)butanoate (DLin-M-C3-DMA), 3-((6Z,9Z,28Z,31Z)-heptatriaconta-6,9,28,31-tetraen-19-yloxy)-N,N-dimethylpropan-1-amine (MC3 Ether), 4-((6Z,9Z,28Z,31Z)-heptatriaconta-6,9,28,31-tetraen-19-yloxy)-N,N-dimethylbutan-1-amine (MC4 Ether), ((4-hydroxybutyl)azanediyl)bis(hexane-6,1-diyl)bis(2-hexyldecanoate) (ALC-0315), 8-[(2-hydroxyethyl)[6-oxo-6-(undecyloxy)hexyl]amino]octanoic acid 1-octylnonyl ester (SM-102), a compound of Formula I, or any combination of the above cationic lipids.

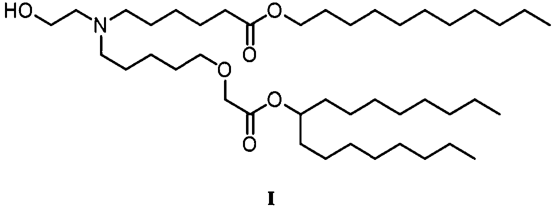

In some embodiments, the cationic lipid of the present disclosure is a compound of Formula I.

In some embodiments, the cationic lipid of the present disclosure is ((4-hydroxybutyl)azanediyl)bis(hexane-6,1-diyl)bis(2-hexyldecanoate) (ALC-0315).

In some embodiments, the cationic lipid of the present disclosure is 8-[(2-hydroxyethyl)[6-oxo-6-(undecyloxy)hexyl]amino]octanoic acid 1-octylnonyl ester (SM-102).

In some embodiments, the cationic lipid of the present disclosure comprises from 10% to 75 mol % of the total lipids present in the lipid nanoparticle, including, but is not limited to, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, or a value between any two of the above values.

In some embodiments, the cationic lipid of the present disclosure comprises from 15% to 49 mol % of the total lipids present in the lipid nanoparticle, including, but is not limited to, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or a value between any two of the above values.

In some embodiments, the cationic lipid of the present disclosure comprises from 20% to 70 mol % of the total lipids present in the lipid nanoparticle.

In some embodiments, the cationic lipid of the present disclosure comprises from 30% to 70 mol % of the total lipids present in the lipid nanoparticle.

In some embodiments, the cationic lipid of the present disclosure comprises from 20% to 60 mol % of the total lipids present in the lipid nanoparticle.

In some embodiments, the cationic lipid of the present disclosure comprises from 30% to 60 mol % of the total lipids present in the lipid nanoparticle.

In some embodiments, the cationic lipid of the present disclosure comprises from 42% to 49 mol % of the total lipids present in the lipid nanoparticle.

In some embodiments, the cationic lipid of the present disclosure comprises 45 mol % of the total lipids present in the lipid nanoparticle.

In some embodiments, the cationic lipid of the present disclosure comprises 46 mol % of the total lipids present in the lipid nanoparticle.

In some embodiments, the cationic lipid of the present disclosure comprises 47 mol % of the total lipids present in the lipid nanoparticle.

In some embodiments, the cationic lipid of the present disclosure comprises 48 mol % of the total lipids present in the lipid nanoparticle.

In some embodiments, the cationic lipid of the present disclosure comprises 49 mol % of the total lipids present in the lipid nanoparticle.

In some embodiments, the cationic lipid of the present disclosure comprises from 5% to 40 mol % of the total lipids present in the lipid nanoparticle, including, but is not limited to, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, or a value between any two of the above values.

In some embodiments, the cationic lipid of the present disclosure comprises from 40% to 70 mol % of the total lipids present in the lipid nanoparticle, including, but is not limited to, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, or a value between any two of the above values.

In some embodiments, the lipid phase of the present disclosure comprises at least one non-cationic lipid.

In some embodiments, the non-cationic lipid of the present disclosure comprises from 20% to 70 mol % of the total lipids present in the lipid nanoparticle, including, but is not limited to, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, or a value between any two of the above values.

In some embodiments, the non-cationic lipid of the present disclosure comprises from 50% to 65 mol % of the total lipids present in the lipid nanoparticle.

In some embodiments, the non-cationic lipid of the present disclosure comprises from 40% to 55 mol % of the total lipids present in the lipid nanoparticle.

In some embodiments, the non-cationic lipid of the present disclosure comprises from 55% to 62 mol % of the total lipids present in the lipid nanoparticle.

In some embodiments, the non-cationic lipid of the present disclosure comprises from 30% to 60 mol % of the total lipids present in the lipid nanoparticle.

In some embodiments, the non-cationic lipid of the present disclosure comprises from 30% to 50 mol % of the total lipids present in the lipid nanoparticle.

In some embodiments, the non-cationic lipid of the present disclosure comprises from 20% to 50 mol % of the total lipids present in the lipid nanoparticle.

In some embodiments, the non-cationic lipid of the present disclosure comprises from 20% to 40 mol % of the total lipids present in the lipid nanoparticle.

In some embodiments, the non-cationic lipid of the present disclosure may be selected from a mixture of a phospholipid and cholesterol or its derivative.

In some embodiments, the phospholipid of the present disclosure may be selected from 1,2-dilinoleoyl-sn-glycero-3-phosphocholine (DLPC), 1,2-dimyristoyl-sn-glycero-phosphocholine (DMPC), 1,2-dioleoyl-sn-glycero-3- phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero- phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2- cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn- glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3- phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl- sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero -3-phospho-rac-(1-glycerol) sodium salt (DOPG), dipalmitoylphosphatidylglycerol (DPPG), palmitoyloleoylphosphatidylethanolamine (POPE), distearoylphosphatidylethanolamine (DSPE), dipalmitoylphosphatidylethanolamine (DPPE), dimyristoylphosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine (LPE), and mixtures thereof.

In some embodiments, the phospholipid of the present disclosure is 1,2-distearoyl-sn-glycero-3-phosphocholine (DSPC).

In some embodiments, the phospholipid of the present disclosure is 1,2-dioleoyl-sn-glycero-3-phosphoethanolamine (DOPE).

In some embodiments, the weight ratio of the phospholipid to the cationic lipid of the present disclosure is 1:20 to 10:1; Preferably, the weight ratio of the phospholipid to the cationic lipid is 1:20 to 5:1; Preferably, the weight ratio of the phospholipid to the cationic lipid is 1:10 to 5:1; More preferably, the weight ratio of the phospholipid to the cationic lipid is 1:10 to 1:1.

In some embodiments, the content of the phospholipid of the present disclosure comprises from 5% to 40 mol % of the total lipids present in the lipid nanoparticle, including, but is not limited to, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, or a value between any two of the above values.

In some embodiments, the content of the phospholipid of the present disclosure comprises from 5% to 20 mol % of the total lipids present in the lipid nanoparticle.

In some embodiments, the content of the phospholipid of the present disclosure comprises 15 mol % of the total lipids present in the lipid nanoparticle.

In some embodiments, the content of the phospholipid of the present disclosure comprises 20 mol % of the total lipids present in the lipid nanoparticle.

In some embodiments, the content of the phospholipid of the present disclosure comprises from 10% to 20 mol % of the total lipids present in the lipid nanoparticle.

In some embodiments, the content of the phospholipid of the present disclosure comprises 10 mol % of the total lipids present in the lipid nanoparticle.

In some embodiments, cholesterol derivatives include, but are not limited to, sitosterol, ergosterol, campesterol, stigmasterol, brassicasterol, tomatine, and ursolic acid.

In another aspect, the cholesterol or its derivative of the present disclosure comprises from 20% to 60 mol % of the total lipids present in the lipid nanoparticle, including, but is not limited to, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, or a value between any two of the above values.

In some embodiments, the content of the cholesterol or its derivative of the present disclosure comprises from 30% to 60 mol % of the total lipids present in the lipid nanoparticle.

In some embodiments, the content of the cholesterol or its derivative of the present disclosure comprises from 20% to 45 mol % of the total lipids present in the lipid nanoparticle.

In some embodiments, the content of the cholesterol or its derivative of the present disclosure comprises from 35% to 45 mol % of the total lipids present in the lipid nanoparticle.

In some embodiments, the content of the cholesterol or its derivative of the present disclosure comprises from 20% to 35 mol % of the total lipids present in the lipid nanoparticle.

In some embodiments, the content of the cholesterol or its derivative of the present disclosure comprises 40.5 mol % of the total lipids present in the lipid nanoparticle.

In some embodiments, the content of the cholesterol or its derivative of the present disclosure comprises 45 mol % of the total lipids present in the lipid nanoparticle.

In some embodiments, the molar ratio of the cholesterol or its derivative to the phospholipid of the present disclosure is 1.0 to 5.0, including, but is not limited to, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or a value between any two of the above values.

In some embodiments, the molar ratio of the cholesterol or its derivative to the phospholipid in the lipid nanoparticle of the present disclosure is 2.0 to 5.0.

In another aspect, the lipid phase of the present disclosure further comprises at least one conjugated lipid; the conjugated lipid includes, but is not limited to, a PEG-modified phosphatidylethanolamine, a PEG-modified phosphatidic acid, a PEG-modified ceramide, a PEG-modified dialkylamine, a PEG-modified diacylglycerol, and a PEG-modified dialkylglycerol.

In some embodiments, the conjugated lipid is selected from distearoylphosphatidylethanolamine-polyethylene glycol 2000 (DSPE-PEG2000), dimyristoylglycerol-3-methoxypolyethylene glycol 2000 (DMG-PEG2000), and methoxypolyethylene glycol ditetradecylacetamide (ALC-0159).

In some embodiments, the conjugated lipid is selected from dimyristoylglycerol-3-methoxypolyethylene glycol 2000.

In some embodiments, the conjugated lipid is selected from distearoylphosphatidylethanolamine-polyethylene glycol 2000.

In some embodiments, the conjugated lipid functions to inhibit aggregation of the lipid nanoparticles (particles).

In some embodiments, the content of the conjugated lipid of the present disclosure comprises from 0.5% to 4 mol % of the total lipids present in the lipid nanoparticle, including, but is not limited to, 0.5%, 0.6%, 0.7%, 0.8%, 0.9%, 1.0%, 1.2%, 1.4%, 1.6%, 1.8%, 2.0%, 2.2%, 2.4%, 2.6%, 2.8%, 3.0%, 3.2%, 3.4%, 3.6%, 3.8%, 4.0%, or a value between any two of the above values.

In some embodiments, the content of the conjugated lipid of the present disclosure comprises from 0.5% to 1.0 mol % of the total lipids present in the lipid nanoparticle.

In some embodiments, the content of the conjugated lipid of the present disclosure comprises from 1.5% to 2.5 mol % of the total lipids present in the lipid nanoparticle.

In some embodiments, the content of the conjugated lipid of the present disclosure comprises from 1% to 2 mol % of the total lipids present in the lipid nanoparticle.

In some embodiments, the content of the conjugated lipid of the present disclosure comprises 1.5 mol % of the total lipids present in the lipid nanoparticle.

In some embodiments, the content of the conjugated lipid of the present disclosure comprises 2.0 mol % of the total lipids present in the lipid nanoparticle.

In some embodiments, the weight ratio of the conjugated lipid to the cationic lipid is 1:100 to 1:1; Preferably, the weight ratio of the conjugated lipid to the cationic lipid is 1:100 to 1:2; Preferably, the weight ratio of the conjugated lipid to the cationic lipid is 1:50 to 1:1; Preferably, the weight ratio of the conjugated lipid to the cationic lipid is 1:50 to 1:2; Preferably, the weight ratio of the conjugated lipid to the cationic lipid is 1:25 to 1:2; Preferably, the weight ratio of the conjugated lipid to the cationic lipid is 1:20 to 1:2.

In some embodiments, the lipid nanoparticle of the present disclosure comprises:
a) a nucleic acid construct;
b) a cationic lipid, wherein the cationic lipid comprises a compound of Formula I or a pharmaceutically acceptable salt thereof, and the cationic lipid comprises from 10% to 75 mol % of the total lipids present in the lipid nanoparticle, 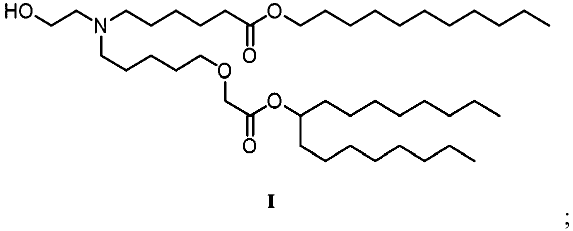
c) a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 15% to 60 mol % of the total lipids present in the lipid nanoparticle;
and d) a conjugated lipid, wherein the conjugate comprises from 0.5% to 2 mol % of the total lipids present in the lipid nanoparticle.

In some embodiments, the lipid nanoparticle of the present disclosure comprises:
a) a nucleic acid construct;
b) a cationic lipid, wherein the cationic lipid comprises a compound of Formula I or a pharmaceutically acceptable salt thereof, and the cationic lipid comprises from 20% to 70 mol % of the total lipids present in the lipid nanoparticle; 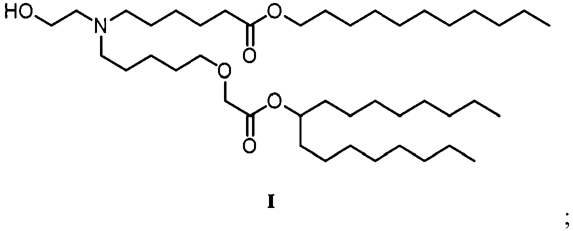
c) a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 15% to 60 mol % of the total lipids present in the lipid nanoparticle;
and d) a conjugated lipid, wherein the conjugate comprises from 0.5% to 2 mol % of the total lipids present in the lipid nanoparticle.

In some embodiments, the lipid nanoparticle of the present disclosure comprises:
a) a nucleic acid construct;
b) a cationic lipid, wherein the cationic lipid comprises a compound of Formula I or a pharmaceutically acceptable salt thereof, and the cationic lipid comprises from 10% to 50 mol % of the total lipids present in the lipid nanoparticle; 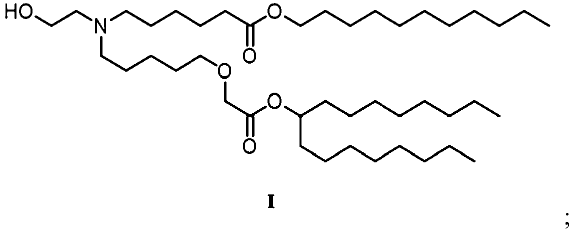
c) a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 30% to 60 mol % of the total lipids present in the lipid nanoparticle;
and d) a conjugated lipid, wherein the conjugate comprises from 0.5% to 2 mol % of the total lipids present in the lipid nanoparticle.

In another aspect, in the nanoparticle preparation of the present disclosure, the average particle size of the nanoparticle preparation is 10 nm to 200 nm.

In some embodiments, the average particle size of the nanoparticle preparation is 40 nm to 150 nm.

In some embodiments, the average particle size of the nanoparticle preparation is 40 nm to 140 nm.

In another aspect, the present disclosure also provides a lyophilized composition comprising the aforementioned lipid nanoparticle.

In some embodiments, the lyophilized preparation of the present disclosure is obtained by freeze-drying the aforementioned lipid nanoparticle or the aforementioned lyophilized composition.

In some embodiments, the lyophilized composition or preparation of the present disclosure comprises the above lipid nanoparticle and sodium chloride.

In some embodiments, the weight ratio of sodium chloride to the cationic lipid in the lyophilized composition or preparation of the present disclosure is 1:10 to 10:1.

In some embodiments, the weight ratio of sodium chloride to the cationic lipid in the lyophilized composition or preparation of the present disclosure is 1:10 to 5:1.

In some embodiments, the weight ratio of sodium chloride to the cationic lipid in the lyophilized composition or preparation of the present disclosure is 1:5 to 10:1.

In some embodiments, the weight ratio of sodium chloride to the cationic lipid in the lyophilized composition or preparation of the present disclosure is 1:5 to 5:1.

In some embodiments, the weight ratio of sodium chloride to the cationic lipid in the lyophilized composition or preparation of the present disclosure is 1:3 to 5:1.

In some embodiments, the weight ratio of sodium chloride to the cationic lipid in the lyophilized composition or preparation of the present disclosure is 1:2 to 5:1.

In some embodiments, the weight ratio of sodium chloride to the cationic lipid in the lyophilized composition or preparation of the present disclosure is 1:2 to 4:1.

In some embodiments, the weight ratio of sodium chloride to the cationic lipid in the lyophilized composition or preparation of the present disclosure is 1:5 to 3:1.

In some embodiments, the weight ratio of sodium chloride to the cationic lipid in the lyophilized composition or preparation of the present disclosure is 1:3 to 3:1.

In some embodiments, the weight ratio of sodium chloride to the cationic lipid in the lyophilized composition or preparation of the present disclosure is 1:3 to 2:1.

In some embodiments, the weight ratio of sodium chloride to the cationic lipid in the lyophilized composition or preparation of the present disclosure is 1:2 to 2:1.

In another aspect, the lyophilized composition or preparation of the present disclosure further comprises at least one lyoprotectant.

In some embodiments, the lyoprotectant is one or more selected from sucrose, trehalose, mannitol, and maltose. Preferably, the lyoprotectant is selected from sucrose, and a combination of sucrose and trehalose.

In some embodiments, the lyoprotectant is sucrose.

In some embodiments, the lyoprotectant is a combination of trehalose and mannitol;

In some embodiments, the weight ratio of the lyoprotectant to the cationic lipid is 200:1 to 1:1; Preferably, the weight ratio of the lyoprotectant to the cationic lipid is 150:1 to 1:1; Preferably, the weight ratio of the lyoprotectant to the cationic lipid is 100:1 to 1:1; Preferably, the weight ratio of the lyoprotectant to the cationic lipid is 100:1 to 20:1; Preferably, the weight ratio of the lyoprotectant to the cationic lipid is 800:1 to 20:1; Preferably, the weight ratio of the lyoprotectant to the cationic lipid is 50:1 to 20:1.

In some embodiments, the lyophilized composition or preparation of the present disclosure comprises the lyoprotectant at a concentration of 5% w/v to 20% w/v, and preferably the lyoprotectant at a concentration of 5% w/v to 15% w/v.

In some embodiments, the lyophilized composition of the present disclosure comprises sucrose at a concentration of 5% w/v to 20% w/v, preferably sucrose at a concentration of 5% w/v to 15% w/v, preferably sucrose at a concentration of 5% w/v to 10% w/v, and preferably sucrose at a concentration of 8% w/v.

In some embodiments, the lyophilized composition or preparation of the present disclosure comprises trehalose at a concentration of 5% w/v to 20% w/v, and preferably trehalose at a concentration of 5% w/v to 15% w/v.

In some embodiments, the lyophilized composition or preparation of the present disclosure comprises sucrose at a concentration of 5% w/v to 10% w/v and trehalose at a concentration of 1% w/v to 10% w/v, and preferably sucrose at a concentration of 5% w/v to 10% w/v and trehalose at a concentration of 2% w/v to 8% w/v.

In another aspect, the lyophilized composition or preparation of the present disclosure further comprises a buffer.

In some embodiments, the buffer is selected from (N-morpholino)propanesulfonic acid (MOPS), 4-hydroxyethylpiperazineethanesulfonic acid (HEPES), tris(hydroxymethyl)aminomethane (TRIS), 4-morpholinoethanesulfonic acid (MES), a citrate, and phosphate-buffered saline (PBS).

In some embodiments, the buffer is TRIS.

In some embodiments, the concentration of the buffer is about 10 mM to about 100 mM; Preferably, the concentration of the buffer is about 15 mM to about 75 mM; More preferably, the concentration of the buffer is about 10 mM to about 40 mM.

In some embodiments, the lyophilized composition or preparation of the present disclosure comprises:
i., a nucleic acid construct;
ii. sodium chloride, wherein the weight ratio of sodium chloride to the cationic lipid is 1:5 to 10:1;
iii. a cationic lipid, wherein the cationic lipid comprises a compound of Formula I or a pharmaceutically acceptable salt thereof, and the cationic lipid comprises from 10% to 75 mol % of the total lipids present in the lipid nanoparticle, 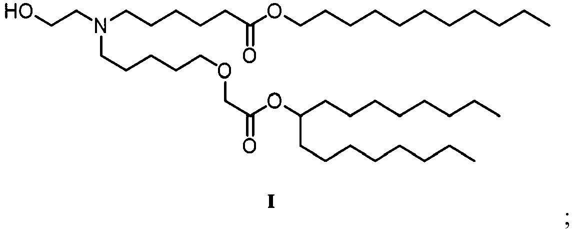
iv. a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 15% to 60 mol % of the total lipids present in the lipid nanoparticle; and
v. a conjugated lipid, wherein the conjugate comprises from 0.5% to 2 mol % of the total lipids present in the lipid nanoparticle.

In some embodiments, the lyophilized composition or preparation of the present disclosure comprises:
i. a nucleic acid construct;
ii. sodium chloride, wherein the weight ratio of sodium chloride to the cationic lipid is 1:5 to 10:1;
iii. a cationic lipid, wherein the cationic lipid comprises a compound of Formula I or a pharmaceutically acceptable salt thereof, and the cationic lipid comprises from 20% to 70 mol % of the total lipids present in the lipid nanoparticle; 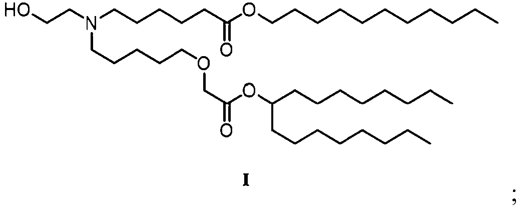
iv. a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 15% to 60 mol % of the total lipids present in the lipid nanoparticle; and
v. a conjugated lipid, wherein the conjugate comprises from 0.5% to 2 mol % of the total lipids present in the lipid nanoparticle.

In some embodiments, the lyophilized composition or preparation of the present disclosure comprises:
i. a nucleic acid construct;
ii. sodium chloride, wherein the weight ratio of sodium chloride to the cationic lipid is 1:5 to 10:1;
iii. a cationic lipid, wherein the cationic lipid comprises a compound of Formula I or a pharmaceutically acceptable salt thereof, and the cationic lipid comprises from 10% to 50 mol % of the total lipids present in the lipid nanoparticle; 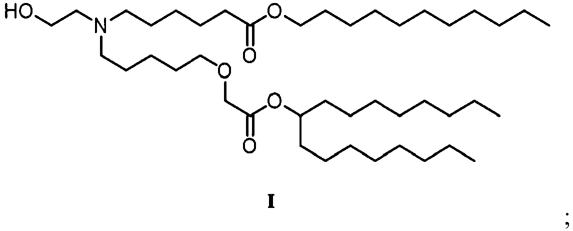
iv. a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 30% to 60 mol % of the total lipids present in the lipid nanoparticle; and
v. a conjugated lipid, wherein the conjugate comprises from 0.5% to 2 mol % of the total lipids present in the lipid nanoparticle.

In some embodiments, the lyophilized composition or preparation of the present disclosure comprises:
1) sodium chloride, wherein the weight ratio of sodium chloride to the cationic lipid is 1:5 to 10:1;
2) a lipid nanoparticle, wherein the lipid nanoparticle comprises:
   a nucleic acid construct;
   a cationic lipid, wherein the cationic lipid comprises a compound of Formula I or a pharmaceutically acceptable salt thereof, and the cationic lipid comprises from 10% to 75 mol % of the total lipids present in the lipid nanoparticle, 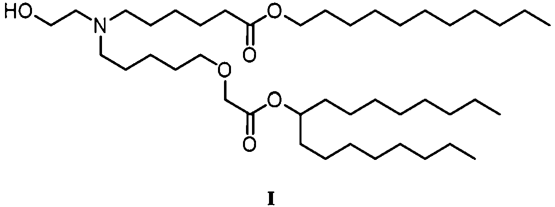
   a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 15% to 60 mol % of the total lipids present in the lipid nanoparticle;
   and a conjugated lipid, wherein the conjugate comprises from 0.5% to 2 mol % of the total lipids present in the lipid nanoparticle.
3) optionally a lyoprotectant, wherein the concentration of the lyoprotectant is 5% w/v to 15% w/v.

In some embodiments, the lyophilized composition or preparation of the present disclosure comprises:
1) sodium chloride, wherein the weight ratio of sodium chloride to the cationic lipid is 1:5 to 10:1;
2) a lipid nanoparticle, wherein the lipid nanoparticle comprises:
   a nucleic acid construct;
   a cationic lipid, wherein the cationic lipid comprises a compound of Formula I or a pharmaceutically acceptable salt thereof, and the cationic lipid comprises from 20% to 70 mol % of the total lipids present in the lipid nanoparticle; 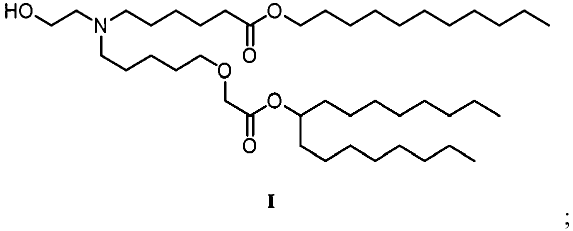
   a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 15% to 60 mol % of the total lipids present in the lipid nanoparticle;
   and a conjugated lipid, wherein the conjugate comprises from 0.5% to 2 mol % of the total lipids present in the lipid nanoparticle;
3) optionally a lyoprotectant, wherein the concentration of the lyoprotectant is 5% w/v to 15% w/v.

In some embodiments, the lyophilized composition or preparation of the present disclosure comprises:
1) sodium chloride, wherein the weight ratio of sodium chloride to the cationic lipid is 1:5 to 10:1;
2) a lipid nanoparticle, wherein the lipid nanoparticle comprises:
   a nucleic acid construct;
   a cationic lipid, wherein the cationic lipid comprises a compound of Formula I or a pharmaceutically acceptable salt thereof, and the cationic lipid comprises from 10% to 50 mol % of the total lipids present in the lipid nanoparticle; 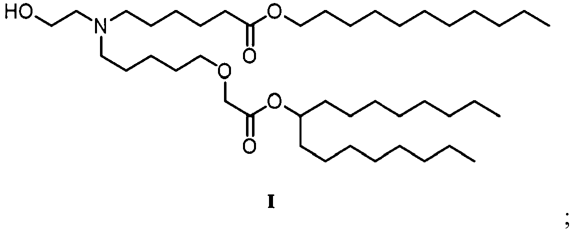
   a non-cationic lipid, wherein the non-cationic lipid is selected from phospholipid and cholesterol or its derivative, wherein the phospholipid comprises from 5% to 40 mol % of the total lipids present in the lipid nanoparticle, and the cholesterol or its derivative comprises from 30% to 60 mol % of the total lipids present in the lipid nanoparticle;
   and a conjugated lipid, wherein the conjugate comprises from 0.5% to 2 mol % of the total lipids present in the lipid nanoparticle;
3) optionally a lyoprotectant, wherein the concentration of the lyoprotectant is 5% w/v to 15% w/v.

In some embodiments, the amount of the active agent in the lyophilized composition or preparation is about 0.01 mg/mL to about 1 mg/mL, about 0.05 mg/mL to about 0.5 mg/mL, about 0.1 mg/mL to about 0.5 mg/mL, or about 0.2 mg/mL, about 0.05 mg/mL, or about 0.02 mg/mL.

In some embodiments, the weight ratio of the active agent to the cationic lipid is 1:10 to 1:60; Preferably, the weight ratio of the active agent to the cationic lipid is 1:10 to 1:50; Preferably, the weight ratio of the active agent to the cationic lipid is 1:10 to 1:40.

In some embodiments, in any one of the aforementioned lipid nanoparticles or lyophilized compositions or preparations, the nucleic acid construct comprises an ORF. In some embodiments, the ORF encodes hepatocyte growth factor (HGF), an antibody, or an antigen-binding fragment thereof. In some specific embodiments, the ORF encodes human hepatocyte growth factor (HGF). In some specific embodiments, the ORF encodes an anti-PD-1 antibody or an antigen-binding fragment thereof.

The present disclosure also provides a method for preparing a lyophilized preparation comprising the following steps of:
a. providing a suspension of the aforementioned lipid nanoparticle in a liquid medium; and
b. adjusting the liquid medium to form a suspension comprising NaCl;
c. freeze-drying.

In some embodiments, in the preparation method of the present disclosure, the liquid medium comprises 5% w/v to 20% w/v of sucrose, and preferably 5% w/v to 15% w/v of sucrose.

In some embodiments, in the preparation method of the present disclosure, the liquid medium comprises 5% w/v to 10% w/v of sucrose and 1% w/v to 10% w/v of trehalose.

In some embodiments, in the preparation method of the present disclosure, the liquid medium comprises 10-200 mM NaCl; preferably 10-150 mM NaCl, more preferably 20-150 mM NaCl, more preferably 20-120 mM NaCl, more preferably 50-150 mM NaCl, more preferably 50-120 mM NaCl, and more preferably 70-120 mM NaCl.

The present disclosure also provides a method for reducing the average particle size of the lipid nanoparticle in a lyophilized composition or lyophilized preparation comprising adding or using sodium chloride during the freeze-drying process.

The present disclosure also provides a method for reducing the average particle size of the lipid nanoparticle after reconstitution of a lyophilized composition or lyophilized preparation comprising adding or using sodium chloride during the freeze-drying process.

The present disclosure also provides a use of sodium chloride in reducing the average particle size of the lipid nanoparticle in a lyophilized composition or lyophilized preparation.

The present disclosure also provides a use of sodium chloride in reducing the average particle size of the lipid nanoparticle after reconstitution of a lyophilized composition or lyophilized preparation, comprising adding or using sodium chloride during the freeze-drying process.

In some embodiments, after the lyophilized composition or lyophilized preparation of the present disclosure is reconstituted, the increase of the average particle size of the lipid nanoparticle does not exceed 100%. In some embodiments, the increase of the average particle size of the lipid nanoparticle does not exceed 80%. In some embodiments, the increase of the average particle size of the lipid nanoparticle does not exceed 60%.

In some embodiments, in the lyophilized composition or lyophilized preparation of the present disclosure, the mRNA integrity is tested immediately after being taken out of the freeze-dryer, wherein the mRNA integrity is not lower than 75%, or the mRNA integrity is not lower than 80%, or the mRNA integrity is not lower than 85%, or the mRNA integrity is not lower than 86%, or the mRNA integrity is not lower than 87%, or the mRNA integrity is not lower than 88%, or the mRNA integrity is not lower than 89%, or the mRNA integrity is not lower than 90%. The immediate test is performed within 1 hour after being taken out.

In some embodiments, in the lyophilized composition or lyophilized preparation of the present disclosure, the mRNA integrity is tested after being placed at 37°C for 72 hours after being taken out of the freeze-dryer, wherein the mRNA integrity is not lower than 75%, or the mRNA integrity is not lower than 80%, or the mRNA integrity is not lower than 85%, or the mRNA integrity is not lower than 86%, or the mRNA integrity is not lower than 87%, or the mRNA integrity is not lower than 88%, or the mRNA integrity is not lower than 89%, or the mRNA integrity is not lower than 90%.

The present disclosure provides a reconstituted solution obtained by reconstituting the aforementioned lyophilized composition or preparation with a reconstitution solvent. In certain embodiments, the reconstituted solution is selected from, but not limited to, the group consisting of water for injection, physiological saline, glucose solution, or the reconstitution solvent described below.

In another aspect, the present disclosure also provides a pharmaceutical composition comprising the lipid nanoparticle and a reconstitution solvent.

In another aspect, the present disclosure also provides a pharmaceutical composition comprising the lyophilized composition and a reconstitution solvent.

In another aspect, the present disclosure also provides a pharmaceutical composition comprising the lipid nanoparticle, sodium chloride, and a reconstitution solvent.

The present disclosure provides a method for preparing the aforementioned pharmaceutical composition comprising adding or using sodium chloride in the preparation of the lyophilized composition. The present disclosure provides a method for preparing the aforementioned pharmaceutical composition comprising a step of reconstituting the lyophilized composition or preparation.

The present disclosure provides a method for preparing a reconstituted solution comprising preparing the aforementioned lyophilized composition, and subjecting the lyophilized composition to a freeze-drying treatment to obtain the lyophilized preparation; and
reconstituting the lyophilized preparation to obtain the reconstituted solution.

The present disclosure provides a method for preparing a lyophilized preparation comprising preparing the aforementioned lyophilized composition, and subjecting the lyophilized composition to a freeze-drying treatment to obtain the lyophilized preparation.

In some embodiments, the reconstitution solvent of the present disclosure comprises a dispersant.

In some embodiments, the dispersant of the present disclosure may be selected from poloxamer, polyvinyl alcohol, hydroxyethyl cellulose, polyethylene glycol, polysorbate, polyoxyethylene fatty acid ester, sorbitan fatty acid ester, polyoxyethylene fatty alcohol ether, polyoxyethylene hydrogenated castor oil, and N-alkylpyrrolidone.

In some embodiments, the dispersant of the present disclosure may be selected from poloxamer 188, polyvinyl alcohol, hydroxyethyl cellulose, polyethylene glycol 400, polysorbate 20 (Tween 20), sorbitan laurate (Span 20), polyoxyethylene monostearate (Myrj 45), polyoxyethylene lauryl ether (Brij 35), and N-ethylpyrrolidone (NMP).

In some embodiments, the dispersant of the present disclosure may be selected from poloxamer 188.

In some embodiments, the weight percentage of the dispersant of the present disclosure relative to the reconstitution solvent is 0.01 to 5.0%.

In some embodiments, the weight percentage of the dispersant of the present disclosure relative to the reconstitution solvent is 0.01 to 3.0%.

In some embodiments, the weight percentage of the dispersant of the present disclosure relative to the reconstitution solvent is 0.05 to 3.0%.

In some embodiments, the weight percentage of the dispersant of the present disclosure relative to the reconstitution solvent is 0.05 to 2.0%.

In some embodiments, the weight percentage of the dispersant of the present disclosure relative to the reconstitution solvent is 0.05 to 1.0%.

In some embodiments, the reconstitution solvent of the present disclosure further comprises a thickener.

In some embodiments, the thickener of the present disclosure may be selected from xanthan gum (Xc), Polyvinylpyrrolidone (PVP), sodium hyaluronate (HA), sodium carboxymethyl cellulose (CMC-Na), and chitosan (CTS).

In some embodiments, the thickener of the present disclosure may be selected from xanthan gum (Xc), Polyvinylpyrrolidone (PVP), sodium hyaluronate (HA), and sodium carboxymethyl cellulose (CMC-Na).

In some embodiments, the Polyvinylpyrrolidone (PVP) of the present disclosure is Polyvinylpyrrolidone K90 (PVP-K90).

In some embodiments, the weight percentage of the thickener of the present disclosure relative to the reconstitution solvent is 0.01 to 5.0%.

In some embodiments, the weight percentage of the thickener of the present disclosure relative to the reconstitution solvent is 0.01 to 3.0%.

In some embodiments, the weight percentage of the thickener of the present disclosure relative to the reconstitution solvent is 0.05 to 3.0%.

In some embodiments, the weight percentage of the thickener of the present disclosure relative to the reconstitution solvent is 0.05 to 2.0%.

The present disclosure also provides a product comprising a container, the container contains the aforementioned lipid nanoparticle, the aforementioned lyophilized composition, the aforementioned pharmaceutical composition, or the aforementioned reconstituted solution.

The present disclosure also provides a product comprising:
1) the aforementioned lyophilized composition or lyophilized preparation; and
2) a reconstitution solvent.

The present disclosure also provides a spray preparation comprising the aforementioned lyophilized composition, lyophilized preparation, or reconstituted solution.

In certain embodiments, the product comprises the lyophilized composition or preparation and the reconstitution solvent in separate packages.

In certain embodiments, the container is a neutral borosilicate glass tubular injection vial or a device for nebulization administration. In certain embodiments, the product comprises a package insert.

The aforementioned lipid nanoparticle, the aforementioned lyophilized composition, the aforementioned pharmaceutical composition, or the aforementioned reconstituted solution provided by the present disclosure has good stability, controllable particle size increase, and excellent mRNA expression.

The present disclosure also provides the aforementioned lipid nanoparticle, the aforementioned lyophilized composition, the aforementioned pharmaceutical composition, or the aforementioned reconstituted solution for use as a medicament for treating or alleviating a disease or disorder.

In some embodiments, any one of the aforementioned lipid nanoparticles comprises a nucleic acid construct. The nucleic acid construct comprises: at least one nucleic acid element capable of regulating the expression of a gene of interest, and the nucleic acid element is a UTR. Further, the nucleic acid construct may contain one or more genes of interest, such as HGF.

### Nucleic Acid Construct

The present disclosure provides a nucleic acid construct comprising:
(a) an open reading frame (ORF), and
(b) an untranslated region element (UTR).

In some embodiments, the nucleic acid construct is a DNA molecule; in some embodiments, the nucleic acid construct is an RNA molecule (e.g., mRNA).

In some embodiments, the ORF is a polynucleotide sequence encoding a gene of interest.

In some embodiments, the gene of interest is heterologous. In some other embodiments, the gene of interest is endogenous. In some embodiments, there are one or more genes of interest (e.g., 2, 3, 4).

In some embodiments, the UTR is derived from the UTR of the gene ACTG1, ATP6V0B, ATP6V0E1, CFL1, COX4I1, CTSB, FAM166A, NDUFB9, CHCHD10, SLC38A2, NDUFA11, NDUFV3, PRDX5, GUK1, IAH1, ABHD16A, SLC25A39, ATPIF1, ANAPC11, CCDC12, MRPL14, or APOA1BP. In some embodiments, the above genes are human genes.

In some embodiments, the UTR is a 3' untranslated region element (3'UTR) or a 5' untranslated region element (5'UTR).

In some embodiments, the 3'UTR and 5'UTR are of the same or different origins, for example, they are derived from the same or different genes. For example, the 3'UTR is derived from the 3'UTR of the gene ACTG1, and the 5'UTR is derived from the 5'UTR of the gene ACTG1. For another example, the 3'UTR is derived from the 3'UTR of the gene CTSB, and the 5'UTR is derived from the 5'UTR of the gene CHCHD10. In some embodiments, the 5'UTR and 3'UTR are derived from the same species or different species.

In some embodiments, the 5'UTR is located upstream of the ORF. In some embodiments, the 5'UTR in the nucleic acid construct is located at the 5' end of the ORF. In some embodiments, the 3'UTR is located downstream of the ORF. In some embodiments, the 3'UTR in the nucleic acid construct is located at the 3' end of the ORF.

In some embodiments, the aforementioned nucleic acid construct comprises:
(a) an open reading frame (ORF),
(b-1) a 3'UTR, wherein the 3'UTR is derived from the 3'UTR of the gene ACTG1, ATP6V0B, ATP6V0E1, CFL1, COX4I1, CTSB, FAM166A, or NDUFB9; and
(b-2) a 5'UTR, wherein the 5'UTR is derived from the 5'UTR of the gene ACTG1, ATP6V0B, ATP6V0E1, CFL1, COX4I1, CTSB, FAM166A, NDUFB9, CHCHD10, SLC38A2, NDUFA11, NDUFV3, PRDX5, GUK1, IAH1, ABHD16A, SLC25A39, ATPIF1, ANAPC11, CCDC12, MRPL14, or APOA1BP.

In some embodiments, the aforementioned nucleic acid construct comprises:
(a) an open reading frame (ORF),
(b-1) a 3'UTR, wherein the 3'UTR is derived from the 3'UTR of the gene CTSB, FAM166A, or NDUFB9; and
(b-2) a 5'UTR, wherein the 5'UTR is derived from the 5'UTR of the gene ACTG1, CHCHD10, or NDUFA11.

In some embodiments, in the aforementioned nucleic acid construct (e.g., a DNA or RNA molecule), the 3'UTR is derived from the 3'UTR of the gene ACTG1 comprising a sequence set forth in SEQ ID NO: 1 or SEQ ID NO: 58 or a sequence having identity with any thereof;
the 3'UTR is derived from the 3'UTR of the gene ATP6V0B comprising a sequence set forth in SEQ ID NO: 2, 3 or SEQ ID NO: 59, 60 or a sequence having identity with any thereof;
the 3'UTR is derived from the 3'UTR of the gene ATP6V0E1 comprising a sequence set forth in SEQ ID NO: 4, 5 or SEQ ID NO: 61, 62 or a sequence having identity with any thereof;
the 3'UTR is derived from the 3'UTR of the gene CFL1 comprising a sequence set forth in SEQ ID NO: 6, 7, 8 or SEQ ID NO: 63, 64, 65 or a sequence having identity with any thereof;
the 3'UTR is derived from the 3'UTR of the gene COX4I1 comprising a sequence set forth in SEQ ID NO: 9, 10, 11 or SEQ ID NO: 66, 67, 68 or a sequence having identity with any thereof;
the 3'UTR is derived from the 3'UTR of the gene CTSB comprising a sequence set forth in SEQ ID NO: 12 or SEQ ID NO: 69 or a sequence having identity with any thereof;
the 3'UTR is derived from the 3'UTR of the gene FAM166A comprising a sequence set forth in SEQ ID NO: 13 or SEQ ID NO: 70 or a sequence having identity with any thereof; or
the 3'UTR is derived from the 3'UTR of the gene NDUFB9 comprising a sequence set forth in SEQ ID NO: 14 or SEQ ID NO: 71 or a sequence having identity with any thereof.

In some embodiments, in the aforementioned nucleic acid construct (e.g., a DNA or RNA molecule), the 5'UTR is derived from the 5'UTR of the gene ACTG1 comprising a sequence set forth in SEQ ID NO: 15 or SEQ ID NO: 72 or a sequence having identity with any thereof;
the 5'UTR is derived from the 5'UTR of the gene ATP6V0B comprising a sequence set forth in SEQ ID NO: 16, 17 or SEQ ID NO: 73, 74 or a sequence having identity with any thereof;
the 5'UTR is derived from the 5'UTR of the gene ATP6V0E1 comprising a sequence set forth in SEQ ID NO: 18, 19 or SEQ ID NO: 75, 76 or a sequence having identity with any thereof;
the 5'UTR is derived from the 5'UTR of the gene CFL1 comprising a sequence set forth in SEQ ID NO: 20, 21, 22 or SEQ ID NO: 77, 78, 79 or a sequence having identity with any thereof;
the 5'UTR is derived from the 5'UTR of the gene COX4I1 comprising a sequence set forth in SEQ ID NO: 23, 24, 25 or SEQ ID NO: 80, 81, 82 or a sequence having identity with any thereof;
the 5'UTR is derived from the 5'UTR of the gene CTSB comprising a sequence set forth in SEQ ID NO: 26 or SEQ ID NO: 83 or a sequence having identity with any thereof;
the 5'UTR is derived from the 5'UTR of the gene FAM166A comprising a sequence set forth in SEQ ID NO: 27 or SEQ ID NO: 84 or a sequence having identity with any thereof;
the 5'UTR is derived from the 5'UTR of the gene NDUFB9 comprising a sequence set forth in SEQ ID NO: 28 or SEQ ID NO: 85 or a sequence having identity with any thereof;
the 5'UTR is derived from the 5'UTR of the gene CHCHD10 comprising a sequence set forth in SEQ ID NO: 29, 30 or SEQ ID NO: 86, 87 or a sequence having identity with any thereof;
the 5'UTR is derived from the 5'UTR of the gene SLC38A2 comprising a sequence set forth in SEQ ID NO: 31 or SEQ ID NO: 88 or a sequence having identity with any thereof;
the 5'UTR is derived from the 5'UTR of the gene NDUFA11 comprising a sequence set forth in SEQ ID NO: 32 or SEQ ID NO: 89 or a sequence having identity with any thereof;
the 5'UTR is derived from the 5'UTR of the gene NDUFV3 comprising a sequence set forth in SEQ ID NO: 33 or SEQ ID NO: 90 or a sequence having identity with any thereof;
the 5'UTR is derived from the 5'UTR of the gene PRDX5 comprising a sequence set forth in SEQ ID NO: 34 or SEQ ID NO: 91;
the 5'UTR is derived from the 5'UTR of the gene GUK1 comprising a sequence set forth in SEQ ID NO: 35, 36, 37 or SEQ ID NO: 92, 93, 94 or a sequence having identity with any thereof;
the 5'UTR is derived from the 5'UTR of the gene IAH1 comprising a sequence set forth in SEQ ID NO: 38 or SEQ ID NO: 95 or a sequence having identity with any thereof;
the 5'UTR is derived from the 5'UTR of the gene ABHD16A comprising a sequence set forth in SEQ ID NO: 39 or SEQ ID NO: 96 or a sequence having identity with any thereof;
the 5'UTR is derived from the 5'UTR of the gene SLC25A39 comprising a sequence set forth in SEQ ID NO: 40 or SEQ ID NO: 97 or a sequence having identity with any thereof;
the 5'UTR is derived from the 5'UTR of the gene ATPIF1 comprising a sequence set forth in SEQ ID NO: 41 or SEQ ID NO: 98 or a sequence having identity with any thereof;
the 5'UTR is derived from the 5'UTR of the gene ANAPC11 comprising a sequence set forth in SEQ ID NO: 42, 43 or SEQ ID NO: 99, 100 or a sequence having identity with any thereof;
the 5'UTR is derived from the 5'UTR of the gene CCDC12 comprising a sequence set forth in SEQ ID NO: 44 or SEQ ID NO: 101 or a sequence having identity with any thereof;
the 5'UTR is derived from the 5'UTR of the gene MRPL14 comprising a sequence set forth in SEQ ID NO: 45 or SEQ ID NO: 102 or a sequence having identity with any thereof; or
the 5'UTR is derived from the 5'UTR of the gene APOA1BP comprising a sequence set forth in SEQ ID NO: 46, 47 or SEQ ID NO: 103, 104 or a sequence having identity with any thereof.

In some embodiments, a nucleic acid construct (e.g., a DNA or RNA molecule) is provided, which comprises:
(a) an open reading frame (ORF),
(b-1) a 3'UTR, and
(b-2) a 5'UTR;
wherein the 3'UTR and the 5'UTR are selected from any one of the following combinations:
   1) the 3'UTR comprises a sequence set forth in SEQ ID NO: 1 or SEQ ID NO: 58 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   2) the 3'UTR comprises a sequence set forth in SEQ ID NO: 2 or SEQ ID NO: 59 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   3) the 3'UTR comprises a sequence set forth in SEQ ID NO: 3 or SEQ ID NO: 60 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   4) the 3'UTR comprises a sequence set forth in SEQ ID NO: 4 or SEQ ID NO: 61 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   5) the 3'UTR comprises a sequence set forth in SEQ ID NO: 5 or SEQ ID NO: 62 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   6) the 3'UTR comprises a sequence set forth in SEQ ID NO: 6 or SEQ ID NO: 63 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   7) the 3'UTR comprises a sequence set forth in SEQ ID NO: 7 or SEQ ID NO: 64 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   8) the 3'UTR comprises a sequence set forth in SEQ ID NO: 8 or SEQ ID NO: 65 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   9) the 3'UTR comprises a sequence set forth in SEQ ID NO: 9 or SEQ ID NO: 66 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   10) the 3'UTR comprises a sequence set forth in SEQ ID NO: 10 or SEQ ID NO: 67 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   11) the 3'UTR comprises a sequence set forth in SEQ ID NO: 11 or SEQ ID NO: 68 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   12) the 3'UTR comprises a sequence set forth in SEQ ID NO: 12 or SEQ ID NO: 69 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   13) the 3'UTR comprises a sequence set forth in SEQ ID NO: 13 or SEQ ID NO: 70 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   14) the 3'UTR comprises a sequence set forth in SEQ ID NO: 14 or SEQ ID NO: 71 or a sequence having identity with any thereof, and the 5'UTR comprises a sequence set forth in any one of SEQ ID NOs: 15 to 47 or any one of SEQ ID NOs: 72 to 104 or a sequence having identity with any thereof;
   15) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 15 or SEQ ID NO: 72 or a sequence having identity with any thereof;
   16) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 16 or SEQ ID NO: 73 or a sequence having identity with any thereof;
   17) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 17 or SEQ ID NO: 74 or a sequence having identity with any thereof;
   18) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 18 or SEQ ID NO: 75 or a sequence having identity with any thereof;
   19) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 19 or SEQ ID NO: 76 or a sequence having identity with any thereof;
   20) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 20 or SEQ ID NO: 77 or a sequence having identity with any thereof;
   21) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 21 or SEQ ID NO: 78 or a sequence having identity with any thereof;
   22) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 22 or SEQ ID NO: 79 or a sequence having identity with any thereof;
   23) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 23 or SEQ ID NO: 80 or a sequence having identity with any thereof;
   24) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 24 or SEQ ID NO: 81 or a sequence having identity with any thereof;
   25) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 25 or SEQ ID NO: 82 or a sequence having identity with any thereof;
   26) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 26 or SEQ ID NO: 83 or a sequence having identity with any thereof;
   27) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 27 or SEQ ID NO: 84 or a sequence having identity with any thereof;
   28) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 28 or SEQ ID NO: 85 or a sequence having identity with any thereof;
   29) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 29 or SEQ ID NO: 86 or a sequence having identity with any thereof;
   30) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 30 or SEQ ID NO: 87 or a sequence having identity with any thereof;
   31) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 31 or SEQ ID NO: 88 or a sequence having identity with any thereof;
   32) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 32 or SEQ ID NO: 89 or a sequence having identity with any thereof;
   33) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 33 or SEQ ID NO: 90 or a sequence having identity with any thereof;
   34) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 34 or SEQ ID NO: 91 or a sequence having identity with any thereof;
   35) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 35 or SEQ ID NO: 92 or a sequence having identity with any thereof;
   36) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 36 or SEQ ID NO: 93 or a sequence having identity with any thereof;
   37) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 37 or SEQ ID NO: 94 or a sequence having identity with any thereof;
   38) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 38 or SEQ ID NO: 95 or a sequence having identity with any thereof;
   39) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 39 or SEQ ID NO: 96 or a sequence having identity with any thereof;
   40) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 40 or SEQ ID NO: 97 or a sequence having identity with any thereof;
   41) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 41 or SEQ ID NO: 98 or a sequence having identity with any thereof;
   42) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 42 or SEQ ID NO: 99 or a sequence having identity with any thereof;
   43) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 43 or SEQ ID NO: 100 or a sequence having identity with any thereof;
   44) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 44 or SEQ ID NO: 101 or a sequence having identity with any thereof;
   45) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 45 or SEQ ID NO: 102 or a sequence having identity with any thereof;
   46) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 46 or SEQ ID NO: 103 or a sequence having identity with any thereof; or
   47) the 3'UTR comprises a sequence set forth in any one of SEQ ID NOs: 1 to 14 or any one of SEQ ID NOs: 58 to 71 or a sequence having identity with any thereof, and the 5'UTR comprises a sequence set forth in SEQ ID NO: 47 or SEQ ID NO: 104 or a sequence having identity with any thereof.

In some embodiments, a nucleic acid construct (e.g., a DNA or RNA molecule) is provided, which comprises:
(a) an open reading frame (ORF),
(b-1) a 3'UTR, and
(b-2) a 5'UTR;
the 3'UTR is derived from the 3'UTR of the gene CTSB, FAM166A, or NDUFB9, and the 5'UTR is derived from the 5'UTR of the gene ACTG1, CHCHD10, or NDUFA11;
for example, the 3'UTR comprises a sequence set forth in any one of SEQ ID NOs: 12, 13, 14 or any one of SEQ ID NOs: 69, 70, 71 or a sequence having identity with any thereof, and the 5'UTR comprises a sequence set forth in any one of SEQ ID NOs: 15, 29, 30, 32 or any one of SEQ ID NOs: 72, 86, 87, 89 or a sequence having identity with any thereof;
for another example, the 3'UTR comprises a sequence set forth in SEQ ID NO: 12 or SEQ ID NO: 69 or a sequence having identity with any thereof, and the 5'UTR comprises a sequence set forth in SEQ ID NO: 15 or SEQ ID NO: 72 or a sequence having identity with any thereof;
the 3'UTR comprises a sequence set forth in SEQ ID NO: 13 or SEQ ID NO: 70 or a sequence having identity with any thereof, and the 5'UTR comprises a sequence set forth in SEQ ID NO: 15 or SEQ ID NO: 72 or a sequence having identity with any thereof;
the 3'UTR comprises a sequence set forth in SEQ ID NO: 14 or SEQ ID NO: 71 or a sequence having identity with any thereof, and the 5'UTR comprises a sequence set forth in SEQ ID NO: 15 or SEQ ID NO: 72 or a sequence having identity with any thereof;
the 3'UTR comprises a sequence set forth in SEQ ID NO: 12 or SEQ ID NO: 69 or a sequence having identity with any thereof, and the 5'UTR comprises a sequence set forth in SEQ ID NO: 29 or SEQ ID NO: 86 or a sequence having identity with any thereof;
the 3'UTR comprises a sequence set forth in SEQ ID NO: 13 or SEQ ID NO: 70 or a sequence having identity with any thereof, and the 5'UTR comprises a sequence set forth in SEQ ID NO: 29 or SEQ ID NO: 86 or a sequence having identity with any thereof;
the 3'UTR comprises a sequence set forth in SEQ ID NO: 14 or SEQ ID NO: 71 or a sequence having identity with any thereof, and the 5'UTR comprises a sequence set forth in SEQ ID NO: 29 or SEQ ID NO: 86 or a sequence having identity with any thereof;
the 3'UTR comprises a sequence set forth in SEQ ID NO: 12 or SEQ ID NO: 69 or a sequence having identity with any thereof, and the 5'UTR comprises a sequence set forth in SEQ ID NO: 30 or SEQ ID NO: 87 or a sequence having identity with any thereof;
the 3'UTR comprises a sequence set forth in SEQ ID NO: 13 or SEQ ID NO: 70 or a sequence having identity with any thereof, and the 5'UTR comprises a sequence set forth in SEQ ID NO: 30 or SEQ ID NO: 87 or a sequence having identity with any thereof;
the 3'UTR comprises a sequence set forth in SEQ ID NO: 14 or SEQ ID NO: 71 or a sequence having identity with any thereof, and the 5'UTR comprises a sequence set forth in SEQ ID NO: 30 or SEQ ID NO: 87 or a sequence having identity with any thereof;
the 3'UTR comprises a sequence set forth in SEQ ID NO: 12 or SEQ ID NO: 69 or a sequence having identity with any thereof, and the 5'UTR comprises a sequence set forth in SEQ ID NO: 32 or SEQ ID NO: 89 or a sequence having identity with any thereof;
the 3'UTR comprises a sequence set forth in SEQ ID NO: 13 or SEQ ID NO: 70 or a sequence having identity with any thereof, and the 5'UTR comprises a sequence set forth in SEQ ID NO: 32 or SEQ ID NO: 89 or a sequence having identity with any thereof; or
the 3'UTR comprises a sequence set forth in SEQ ID NO: 14 or SEQ ID NO: 71 or a sequence having identity with any thereof, and the 5'UTR comprises a sequence set forth in SEQ ID NO: 32 or SEQ ID NO: 89 or a sequence having identity with any thereof.

In the above embodiments, "having ... identity" covers having an identity of at least 80%, 85%, 90%, 95%, 96%, 97%, 98%, 99%, 100%, and a range between any two of the aforementioned values, including integers and decimals. For example, "having at least 90% identity" or "having at least 95% identity".

In some embodiments, the 3'UTR and/or 5'UTR is a variant of the aforementioned 3'UTR and/or 5'UTR, the variant is, for example, a truncation or a nucleotide mutant, the variant still retains an activity or function similar to the aforementioned 3'UTR and/or 5'UTR of the present disclosure, for example, it still retains the function of regulating the expression of a target protein encoded by the ORF of the gene of interest.

In some embodiments, the aforementioned nucleic acid construct provided by the present disclosure further comprises:
(c) a polyadenylation (poly-A) tail.

In some specific embodiments, the poly-A tail in the nucleic acid construct is located downstream of the 3'UTR. In some specific embodiments, the poly-A tail in the nucleic acid construct is located at the 3' end of the 3'UTR. In some specific embodiments, the poly-A tail is at the 3' end of the nucleic acid construct. In some specific embodiments, the length of the poly-A tail is at least about 50, 100, 120, 150, 200, 300, 400, or 500 nucleotides.

In some specific embodiments, the poly-A tail is selected from A120, A30L70, HGH polyA, SV40 polyA, BGH polyA, rbGlob polyA, or SV40late polyA. For example, the A30L70 is a sequence set forth in SEQ ID NO: 52; and the A120 comprises 120 adenine nucleotides.

In some embodiments, in the aforementioned nucleic acid construct provided by the present disclosure, the expressed gene of interest (*i.e.*, the open reading frame, ORF) is hepatocyte growth factor (HGF), an antibody, or an antigen-binding fragment thereof. For example, an antibody or an antigen-binding fragment thereof binding to a tumor antigen, an antibody or an antigen-binding fragment thereof binding to a viral antigen, and the like.

In some specific embodiments, the polypeptide or protein encoded by the ORF is a fluorescent protein or luciferase, such as a sequence set forth in SEQ ID NO: 126.

In some specific embodiments, the HGF is human hepatocyte growth factor (hHGF).

In some specific embodiments, the coding sequence of hHGF comprises any one selected from the following 1) to 3):
1) a nucleic acid sequence encoding an amino acid sequence set forth in SEQ ID NO: 109 or a codon-optimized nucleic acid sequence;
2) a DNA sequence set forth in SEQ ID NOs: 110 to 113 or a DNA sequence having an identity of at least 80%, 85%, 90%, 95%, 96%, 97%, 98%, 99%, or 100% therewith;
3) an RNA sequence set forth in any one of SEQ ID NOs: 128 to 131 or an RNA sequence having an identity of at least 80%, 85%, 90%, 95%, 96%, 97%, 98%, 99%, or 100% therewith.

In some specific embodiments, the nucleic acid construct expressing HGF as the gene of interest comprises a sequence set forth in any one of SEQ ID NO: 115, 116, 127 or a sequence having an identity of at least 80%, 85%, 90%, 95%, 96%, 97%, 98%, 99%, or 100% therewith.

In some specific embodiments, the antibody or the antigen-binding fragment thereof is an anti-PD-1 antibody or an antigen-binding fragment thereof.

In some specific embodiments, the coding sequence of the anti-PD-1 antibody or the antigen-binding fragment thereof comprises any one selected from the following 1) to 4):
1) a nucleic acid sequence or codon-optimized nucleic acid sequence encoding a heavy chain amino acid sequence set forth in SEQ ID NO: 117, and a nucleic acid sequence or codon-optimized nucleic acid sequence encoding a light chain amino acid sequence set forth in SEQ ID NO: 118;
2) a nucleic acid sequence or codon-optimized nucleic acid sequence encoding HCDR1, HCDR2, and HCDR3 in the heavy chain amino acid sequence set forth in SEQ ID NO: 117, and a nucleic acid sequence or codon-optimized nucleic acid sequence encoding LCDR1, LCDR2, and LCDR3 in the light chain amino acid sequence set forth in SEQ ID NO: 118, wherein the CDRs are defined according to a Kabat, IMGT, Chothia, AbM, or Contact numbering system, for example, defined according to a Kabat numbering system;
3) a DNA sequence set forth in SEQ ID NO: 119 or a DNA sequence having an identity of at least 80%, 85%, 90%, 95%, 96%, 97%, 98%, 99%, or 100% therewith, and a DNA sequence set forth in SEQ ID NO: 120 or a DNA sequence having an identity of at least 80%, 85%, 90%, 95%, 96%, 97%, 98%, 99%, or 100% therewith;
4) a DNA sequence set forth in SEQ ID NO: 121 or a DNA sequence having an identity of at least 80%, 85%, 90%, 95%, 96%, 97%, 98%, 99%, or 100% therewith, and a DNA sequence set forth in SEQ ID NO: 122 or a DNA sequence having an identity of at least 80%, 85%, 90%, 95%, 96%, 97%, 98%, 99%, or 100% therewith.

In some specific embodiments, the nucleic acid construct expressing the anti-PD-1 antibody or the antigen-binding fragment thereof comprises the sequences set forth in SEQ ID NO: 124 and 125, or sequences having an identity of at least 80%, 85%, 90%, 95%, 96%, 97%, 98%, 99%, or 100% with SEQ ID NO: 124 and 125.

In some embodiments, the aforementioned nucleic acid construct provided by the present disclosure is DNA or RNA, for example, mRNA.

In some embodiments, from a 5' to 3' direction, the aforementioned nucleic acid construct (DNA or RNA) provided by the present disclosure comprises any one of the following 1) to 6):
1) a 5'UTR and an ORF;
2) an ORF and a 3'UTR;
3) a 5'UTR, an ORF, and a 3'UTR;
4) a 5'UTR, an ORF, a 3'UTR, and a poly-A tail;
5) a 5'UTR, an ORF, and a poly-A tail;
6) an ORF, a 3'UTR, and a poly-A tail;
   the 5'UTR and 3'UTR may be derived from the same or different genes.

In some embodiments, from a 5' to 3' direction, the aforementioned nucleic acid construct (DNA) provided by the present disclosure comprises any one of the following 1) to 4):
1) a 5'UTR and an ORF;
2) an ORF and a 3'UTR;
3) a 5'UTR, an ORF, and a 3'UTR;
4) a 5'UTR, an ORF, a 3'UTR, and a poly-A tail;
the 5'UTR and 3'UTR may be derived from the same or different genes.

In some specific embodiments, the 5'UTR in 1), 3), and 4) comprises or is a nucleotide sequence set forth in any one of SEQ ID NOs: 15 to 47. In some specific embodiments, the ORF in 1) to 4) comprises or is a nucleotide sequence set forth in SEQ ID NO: 110 or a codon-optimized nucleotide sequence thereof (e.g., SEQ ID NOs: 111 to 113). In some specific embodiments, the ORF in 1) to 4) comprises or is the nucleotide sequences set forth in SEQ ID NOs: 119 and 120. In some specific embodiments, the 3'UTR in 2) to 4) comprises or is a nucleotide sequence set forth in any one of SEQ ID NOs: 1 to 14.

In some embodiments, the aforementioned nucleic acid construct (RNA or mRNA) provided by the present disclosure further comprises:
(d) a 5' cap structure (5'Cap).

In some specific embodiments, the 5'Cap structure in the RNA molecule is located upstream of the 5'UTR. In some embodiments, the 5'Cap structure in the RNA molecule is located at the 5' end of the 5'UTR. In some embodiments, the 5' cap structure is a cap structure known to those skilled in the art, such as Cap0 (methylation of the first nucleobase, e.g., m⁷GpppN), Cap1 (additional methylation of the ribose of the adjacent nucleotide of m⁷GpppN, e.g., m⁷G(5')ppp(5')(2'OMeA)pG), Cap2 (additional methylation of the ribose of the 3^{rd} nucleotide downstream of m⁷GpppN), Cap3 (additional methylation of the ribose of the 3^{rd} nucleotide downstream of m⁷GpppN), Cap4 (additional methylation of the ribose of the 4^{th} nucleotide downstream of m⁷GpppN), ARCA (anti-reverse cap analog), modified ARCA (e.g., phosphorothioate-modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In some specific embodiments, chemical RNA synthesis or *in vitro* RNA transcription (co-transcriptional capping) is used to form a 5'Cap structure (such as Cap0 or Cap1).

In some specific embodiments, a capping enzyme (e.g., vaccinia virus capping enzyme and/or cap-dependent 2'-O methyltransferase) is used via enzymatic capping to form a 5'-cap structure (such as Cap0 or Cap1). In some embodiments, an immobilized capping enzyme is used to add a 5' cap structure (Cap0 or Cap1). The capping methods and means in WO2016/193226 are incorporated herein by reference in their entirety.

In some specific embodiments, the 5'Cap is selected from ARCA, 3'-O-Mem⁷G(5')ppp(5')G, m⁷G(5')ppp(5')(2'OMeA)pU, m⁷Gppp(A2'O-MOE)pG, m⁷G(5')ppp(5')(2'OMeA)pG, m⁷G(5')ppp(5')(2'OMeG)pG, m⁷(3'OMeG)(5')ppp(5')(2'OMeG)pG and m⁷(3'OMeG)(5')ppp(5')(2'OMeA)pG. In some specific embodiments, the 5'Cap is 3'-O-Me-m⁷G(5')ppp(5')G or m⁷G(5')ppp(5')(2'OMeA)pG.

In some embodiments, from a 5' to 3' direction, the nucleic acid construct (RNA or mRNA) provided by the present disclosure comprises any one of the following 1) to 5):
1) a 5'UTR and an ORF;
2) an ORF and a 3'UTR;
3) a 5'UTR, an ORF, and a 3'UTR;
4) a 5'UTR, an ORF, a 3'UTR, and a poly-A tail;
5) a 5'Cap, a 5'UTR, an ORF, a 3'UTR, and a poly-A tail;
the 5'UTR and 3'UTR may be derived from the same or different genes.

In some specific embodiments, the 5'UTR in 1), 3), 4), and 5) comprises or is a nucleotide sequence set forth in any one of SEQ ID NOs: 72 to 104. In some specific embodiments, the ORF in 1) to 5) comprises or is a nucleotide sequence set forth in any one of SEQ ID NOs: 128 to 131. In some specific embodiments, the 3'UTR in 2) to 5) comprises or is a nucleotide sequence set forth in any one of SEQ ID NOs: 58 to 71. In some specific embodiments, the structures comprise, but are not limited to, Cap0, Cap1 (e.g., m⁷G(5')ppp(5')(2'OMeA)pG), Cap2, Cap3, Cap4, ARCA.

In some embodiments, the present disclosure provides a nucleic acid construct (DNA) sequentially comprising, from a 5' to 3' direction, a 5'UTR, an ORF, and a 3'UTR, and optionally further comprising a poly-A tail in the 3' direction. In some embodiments, the 5'UTR comprises or is a nucleotide sequence set forth in any one of SEQ ID NOs: 15 to 47, the ORF comprises or is a nucleotide sequence set forth in SEQ ID NO: 110 or a codon-optimized nucleotide sequence thereof (e.g., SEQ ID NOs: 111 to 113), the 3'UTR comprises or is a nucleotide sequence set forth in any one of SEQ ID NOs: 1 to 14. In some embodiments, the 5'UTR comprises or is a nucleotide sequence set forth in any one of SEQ ID NOs: 15 to 47, the ORF comprises or is the nucleotide sequences set forth in SEQ ID NOs: 119 and 120, the 3'UTR comprises or is a nucleotide sequence set forth in any one of SEQ ID NOs: 1 to 14. In some specific embodiments, the nucleic acid construct comprises a nucleotide sequence set forth in SEQ ID NO: 115, 116, or 127.

In some embodiments, the present disclosure provides a nucleic acid construct (RNA or mRNA) sequentially comprising, from a 5' to 3' direction, a 5'UTR, an ORF, and a 3'UTR, and optionally further comprising a poly-A tail in the 3' direction. In some embodiments, the 5'UTR comprises or is a nucleotide sequence set forth in any one of SEQ ID NOs: 72 to 104, the ORF comprises or is a nucleotide sequence set forth in any one of SEQ ID NOs: 128 to 131, the 3'UTR comprises or is a nucleotide sequence set forth in any one of SEQ ID NOs: 58 to 71, and the poly-A tail comprises or is 120 consecutive adenylates or a nucleotide sequence set forth in SEQ ID NO: 52. In some specific embodiments, the nucleic acid construct comprises a nucleotide sequence set forth in SEQ ID NO: 132.

In some embodiments, the 5'UTR comprises or is a nucleotide sequence set forth in any one of SEQ ID NOs: 72 to 104, the ORF comprises or is the nucleotide sequences set forth in SEQ ID NOs: 121 and 122, the 3'UTR comprises or is a nucleotide sequence set forth in any one of SEQ ID NOs: 58 to 71, and the poly-A tail comprises or is 120 consecutive adenylates or a nucleotide sequence set forth in SEQ ID NO: 52. In some specific embodiments, the nucleic acid construct comprises a nucleotide sequence set forth in SEQ ID NO: 124 or 125.

In some embodiments, in any one of the aforementioned nucleic acid constructs (DNA or RNA molecules), the UTR is used to increase the expression level of the protein encoded by the ORF. Illustratively, in some specific embodiments, compared to the 5'UTR set forth in SEQ ID NO: 48 or 50, the 5'UTR set forth in any one of SEQ ID NOs: 15 to 47 and 72 to 104 of the present disclosure has an increased expression level of the target protein expressed by regulating the ORF. In some specific embodiments, compared to the 5'UTR set forth in SEQ ID NO: 49 or 51, the 3'UTR set forth in any one of SEQ ID NOs: 1 to 14, 58 to 71 of the present disclosure has an increased expression level of the target protein expressed by regulating the ORF. In some specific embodiments, compared to the combination of the 5'UTR and 3'UTR set forth in SEQ ID NOs: 48 and 49, the combination of the 5'UTR set forth in any one of SEQ ID NOs: 15 to 47, 72 to 104 and the 3'UTR set forth in any one of SEQ ID NOs: 1 to 14 and 58 to 71 of the present disclosure has an increased expression level of the target protein expressed by regulating the ORF. In some specific embodiments, compared to the combination of the 5'UTR and 3'UTR set forth in SEQ ID NOs: 50 and 51, the combination of the 5'UTR set forth in any one of SEQ ID NOs: 15 to 47 and 72 to 104 and the 3'UTR set forth in any one of SEQ ID NOs: 1 to 14 and 58 to 71 of the present disclosure has an increased expression level of the target protein expressed by regulating the ORF.

In some embodiments, the expression level of the target protein of the nucleic acid construct of the present disclosure is about 1 to about 20 times that of the BioN vector, for example, about 1 time, about 1.1 times, about 1.2 times, about 1.3 times, about 1.4 times, about 1.5 times, about 1.6 times, about 1.7 times, about 1.8 times, about 1.9 times, about 2 times, about 2.1 times, about 2.3 times, about 2.5 times, about 2.8 times, about 3 times, about 3.2 times, about 3.4 times, about 3.8 times, about 4 times, about 4.5 times, about 5 times, about 5.2 times, about 5.5 times, about 5.8 times, about 6 times, about 7 times, about 8 times, about 10 times, about 12 times, about 15 times, etc. In some specific embodiments, the nucleic acid construct of the present disclosure is an mRNA molecule expressing a target protein, the expression level of the target protein of the mRNA molecule is about 1 to about 20 times that of the BioN vector. In some specific embodiments, the BioN vector comprises a 5'UTR set forth in SEQ ID NO: 50 or 107; and/or comprises a 3'UTR set forth in SEQ ID NO: 51 or 108.

In some embodiments, the expression level of the target protein of the nucleic acid construct of the present disclosure is about 1 to about 20 times that of the Mod vector, for example, about 1 time, about 1.1 times, about 1.2 times, about 1.3 times, about 1.4 times, about 1.5 times, about 1.6 times, about 1.7 times, about 1.8 times, about 1.9 times, about 2 times, about 2.1 times, about 2.3 times, about 2.5 times, about 2.8 times, about 3 times, about 3.2 times, about 3.4 times, about 3.8 times, about 4 times, about 4.5 times, about 5 times, about 5.2 times, about 5.5 times, about 5.8 times, about 6 times, about 7 times, about 8 times, about 10 times, about 12 times, about 15 times, etc. In some specific embodiments, the nucleic acid construct of the present disclosure is an mRNA molecule expressing a target protein, the expression level of the target protein of the mRNA molecule is about 1 to about 20 times that of the BioN vector. In some specific embodiments, the Mod vector comprises a 5'UTR set forth in SEQ ID NO: 48 or 105; and/or comprises a 3'UTR set forth in SEQ ID NO: 49 or 106.

In some embodiments, the nucleic acid construct of the present disclosure is delivered into a subject, and the target protein (e.g., hHGF protein) is expressed after about 0.5 to 1.5 h. In some embodiments, the nucleic acid construct of the present disclosure is delivered into a subject, and reaches an expression peak at about 2 h to 10 h (e.g., about 2 h, about 3 h, about 4 h, about 5 h, about 6 h, about 7 h, about 8 h, about 10 h, etc.). In some specific embodiments, the nucleic acid construct of the present disclosure is an mRNA molecule expressing a target protein, for example, the nucleotide construct is an mRNA molecule expressing the hHGF protein.

In some embodiments, the nucleic acid construct of the present disclosure is delivered into a subject, and has higher pharmacokinetic performance than the Collategene plasmid. In some specific embodiments, the nucleic acid construct is an mRNA molecule; compared with the Collategene plasmid, the mRNA molecule has improved pharmacokinetic performance (e.g., Cmax, AUC_{0-inf}, MRT_{0-inf}).

In some embodiments, the nucleic acid construct of the present disclosure (e.g., mA16-B12 (hHGF) expressing the hHGF protein) is capable of improving the blood perfusion ratio of the ischemic hindlimb in a subject. Illustratively, an improved blood perfusion ratio could be achieved in hindlimb ischemia mice injected with 50 ng/mouse and 500 ng/mouse of m-A16-B12 (hHGF). The hindlimb ischemia mice injected with about 500 ng/mouse of m-A16-B12 (hHGF) could achieve a restoration of blood perfusion to over about 90%.

In some embodiments, the nucleic acid construct of the present disclosure (e.g., mA16-B12 (hHGF) expressing the hHGF protein) is capable of improving ischemic necrosis in a subject induced with hindlimb ischemia. Illustratively, the hindlimbs of hindlimb ischemia mice injected with 50 ng/mouse and 500 ng/mouse of m-A16-B12 (hHGF) both maintained good integrity, and no hindlimb necrosis occurred.

In some embodiments, the nucleic acid construct of the present disclosure (e.g., mA16-B12 (hHGF) expressing the hHGF protein) can promote angiogenesis in the ischemic hindlimb muscles of each group. Illustratively, in the cases where 50 ng/mouse and 500 ng/mouse of m-A16-B12 (hHGF) were administered, angiogenesis in the ischemic hindlimb muscles of each group was significantly promoted, and the number of newly formed blood vessels had a significant statistical difference compared with the PBS group (*p* < 0.05).

In some embodiments, the nucleic acid construct of the present disclosure (e.g., mA16-B12 (hHGF) expressing the hHGF protein) is capable of improving wound healing in a diabetic subject. Illustratively, in Db/Db diabetic mouse models injected with 50 ng/mouse, 200 ng/mouse, and 500 ng/mouse of m-A16-B12 (hHGF), the wounds of the mice were all well healed. On day 14, the wound healing rate for the 50 ng/mouse dose of m-A16-B12 (hHGF) reached 66%, and the 200 ng/mouse and 500 ng/mouse doses of m-A16-B12 (hHGF) achieved 100% wound healing.

The mRNA provided by the present disclosure comprises 5'UTRs and 3'UTRs of a novel structure, which reduces early degradation of the mRNA or stabilizes degradation of the mRNA, but without losing or rather enhancing protein translation efficiency. The mRNA has higher stability and can be applied in gene therapy and genetic vaccination. In addition, the present disclosure provides an mRNA capable of expressing human hepatocyte growth factor (hHGF) and a lipid nanoparticle (LNP) delivery system thereof, which can achieve efficient and rapid *in vivo* transformation of exogenous hHGF protein, it has the advantages of no integration risk and easy industrial-scale scale-up. It is an ideal treatment regimen compared to a naked plasmid, and can be used as a gene therapy drug for a variety of diseases such as CLI and DFU.

### Polynucleotide

The present disclosure also provides an isolated polynucleotide comprising (a) an open reading frame (ORF). Illustratively, the open reading frame (ORF) encodes hepatocyte growth factor (HGF), such as human hepatocyte growth factor (hHGF).

In some embodiments, the coding sequence of the hHGF comprises any one selected from the following 1) to 3):
1) a nucleic acid sequence encoding an amino acid sequence set forth in SEQ ID NO: 109 or a codon-optimized sequence thereof;
2) a DNA sequence set forth in any one of SEQ ID NOs: 110 to 113 or a DNA sequence having an identity of at least 80%, 85%, 90%, 95%, 96%, 97%, 98%, 99%, or 100% therewith;
3) an RNA sequence set forth in any one of SEQ ID NOs: 128 to 131 or an RNA sequence having an identity of at least 80%, 85%, 90%, 95%, 96%, 97%, 98%, 99%, or 100% therewith.

In some specific embodiments, the 5' end of the polynucleotide may comprise any 5'UTR provided by the present disclosure, and/or the 3' end of the polynucleotide may comprise any 3'UTR provided by the present disclosure.

In some specific embodiments, the 5' end of the polynucleotide may comprise any 5'Cap provided by the present disclosure, and/or the 3' end of the polynucleotide may comprise any poly-A tail provided by the present disclosure.

In some embodiments, the polynucleotide is RNA, such as mRNA.

### Modification

In order to further improve the stability of the RNA or polynucleotide of the present disclosure regarding protein expression, the RNA or polynucleotide may further comprise one or more modifications (including chemical modifications), for example, a backbone modification, a glycosyl modification, a base modification, and/or a lipid modification. In some embodiments, the RNA or polynucleotide is uniformly modified into a specific modification (e.g., completely modified throughout the sequence). For example, the RNA can be uniformly modified with pseudouridine (e.g., N1-methylpseudouridine) such that every U in the sequence is pseudouridine.

The backbone modification related to the present disclosure refers to a chemical modification of the phosphate of the backbone of the nucleotide contained in the RNA or polynucleotide of the present disclosure. In some embodiments, the backbone modification includes, but is not limited to, completely replacing the unmodified phosphate moiety in the backbone with a modified phosphate, for example, the phosphate group of the backbone can be modified by replacing one or more oxygen atoms with different substituents. In some embodiments, the modified phosphate includes, but is not limited to, phosphorothioate, phosphoroselenoate, boranophosphate, boranophosphate, hydrogen phosphonate, phosphoramidate, alkyl or aryl phosphonate, and phosphotriester.

The glycosyl modification related to the present disclosure refers to a chemical modification of the sugar of the nucleotide contained in the RNA or polynucleotide of the present disclosure. In some embodiments, the glycosyl modification includes, but is not limited to, modifying or replacing the 2' hydroxy (OH) of the RNA molecule with many different "oxy" or "deoxy" substituents. In some embodiments, the "oxy" modification includes, but is not limited to, a substitution modification with an alkoxy, an aryloxy, polyethylene glycol (PEG), and the like. In some embodiments, the "deoxy" modification includes, but is not limited to, modification with a hydrogen, an amino (e.g., NH₂, alkylamino, dialkylamino, heterocyclyl, arylamino, diarylamino, heteroarylamino, diheteroarylamino, or amino acid).

The base modification related to the present disclosure refers to a chemical modification of the base portion of the nucleotide contained in the RNA or polynucleotide of the present disclosure. In some embodiments, the base modification includes modifications to adenine, guanine, cytosine, and uracil in the nucleotide. For example, the nucleosides and nucleotides described herein can be chemically modified on the major groove surface. In some embodiments, the major groove chemical modification may include an amino, thiol, alkyl, or halogen group. In some embodiments, the base modification includes, but is not limited to, a modification with pseudouridine, 1-methyl-pseudouridine, 5-azacytidine, 5-methylcytosine-5'-triphosphate, or 2-methoxyadenine. In some embodiments, the base modification is a pseudouridine modification. For example, the RNA can be uniformly modified with pseudouridine such that every U in the sequence is pseudouridine.

The lipid modification related to the present disclosure refers to a lipid modification in the RNA or polynucleotide of the present disclosure. In some embodiments, the lipid modification includes, but is not limited to, the RNA or polynucleotide of the present disclosure being covalently linked to at least one linker, and the corresponding linker being covalently linked to at least one lipid. In some embodiments, the lipid modification includes, but is not limited to, the RNA or polynucleotide of the present disclosure being covalently linked to at least one lipid (without a linker).

### UTRs

UTRs (5'UTR and/or 3'UTR) can be provided as flanking regions to the nucleic acid construct, RNA, or polynucleotide molecule of the present disclosure. The UTRs may be homologous or heterologous to the coding region in the nucleic acid construct, RNA, or polynucleotide molecule of the present disclosure. The flanking region may comprise one or more 5'UTRs and/or 3'UTRs, the UTRs may be the same or different sequences. Any portion of the flanking region can be codon-optimized. Before and/or after codon optimization, any portion of the flanking region may independently comprise one or more different structural or chemical modifications.

In order to alter one or more properties of the nucleic acid construct, RNA, or polynucleotide of the present disclosure, UTRs heterologous to the ORF of the present disclosure are introduced or engineered and synthesized into the nucleic acid construct, RNA, or polynucleotide of the present disclosure. The recombinant nucleic acid construct, RNA, or polynucleotide is then administered to a cell, tissue, or organism, and the results (such as protein level, localization, and/or half-life) are measured to evaluate the beneficial effects produced by the heterologous UTR on the RNA or polynucleotide of the present disclosure. In some embodiments, the UTR includes a wild-type UTR or a variant thereof, the UTR variant includes adding or removing one or more nucleotides (including A, T, C, or G) at the end. In some embodiments, the UTR variant also includes codon optimization or modification performed in any manner. In some embodiments, the UTR variant also includes a derived sequence of any embodiment of the present disclosure; for example, based on a natural UTR sequence, part of the nucleotides is subjected to point mutation, the target gene expression level and stability of the mutated variant are maintained or improved. The detection methods for the target gene expression level and stability are conventional in the art, such as the detection methods in Examples 3 and 4 of the present disclosure.

### Vector

The present disclosure also provides a vector comprising any one of the aforementioned nucleic acid constructs, RNAs, or polynucleotides. The nucleic acid construct, RNA, or polynucleotide may be present in the vector and/or may be a part of the vector; the vector is, for example, a plasmid, a cosmid, a YAC, or a viral vector. The vector may be an expression vector, that is, a vector that can provide expression of a polypeptide encoded by the nucleic acid construct, RNA, or polynucleotide. The expression vector generally comprises at least one nucleic acid of the present disclosure operably linked to one or more suitable expression regulatory elements (e.g., a promoter, a terminator, etc.). It is common knowledge for those skilled in the art to select the elements and sequences thereof for expression in a specific host. Regulatory elements and other elements useful or necessary for the expression of the encoded polypeptide of the present disclosure are, for example, a promoter, a terminator, a selectable marker, a leader sequence, a reporter gene, and the like.

In some embodiments, the vector is a therapeutic vector capable of expressing the target gene of the present disclosure (e.g., HGF), such as a plasmid (e.g., a naked plasmid), an adenoviral vector, an adeno-associated viral vector, and a lentiviral vector.

The nucleic acid construct of the present disclosure can be prepared or obtained by a known manner (e.g., by automated DNA synthesis and/or recombinant DNA technology) based on the information of the nucleotide sequence of the present disclosure, and/or can be isolated from a suitable natural source.

In some embodiments, the vector of the present disclosure further comprises a promoter; for example, the promoter is at the 5' end of the 5'UTR of the nucleic acid construct; for example, the promoter is a T7 promoter, a T7 lac promoter, a Tac promoter, a Lac promoter, a Trp promoter.

### Host Cell

The present disclosure also provides a host cell comprising any one of the aforementioned nucleic acid constructs, RNAs, or polynucleotides. In some embodiments, the cell is capable of expressing one or more polypeptides encoded by the nucleic acid construct, RNA, or polynucleotide of the present disclosure. In some embodiments, the host cell is a bacterial cell, a fungal cell, or a mammalian cell.

Bacterial cells include, for example, cells of Gram-negative bacterial strains (such as *Escherichia coli* strains, *Proteus* strains, and *Pseudomonas* strains) and Gram-positive bacterial strains (such as *Bacillus* strains, *Streptomyces* strains, *Staphylococcus* strains, and *Lactococcus* strains).

Fungal cells include, for example, cells of species of *Trichoderma*, *Neurospora*, and *Aspergillus*; or cells of species of *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*), and *Hansenula*.

Mammalian cells include, for example, HEK293 cells, CHO cells, BHK cells, HeLa cells, COS cells, and the like.

However, amphibian cells, insect cells, plant cells, and any other cells used for expressing heterologous proteins in the art can also be used in the present disclosure.

### Production or Preparation Method

The present disclosure provides a method for preparing the nucleic acid construct, RNA, or polynucleotide of the present disclosure, and a method for preparing a polypeptide encoded thereby.

Methods and reagents used for preparing the nucleic acid construct, RNA, or polynucleotide, and the polypeptide encoded thereby, such as specifically suitable vectors, transformation or transfection methods, selectable markers, methods for inducing protein expression, culture conditions, and the like, are known in the art. Similarly, protein isolation and purification techniques suitable for use in the methods for producing the encoded polypeptide of the present disclosure are well known to those skilled in the art.

In some embodiments, the method for preparing the nucleic acid construct or polynucleotide comprises culturing the aforementioned host cell, and recovering the produced nucleic acid construct or polynucleotide from the culture. The nucleic acid construct or polynucleotide of the present disclosure, and the polypeptide encoded thereby can also be obtained by other production methods known in the art, such as chemical synthesis, including solid-phase or liquid-phase synthesis.

In some embodiments, the method for preparing an RNA molecule comprises: preparing a nucleic acid construct or a vector, and then performing reverse transcription using the nucleic acid construct or vector to obtain an RNA molecule. In some specific embodiments, the method further comprises adding a 5'Cap to the 5' end of the RNA molecule.

In some embodiments, the RNA may further comprise one or more modifications (including chemical modifications), such as a backbone modification, a glycosyl modification, a base modification, and/or a lipid modification. In some embodiments, the RNA or polynucleotide is uniformly modified into a specific modification (e.g., completely modified throughout the sequence). For example, the RNA can be uniformly modified with pseudouridine (e.g., N1-methylpseudouridine) such that every U in the sequence is pseudouridine (e.g., N1-methylpseudouridine).

### Method of Treating Diseases and Pharmaceutical Use

The present disclosure provides a pharmaceutical use of the aforementioned lipid nanoparticle, lyophilized composition, lyophilized preparation, reconstituted solution, spray preparation, or pharmaceutical composition in the preparation of a medicament for preventing, treating, or alleviating the aforementioned diseases or symptoms.

The present disclosure provides a use and a method of the aforementioned lipid nanoparticle, lyophilized composition, lyophilized preparation, reconstituted solution, spray preparation, or pharmaceutical composition in preventing, treating, or alleviating a disease or symptom.

The present disclosure provides a method for preventing, treating, or alleviating a disease or symptom comprising administering to a patient or a subject a prophylactically and/or therapeutically effective amount of the aforementioned lipid nanoparticle, lyophilized composition, lyophilized preparation, reconstituted solution, spray preparation, or pharmaceutical composition.

In some embodiments, a method comprising administering an effective amount of the aforementioned lipid nanoparticle, lyophilized composition, lyophilized preparation, reconstituted solution, spray preparation, or pharmaceutical composition to a subject in need thereof is used for preventing, treating, or alleviating a disease or symptom.

In some embodiments, the disease or symptom is selected from ischemic disease, metabolic syndrome, diabetes and complications thereof, restenosis, and nerve injury;
preferably, the ischemic disease is selected from coronary artery disease (CAD), peripheral artery disease (PAD), myocardial infarction, limb ischemia, thromboangiitis obliterans (TAO), and diabetic artery obliterans (DAO); more preferably, the limb ischemia is Lower limb ischemia, and most preferably, the limb ischemia is critical limb ischemia (CLI);
preferably, the diabetes and complications thereof are selected from diabetic peripheral neuropathy, diabetic foot ulcer (DFU), and diabetic artery obliterans (DAO);
preferably, the restenosis is selected from post-surgical restenosis and post-perfusion restenosis;
preferably, the nerve injury is selected from neurodegenerative disease, traumatic nerve injury, and peripheral neuropathy, more preferably, the neurodegenerative disease is selected from amyotrophic lateral sclerosis (ALS), Parkinson's disease, and dementia, and the peripheral neuropathy is diabetic peripheral neuropathy.

The pharmaceutical composition of the present disclosure can be used for treating a patient in need of such treatment by means of parenteral administration. The parenteral administration route can be selected from subcutaneous injection, intramuscular injection, and intravenous injection.

### DESCRIPTION OF DRAWINGS

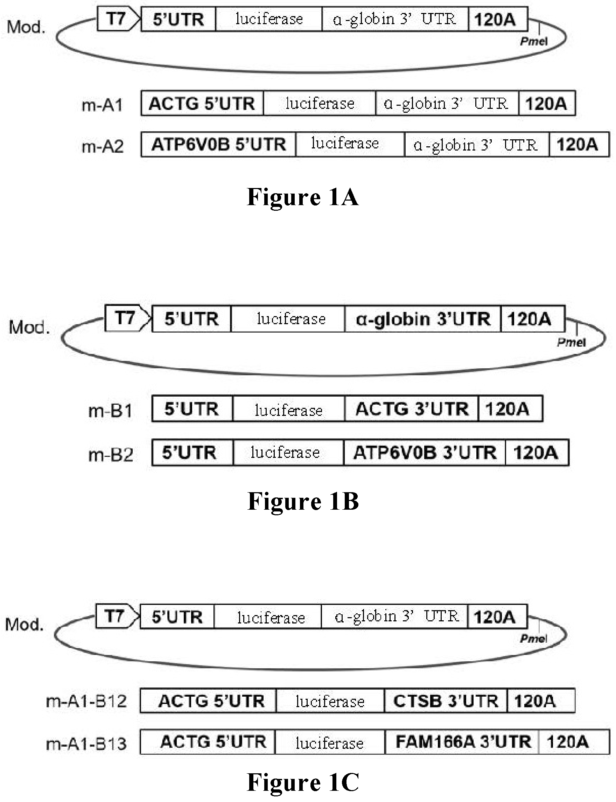

FIG. 1A to FIG. 1C are schematic diagrams of vector construction. FIG. 1A is a schematic diagram of constructing a 5'UTR element screening vector, the control is an mRNA sequence containing 5'UTR and 3'UTR elements from Moderna, namely 5'UTR-Fluc-α globin 3'UTR-120A (abbreviated as Mod.), its 5'UTR is an artificial nucleic acid sequence, and its 3'UTR is derived from human α-globin mRNA. When the 5'UTR screening vector is constructed, the 5'UTR region is replaced through suitable restriction enzyme sites. PmeI is the linearization restriction site. FIG. 1B is a schematic diagram of constructing a 3'UTR element screening vector, the control is Mod. When the 3'UTR screening vector is constructed, the 3'UTR region is replaced through suitable restriction enzyme sites. FIG. 1C is a schematic diagram of constructing a 5' and 3'UTR element combination screening vector, the control is Mod. When the 5'UTR and 3'UTR element combination screening vector is constructed, the 5' and 3'UTR regions were replaced through suitable restriction enzyme sites, or whole gene synthesis is used.

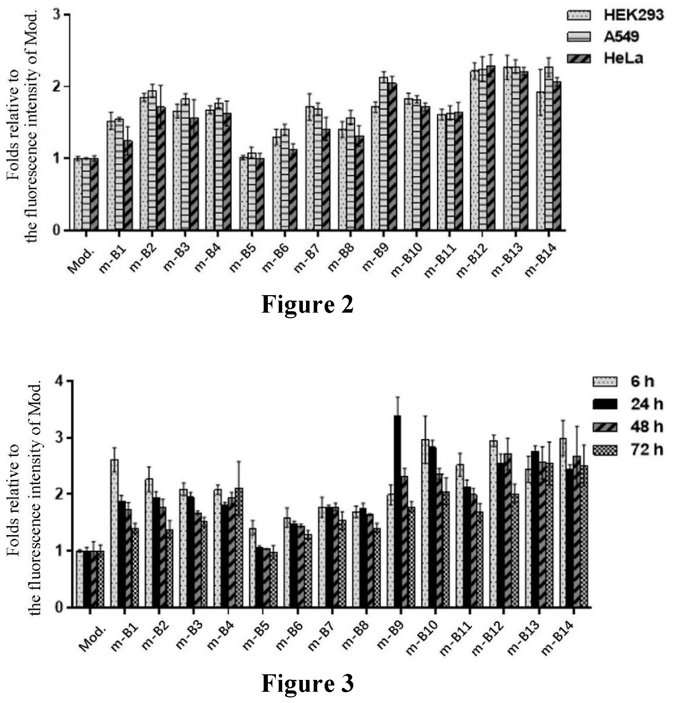

FIG. 2 shows the evaluation results of the effects of different 3'UTR elements of the present disclosure in different cell lines. mRNAs containing different 3'UTR elements were transfected into HEK293, HeLa, and A549 cells. At 24 hours after transfection, luciferase expression was detected to evaluated the effect of the 3'UTR sequence on the protein expression level. Mod. was used as a control, and the expression level of Mod. was set to 1. The results showed that the effects of 3'UTR elements on protein expression levels were consistent across different cell lines.
FIG. 3 shows the effect results of different 3'UTR elements of the present disclosure on mRNA expression efficiency. mRNAs containing different 3'UTR elements were transduced into HEK293 cells via lipofection, the expression level of luciferase was detected at 6 h, 24 h, 48 h, and 72 h after transfection. Mod. was used as a control, and its expression level was set to 1. The results showed that the 3'UTR elements numbered B9, B10, B12, B13, and B14 significantly increased the expression level of the protein.

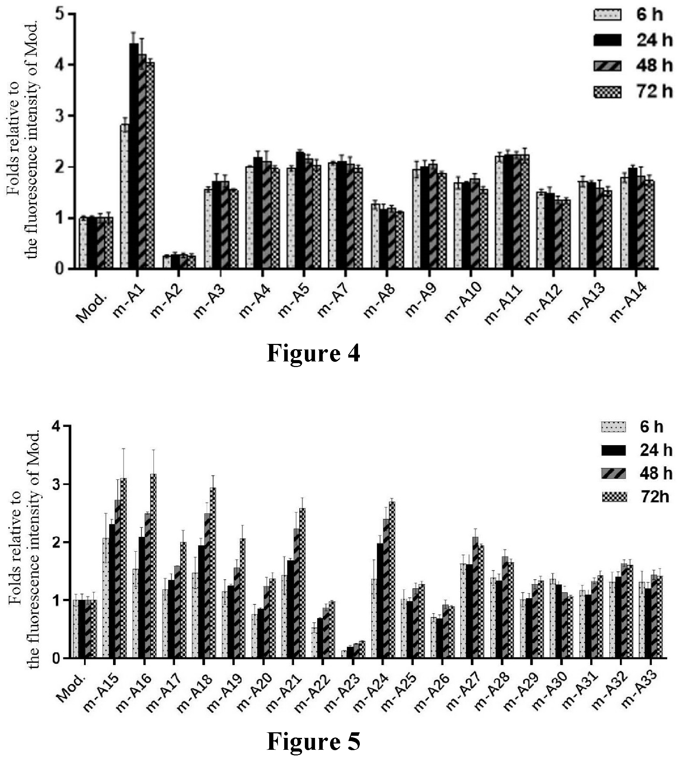

FIG. 4 shows the effect results of different 5'UTR elements of the present disclosure on mRNA expression efficiency. mRNAs containing different 5'UTR elements were transduced into HEK293 cells via lipofection, the expression level of luciferase was detected at 6 h, 24 h, 48 h, and 72 h after transfection. Mod. was used as a control, and its expression level was set to 1. The results showed that the 5'UTR elements numbered A1, A3 to A7, and A9 to A14 significantly increased the expression level of the protein.
FIG. 5 shows the effect results of different 5'UTR elements of the present disclosure on mRNA expression efficiency. mRNAs containing different 5'UTR elements were transduced into HEK293 cells via lipofection, the expression level of luciferase was detected at 6 h, 24 h, 48 h, and 72 h after transfection. Mod. was used as a control, and its expression level was set to 1. The results showed that the 5'UTR elements numbered A15, A16, A18-A19, A21, A24, A27, A28, and A30 to A33 significantly increased the expression level of the protein.

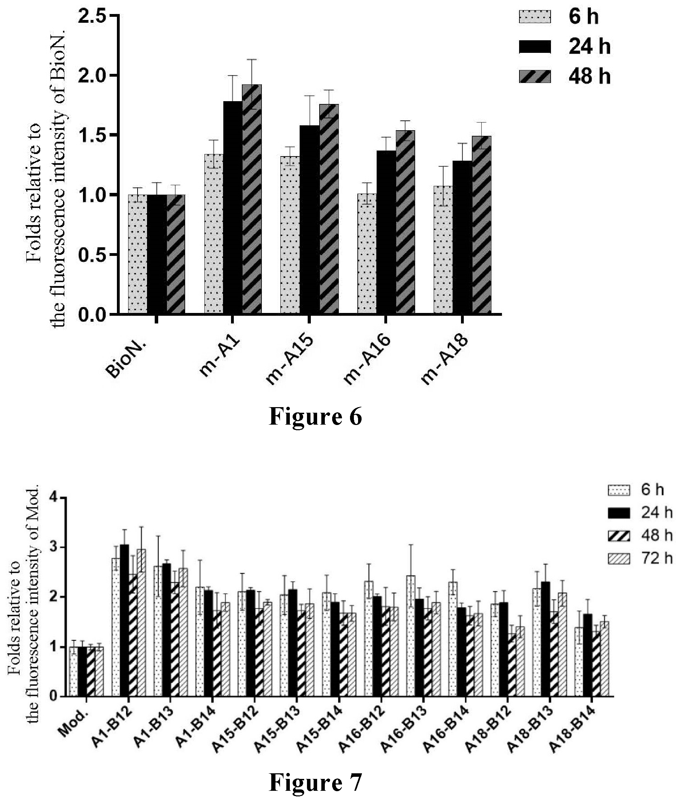

FIG. 6 shows the effect results of different 5'UTR elements of the present disclosure on mRNA expression efficiency. mRNAs containing different 5'UTR elements were transduced into HEK293 cells via lipofection, the expression level of luciferase was detected at 6 h, 24 h, and 48 h after transfection. BioN. was used as a control, and its expression level was set to 1. The results showed that the 5'UTR elements numbered A1, A15, A16, and A18 significantly increased the expression level of the protein compared to the control nucleic acid molecule.
FIG. 7 shows the effect results of UTR element combinations of the present disclosure on mRNA expression efficiency. mRNAs containing different 5'UTR and 3'UTR elements were transduced into HEK293 cells via lipofection, the expression level of luciferase was detected at 6 h, 24 h, 48 h, and 72 h after transfection. Mod. was used as a control, and its expression level was set to 1. The results showed that the combinations of the 5'UTR elements numbered A1, A15, A16, and A18 with the 3'UTR elements numbered B12, B13, and B14 significantly increased the expression level of the protein.

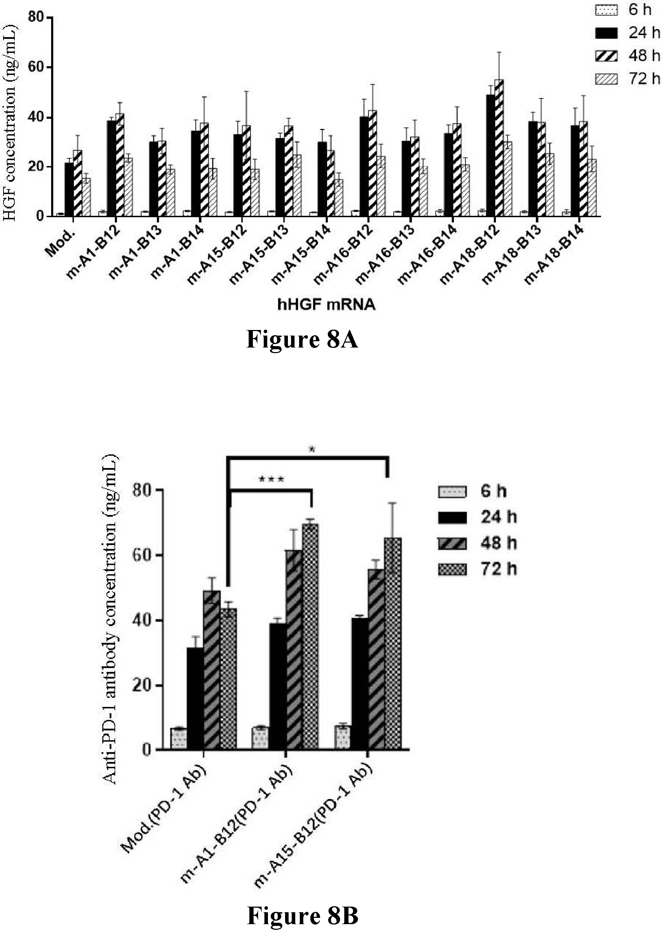

FIG. 8A to FIG. 8B show the effect results of UTR element combinations of the present disclosure on the expression efficiency of different target proteins. FIG. 8A shows the regulation of hHGF expression levels by mRNAs containing the 5'UTR and 3'UTR elements screened of the present disclosure in HEK293 cells. The results showed that compared to the control Mod., the combinations of the 5'UTR elements numbered A1, A15, A16, and A18 with the 3'UTR elements numbered B12, B13, and B14 significantly increased the expression level of hHGF. FIG. 8B shows the regulation of anti-PD-1 antibody expression levels by mRNAs containing the 5'UTR and 3'UTR elements screened of the present disclosure in HEK293 cells. The results showed that compared to the control Mod., the UTR combinations of A1-B12 and A15-B12 significantly increased the expression level of the anti-PD-1 antibody.

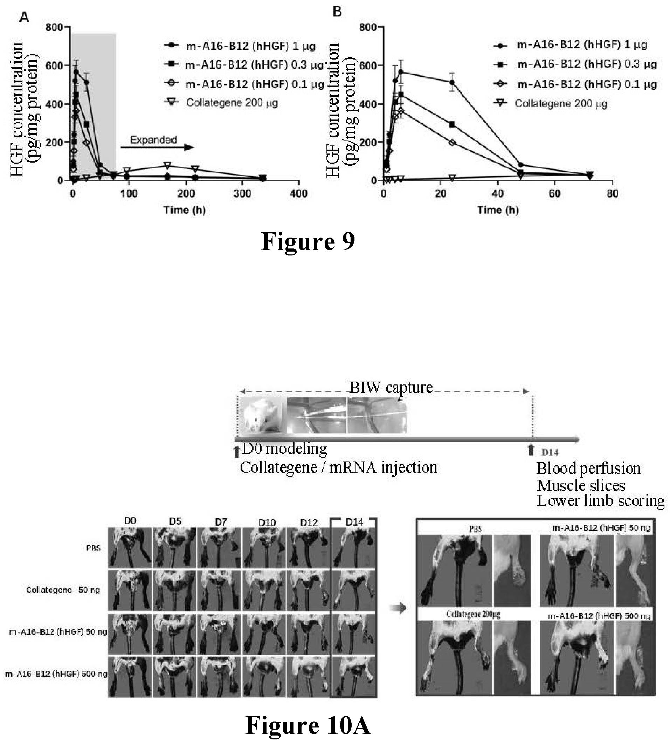

FIG. 9 is a schematic diagram of the experimental results showing the time course of hHGF protein expression levels in mouse muscle tissues for m-A16-B12 (hHGF) of the present disclosure and the control plasmid. The results showed that both the injection of m-A16-B12 (hHGF) and the Collategene plasmid effectively expressed the hHGF protein. The hHGF could be expressed at 1 h after intramuscular injection of m-A16-B12 (hHGF); at 6 h after injection, an expression peak of hHGF appeared in a dose-dependent manner; a low dose of m-A16-B12 (hHGF) could reach a level comparable to the AUCinf (hr*pg/mg protein) of Collategene.

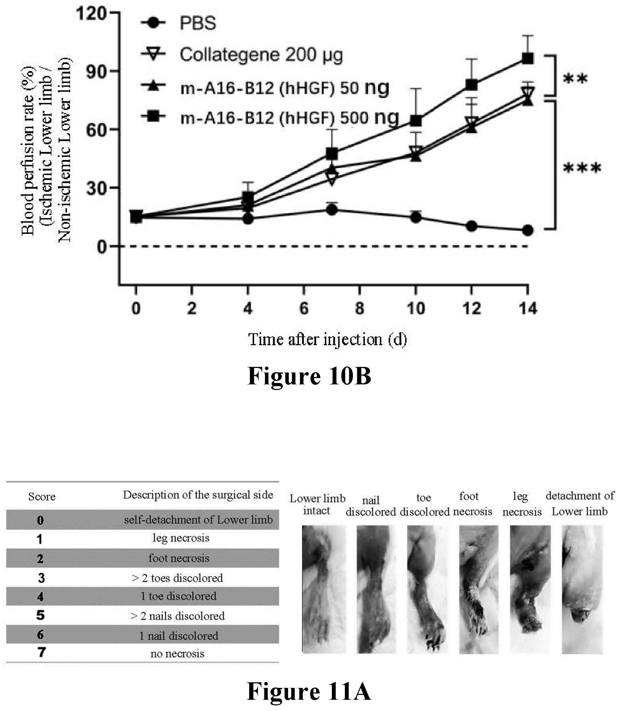

FIG. 10A to FIG. 10B show the therapeutic effect results of m-A16-B12 (hHGF) of the present disclosure and the control plasmid on a hindlimb ischemia mouse model. FIG. 10A is a schematic diagram of the study protocol and experimental result photographs showing the effect of m-A16-B12 (hHGF) and the Collategene plasmid on blood perfusion in the hindlimb ischemia mouse model. FIG. 10B shows the statistical results of the effect of m-A16-B12 (hHGF) and the Collategene plasmid on the blood perfusion ratio in the hindlimb ischemia mouse model. The results showed that under the treatment with 50 ng/mouse and 500 ng/mouse of m-A16-B12 (hHGF) and 200 ng/mouse of Collategene naked plasmid, the blood perfusion ratio of the ischemic hindlimb of each mouse was significantly improved compared with the control group; the blood perfusion ratio of the ischemic hindlimb gradually recovered over time. The 50 ng/mouse m-A16-B12 (hHGF) group showed a blood flow recovery effect similar to that of the 200 ng/mouse Collategene naked plasmid group; the 500 ng/mouse m-A16-B12 (hHGF) group was significantly superior to the 200 ng/mouse Collategene naked plasmid group in blood flow recovery effect, achieving a recovery of more than 90% in blood perfusion.

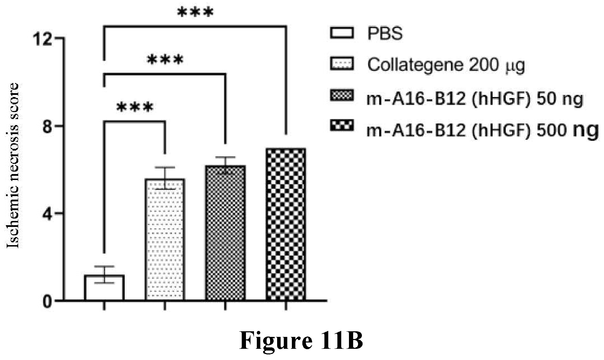

FIG. 11A to FIG. 11B show the therapeutic effect results of m-A16-B12 (hHGF) of the present disclosure and the control plasmid on the hindlimb ischemia mouse model. FIG. 11A shows the scoring criteria for different degrees of hindlimb necrosis in the hindlimb ischemia mouse model and representative photographs. FIG. 11B shows the statistical results of the effect of m-A16-B12 (hHGF) and the Collategene plasmid on hindlimb necrosis in the hindlimb ischemia mouse model. The experimental results showed that in the treatment groups injected with m-A16-B12 (hHGF) and Collategene naked plasmid, the hindlimbs of all mice maintained good integrity, and no hindlimb necrosis occurred.

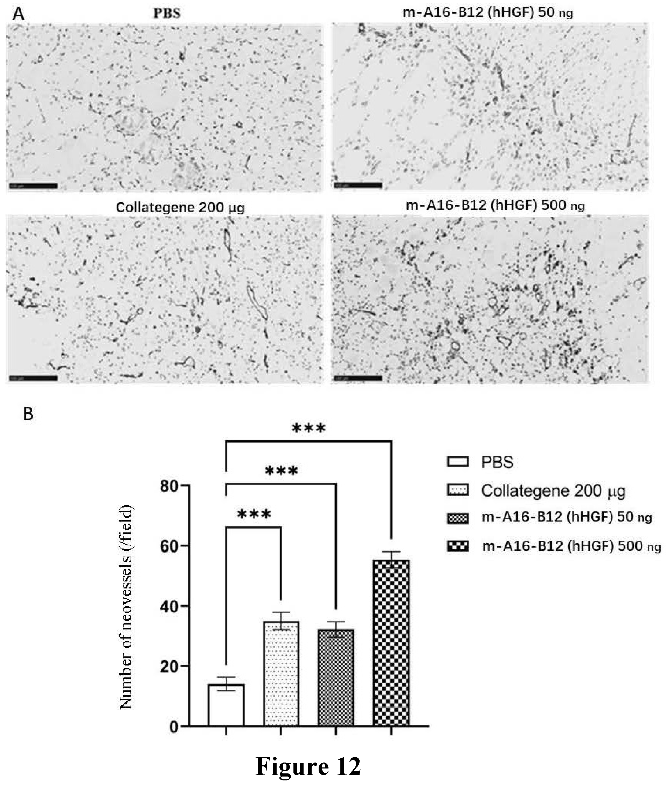

FIG. 12 shows representative photographs and statistical results of the effect of mA16-B12 (hHGF) of the present disclosure and the control plasmid on angiogenesis in the hindlimb ischemia mouse model. The results showed that under the treatment with 50 ng/mouse and 500 ng/mouse of m-A16-B12 (hHGF) and 200 ng/mouse of the Collategene naked plasmid, angiogenesis in the ischemic hindlimb muscles was significantly promoted.

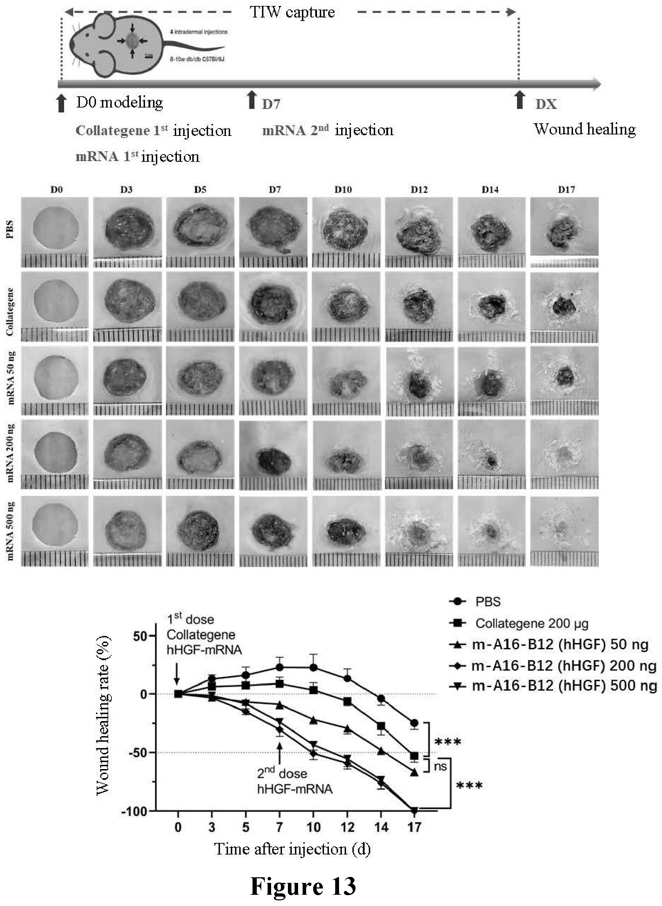

FIG. 13 shows the treatment protocol, representative photographs, and statistical results of the effect of m-A16-B12 (hHGF) of the present disclosure and the control plasmid on wounds in a full-thickness skin injury Db/Db mouse model. The results showed that under the treatment with 50 ng/mouse, 200 ng/mouse, and 500 ng/mouse of m-A16-B12 (hHGF) and 200 µg/mouse of the Collategene naked plasmid, the wounds of the mice were all well healed compared with the control group. The promotion of wound healing by m-A16-B12 (hHGF) was significantly superior to that of 200 µg/mouse of the Collategene naked plasmid.

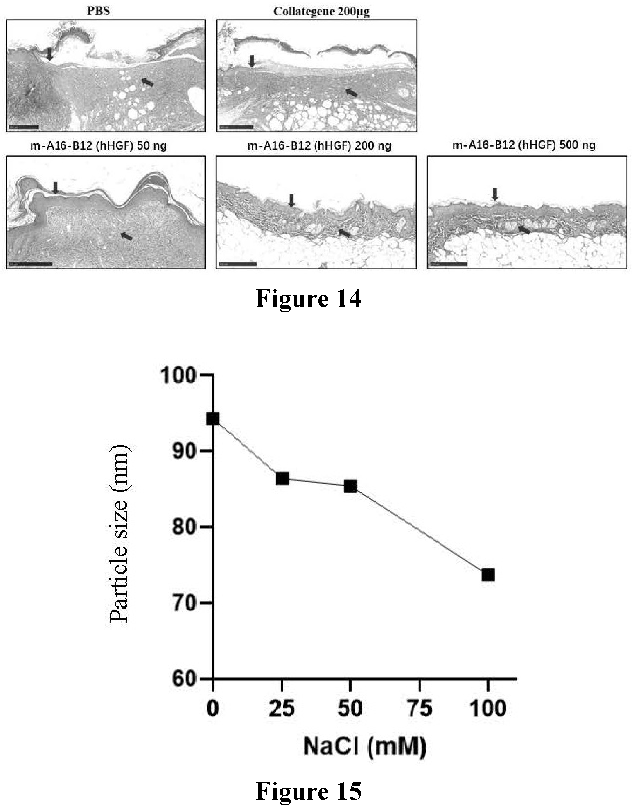

FIG. 14 shows representative photographs of the effect of m-A16-B12 (hHGF) of the present disclosure and the control plasmid on tissue remodeling in the full-thickness skin injury Db/Db mouse model. The results showed that under the treatment with mA16-B12 (hHGF), full epithelial coverage was achieved on the wound epithelium of mice in all groups, and tissue remodeling was completed. However, epithelial re-coverage was not completed in the control group and the 200 µg/mouse Collategene naked plasmid group, abnormal proliferation of collagen and structural disorder occurred at the wounds.
FIG. 15: Particle sizes of preparations 1-4 in different buffers before freeze-drying.

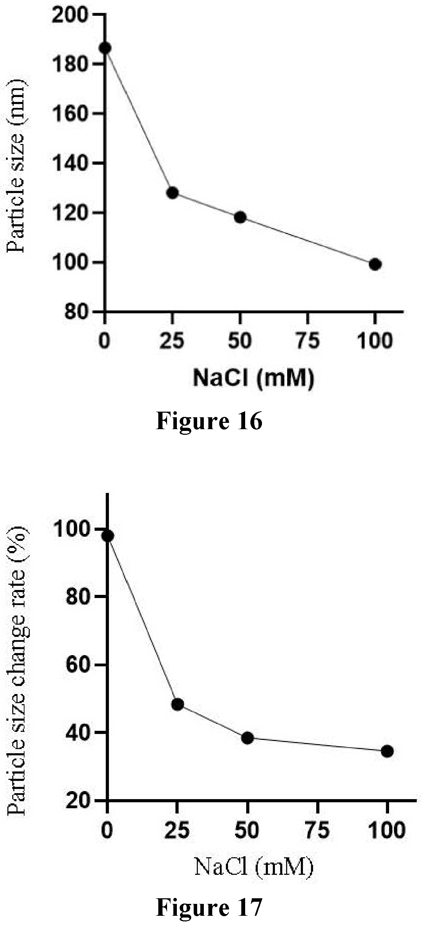

FIG. 16: Particle sizes of preparations 1-4 after freeze-drying and reconstitution.
FIG. 17: Particle size change rates of preparations 1-4 before and after freeze-drying.

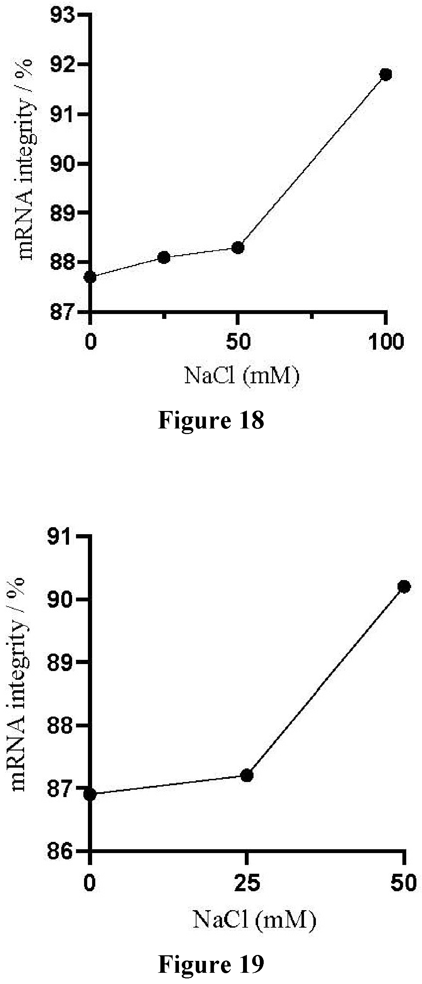

FIG. 18: mRNA integrity of preparations 1-4 after taking out from a freeze-dryer.
FIG. 19: mRNA integrity of preparations 7-9 after taking out from a freeze-dryer.

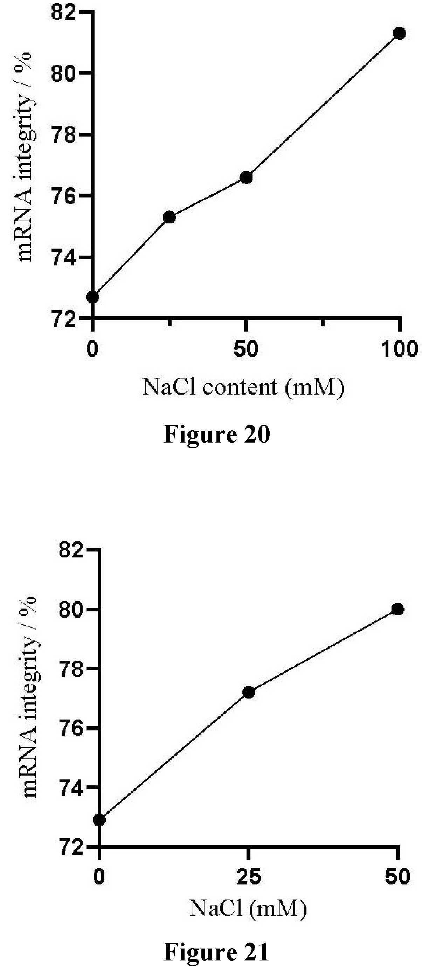

FIG. 20: mRNA integrity of preparations 1-4 under a high-temperature storage condition of 37°C.
FIG. 21: mRNA integrity of preparations 7-9 under a high-temperature storage condition of 37°C.

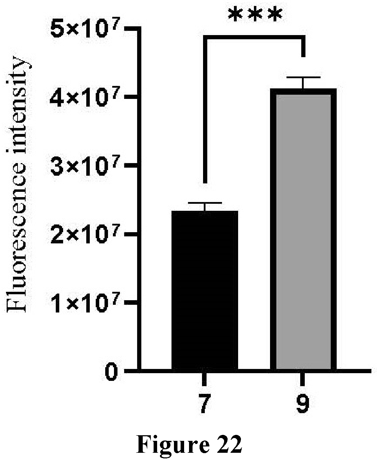

FIG. 22: Fluorescence intensity values of cell transfection expression amounts of freeze-dried products of preparations 7 and 9.

### DETAILED DESCRIPTION

### I. Terms

To make the present disclosure easier to understand, certain technical and scientific terms are specifically defined below. Unless specifically defined otherwise in the present disclosure, all other technical and scientific terms used in the present disclosure have the meanings commonly understood by those of ordinary skill in the art to which the present disclosure belongs. The three-letter codes and one-letter codes for amino acids used in the present disclosure are as described in J. Biol. Chem, 243, p3558 (1968).

"Sequence" should generally be understood to include both the relevant amino acid sequence and the nucleic acid sequence or nucleotide sequence encoding the sequence, unless further restrictive interpretation is required by the present disclosure.

"Homology" or "identity" refers to the sequence similarity between two polynucleotide sequences or between two polypeptides. When positions in two aligned sequences are occupied by the same base or amino acid monomer subunit, for example, if a position in each of two DNA molecules is occupied by adenine, then the molecules are homologous at that position. The percentage of homology between two sequences is a function of the number of matching or homologous positions shared by the two sequences divided by the number of positions compared × 100%. For example, in an optimal sequence alignment, if 6 out of 10 positions in two sequences are matched or homologous, then the two sequences are 60% homologous. Generally, the comparison is performed when the two sequences are aligned to yield the maximum percentage of homology.

"About" or "approximately" means that a numerical value is within an acceptable error range for the specific value as determined by one of ordinary skill in the art, which depends in part on how the value is measured or determined (*i.e.*, the limitations of the measurement system). For example, "about" can mean within 1 or more than 1 standard deviation. Alternatively, "about" or "comprising essentially" can mean a range of up to 20%, such as varying between 1% and 15%, between 1% and 10%, between 1% and 5%, between 0.5% and 5%, or between 0.5% and 1%. In the present disclosure, each instance where a numerical value or range is preceded by the term "about" also includes the embodiment of the given number. Unless otherwise specified, when a specific value appears in the present disclosure and claims, the meaning of "about" or "comprising essentially" should be assumed to be within an acceptable error range for that specific value.

"Buffer" refers to a buffer that tolerates changes in pH through the action of its acid-base conjugate components. Examples of buffers that control the pH within an appropriate range include tris(hydroxymethyl)aminomethane (Tris), acetate, succinate, gluconate, histidine, oxalate, lactate, phosphate, citrate, tartrate, fumarate, glycylglycine, and other organic acid buffers.

A "histidine buffer" is a buffer comprising a histidine ion. Examples of the histidine buffer include histidine-hydrochloride, histidine-acetate, histidine-phosphate, histidine-sulfate buffers, and the like, preferably histidine-hydrochloride buffer or histidine-acetate buffer; the histidine-acetate buffer is prepared from histidine and acetic acid; the histidine-hydrochloride buffer is prepared from histidine and histidine hydrochloride, or from histidine and hydrochloric acid.

A "phosphate buffer" is a buffer comprising a phosphate ion. Examples of the phosphate buffer include disodium hydrogen phosphate-sodium dihydrogen phosphate, disodium hydrogen phosphate-potassium dihydrogen phosphate, disodium hydrogen phosphate-citric acid, and the like. Preferably, the phosphate buffer is disodium hydrogen phosphate-sodium dihydrogen phosphate.

"Pharmaceutical composition" means a mixture comprising one or more of the antibodies described herein and other chemical components, the other components are, for example, physiologically/pharmaceutically acceptable carriers and excipients. The purpose of the pharmaceutical composition is to maintain the stability of the active ingredients, facilitate administration to an organism, and facilitate the absorption of the active ingredients to exert biological activities.

In the present disclosure, "pharmaceutical composition" and "preparation" are not mutually exclusive.

In the solution form of the pharmaceutical composition according to the present disclosure, unless otherwise specified, the solvent is water.

"Lyophilized preparation" means a preparation or pharmaceutical composition obtained after a pharmaceutical composition or a preparation in a liquid or solution form is subjected to a vacuum freeze-drying step.

"Administration", "administering", and "treatment", when applied to an animal, a human, an experimental subject, a cell, a tissue, an organ, or a biological fluid, refer to contact of an exogenous drug, therapeutic agent, diagnostic agent, or composition with the animal, human, subject, cell, tissue, organ, or biological fluid. "Administration", "administering", and "treatment" can refer to, for example, therapeutic, pharmacokinetic, diagnostic, research, and experimental methods. Treatment of a cell includes contact of an agent with the cell, as well as contact of an agent with a fluid, wherein the fluid is in contact with the cell. "Administration", "administering", and "treatment" also mean *in vitro* and *ex vivo* treatment of, for example, a cell via an agent, a diagnostic, a binding composition, or via another cell. "Treatment", when applied to a human, veterinary medicine, or a research subject, refers to therapeutic treatment, prophylactic or preventive measures, research and diagnostic applications.

"Treating" or "treatment" means administering a systemic or topical therapeutic agent to a subject, for example, the therapeutic agent comprises any one of the antibodies or pharmaceutical compositions thereof of the present disclosure, the subject already has, is suspected of having, or is predisposed to having one or more proliferative diseases or symptoms thereof, wherein the therapeutic agent is known to have a therapeutic effect on these symptoms. Usually, the therapeutic agent is administered to the treated subject or population in an amount effective to alleviate one or more disease symptoms, this can be by inducing regression of such symptoms or inhibiting the progression of such symptoms to any clinically measurable degree. The amount of a therapeutic agent effective to alleviate any specific disease symptom (also referred to as a "therapeutically effective amount") can vary depending on a variety of factors, for example, the disease state, age, and weight of the subject, and the ability of the drug to elicit a desired therapeutic effect in the subject. Whether a disease symptom has been alleviated can be evaluated by any clinical test method commonly used by a physician or other professional healthcare provider to evaluate the severity or progression of the symptom. Although embodiments of the present disclosure (e.g., a method of treatment or product) may not be effective in alleviating the target disease symptom in a certain subject, they should alleviate the target disease symptom in a statistically significant number of subjects as determined by any statistical test method known in the art, such as Student's t-test, Chi-square test, Mann-Whitney U test, Kruskal-Wallis test (H test), Jonckheere-Terpstra test, and Wilcoxon test.

"Effective amount" includes an amount sufficient to ameliorate or prevent a symptom or condition of a medical condition. An effective amount also means an amount sufficient to allow or facilitate diagnosis. An effective amount for a subject can vary depending on factors such as the condition to be treated, the overall health of the subject, the method and route of administration, the dose, and the severity of side effects. An effective amount can be the maximum dose or dosing regimen that avoids significant side effects or toxic effects. The subject of the present disclosure can be an animal or a human subject.

"Optional" or "optionally" means that the subsequently described event or circumstance can but need not occur, and this description includes instances where the event or circumstance occurs and instances where it does not.

"Subject" or "patient" means a mammal, especially a primate, and in particular a human.

Unless the context clearly requires otherwise, throughout the specification and the claims, words such as "comprise", "have", "include", and the like should be construed in an inclusive sense rather than an exclusive or exhaustive sense; that is, in the sense of "including but not limited to".

The term "lipid phase" refers to a collective term for all lipids in a lipid nanoparticle, this includes, but is not limited to, a cationic lipid, a non-cationic lipid, a phospholipid, cholesterol or its derivative, a conjugated lipid, and the like, which are well known to those skilled in the art and can be used to prepare a lipid nanoparticle.

The term "lipid" refers to a class of organic compounds, which includes but is not limited to an ester of a fatty acid, and is characterized by being insoluble in water but soluble in many organic solvents. They are generally classified into at least three categories: (1) "simple lipids", which include fats and oils as well as waxes; (2) "compound lipids", which include phospholipids and glycolipids; and (3) "derived lipids" such as steroids.

The term "cationic lipid" refers to any one of a number of lipid species carrying a net positive charge at a selected pH, such as a physiological pH (e.g., pH is about 7.0).

The term "anionic lipid" refers to any lipid that carries a negative charge at a physiological pH. These lipids include, but are not limited to, phosphatidylglycerol, cardiolipin, diacylphosphatidylserine, diacylphosphatidic acid, N-dodecanoylphosphatidylethanolamine, N-succinylphosphatidylethanolamine, N-glutarylphosphatidylethanolamine, lysylphosphatidylglycerol, palmitoyloleoylphosphatidylglycerol (POPG), and other anionic modifying groups linked to neutral lipids.

The term "neutral lipid" refers to any one of a number of lipid species that exist in an uncharged or neutral zwitterionic form at a selected pH. At a physiological pH, such lipids include, for example, diacylphosphatidylcholine, diacylphosphatidylethanolamine, ceramide, sphingomyelin, cephalin, cholesterol, cerebroside, and diacylglycerol.

The term "amphiphilic lipid" partially refers to any suitable material in which a hydrophobic portion of a lipid material is oriented toward a hydrophobic phase, while a hydrophilic portion is oriented toward an aqueous phase. Hydrophilic properties are derived from the presence of polar or charged groups such as carbohydrates, phosphate, carboxyl, sulfate, amino, sulfhydryl, nitro, hydroxy, and other similar groups. Hydrophobicity can be imparted by the inclusion of non-polar groups, including but are not limited to long-chain saturated and unsaturated aliphatic hydrocarbon groups and such groups substituted by one or more aromatic, alicyclic, or heterocyclic groups. Examples of amphiphilic compounds include, but are not limited to, phospholipids, aminolipids, and sphingolipids.

The term "non-cationic lipid" refers to any amphiphilic lipid as well as any other neutral lipid or anionic lipid.

The term "conjugated lipid" refers to a bound lipid that inhibits aggregation of lipid particles. The conjugated lipid includes, but is not limited to, PEG-modified phosphatidylethanolamine, PEG-modified phosphatidic acid, PEG-modified ceramide, PEG-modified dialkylamine, PEG-modified diacylglycerol, and PEG-modified dialkylglycerol.

The term "mammal" refers to any mammalian species such as humans, mice, rats, dogs, cats, hamsters, guinea pigs, rabbits, livestock, and the like.

The "average particle size" (Z-average Size) of the present disclosure, for example, "an average particle size of less than 2000 nm", is a light intensity average value calculated from the light intensity contributed by different kinds of particles. The "average particle size" can be measured by a conventional particle size measurement technique well known to those skilled in the art to measure the average particle size of particles. Such techniques include, for example, sedimentation field flow fractionation, photon correlation spectroscopy, light scattering, and the like.

The Polydispersity Index (PDI) in the present disclosure reflects the uniformity of particle sizes and is an important index for particle size characterization.

The terms "incorporate into" and "mix" mean that the order of adding the components is not limited. For example, incorporating A into B can mean adding A to B, or it can mean adding B to A; Mixing A and B can mean adding A to B for mixing, or it can mean adding B to A for mixing.

Numerical values in the present disclosure are instrument measurement values, and there is a certain degree of error. Generally speaking, ± 10% is within a reasonable error range. Certainly, the context where the numerical value is applied shall be taken into account; for example, for the particle size of an active ingredient, the value is an error change after measurement that does not exceed ±10%, which can be ±9%, ±8%, ±7%, ±6%, ± 5%, ±4%, ± 3%, ±2%, or ± 1%, preferably ± 5%.

The compound of Formula I of the present disclosure was prepared with reference to the method in WO2023125738A, and the relevant contents are incorporated herein for illustration.

### The equipment and methods used in the detection process were as follows:

### Total content and encapsulation efficiency:

Under the action of 2% Triton X-100, mRNA-LNPs could release the mRNA encapsulated in the lipids; the mRNA was bound to RiboGreen; it could be read in a fluorescence mode of a microplate reader by selecting an excitation light of 485 nm and an emission light of 535 nm. A fluorescent dye method was used to detect the mRNA contents in the samples before and after release treated with Triton X-100, and the Free RNA and Total RNA contents were measured respectively; the Total RNA content was the total content; the proportion of the difference between Total RNA and Free RNA to Total RNA was deemed as the encapsulation efficiency of the sample.

Total content and encapsulation efficiency detection instrument: Molecular Devices - multifunctional microplate reader.

### dsRNA detection:

A double-antibody sandwich enzyme-linked immunosorbent assay (ELISA) was applied to detect the content of dsRNA in a stock solution of an *in vitro* transcribed RNA product. A microplate was coated with a capture antibody to form a solid-phase antibody; a dsRNA standard and a sample to be tested were added to the solid-phase antibody microplate; then a detection antibody was added; finally, a horseradish peroxidase-labeled enzyme-labeled secondary antibody was added to form a "coating antibody-antigen-enzyme-labeled detection antibody" complex; after washing, a chromogenic solution was added for color development; under the catalysis of the HRP enzyme, the chromogenic solution was converted to blue and finally converted to yellow under the action of an acid; the depth of the color was positively correlated with the amount of dsRNA in the sample.

dsRNA detection instrument: Molecular Devices - multifunctional microplate reader.

### HGF detection:

In this experiment, an expression supernatant obtained 3 days after infection of HEK293 cells with an mRNA drug (liposome form) was collected as a test sample. A double-antibody sandwich ELISA method was adopted: An anti-human HGF antibody was coated on a microplate; human HGF in the sample and standard bound to the antibody immobilized on the plate, and free components were washed away; subsequently, a biotinylated anti-human HGF detection antibody was added for incubation; after washing to remove unbound substances, streptavidin-labeled horseradish peroxidase (Streptavidin-HRP) was added for incubation. After washing to remove unbound reagents, a TMB substrate solution (chromogenic agent) was added. The color of the solution was positively proportional to the bound target protein; a stop solution was added; the absorbance was determined with a microplate reader.

HGF detection instrument: Molecular Devices - multifunctional microplate reader.

### Examples and Test Examples

The present disclosure is further illustrated in detail by the following examples. These examples are for illustrative purposes only and are not intended to limit the scope of the present disclosure.

For experimental methods where specific conditions are not indicated in the examples of the present disclosure, conventional conditions are generally followed; or conditions suggested by raw material or commercial manufacturers are followed. Refer to Sambrook et al., Molecular Cloning: A Laboratory Manual, Cold Spring Harbor Laboratory; Current Protocols in Molecular Biology, edited by Ausubel et al., Greene Publishing Associates, Wiley Interscience, NY. Reagents whose specific sources are not indicated are conventional reagents purchased from the market.

### Example 1. Preparation of mRNA

### 1.1 Screening of 5'UTR and 3'UTR

In order to screen out a 3'-untranslated region element (3'UTR element) and a 5'-untranslated region element (5'UTR element) that could improve protein expression efficiency, UTR sequences in housekeeping gene mRNAs were screened in this example.

First, 301 candidate human housekeeping genes including Abhd16a and the like were identified through a database (https://esbl.nhlbi.nih.gov/helixweb/Database/ NephronRNAseq/Housekeeping_Genes.htmL). Then, the expression levels of the above genes were analyzed and ranked by expression amount through bioinformatics methods, transcript and UTR sequence information of the genes was retrieved. The UTR elements were further screened according to the expression level of the genes and the length of the UTR sequences; multiple candidate UTR sequences were obtained; among them, 33 5'UTRs numbered A1 to A33 and 14 3'UTRs numbered B1 to B14 were screened out. The gene sources and sequence numbers are shown in Table 1 and Table 2.

**Table 1. Novel 3'UTRs obtained by screening**

| 3'UTR No. | Gene source (All are from Homo sapiens) | Corresponding DNA sequence No. | Corresponding mRNA sequence No. |
|---|---|---|---|
| B1 | ACTG1 | SEQ ID NO:1 | SEQ ID NO:58 |
| B2 | ATP6V0B | SEQ ID NO:2 | SEQ ID NO:59 |
| B3 | ATP6V0B | SEQ ID NO:3 | SEQ ID NO:60 |
| B4 | ATP6V0E1 | SEQ ID NO:4 | SEQ ID NO:61 |
| B5 | ATP6V0E1 | SEQ ID NO:5 | SEQ ID NO:62 |
| B6 | CFL1 | SEQ ID NO:6 | SEQ ID NO:63 |
| B7 | CFL1 | SEQ ID NO:7 | SEQ ID NO:64 |
| B8 | CFL1 | SEQ ID NO:8 | SEQ ID NO:65 |
| B9 | COX4I1 | SEQ ID NO:9 | SEQ ID NO:66 |
| B10 | COX4I1 | SEQ ID NO:10 | SEQ ID NO:67 |
| B11 | COX4I1 | SEQ ID NO:11 | SEQ ID NO:68 |
| B12 | CTSB | SEQ ID NO:12 | SEQ ID NO:69 |
| B13 | FAM166A | SEQ ID NO:13 | SEQ ID NO:70 |
| B14 | NDUFB9 | SEQ ID NO:14 | SEQ ID NO:71 |

**Table 2. Novel 5'UTRs obtained by screening**

| 5'UTR No. | Gene source (All are from Homo sapiens) | Corresponding DNA sequence No. | Corresponding mRNA sequence No. |
|---|---|---|---|
| A1 | ACTG1 | SEQ ID NO:15 | SEQ ID NO:72 |
| A2 | ATP6V0B | SEQ ID NO:16 | SEQ ID NO:73 |
| A3 | ATP6V0B | SEQ ID NO:17 | SEQ ID NO:74 |
| A4 | ATP6V0E1 | SEQ ID NO:18 | SEQ ID NO:75 |
| A5 | ATP6V0E1 | SEQ ID NO:19 | SEQ ID NO:76 |
| A6 | CFL1 | SEQ ID NO:20 | SEQ ID NO:77 |
| A7 | CFL1 | SEQ ID NO:21 | SEQ ID NO:78 |
| A8 | CFL1 | SEQ ID NO:22 | SEQ ID NO:79 |
| A9 | COX4I1 | SEQ ID NO:23 | SEQ ID NO:80 |
| A10 | COX4I1 | SEQ ID NO:24 | SEQ ID NO:81 |
| A11 | COX4I1 | SEQ ID NO:25 | SEQ ID NO:82 |
| A12 | CTSB | SEQ ID NO:26 | SEQ ID NO:83 |
| A13 | FAM166A | SEQ ID NO:27 | SEQ ID NO:84 |
| A14 | NDUFB9 | SEQ ID NO:28 | SEQ ID NO:85 |
| A15 | CHCHD10 | SEQ ID NO:29 | SEQ ID NO:86 |
| A16 | CHCHD10 | SEQ ID NO:30 | SEQ ID NO:87 |
| A17 | SLC38A2 | SEQ ID NO:31 | SEQ ID NO:88 |
| A18 | NDUFA11 | SEQ ID NO:32 | SEQ ID NO:89 |
| A19 | NDUFV3 | SEQ ID NO:33 | SEQ ID NO:90 |
| A20 | PRDX5 | SEQ ID NO:34 | SEQ ID NO:91 |
| A21 | GUK1 | SEQ ID NO:35 | SEQ ID NO:92 |
| A22 | GUK1 | SEQ ID NO:36 | SEQ ID NO:93 |
| A23 | GUK1 | SEQ ID NO:37 | SEQ ID NO:94 |
| A24 | IAH1 | SEQ ID NO:38 | SEQ ID NO:95 |
| A25 | ABHD16A | SEQ ID NO:39 | SEQ ID NO:96 |
| A26 | SLC25A39 | SEQ ID NO:40 | SEQ ID NO:97 |
| A27 | ATPIF1 | SEQ ID NO:41 | SEQ ID NO:98 |
| A28 | ANAPC11 | SEQ ID NO:42 | SEQ ID NO:99 |
| A29 | ANAPC11 | SEQ ID NO:43 | SEQ ID NO:100 |
| A30 | CCDC12 | SEQ ID NO:44 | SEQ ID NO:101 |
| A31 | MRPL14 | SEQ ID NO:45 | SEQ ID NO:102 |
| A32 | APOA1BP | SEQ ID NO:46 | SEQ ID NO:103 |
| A33 | APOA1BP | SEQ ID NO:47 | SEQ ID NO:104 |

### 1.2 Preparation of mRNA

The UTRs obtained by screening in 1.1 were constructed into a DNA vector for *in vitro* transcription to obtain an mRNA stably expressing the 5'UTR and 3'UTR elements. The vector contained a T7 promoter, a sequence encoding Firefly Luciferase (Fluc) as an open reading frame (ORF) (SEQ ID NO: 126), and a polyadenylation (polyA) sequence, the polyA sequence was followed by a restriction site used for linearizing the vector. The polyA was selected from A120 (*i.e.*, 120 consecutive adenylates) and A30L70 (SEQ ID NO: 52). The 5'UTR and 3'UTR elements were respectively constructed to the 5' end and 3' end of the open reading frame ORF (Fluc) through appropriate restriction enzyme sites. Examples of the constructed vectors are shown in FIG. 1A to FIG. 1C, the combinations of 5'UTR and 3'UTR are shown in Table 3.

The control vectors used in this example included Mod.-120A with a polyA of A120 (SEQ ID NO: 53), Mod.-A30L70 with a polyA of A30L70 (SEQ ID NO: 54), and BioN.-A30L70 (SEQ ID NO: 55), with the target genes were all Fluc, which were constructed through whole gene synthesis. Different UTR elements were respectively constructed into the above vectors by means of restriction enzyme digestion. Among them, the construction vector for vectors V-B1 to V-B14 was Mod.-120A, the restriction enzyme sites selected were *Age*I and *Sac*II, their polyA were all A120, the control used was Mod.-120A (SEQ ID NO: 53). The construction vector for vectors V-A1 to V-A14 was Mod.-120A (SEQ ID NO: 53), the restriction enzyme sites selected were *Bam*HI and *Nhe*I, their polyA were all A120, the control used was also Mod.-120A (SEQ ID NO: 53). The construction vector for V-A15 to V-A33 was Mod.-A30L70 (SEQ ID NO: 54), the restriction enzyme sites selected were *Bam*HI and *Nhe*I, their polyA were all A30L70, the control used was Mod.-A30L70 (SEQ ID NO: 54). The construction vector for vectors V-A1-B12, V-A1-B13, and V-A1-B14 was Mod.-A30L70 (SEQ ID NO: 54), the restriction enzyme sites selected were *Bam*HI and *Sac*II, their polyA were all A30L70, the controls used were Mod.-A30L70 (SEQ ID NO: 54) and BioN.-A30L70 (SEQ ID NO: 55). The inserted UTR element gene fragments were all whole-gene synthesized (Suzhou Genewiz Biological Technology Co., Ltd.). The fragments were constructed into the corresponding vectors through restriction enzyme digestion and ligation. After the construction of the vectors was completed, restriction enzyme digestion and sequencing identification were required. When the identification was correct, they were used for the next experiment.

**Table 3. Combinations of 5'UTR and 3'UTR in vectors**

| Vector No. (Corresponding mRNA No.) | 5'UTR | 3'UTR |
|---|---|---|
| Mod. | Mod. 5'UTR (SEQ ID NO:48) | α-globin 3'UTR (SEQ ID NO:49) |
| V-B1 (m-B1) | | B1 (SEQ ID NO:1) |
| V-B2 (m-B2) | | B2 (SEQ ID NO:2) |
| V-B3 (m-B3) | | B3 (SEQ ID NO:3) |
| V-B4 (m-B4) | | B4 (SEQ ID NO:4) |
| V-B5 (m-B5) | | B5 (SEQ ID NO:5) |
| V-B6 (m-B6) | | B6 (SEQ ID NO:6) |
| V-B7 (m-B7) | | B7 (SEQ ID NO:7) |
| V-B8 (m-B8) | | B8 (SEQ ID NO:8) |
| V-B9 (m-B9) | | B9 (SEQ ID NO:9) |
| V-B10 (m-B10) | | B10 (SEQ ID NO:10) |
| V-B11 (m-B11) | | B11 (SEQ ID NO:11) |
| V-B12 (m-B12) | | B12 (SEQ ID NO:12) |
| V-B13 (m-B13) | | B13 (SEQ ID NO:13) |
| V-B14 (m-B14) | | B14 (SEQ ID NO:14) |
| V-A1 (m-A1) | A1 (SEQ ID NO:15) | α-globin 3'UTR (SEQ ID NO:49 |
| V-A2 (m-A2) | A2 (SEQ ID NO:16) | |
| V-A3 (m-A3) | A3 (SEQ ID NO:17) | |
| V-A4 (m-A4) | A4 (SEQ ID NO:18) | |
| V-A5 (m-A5) | A5 (SEQ ID NO:19) | |
| V-A6 (m-A6) | A6 (SEQ ID NO:20) | |
| V-A7 (m-A7) | A7 (SEQ ID NO:21) | |
| V-A8 (m-A8) | A8 (SEQ ID NO:22) | |
| V-A9 (m-A9) | A9 (SEQ ID NO:23) | |
| V-A10 (m-A10) | A10 (SEQ ID NO:24) | |
| V-A11 (m-A11) | A11 (SEQ ID NO:25) | |
| V-A12 (m-A12) | A12 (SEQ ID NO:26) | |
| V-A13 (m-A13) | A13 (SEQ ID NO:27) | |
| V-A14 (m-A14) | A14 (SEQ ID NO:28) | |
| V-A15 (m-A15) | A15 (SEQ ID NO:29) | |
| V-A16 (m-A16) | A16 (SEQ ID NO:30) | |
| V-A17 (m-A17) | A17 (SEQ ID NO:31) | |
| V-A18 (m-A18) | A18 (SEQ ID NO:32) | |
| V-A19 (m-A19) | A19 (SEQ ID NO:33) | |
| V-A20 (m-A20) | A20 (SEQ ID NO:34) | |
| V-A21 (m-A21) | A21 (SEQ ID NO:35) | |
| V-A22 (m-A22) | A22 (SEQ ID NO:36) | |
| V-A23 (m-A23) | A23 (SEQ ID NO:37) | |
| V-A24 (m-A24) | A24 (SEQ ID NO:38) | |
| V-A25 (m-A25) | A25 (SEQ ID NO:39) | |
| V-A26 (m-A26) | A26 (SEQ ID NO:40) | |
| V-A27 (m-A27) | A27 (SEQ ID NO:41) | |
| V-A28 (m-A28) | A28 (SEQ ID NO:42) | |
| V-A29 (m-A29) | A29 (SEQ ID NO:43) | |
| V-A30 (m-A30) | A30 (SEQ ID NO:44) | |
| V-A31 (m-A31) | A31 (SEQ ID NO:45) | |
| V-A32 (m-A32) | A32 (SEQ ID NO:46) | |
| V-A33 (m-A33) | A33 (SEQ ID NO:47) | |
| V-A1-B12 (m-A1-B12) | A1 (SEQ ID NO:15) | B12 (SEQ ID NO:12) |
| V-A1-B13 (m-A1-B13) | A1 (SEQ ID NO:15) | B13 (SEQ ID NO:13) |
| V-A1-B14 (m-A1-B14) | A1 (SEQ ID NO:15) | B14 (SEQ ID NO:14) |
| BioN. | α-1-globin 5'UTR (SEQ ID NO:50) | haplogroup U8b1b1 mitochondrion (SEQ ID NO:51) |

The vectors (*i.e.*, DNA templates) in Table 3 were digested by a restriction endonuclease to linearize the DNA templates, then T7-RNA polymerase was used for *in vitro* transcription. For *in vitro* transcription, T7 RNA polymerase (Roche), corresponding reaction buffers, pyrophosphatase, RNase inhibitors, and NTPs were used. In order to effectively cap RNA, an excess amount of cap analog compound ARCA (3'-O-Mem⁷G(5')ppp(5')G, Trilink, N-7003-1) or CleanCap (m7G(5')ppp(5')(2'OMeA)pG, Trilink, N-7113) was added to the reaction, wherein ARCA was selected for capping in the RNA production in FIG. 2 to FIG. 5, and CleanCap was selected for capping the RNA in FIG. 6 to FIG. 9. At the same time, in order to reduce the immunogenicity of mRNA and improve translation efficiency, the mRNAs of the present disclosure were all subjected to nucleic acid modification, wherein all uridine triphosphate (abbreviated as UTP) in the reaction system was replaced with N1-methyl-pseudouridine triphosphate (abbreviated as 1m-ψUTP, purchased from ThermoFisher). This modification method referred to the patent application US 2014/0194494 A1. After the *in vitro* transcription system was incubated at 37°C for 2.5 hours, the RNA was purified through carboxylated magnetic beads (Invitrogen) and resuspended in RNase-free water; the concentration and quality of RNA were evaluated by spectrophotometry and analysis on a 5200 Bioanalyzer (Agilent). Upon detection, the target mRNA was obtained.

The sequences from 5'UTR to polyA of Mod. of the present disclosure are set forth in SEQ ID NOs: 53 and 54; the sequence from 5'UTR to polyA of BioN. is set forth in SEQ ID NO: 55. The 5'UTR and 3'UTR sequences in Mod. are consistent with the sequences in the patents and published literature of Moderna Inc. (US10849920B2, WO2013151667A1, US10730924B2, DOI: 10.1016/j.cell.2017.02.017). The 5'UTR and 3'UTR sequences of BioN. are derived from the patent application WO 2018/160540.

The linearized sequence of V-B1 is provided exemplarily, and the sequence from 5'UTR to polyA is set forth in SEQ ID NO: 56; for the linearized sequence of V-A1, the sequence from 5'UTR to polyA is set forth in SEQ ID NO: 57.

### 1.3 Functional validation of 5'UTR, 3'UTR, and combinations thereof

In this example, an mRNA lipofection-based luciferase expression system was used for functional validation of UTRs.

Experimental methods: Human embryonic kidney cells (HEK293, purchased from ATCC) were seeded in a 96-well plate at a density of 4×10⁴ cells/well. The next day, transfection was performed through a Lipofectamine^{™} MessengerMAX^{™} mRNA transfection reagent, 100 ng of the capped mRNA prepared in Example 2 above was transfected into each well. After 6 hours of transfection, fresh medium was replaced. 100 µL of medium was aspirated and 50 µL of a luciferase substrate was added; the luminescence intensity was detected using a PerkinElmer multifunctional microplate reader. The experimental methods for human cervical cancer cells (HeLa, purchased from ATCC) and human lung cancer cells (A549, purchased from ATCC) were the same as those for HEK293.

Evaluation methods: In Table 4 to Table 7 and Table 9 to Table 11, the luciferin expression amount of the reference positive control Mod. in different cell lines was set to 1; the expression amount folds of different mRNAs relative to the positive control Mod. were calculated. In Table 8, the luciferin expression amount of the reference positive control BioN. in different cell lines was set to 1. The expression amount folds of different mRNAs relative to the positive control BioN. were calculated. Alle numerical values in the tables are folds of the detection average value.

### 1) Determination of the ability of 3'UTRs to regulate target gene expression in different cell lines

To verify the ability of different 3'UTRs to regulate the expression of target genes in different cell lines, mRNA expressing luciferase (Fluc) was prepared using the method in Example 2; the obtained RNA was capped with ARCA.; the polyA tail structure was A120. The RNA was transfected into human HeLa, HEK293, and A549 cells, and luciferase levels were measured 24 hours after transfection. The results are shown in Table 4 and FIG. 2.

**Table 4. Expression amounts of luciferase (Fluc) expressed by mRNAs carrying different 3'UTRs**

| (ARCA capping, target genes were all Fluc, polyA were all A120) | | | | | |
|---|---|---|---|---|---|
| mRNA No. | mRNA sequence number of 5'UTR | mRNA sequence number of 3'UTR | Cell line | | |
| | | | HeLa | HEK293 | A549 |
| Mod. | SEQ ID NO: 105 | SEQ ID NO: 106 | 1 | 1 | 1 |
| m-B1 | | SEQ ID NO: 58 | 1.25 | 1.52 | 1.55 |
| m-B2 | | SEQ ID NO: 59 | 1.72 | 1.85 | 1.94 |
| m-B3 | | SEQ ID NO: 60 | 1.56 | 1.66 | 1.83 |
| m-B4 | | SEQ ID NO: 61 | 1.63 | 1.68 | 1.77 |
| m-B5 | | SEQ ID NO: 62 | 0.99 | 1.01 | 1.08 |
| m-B6 | | SEQ ID NO: 63 | 1.13 | 1.30 | 1.40 |
| m-B7 | | SEQ ID NO: 64 | 1.41 | 1.72 | 1.69 |
| m-B8 | | SEQ ID NO: 65 | 1.31 | 1.40 | 1.57 |
| m-B9 | | SEQ ID NO: 66 | 2.04 | 1.71 | 2.13 |
| m-B10 | | SEQ ID NO: 67 | 1.72 | 1.83 | 1.82 |
| m-B11 | | SEQ ID NO: 68 | 1.65 | 1.61 | 1.63 |
| m-B12 | | SEQ ID NO: 69 | 2.29 | 2.22 | 2.24 |
| m-B13 | | SEQ ID NO: 70 | 2.21 | 2.27 | 2.28 |
| m-B14 | | SEQ ID NO: 71 | 2.07 | 1.92 | 2.27 |

The results showed that in different cell lines, compared to the Mod. positive control, the effects of the 3'UTRs of B1 to B14 on protein expression amounts were consistent; there was no significant difference between different cell lines (*p* > 0.05). This indicated that the effects of the 3'UTR elements obtained by screening in the present disclosure on protein expression amounts were universally applicable and could be used to improve the protein expression level of target genes.

### 2) Determination of the duration of regulating target gene expression by 3'UTRs in cells

To determine the ability of different 3'UTRs to regulate target gene expression duration in cells, the mRNAs in Table 4 were transfected into HEK293 cells; luciferase expression levels were measured at 6 h, 24 h, 48 h, and 72 h after transfection. The results are shown in Table 5 and FIG. 3.

**Table 5. Expression amounts of luciferase (Fluc) expressed by mRNAs carrying different 3'UTRs**

| mRNA No. | HEK293 | | | |
|---|---|---|---|---|
| | 6h | 24h | 48h | 72h |
| Mod. | 1 | 1 | 1 | 1 |
| m-B1 | 2.61 | 1.88 | 1.73 | 1.40 |
| m-B2 | 2.26 | 1.94 | 1.78 | 1.38 |
| m-B3 | 2.09 | 1.95 | 1.67 | 1.51 |
| m-B4 | 2.08 | 1.80 | 1.93 | 2.10 |
| m-B5 | 1.41 | 1.05 | 1.04 | 0.98 |
| m-B6 | 1.59 | 1.47 | 1.44 | 1.29 |
| m-B7 | 1.77 | 1.76 | 1.78 | 1.53 |
| m-B8 | 1.69 | 1.75 | 1.64 | 1.41 |
| m-B9 | 1.99 | 3.39 | 2.32 | 1.78 |
| m-B10 | 2.96 | 2.83 | 2.36 | 2.05 |
| m-B11 | 2.52 | 2.13 | 1.99 | 1.68 |
| m-B12 | 2.95 | 2.54 | 2.71 | 2.01 |
| m-B13 | 2.44 | 2.77 | 2.56 | 2.54 |
| m-B14 | 2.99 | 2.44 | 2.68 | 2.51 |

The results showed that in HEK293 cells, changes in 3'UTR elements could affect the expression amount of target proteins. Except for the B5-3'UTR element, other 3'UTR elements could increase the protein expression amount by more than 1.2 folds, and there were significant differences at different detection time points (*p* < 0.05). Among them, the 3'UTR elements of B12, B13, and B14 showed the best effect, which could increase the protein expression amount by more than 2 folds and had good continuity over time.

### 3) Determination of regulation of target gene expression by 5'UTRs in cells

To determine the ability of different 5'UTRs to regulate target gene expression duration in cells, mRNA expressing luciferase (Fluc) was prepared by the aforementioned method. The RNAs used in Table 6 and FIG. 4 were capped with ARCA, and the polyA tail structure was A120. The RNAs used in Table 7 and FIG. 5 were capped with CleanCap, and the polyA tail structure was A30L70. The above RNAs were respectively transfected into HEK293 cells; luciferase expression levels were measured at 6 h, 24 h, 48 h, and 72 h after transfection. The results are shown in Table 6, Table 7, and FIG. 4, FIG. 5.

**Table 6. Expression amounts of luciferase (Fluc) expressed by mRNAs carrying different 5'UTRs**

| (ARCA capping, target genes were all Fluc, polyA were all A120) | | | | | | |
|---|---|---|---|---|---|---|
| mRNA No. | mRNA sequence number of 5'UTR | mRNA sequence number of 3'UTR | HEK293 | | | |
| | | | 6h | 24h | 48h | 72h |
| Mod. | SEQ ID NO:105 | α-globin (SEQ ID NO:106) | 1 | 1 | 1 | 1 |
| m-A1 | SEQ ID NO:72 | | 2.83 | 4.42 | 4.22 | 4.05 |
| m-A2 | SEQ ID NO:73 | | 0.25 | 0.27 | 0.26 | 0.25 |
| m-A3 | SEQ ID NO:74 | | 1.56 | 1.72 | 1.72 | 1.55 |
| m-A4 | SEQ ID NO:75 | | 2.02 | 2.18 | 2.12 | 1.97 |
| m-A5 | SEQ ID NO:76 | | 1.97 | 2.28 | 2.17 | 2.04 |
| m-A7 | SEQ ID NO:78 | | 2.08 | 2.10 | 2.06 | 1.97 |
| m-A8 | SEQ ID NO:79 | | 1.26 | 1.17 | 1.18 | 1.11 |
| m-A9 | SEQ ID NO:80 | | 1.95 | 1.99 | 2.05 | 1.87 |
| m-A10 | SEQ ID NO:81 | | 1.69 | 1.70 | 1.78 | 1.55 |
| m-A11 | SEQ ID NO:82 | | 2.20 | 2.23 | 2.24 | 2.23 |
| m-A12 | SEQ ID NO:83 | | 1.51 | 1.48 | 1.36 | 1.34 |
| m-A13 | SEQ ID NO:84 | | 1.72 | 1.70 | 1.59 | 1.52 |
| m-A14 | SEQ ID NO:85 | | 1.80 | 1.97 | 1.81 | 1.73 |

**Table 7. Expression amounts of luciferase (Fluc) expressed by mRNAs carrying different 5'UTRs**

| (CleanCap capping, target genes were all Fluc, polyA were all A30L70) | | | | | | |
|---|---|---|---|---|---|---|
| mRNA No. | mRNA sequence number of 5'UTR | mRNA sequence number of 3'UTR | HEK293 | | | |
| | | | 6h | 24h | 48h | 72h |
| Mod. | SEQ ID NO:105 | α-globin (SEQ ID NO:106) | 1 | 1 | 1 | 1 |
| m-A15 | SEQ ID NO:86 | | 2.07 | 2.31 | 2.73 | 3.11 |
| m-A16 | SEQ ID NO:87 | | 1.54 | 2.09 | 2.50 | 3.17 |
| m-A17 | SEQ ID NO:88 | | 1.19 | 1.36 | 1.59 | 2.01 |
| m-A18 | SEQ ID NO:89 | | 1.47 | 1.95 | 2.50 | 2.94 |
| m-A19 | SEQ ID NO:90 | | 1.14 | 1.25 | 1.56 | 2.06 |
| m-A20 | SEQ ID NO:91 | | 0.76 | 0.86 | 1.24 | 1.38 |
| m-A21 | SEQ ID NO:92 | | 1.43 | 1.69 | 2.23 | 2.59 |
| m-A22 | SEQ ID NO:93 | | 0.53 | 0.69 | 0.88 | 0.97 |
| m-A23 | SEQ ID NO:94 | | 0.13 | 0.20 | 0.24 | 0.29 |
| m-A24 | SEQ ID NO:95 | | 1.37 | 1.98 | 2.40 | 2.70 |
| m-A25 | SEQ ID NO:96 | | 1.03 | 0.98 | 1.20 | 1.27 |
| m-A26 | SEQ ID NO:97 | | 0.70 | 0.69 | 0.93 | 0.89 |
| m-A27 | SEQ ID NO:98 | | 1.62 | 1.62 | 2.09 | 1.94 |
| m-A28 | SEQ ID NO:99 | | 1.39 | 1.34 | 1.76 | 1.65 |
| m-A29 | SEQ ID NO:100 | | 1.01 | 1.04 | 1.28 | 1.34 |
| m-A30 | SEQ ID NO:101 | | 1.37 | 1.27 | 1.13 | 1.06 |
| m-A31 | SEQ ID NO:102 | | 1.17 | 1.09 | 1.34 | 1.43 |
| m-A32 | SEQ ID NO:103 | | 1.31 | 1.41 | 1.64 | 1.60 |
| m-A33 | SEQ ID NO:104 | | 1.31 | 1.20 | 1.44 | 1.42 |

Different capping methods and polyA tail structures were used for screening in FIG. 4 and FIG. 5, respectively. The results showed that in different screening vectors, changes in 5'UTR elements could affect the expression amount of target proteins, 5'UTR elements possessed universality in increasing the expression levels of target proteins. Among them, the 5'UTR elements numbered A1, A3 to A7, A9 to A16, A18, A21, A24, A27, A28, A32, and A33 could all increase the protein expression amount by more than 1.2 folds, and there were significant differences at the detection time points of 24 h, 48 h, and 72 h (*p* < 0.05). Among them, the 5'UTR elements A1, A15, A16, and A18 showed the best effect, which could increase the protein expression amount by more than 2 folds and had good continuity over time.

### 4) Determination of the regulation of target gene expression intensity by candidate 5'UTRs compared with the positive control BioN. vector

To determine the ability of different candidate 5'UTRs to regulate target gene expression intensity compared with the positive control BioN. vector, the previously screened optimal 5'UTR elements A1, A15, A16, and A18 were constructed into a vector containing an A30L70 polyA tail element. mRNA expressing luciferase (Fluc) was prepared using the aforementioned method and transfected into HEK293 cells. Luciferase expression levels were measured at 6 h, 24 h, 48 h, and 72 h after transfection. The results are shown in Table 8 and FIG. 6.

**Table 8. Expression amounts of luciferase (Fluc) expressed by mRNAs carrying different 5'UTRs**

| (CleanCap capping, target genes were all Fluc, polyA were all A30L70) | | | | | |
|---|---|---|---|---|---|
| mRNA No. | mRNA sequence number of 5'UTR | mRNA sequence number of 3'UTR | HEK293 | | |
| | | | 6h | 24h | 48h |
| BioN. | SEQ ID NO:107 | SEQ ID NO:108 | 1 | 1 | 1 |
| m-A1 | SEQ ID NO:72 | α-globin (SEQ ID NO:106) | 1.34 | 1.78 | 1.92 |
| m-A15 | SEQ ID NO:86 | | 1.32 | 1.58 | 1.76 |
| m-A16 | SEQ ID NO:87 | | 1.01 | 1.37 | 1.54 |
| m-A18 | SEQ ID NO:89 | | 1.07 | 1.29 | 1.49 |

The results showed that all candidate 5'UTR elements could increase the protein expression amount by at least more than 1.2 folds at 24 h and 48 h compared with the BioN. vector, and all showed significant differences (*p* < 0.05).

### 5) Determination of regulation of target gene expression in cells by different combinations of 5'UTRs and 3'UTRs

To study the effects of different combinations of 5'UTRs and 3'UTRs on the expression of target proteins by mRNAs, mRNAs containing different combinations of 5'UTR and 3'UTR elements were compared with the 5'UTR and 3'UTR used by Moderna. The results are shown in Table 9 and FIG. 7.

**Table 9. Expression amounts of mRNAs carrying different combinations of 5'UTRs and 3'UTRs**

| (CleanCap capping, target genes were all Fluc, polyA were all A30L70) | | | | | | |
|---|---|---|---|---|---|---|
| mRNA No. | mRNA sequence number of 5'UTR | mRNA sequence number of 3'UTR | HEK293 | | | |
| | | | 6h | 24h | 48h | 72h |
| Mod. | SEQ ID NO:105 | SEQ ID NO:106 | 1 | 1 | 1 | 1 |
| m-A1-B12 | SEQ ID NO:72 | SEQ ID NO:69 | 2.78 | 3.06 | 2.46 | 2.96 |
| m-A1-B13 | | SEQ ID NO:70 | 2.62 | 2.67 | 2.30 | 2.57 |
| m-A1-B14 | | SEQ ID NO:71 | 2.20 | 2.14 | 1.74 | 1.90 |
| m-A15-B12 | SEQ ID NO:86 | SEQ ID NO:69 | 2.11 | 2.14 | 1.78 | 1.90 |
| m-A15-B13 | | SEQ ID NO:70 | 2.04 | 2.15 | 1.74 | 1.87 |
| m-A15-B14 | | SEQ ID NO:71 | 2.09 | 1.90 | 1.68 | 1.68 |
| m-A16-B12 | SEQ ID NO:87 | SEQ ID NO:69 | 2.32 | 2.02 | 1.82 | 1.81 |
| m-A16-B13 | | SEQ ID NO:70 | 2.43 | 1.96 | 1.78 | 1.89 |
| m-A16-B14 | | SEQ ID NO:71 | 2.30 | 1.79 | 1.63 | 1.68 |
| m-A18-B12 | SEQ ID NO:89 | SEQ ID NO:69 | 1.87 | 1.90 | 1.27 | 1.41 |
| m-A18-B13 | | SEQ ID NO:70 | 2.17 | 2.31 | 1.71 | 2.08 |
| m-A18-B14 | | SEQ ID NO:71 | 1.40 | 1.66 | 1.31 | 1.51 |

The results showed that compared with the 5'UTR and 3'UTR elements used by Moderna, the combinations of 5'UTR and 3'UTR elements screened in the present disclosure could all increase the protein expression amount by more than 1.3 folds at the detection time points of 24 h, 48 h, and 72 h, and all showed significant differences (*p* < 0.05). The target proteins in 1) to 5) above were all luciferase (Fluc); the target proteins in 6) below were secretory proteins (e.g., hHGF, anti-PD-1 antibody).

### 6) Determination of regulation of secretory target protein expression by different combinations of 5'UTRs and 3'UTRs

To study the effects of different combinations of 5'UTRs and 3'UTRs on the expression of secretory proteins by mRNAs, the mRNA was constructed herein such that the target protein expressed by the ORF was human hepatocyte growth factor (hHGF) or an anti-PD-1 antibody.

The amino acid sequence of the hHGF is as set forth in SEQ ID NO: 109, and its DNA sequence is as set forth in SEQ ID NO: 110; after codon optimization, mRNAs OS1, OS2, and OS3 were obtained, and their optimized codon sequences are as set forth in SEQ ID NOs: 129 to 131.

The amino acid sequences of the heavy chain and light chain of the PD-1 antibody are as set forth in SEQ ID NOs: 117 and 118, respectively; the DNA sequences of the heavy chain and light chain are as set forth in SEQ ID NOs: 119 and 120, respectively; the mRNA sequences of the heavy chain and light chain are as set forth in SEQ ID NOs: 121 and 122, respectively.

First, in order to improve the translation efficiency of the hHGF protein, codon optimization on the nucleotide sequence of wild-type hHGF (SEQ ID NO: 110) was conducted. The optimized sequences included hHGF-OS1 (SEQ ID NO: 111), hHGF-OS2 (SEQ ID NO: 112), and hHGF-OS3 (SEQ ID NO: 113), and the corresponding mRNA sequences are as set forth in SEQ ID NOs: 129 to 131, respectively. The aforementioned natural hHGF and codon-optimized hHGF sequences were constructed into vectors to produce mRNAs. The expression amount of the hHGF protein was detected by ELISA, demonstrating that the hHGF codon-optimized sequences could increase the expression amount of the hHGF protein.

The mRNA expressing hHGF-OS2 containing different combinations of 5'UTR and 3'UTR elements (the corresponding DNA sequence is SEQ ID NO: 112) was compared with mRNA Mod.(hHGF) expressing hHGF-OS2 containing a combination of 5'UTR and 3'UTR elements of Moderna (the corresponding DNA sequence is SEQ ID NO: 114). HEK293 cells were transfected with the mRNA encoding hHGF. Supernatants were collected at 6 h, 24 h, 48 h, and 72 h after transfection, and the expression amount of the hHGF protein was determined by ELISA to evaluate the increase/prolongation of HGF expression by the candidate vectors. The results are shown in Table 10 and FIG. 8A.

**Table 10. Target protein expression amounts of mRNAs carrying different combinations of 5'UTRs and 3'UTRs**

| (CleanCap capping, target genes were all hHGF, polyA were all A30L70) | | | | | | |
|---|---|---|---|---|---|---|
| mRNA No. (sequence number) | mRNA sequence number of 5'UTR | mRNA sequence number of 3'UTR | hHGF protein amount (ng/mL) | | | |
| | | | 6h | 24h | 48h | 72h |
| Mod.(hHGF) (SEQ ID NO:114) | SEQ ID NO:105 | SEQ ID NO:106 | 1.16 | 21.83 | 26.89 | 15.37 |
| m-A1-B12(hHGF) (SEQ ID NO:115) | SEQ ID NO:72 | SEQ ID NO:69 | 2.06 | 38.65 | 41.49 | 23.76 |
| m-A1-B13(hHGF) | | SEQ ID NO:70 | 1.98 | 30.19 | 30.57 | 19.00 |
| m-A1-B14(hHGF) | | SEQ ID NO:71 | 2.27 | 34.61 | 37.75 | 19.33 |
| m-A15-B12(hHGF) (SEQ ID NO:116) | SEQ ID NO:86 | SEQ ID NO:69 | 1.84 | 33.15 | 36.79 | 19.06 |
| m-A15-B13(hHGF) | | SEQ ID NO:70 | 2.14 | 31.68 | 36.60 | 25.04 |
| m-A15-B14(hHGF) | | SEQ ID NO:71 | 1.74 | 30.08 | 26.85 | 14.81 |
| m-A16-B12(hHGF) | SEQ ID NO:87 | SEQ ID NO:69 | 2.34 | 40.39 | 42.82 | 24.50 |
| (SEQ ID NO:127) | | | | | | |
| m-A16-B13(hHGF) | | SEQ ID NO:70 | 1.95 | 30.53 | 32.31 | 20.36 |
| m-A16-B14(hHGF) | | SEQ ID NO:71 | 2.22 | 33.56 | 37.61 | 21.10 |
| m-A18-B12(hHGF) | SEQ ID NO:89 | SEQ ID NO:69 | 2.45 | 49.06 | 55.14 | 30.24 |
| m-A18-B13(hHGF) | | SEQ ID NO:70 | 1.94 | 38.38 | 38.05 | 25.52 |
| m-A18-B14(hHGF) | | SEQ ID NO:71 | 1.93 | 36.75 | 38.37 | 23.25 |

The full-length mRNA expressing anti-PD-1 antibody containing different combinations of 5'UTR and 3'UTR elements (the corresponding DNA sequences are SEQ ID NOs: 124 to 125) was compared with the mRNA expressing anti-PD-1 antibody containing a combination of 5'UTR and 3'UTR elements of Moderna (the corresponding DNA sequence is SEQ ID NO: 123). HEK293 cells were transfected with the full-length mRNA encoding anti-PD-1 antibody. Supernatants were collected at 6 h, 24 h, 48 h, and 72 h after transfection, and the expression amount of the anti-PD-1 antibody was determined by ELISA to evaluate the increase/prolongation of anti-PD-1 antibody expression by the candidate vectors. The results are shown in Table 11 and FIG. 8B.

**Table 11. Target protein expression amounts of mRNAs carrying different combinations of 5'UTRs and 3'UTRs**

| (CleanCap capping, target genes were all heavy chain and light chain of anti-PD-1 antibody, polyA were all A30L70) | | | | |
|---|---|---|---|---|
| mRNA No. (corresponding DNA sequence number) | Anti-PD-1 antibody (ng/mL) | | | |
| | 6h | 24h | 48h | 72h |
| Mod.(PD-1 Ab) (SEQ ID NO:123) | 2.79 | 23.11 | 30.04 | 28.23 |
| m-A1-B12(PD-1 Ab) (SEQ ID NO:124) | 3.22 | 33.47 | 52.50 | 43.19 |
| m-A15-B12(PD-1 Ab) (SEQ ID NO:125) | 2.77 | 34.39 | 45.72 | 49.90 |

The above results showed that after continuously expressing the secretory proteins hHGF and anti-PD-1 antibody for 72 hours, the combinations of 5'UTR and 3'UTR screened in the present disclosure could significantly increase the expression amounts of secretory proteins compared with the 5'UTR and 3'UTR elements used by Moderna (*p* < 0.05).

### 1.4 Validation of hHGF protein expression in vivo in mice by mRNA expressing hHGF

In order to determine the ability of the mRNA molecule m-A16-B12 (hHGF) (corresponding DNA sequence is SEQ ID NO: 127) to express hHGF *in vivo* in animals, lipid nanoparticles LNPs (comprising 50 mol% ionizable lipid (SM-102), 10 mol% DSPC, 38.5 mol% cholesterol, 1.5 mol% PEG-DMG) were used for mRNA delivery. The same lipid nanoparticles LNPs were also used in Example 5 and Example 6 below. The positive control was the Collategene plasmid (AnGes, Inc.), which was administered as a naked plasmid. Balb/c mice (6-8 w, male) were randomly divided into 4 groups with 33 mice in each group. Different doses of m-A16-B12 (hHGF) and the Collategene plasmid were injected intramuscularly into the gastrocnemius muscles of the mice. The experimental groups were arranged according to the protocol in Table 12 as follows: 1) 1.0 µg/mouse of m-A16-B12 (hHGF) was injected; 2) 0.3 µg/mouse of m-A16-B12 (hHGF) was injected; 3) 0.1 µg/mouse of m-A16-B12 (hHGF) was injected; 4) 200 µg/mouse of Collategene naked plasmid was injected. Mouse gastrocnemius muscle samples were obtained at 1 h, 2 h, 4 h, 6 h, 24 h, 48 h, 72 h, 96 h, 168 h, 216 h, and 336 h. Muscle tissues were homogenized and lysed using a RIPA lysis buffer containing protease inhibitors. The supernatant was taken after centrifugation, the hHGF protein concentration was determined using a BCA protein concentration assay kit. The expression level of hHGF was evaluated using an ELISA method. Data of each group is expressed as mean ± standard deviation (Mean ± SD), GraphPad Prism 9.0 software was used for plotting and statistics.

**Table 12. Experimental grouping of hHGF expression in vivo by m-A16-B12 (hHGF) and Collategene**

| Group | G1 | G2 | G3 | G4 |
|---|---|---|---|---|
| Animal numbers | 33 | 33 | 33 | 33 |
| Agent tested | Plasmid (Collategene) | m-A16-B12 (hHGF) | m-A16-B12 (hHGF) | m-A16-B12 (hHGF) |
| Dosage | 200 µg | 0.1 µg | 0.3 µg | 1.0 µg |
| Administration route | i.m. | | | |
| Administration frequency | Single-dose | | | |
| Indicator | Plasma and muscle samples were collected to determine hHGF protein content | | | |
| Sample collection | Samples were taken 1 h, 2 h, 4 h, 6 h, 24 h, 48 h, 72 h, 96 h, D7, D10, D14 after administration | | | |

The experimental results are shown in A of FIG. 9. m-A16-B12 (hHGF) could express the hHGF protein at 1 h after intramuscular injection, an expression peak was reached at 6 h after injection in a dose-dependent manner. As shown in B of FIG. 9, Collategene reached an expression peak at 7 days after injection. The protein expression amount decreased at 48 h after the delivery of m-A16-B12 (hHGF). The protein expression amount at the dose of 1 µg/mouse m-A16-B12 (hHGF) at 72 h was comparable to that of Collategene. At the same time, statistics on the PK data of each group found that even for the lowest dose of 0.1 µg of m-A16-B12 (hHGF), its Cₘₐₓ was more than 5 times that of Collategene, and the AUC_{inf (hr*pg/mg protein)} of 0.1 µg of m-A16-B12 (hHGF) was also comparable to that of Collategene (Table 13). Therefore, m-A16-B12 (hHGF) could achieve high-efficiency expression of hHGF *in vivo* in animals, and no hHGF expression was detected in plasma at each time point.

**Table 13. Results of hHGF expression in vivo by m-A16-B12 (hHGF) and Collategene**

| Route | Agent tested | Dosage (µg) | Cₘₐₓ (pg/mg protein) | AUC_{0-inf} (hr*pg/mg protein) | MRT_{0-inf}(h) |
|---|---|---|---|---|---|
| | m-A16-B12 (hHGF) | 0.1 µg | 369.1±24.8 | 16158.3±978.7 | 201.5±73.8 |
| I.M. | | 0.3 µg | 463.1±16.5 | 20133.6±770.2 | 125.9±26.4 |
| | | 1 µg | 570.6±42.5 | 29652.8±2555.9 | 142.8±71.9 |
| | Collategene | 200 µg | 78.0±5.7 | 15300.4±491.2 | 183.5±3.0 |

### 1.5 Validation of the therapeutic function of mRNA expressing hHGF on a hindlimb ischemia mouse model

The hindlimb ischemia mouse model is a classic mouse model simulating critical limb ischemia in humans. Male Balb/c mice at 6 to 8 weeks were used for modeling as follows: 1) The animal was anesthetized and placed in a supine position on an operating table.; the hindlimbs were thoroughly depilated and fixed, and the skin at the surgical site was disinfected; 2) A skin incision approximately 1 cm long was made from the knee to the inner thigh; the subcutaneous fat tissue was incised and dissected in turn to fully expose the femoral artery; 3) The membranous femoral sheath was gently pierced with microscissors to expose the neurovascular bundle; the proximal position of the femoral artery from the femoral vein and nerve near the groin was isolated; after clean isolation, the proximal end of the femoral artery was ligated with a 6-0 suture under the proximal end of the femoral artery; 4) The femoral artery and femoral vein were separated at a distal end near the knee; the distal end of the femoral artery was ligated under the distal end with a 6-0 suture at the proximal end of the popliteal artery; 5) The wound was sutured.

The LNPs in Example 1.4 were used as a vector to deliver m-A16-B12 (hHGF); Collategene was administered in the form of a naked plasmid. The hindlimb ischemia model mice were randomly divided into 4 groups with 5 mice in each group. m-A16-B12 (hHGF) and the Collategene naked plasmid were injected intramuscularly into the gastrocnemius muscles of the mice respectively. The experimental grouping was as follows: 1) 500 ng/mouse of m-A16-B12 (hHGF) was injected; 2) 50 ng/mouse of mA16-B12 (hHGF) was injected; 3) 200 µg/mouse of Collategene naked plasmid was injected; 4) an equal volume of PBS was injected as a control group. The day of modeling was counted as day 0. The leg conditions were observed on day 0, day 4, day 7, day 10, day 12, and day 14 after treatment in each group. The blood perfusion condition in the hindlimbs of mice was detected using a blood flow meter, and photographs were recorded.

The blood perfusion ratio of each group was calculated according to the following formula: blood perfusion ratio = blood perfusion volume in the hindlimb of a mouse on the day / blood perfusion volume in the hindlimb of the mouse on day 0 × 100%. Data of each group is expressed as mean ± standard deviation (Mean ± SD), GraphPad Prism 9.0 software was used for plotting and statistics.

The results are shown in FIG. 10A and FIG. 10B; in the case where 50 ng/mouse and 500 ng/mouse of m-A16-B12 (hHGF) and 200 ng/mouse of the Collategene naked plasmid were injected for treatment, the blood perfusion ratio of the ischemic hindlimbs of each mouse was significantly improved relative to the control group, and the blood perfusion ratio of the ischemic hindlimbs gradually recovered over time. Among them, the group injected with 50 ng/mouse of m-A16-B12 (hHGF) showed a blood flow recovery effect similar to that of the group injected with 200 ng/mouse of the Collategene naked plasmid. In particular, the group injected with 500 ng/mouse of m-A16-B12 (hHGF) was significantly superior to the group injected with 200 ng/mouse of the Collategene naked plasmid in blood flow recovery effect, achieving a recovery of more than 90% in blood perfusion.

In order to further determine the therapeutic effect of m-A16-B12 (hHGF) on the degree of hindlimb necrosis in the hindlimb ischemia mouse model, the leg conditions of the mice were observed and photographed on day 14 after administration, and the degree of hindlimb necrosis of the mice was scored by the criteria as shown in FIG. 11A. The criteria used were as follows: 0 = self-detachment of hindlimb; 1 = leg necrosis; 2 = foot necrosis; 3 = > 2 toes discolored; 4 = 1 toe discolored; 5 = > 2 nails discolored; 6 = 1 nail discolored; 7 = no necrosis. Data of each group is expressed as mean ± standard deviation (Mean ± SD), GraphPad Prism 9.0 software was used for plotting and statistics.

The results are shown in FIG. 11B. After inducing hindlimb ischemia in mice, the mice in the control group experienced obvious ischemic necrosis in the hindlimbs within 2 weeks after surgery, and by day 14, the hindlimbs of some mice completely fell off. In the treatment groups injected with m-A16-B12 (hHGF) and the Collategene naked plasmid, the hindlimbs of all mice could maintain good integrity and no hindlimb necrosis occurred, indicating that m-A16-B12 (hHGF) possessed an ability to improve hindlimb ischemia comparable to that of the control Collategene naked plasmid.

CD31 is an important marker for angiogenesis. To determine the effect of m-A16-B12 (hHGF) in promoting angiogenesis in the hindlimb ischemia mouse model, gastrocnemius muscle samples near the ischemic tissues of the mice were taken on day 14 after administration, and paraffin sections were prepared. CD31 in the section samples was subjected to immunohistochemical staining with a CD31 antibody and photographed. The images were analyzed using ImageJ (NIH) software, and the CD31 staining area was calculated. Data of each group is expressed as mean ± standard deviation (Mean ± SD), GraphPad Prism 9.0 software was used for plotting and statistics.

The results are shown in FIG. 12. In the cases where 50 ng/mouse and 500 ng/mouse of m-A16-B12 (hHGF) and 200 ng/mouse of the Collategene naked plasmid were administered for treatment, angiogenesis in the ischemic hindlimb muscles of each group was significantly promoted, and the number of newly formed blood vessels had a significant statistical difference relative to the PBS group (*p* < 0.05). The 50 ng/mouse mA16-B12 (hHGF) group showed an angiogenesis-promoting effect comparable to that of 200 ng/mouse of the Collategene naked plasmid; the m-A16-B12 (hHGF) administration groups showed dose-dependent angiogenesis promotion.

### 1.6 Validation of the therapeutic function of mRNA expressing hHGF on a full-thickness skin injury Db/Db mouse model

The Db/Db mouse model is a classic diabetic mouse model, which is a mouse with a defect in the Leptin receptor gene. As a result, the mice exhibit characteristics similar to those of diabetic patients, such as hyperglycemia, hyperlipidemia, and insulin resistance, as their age-in-week increases. In this experiment, a full-thickness skin injury model was used to simulate skin injuries in patients with diabetic foot that are difficult to heal. The method for modeling the aforementioned Db/Db mouse model was as follows: Db/Db mice were anesthetized and depilated with depilatory cream, and disinfected with 75% alcohol cotton balls. A 8 mm diameter skin biopsy punch was used to create full-thickness skin wounds on the lower back.

In order to determine the therapeutic effect of m-A16-B12 (hHGF) on wound healing in the full-thickness skin injury mouse model, the LNPs in Example 1.4 were used to deliver m-A16-B12 (hHGF); the control Collategene was administered in the form of a naked plasmid. The administration method was subcutaneous injection at 4 points. The Db/Db mice of the full-thickness skin injury model were divided into 5 groups according to body weight and blood glucose level, with 7 mice in each group. The experimental grouping was as follows: 1) 500 ng/mouse of m-A16-B12 (hHGF) was injected; 2) 200 ng/mouse of m-A16-B12 (hHGF) was injected; 3) 50 ng/mouse of m-A16-B12 (hHGF) was injected; 4) 200 µg/mouse of Collategene naked plasmid was injected; 5) a control group injected with an equal volume of PBS. The day of modeling was counted as day 0. After treatments were performed for each group, the wound recovery conditions were observed on day 0, day 3, day 5, day 7, day 10, day 12, and day 14, respectively. The wound sites were photographed, the images were analyzed using ImageJ (NIH) software, and the wound area was calculated.

The wound healing percentage was calculated according to the following formula: P = (AD - A0) / A0 × 100% (P: wound healing percentage; AD: wound area on the day of photographing; A0: wound area on the day after surgery).

The wound healing percentage of each group is expressed as mean ± standard deviation (Mean ± SD), GraphPad Prism 9.0 software was used for plotting and statistics.

The results are shown in FIG. 13. Under the treatments with 50 ng/mouse, 200 ng/mouse, and 500 ng/mouse of m-A16-B12 (hHGF) and 200 µg/mouse of the Collategene naked plasmid, the wounds of the mice were all well healed compared with the control group. Moreover, the promotion of mouse wound healing by m-A16-B12 (hHGF) was dose-dependent. On day 14, the wound healing rate at the dose of 50 ng/mouse m-A16-B12 (hHGF) reached 66%, and the dose of 200 ng/mouse and 500 ng/mouse m-A16-B12 (hHGF) could achieve a 100% wound healing. Moreover, the promotion of wound healing by m-A16-B12 (hHGF) was significantly superior to that of 200 µg/mouse of the Collategene naked plasmid (*p* < 0.05). Even the lower dose (50 ng/mouse) of m-A16-B12 (hHGF) showed a wound healing percentage with a significant difference from that of 200 µg/mouse Collategene naked plasmid (*p* < 0.05).

In order to determine the therapeutic effect of m-A16-B12 (hHGF) on tissue remodeling in the full-thickness skin injury mouse model, epithelial regeneration and tissue remodeling were evaluated through Masson's staining. On day 17 after administration, full-thickness skin samples of the mice were collected, and paraffin sections were prepared. Masson's staining was used to stain the skin tissues of the mice.

The results are shown in FIG. 14. Under the treatment of m-A16-B12 (hHGF), full epithelial coverage was achieved on the wound epithelium of the mice in all groups. The epithelium at the wounds of the low-dose group was abnormally thickened, and collagen proliferated. The epithelium of the medium- and high-dose groups recovered to normal thickness, and collagen was arranged orderly, completing tissue remodeling. However, the control group and the 200 µg/mouse Collategene naked plasmid group did not complete epithelial re-coverage, and abnormal proliferation of collagen and structural disorder occurred at the wounds.

### Example 2:

### Preparation of LNP lipid particles

Compound 1 (the compound of Formula I, prepared with reference to WO2023125738A), DSPC, cholesterol, and DMG-PEG 2000 solution were mixed at a certain molar percentage (mol %, that is, the percentage of the molar amount of each component to the molar amount of total lipids present in the lipid nanoparticle) to formulate an ethanol lipid solution. The molar ratio of the four components was formulated as shown in Table 14. The mRNA encoding the luciferase protein was dissolved in a pH 4 acetate buffer to formulate an aqueous mRNA solution. The ethanol lipid solution and the aqueous mRNA solution were mixed at a volume ratio of 1:3 through a microfluidic device, and liposome was prepared at a weight ratio of total lipids to mRNA of about 20:1. Ethanol was removed by dialysis in a 20 mM Tris solution to obtain a liposome nanoparticle composition encapsulating the mRNA.

### Characterization of Lipid Particle Composition

### Characterization Methods

The nanoparticle size and polydispersity index (PDI) of the liposome nanoparticles were determined by dynamic light scattering using a Malvern Zetasizer Pro in a 173° backscatter detection mode.

The encapsulation efficiency of the liposome nanoparticles was determined using a Quant-iT RiboGreen RNA quantitative Assay Kit.

**Table 14**

| No. | Compound 1 mol % | DSPC mol % | Cholesterol mol % | PEG-DMG mol % | Particle size nm | PDI | Encapsulation efficiency % |
|---|---|---|---|---|---|---|---|
| 1 | 48.0 | 10.0 | 40.5 | 1.5 | 90.8 | 0.12 | 97 |

### Example 3:

### Freeze-drying of lipid particle compositions

Lipid nanoparticles were prepared according to Example 2. The prepared lipid nanoparticles were exchanged into a lyophilized preparation buffer (containing 20 mM Tris, pH 7.5, 8% sucrose, 5% trehalose, and sodium chloride solutions of different concentrations corresponding to the table below) through ultrafiltration. The lyophilized preparation solution of the lipid nanoparticles was filled into 2 mL vials at 0.6 mL/vial, with a concentration of the cationic lipid in each vial was 1.78 mg/mL. The vials were placed in a freeze-dryer, pre-freezing was performed at -45°C for 3 hours, primary drying was performed at -40°C for 30 hours, and secondary drying was performed at 30°C for 10 hours, respectively, at a vacuum degree of 0.05 mmbar. The final lyophilized product was obtained after being taken out of the freeze-dryer.

The lyophilized product was taken and reconstituted by adding 0.6 mL of water for injection. The particle size of the lipid nanoparticles was determined according to the aforementioned method. The particle sizes of the lipid nanoparticles before and after freeze-drying were compared. The results are shown in Table 15 and FIG. 15 to FIG. 17.

**Table 15**

| No. | NaCl concentration (mM) | Mass ratio of sodium chloride to cationic lipid | Particle size before freeze-drying (nm) | Particle size (nm) after freeze-drying and reconstitution | Rate of change (%) |
|---|---|---|---|---|---|
| Formula 1 | 0 | 0 | 94.3 | 186.7 | 98.0 |
| Formula 2 | 25 | 1:1.2 | 86.4 | 128.2 | 48.4 |
| Formula 3 | 50 | 1.6:1 | 85.4 | 118.3 | 38.5 |
| Formula 4 | 100 | 3.3:1 | 73.7 | 99.2 | 34.6 |

The stock solutions of the lipid nanoparticles of the above were diluted to 1/2 and 1/3 concentrations using the lyophilized preparation buffer, respectively. The obtained lipid nanoparticles were filled and freeze-dried according to the above experimental operations. The particle sizes of the lipid nanoparticles after freeze-drying and reconstitution were determined respectively. The results are shown in Table 16.

**Table 16**

| No. | NaCl concentration (mM) | Mass ratio of sodium chloride to cationic lipid | Particle size before freeze-drying (nm) | Particle size (nm) after freeze-drying and reconstitution | Rate of change (%) |
|---|---|---|---|---|---|
| Formula 5 | 100 | 6.6:1 | 74.7 | 101.4 | 35.7 |
| Formula 6 | 100 | 9.9:1 | 73.4 | 100.4 | 36.8 |

The well-prepared lipid nanoparticles were exchanged into another lyophilized preparation buffers (containing 20 mM Tris, pH 7.5, 8% sucrose, and sodium chloride solutions of different concentrations corresponding to the table below) through ultrafiltration for liquid exchange. The lyophilized preparation solution of the lipid nanoparticles was filled into 2 mL vials at 0.6 mL/vial, with a concentration of the cationic lipid in each vial was 2.38 mg/mL. The vials were placed in a freeze-dryer. The pre-freezing was performed at -45°C for 3 hours, primary drying was performed at -40°C for 30 hours, and secondary drying was performed at 30°C for 10 hours, respectively, at a vacuum degree of 0.05 mmbar. The final lyophilized product was obtained after being taken out of the freeze-dryer.

The lyophilized product was taken and reconstituted by adding 0.6 mL of water for injection.; the particle size of the lipid nanoparticles was determined according to the aforementioned method. The particle sizes of the lipid nanoparticles before and after freeze-drying were compared. The results are shown in Table 17.

**Table 17**

| No. | NaCl concentration (mM) | Mass ratio of sodium chloride to cationic lipid | Particle size before freeze-drying (nm) | Particle size (nm) after freeze-drying and reconstitution | Rate of change (%) |
|---|---|---|---|---|---|
| Formula 7 | 0 | 0 | 93.5 | 162.6 | 73.9 |
| Formula 8 | 25 | 1:1.6 | 81.5 | 121.8 | 49.4 |
| Formula 9 | 50 | 1.2:1 | 82.0 | 121.7 | 48.4 |

### Example 4:

### Evaluation of mRNA integrity in lipid nanoparticle compositions

The lyophilized lipid nanoparticles were prepared according to Example 3. The mRNA integrity in each group of samples was determined using a capillary electrophoresis instrument (Agilent 5200 Fragment Analyzer), respectively.

mRNA is easily degraded and is affected by temperature.; as the temperature increases, the stability of mRNA decreases. With the prolongation of storage time, degradation occurs and integrity decreases under high-temperature conditions. During the freeze-drying process, there is a temperature rise from primary drying to secondary drying, and the secondary drying temperature is relatively high. Therefore, the effects of sodium chloride in different preparations on mRNA integrity during the freeze-drying process and under high-temperature storage conditions were evaluated. As shown in Table 18 and FIG. 18 to FIG. 21, with the increase of sodium chloride concentration, the mRNA integrity after being taken out of the freeze-dryer increased. Similarly, under the high-temperature storage condition of 37°C, with the increase of sodium chloride concentration, the mRNA integrity increased.

**Table 18**

| No. | NaCl concentration (mM) | mRNA integrity (%) | |
|---|---|---|---|
| | | After being taken out of the freeze-dryer | After 72 hours at 37°C |
| Formula 1 | 0 | 87.7 | 72.7 |
| Formula 2 | 25 | 88.1 | 75.3 |
| Formula 3 | 50 | 88.3 | 76.6 |
| Formula 4 | 100 | 91.8 | 81.3 |
| Formula 7 | 0 | 86.9 | 72.9 |
| Formula 8 | 25 | 87.2 | 77.2 |
| Formula 9 | 50 | 90.2 | 80.0 |

### Example 5:

### Evaluation of mRNA delivery efficiency of lipid nanoparticles in cells

HEK 293 cells were seeded into a 96-well plate and cultured overnight, until the cell density reached more than 80%, the lipid nanoparticle solutions encapsulating the luciferase mRNA (the lyophilized product of formula 7 and the lyophilized product of formula 9) were added into the culture medium in the wells of the cell plate. The mRNA dose was 100 ng/well. After 24 hours, the fluorescence intensity of the expressed luciferase protein was determined using a luciferase reporter assay kit (Promega) and a microplate reader. The fluorescence intensity value was the fluorescence value determined by the microplate reader, which represented the expression amount of the luciferase protein. The higher the fluorescence intensity, the higher the protein expression amount. The average fluorescence intensity for the lipid nanoparticles corresponding to each formula was calculated from at least 3 replicates.

**Table 19**

| No. | Average fluorescence intensity |
|---|---|
| Formula 7 | 2.34E+07 |
| Formula 9 | 4.12E+07 |

Conclusion: As shown by the fluorescence intensities in Table 19 and FIG. 22, the expression amount of the lyophilized lipid nanoparticle product corresponding to formula 9 was significantly higher than that of formula 7. The *** in FIG. 22 represents a statistically significant difference, with P < 0.001.

### Example 6. Preparation process of HGF-mRNA-LNP spray preparation

Preparation of HGF-mRNA-LNP lyophilized powder. HGF-mRNA-LNP (0.09 mg of m-A16-B12, 1.07 mg of the cationic lipid represented by Formula I; the molar percentages of the cationic lipid, DSPC, cholesterol, and DMG-PEG 2000 relative to the total moles of the lipid nanoparticles were 48 mol %, 10 mol %, 40.5 mol %, and 1.5 mol %, respectively) was prepared according to the method in Example 2. The HGF-mRNA-LNP lyophilized powder (formula 10) was prepared according to formula 4 in Example 3. The lyophilized product was taken and reconstituted by adding 0.6 mL of water for injection. The particle size of the lipid nanoparticles was determined according to the aforementioned method. The particle sizes of the lipid nanoparticles before and after freeze-drying were compared. The results are shown in Table 20.

**Table 20**

| No. | NaCl concentration (mM) | Mass ratio of sodium chloride to cationic lipid | Particle size before freeze-drying (nm) | Particle size (nm) after freeze-drying and reconstitution | Rate of change (%) |
|---|---|---|---|---|---|
| Formula 10 | 100 | 3.3:1 | 76.8 | 102.5 | 33.5 |

A reconstitution solvent was formulated and subjected to sterilizing filtration. This was used to reconstitute the above HGF-mRNA-LNP lyophilized powder to formulate a spray preparation.

### Example 7. Screening of reconstitution solvent component - dispersant

When mRNA-LNP exists in a solution form (after reconstitution of the lyophilized powder), its particles have relatively large stability issues, which are manifested by a tendency of particle aggregation and particle size increase. When LNP is nebulized through an nebulization administration device, the LNP particles will have a further increased possibility of aggregation under external mechanical squeezing or shear forces. To reduce the risk of particle aggregation occurring during the nebulization process of LNP, a dispersant needs to be added to the reconstitution solvent. Reconstitution solvents of WFI, 0.2% PF68 (poloxamer 188), 0.2% PF127 (poloxamer 407), 0.25% PVA (polyvinyl alcohol), 0.1% HEC (hydroxyethyl cellulose), and 1% PEG400 (polyethylene glycol 400) were respectively formulated (by weight ratio). nebulization was performed using a disposable nasal administration device, the nebulized solution was collected at a position 3 cm away from the nebulization nozzle. The particle size, PDI, total mRNA content, and encapsulation efficiency of the collected solution after nebulization and the reconstituted solution before nebulization were determined as evaluation indicators.

The results are shown in Table 21. When different reconstitution solvents were used to reconstitute the LNP lyophilized powder, compared with reconstitution with water for injection (WFI), only reconstitution with 0.2% PF127 resulted in a significant decrease in the LNP particle size (81.3 nm vs 96.7 nm), indicating that the structure of the LNP might have been destroyed after reconstitution with 0.2% PF127. After reconstitution with other reconstitution solvents, the LNP particle size had no significant difference compared with the particle size after WFI reconstitution. Moreover, the total mRNA content and encapsulation efficiency of the solution after reconstitution with other reconstitution solvents were comparable to those of the solution after WFI reconstitution. Regarding the changes in the particle size, PDI, total mRNA content, and encapsulation efficiency of the solution before and after spraying, there was no significant difference in these indicators for the solution before and after spraying after reconstitution with each reconstitution solvent. This indicated that the risk levels of LNP particle structure damage and medicine adsorption during the spraying process using the disposable nasal administration device were all relatively low.

Taken together, 0.2% PF68, 0.25% PVA, 0.1% HEC, and 1% PEG400 can all be used as reconstitution solvent component - dispersant.

**Table 21. Screening results of reconstitution solvent component - dispersant**

| Reconstitution solvent formula | | Particle size (nm) | PDI | Total mRNA content (µg/ml) | Encapsulation efficiency |
|---|---|---|---|---|---|
| WFI | Before | 96.7 | 0.22 | 66.88 | 81.7% |
| | After spraying | 98.6 | 0.21 | 67.20 | 77.5% |
| 0.2% PF68 | Before | 96.7 | 0.19 | 79.27 | 86.7% |
| | After spraying | 98.1 | 0.22 | 81.24 | 81.2% |
| 0.2% PF127 | Before | 81.3 | 0.16 | 82.27 | 83.0% |
| | After spraying | 84.1 | 0.18 | 84.02 | 81.2% |
| 0.25% PVA | Before | 98.7 | 0.17 | 77.64 | 86.4% |
| | After spraying | 103.3 | 0.22 | 76.17 | 79.4% |
| 0.1% HEC | Before | 101.5 | 0.17 | 78.47 | 87.5% |
| | After spraying | 105.6 | 0.23 | 83.97 | 83.9% |
| 1% PEG400 | Before | 100.7 | 0.18 | 72.93 | 79.8% |
| | After spraying | 103.0 | 0.20 | 73.66 | 83.8% |

### Example 8. Screening of reconstitution solvent component - thickener

As an external spray preparation, the preparation needs to have a certain viscosity to increase the retention time of the preparation at the administration site. Xanthan gum (Xc), Polyvinylpyrrolidone K90 (PVP-K90), sodium hyaluronate (HA), sodium carboxymethyl cellulose (CMC-Na), and chitosan (CTS) were screened as thickeners. The specific preparations are as follows (by weight ratio):
1) 0.24% xanthan gum (Xc);
2) 0.5% Polyvinylpyrrolidone K90 (PVP-K90);
3) 0.1% sodium hyaluronate (HA);
4) 0.5% sodium carboxymethyl cellulose (CMC-Na);
5) 0.2% chitosan (CTS).

Each of the above reconstitution solvents was formulated, filtered, filled, stoppered, and capped for later use. A disposable nasal administration device was used to transfer each of the above reconstitution solvents to reconstitute the lyophilized powder and then perform spraying, the sprayed samples were collected. The particle size, PDI, encapsulation efficiency, and total mRNA content of the samples before and after spraying were determined. The results are shown in Table 22 below.

**Table 22. Screening of reconstitution solvent component - thickener**

| Reconstitution solvent formula | | Particle size (nm) | PDI | Total mRNA content (µg/ml) | Encapsulation efficiency |
|---|---|---|---|---|---|
| WFI | Before | 96.7 | 0.22 | 66.88 | 81.7% |
| | After spraying | 98.6 | 0.21 | 67.20 | 77.5% |
| 0.24% Xc | Before | 101.7 | 0.15 | 70.42 | 82.7% |
| | After spraying | 103.2 | 0.21 | 72.12 | 81.2% |
| 0.5% PVPK90 | Before | 98.4 | 0.13 | 73.14 | 83.3% |
| | After spraying | 100.1 | 0.18 | 74.02 | 82.2% |
| 0.1% HA | Before | 98.6 | 0.16 | 71.69 | 84.4% |
| | After spraying | 97.1 | 0.21 | 76.37 | 81.4% |
| 0.5% CMC-Na | Before | 104.5 | 0.16 | 80.13 | 84.5% |
| | After spraying | 100.5 | 0.20 | 83.87 | 83.9% |
| 0.2% CTS | Before | 99.2 | 0.18 | 72.23 | 83.8% |
| | After spraying | 107.5 | 0.25 | 69.66 | 79.8% |

According to the above results, when 0.2% chitosan (CTS) was used as the reconstitution solvent and the reconstituted sample was sprayed, its particle size and PDI showed an increasing trend compared with those before spraying (107.5 nm vs 99.2 nm), suggesting that particle aggregation might have occurred in the sample after spraying. Several other reconstitution solvents (0.24% Xc, 0.5% PVPK90, 0.1% HA, 0.5% CMC-Na) could maintain the stability of the LNP particle structure before and after spraying.

At the same time, the above reconstitution solvents were screened using the solution viscosity and spray particle size after spraying as evaluation indicators. The results are shown in Table 23 below:

**Table 23. Screening of reconstitution solvent component - thickener (spray morphology and pattern)**

| Reconstitution solvent formula | Viscosity (mPa·s) | Spray area (mm²) | Ellipticity ratio | Dmax (mm) | Dmin (mm) |
|---|---|---|---|---|---|
| WFI | 1.42 | 2553.2 | 1.205 | 62.64 | 52.00 |
| 0.24% Xc | 1.98 | 2628.5 | 1.135 | 60.24 | 53.07 |
| 0.5% PVPK90 | 2.02 | 1832.4 | 1.453 | 71.82 | 49.43 |
| 0.1% HA | 1.37 | 2231.2 | 1.217 | 67.44 | 55.41 |
| 0.5% CMC-Na | 1.64 | 2472.2 | 1.189 | 63.04 | 53.02 |
| 0.2% CTS | 2.21 | 1923.9 | 1.365 | 72.08 | 52.81 |

It can be seen from the above results that, compared with reconstitution and spraying with water for injection, the solution after reconstitution and spraying with 0.5% PVPK90 and 0.2% CTS had a higher viscosity, a smaller spray area, and a higher ellipticity ratio. This indicated that the solution was more viscous after reconstitution with 0.5% PVPK90 and 0.2% CTS, making it difficult to obtain a better spray morphology (spray area and ellipticity ratio). There was no significant difference in the spray effects of the other reconstitution solvents (0.24% Xc, 0.1% HA, 0.2% CTS) compared with that of water for injection (WFI).

### Example 9. Determination of reconstitution solvent components

The reconstitution solvent components need to be comprehensively considered for the viscosity of the sprayed solution, the stability of the LNP structure, the spray morphology and pattern, etc. The preparation of the reconstitution solvent components was performed according to Table 24 below. Then a disposable nasal administration device was used to perform spraying, the sprayed samples were collected. The viscosity, particle size, PDI, total mRNA content, and encapsulation efficiency after spraying were determined and used as evaluation indicators to screen the reconstitution solvent components. The test results are shown in Table 25 below.

**Table 24. Experimental design for reconstitution solvent components screening**

| Batch No. | Reconstitution solvent component - dispersant | Reconstitution solvent component - thickener | Evaluation indicator |
|---|---|---|---|
| 1 | WFI | WFI | Viscosity, particle size, PDI, total mRNA content, and encapsulation efficiency before and after spraying |
| 2 | 1% PEG-400 | 1% PVP-K90 | |
| 3 | 0.2% PF68 | 1% PVP-K90 | |
| 4 | 1% PEG-400 | 0.5% PVP-K90 | |
| 5 | 0.2% PF68 | 0.5% PVP-K90 | |
| 6 | 1% PEG-400 | 0.25% PVP-K90 | |
| 7 | 0.2% PF68 | 0.25% PVP-K90 | |

**Table 25. Results for reconstitution solvent components screening**

| Reconstitution solvent formula | | Viscosity (mPa·s) | Particle size (nm) | PDI | Total mRNA content (µg/ml) | Encapsulation efficiency |
|---|---|---|---|---|---|---|
| WFI | Before spraying | 1.42 | 95.85 | 0.21 | 59.64 | 81.9% |
| | After spraying | | 98.75 | 0.22 | 57.60 | 82.4% |
| 1% PEG-400+ 1% PVP-K90 | Before spraying | 3.72 | 106.4 | 0.21 | 77.74 | 85.1% |
| | After spraying | | 103.6 | 0.22 | 63.06 | 76.7% |
| 0.2% PF68+ 1% PVP-K90 | Before spraying | 3.83 | 106.5 | 0.22 | 84.42 | 85.5% |
| | After spraying | | 106.5 | 0.20 | 72.04 | 85.5% |
| 1% PEG-400+ | Before spraying | 2.19 | 102.7 | 0.23 | 74.41 | 86.1% |
| 0.5% PVP-K90 | After spraying | | 105.6 | 0.24 | 67.01 | 77.2% |
| 0.2% PF68+ 0.5% PVP-K90 | Before spraying | 2.05 | 98.99 | 0.20 | 82.19 | 86.9% |
| | After spraying | | 105.9 | 0.23 | 72.36 | 81.1% |
| 1% PEG-400+ 0.25% PVP- | Before spraying | 1.73 | 96.95 | 0.16 | 73.53 | 87.0% |
| | After spraying | | 105.7 | 0.23 | 64.34 | 73.5% |
| 0.2% PF68+ 0.25% PVP-K90 | Before spraying | 1.78 | 97.4 | 0.16 | 82.48 | 88.9% |
| | After spraying | | 105.7 | 0.20 | 73.03 | 80.0% |

Judging from the test results, the reconstitution solvent containing 1% PVP-K90 had the highest viscosity, followed by the one containing 0.5% PVP-K90. The reconstitution solvent containing 0.25% PVP-K90 had the lowest viscosity. From the perspective of the changes in particle size, PDI, total mRNA content, and encapsulation efficiency before and after spraying, and the comparison with WFI, the encapsulation efficiency and total mRNA content of the reconstitution solvent containing 1% PEG-400 decreased after spraying compared with those before spraying. Therefore, 0.2% PF68 + 1% PVP-K90, 0.2% PF68 + 0.5% PVP-K90, and 0.2% PF68 + 0.25% PVP-K90 were preferred as the reconstitution solvent components.

### Example 10. Determination of reconstitution solvent components - HGF expression

Furthermore, it is necessary to introduce the spray morphology and pattern, as well as the biological activity of the main component (LNP) of the solution after spraying, as key indicators to further screen and evaluate the reconstitution solvent components. The reconstitution solvent components are as follows:
1) 1% PVP-K90 + 1% PEG-400
2) 1% PVP-K90 + 0.2% PF68
3) 0.5% PVP-K90 + 1% PEG-400
4) 0.5% PVP-K90 + 0.2% PF68
5) 0.25% PVP-K90 + 1% PEG-400
6) 0.25% PVP-K90 + 0.2% PF68
7) 0.2% PF68
8) 1.5% PK90
9) 1% PEG-400
10) 0.1% HEC
11) 0.25% PVA
12) 0.2% PF127
13) WFI

Each reconstitution solvent was formulated according to the above preparations, filtered, filled, stoppered, and capped for later use. A disposable nasal administration device was used to transfer each of the above reconstitution solvents to reconstitute the lyophilized powder and then perform spraying, the samples after spraying were collected. The viscosity, osmolality, particle size, PDI, encapsulation efficiency, total mRNA content, and spray pattern of the samples after spraying were determined. At the same time, the collected samples were tested for their HGF expression in HEK293 (CCTCC) cells. The results are shown in Table 26 below.

**Table 26. Reconstitution solvent components screening (biological activity)**

| Reconstitution solvent formula | Viscosity (mPa·s) | Osmolality (mOsm) (LNP) | Spray particle size (3 cm air gap distance, D50, µm) | Spray area (mm^2) | HGF expression (ng/mL) |
|---|---|---|---|---|---|
| 1 | 3.72 | 347 | 177.3 | 86.4 | 117.2 |
| 2 | 3.83 | 334 | 193.3 | 72.2 | 201.5 |
| 3 | 2.19 | 354 | 68.84 | 549.3 | 259.9 |
| 4 | 2.05 | 312 | 60.70 | 500.3 | 308.1 |
| 5 | 1.73 | 346 | 148.2 | 83.8 | 287.3 |
| 6 | 1.78 | 311 | 39.24 | 756.0 | 278.1 |
| 7 | 1.24 | 321 | 27.08 | 1766.7 | 279.2 |
| 8 | 5.97 | 334 | 337.2 | 13.9 | 378.2 |
| 9 | 1.02 | 348 | 25.13 | 1453.0 | 228.9 |
| 10 | 1.25 | 318 | 26.33 | 1615.3 | 227.7 |
| 11 | 1.16 | 316 | 30.81 | 1255.0 | 320.0 |
| 12 | 1.12 | 324 | 28.43 | 1676.3 | 0.1 |
| 13 | 1..42 | 295 | 24.51 | 1812.7 | 220.2 |

It can be seen from the results in Table 26 that after formula 12 (0.2% PF127) was reconstituted and sprayed, the HGF expression of the sample was almost 0. This showed that this reconstitution solvent might have a great destructive effect on LNP, causing it to lose its biological activity after reconstitution and spraying. After reconstitution and spraying, formula 1 (1% PVP-K90 + 1% PEG-400), formula 2 (1% PVP-K90 + 0.2% PF68), formula 5 (0.25% PVP-K90 + 1% PEG-400), and formula 8 (1.5% PVP-K90) had a large spray particle size, a small spray area, and a high viscosity. The reconstitution solvents of the remaining preparations all exhibited a high HGF expression level, moderate viscosity, a large spray area, and a small spray particle size.

### Sequence Listing

>B1 (ACTG1 3'UTR) 
CCGACTACCTCATGAAGATCCTCACTGAGCGAGGCTACAGCTTCACCACCACGGCCGAG
CGGGAAATCGTGCGCGACATCAAGGAGAAGCTGTGCTACGTCGCCCTGGACTTCGAGC
AGGAGATGGCCACCGCCGCATCCTCCTCTTCTCTGGAGAAGAGCTACGAGCTGCCCGAT
GGCCAGGTCATCACCATTGGCAATGAGCGGTTCCGGTGTCCGGAGGCGCTGTTCCAGCC
TTCCTTCCTGGGTATGGAATCTTGCGGCATCCACGAGACCACCTTCAACTCCATCATGA
AGTGTGACGTGGACATCCGCAAAGACCTGTACGCCAACACGGTGCTGTCGGGCGGCAC
CACCATGTACCCGGGCATTGCCGACAGGATGCAGAAGGAGATCACCGCCCTGGCGCCC
AGCACCATGAAGATCAAGATCATCGCACCCCCAGAGCGCAAGTACTCGGTGTGGATCG
GTGGCTCCATCCTGGCCTCACTGTCCACCTTCCAGCAGATGTGGATTAG
SEQ ID NO:1
>B2 (ATP6V0B 3'UTR) 
ATGATATGTGTGGGTGGGGCCGTGCCTCACTTTTATTTATTGCTGGTTTTCCTGGGACAG
CTGGAGCTGTGTCCCTTAGCCTTTCAGAGGCTTGGTGTTCAGGGCCCTCCCTGCACTCCC
CTCTTGCTGCGTGTTGATTTGGAGGCACTGCAGTCCAGGCCGAGTCCTCAGTGCGGGGA
GCAGGCTGCTGCTGCTGACTCTGTGCAGCTGCGCACCTGTGTCCCCCACCTCCACCCTCA
ACCCATCTTCCTAGTGTTTGTGAAATAAACTTGGTATTTGTCT
SEQ ID NO:2
>B3 (ATP6V0B 3'UTR) 
GTTGAAGAACTTCTCTCTACCGAGCAGCCCGTAACCCCCTTTTTCTCCCTCACTGCTGCA
GGAGTCTGCTACACCATTTTTGATTTGGGCTTCCGCTTTGATGTGGCATGGTTCCTGACG
GAGACTTCGCCCTTCATGTGGTCCAACCTGGGCATTGGCCTAGCTATCTCCCTGTCTGTG
GTTGGGGCAGCCTGGGGCATCTATATTACCGGCTCCTCCATCATTGGTGGAGGAGTGAA
GGCCCCCAGGATCAAGACCAAGAACCTGGTCAGCATCATCTTCTGTGAGGCTGTGGCCA
TCTACGGCAT

SEQ ID NO:3
>B4 (ATP6V0E1 3'UTR) 
GGAAGAAGACATGCTCTACAGTGCTCAGTCTTTGAGGTGACTATGCTTGTGACCTTTCTT
ATCAGTATGGTCAGGCAGTCAGAGTTTGCCTTGACTTTTTCTCACATTTTATGGCCATTT
TAACACGTGGCAAGGGCAAGCTGCTGAATGCAAACACGATCTGAAGAGCAGAAATAAA
ATTGGAACTTAAGCCTTAGCTTACCTAAATCTCATTAAGATTCTTTTT
SEQ ID NO:4
>B5 (ATP6V0E1 3'UTR) 
ATGCAAAATCACCTCCAAACCAGACCACTTTTCTTGACTTGCCTGTTTTGGCCATTAGCT
GCCTTAAACGTTAACAGCACATTTGAATGCCTTATTCTACAATGCAGCGTGTTTTCCTTT
GCCTTTTTTGCACTTTGGTGAATTACGTGCCTCCATAACCTGAACTGTGCCGACTCCACA
AAACGATTATGTACTCTTCTGAGATAGAAGATGCTGTTCTTCTGAGAGATACGTTACTCT
CTCCTTGGAATCTGTGGATTTGAAGATGGCTCCTGCCTTCTCACGTGGGAATCAGTGAA
GTGTTTAGAAACTGCTGCAAGACAAACAAGACTCCAGTGGGGTGGTCAGTAGGAGAGC
ACGTTCAGAGGGAAGAGCCATCTCAACAGAATCGCACCAAACTATACTTTCAGGATGA
ATTTCTTCTTTCTGCCATCTTTTGGAATAAATATTTTCCTCCTTTC
SEQ ID NO:5
>B6 (CFL1 3'UTR) 
GCCCCTTCTGGCCCCCTGCCTGGAGCATCTGGCAGCCCCACACCTGCCCTTGGGGGTTG
CAGGCTGCCCCCTTCCTGCCAGACCGGAGGGGCTGGGGGGATCCCAGCAGGGGGAGGG
CAATCCCTTCACCCCAGTTGCCAAACAGACCCCCCACCCCCTGGATTTTCCTTCTCCCTC
CATCCCTTGACGGTTCTGGCCTTCCCAAACTGCTTTTGATCTTTTGATTCCTCTTGGGCTG
AAGCAGACCAAGTTCCCCCCAGGCACCCCAGTTGTGGGGGAGCCTGTATTTTTTTTAAC
AACATCCCCATTCCCCACCTGGTCCTCCCCCTTCCCATGCTGCCAACTTCTAACCGCAAT
AGTGACTCTGTGCTTGTCTGTTTAGTTCTGTGTATAAATGGAATGTTGTGGAGATGACCC
CTCCCTGTGCCGGCTGGTTCCTCTCCCTTTTCCCCTGGTCACGGCTACTCATGGAAGCAG
GACCAGTAAGGGACCTTCGATT
SEQ ID NO:6
>B7 (CFL1 3'UTR) 
GCCCCTTCTGGCCCCCTGCCTGGAGCATCTGGCAGCCCCACACCTGCCCTTGGGGGTTG
CAGGCTGCCCCCTTCCTGCCAGACCGGAGGGGCTGGGGGGATCCCAGCAGGGGGAGGG
CAATCCCTTCACCCCAGTTGCCAAACAGACCCCCCACCCCCTGGATTTTCCTTCTCCCTC
CATCCCTTGACGGTTCTGGCCTTCCCAAACTGCTTTTGATCTTTTGATTCCTCTTGGGCTG
AAGCAGACCAAGTTCCCCCCAGGCACCCCAGTTGTGGGGGAGCC
SEQ ID NO:7
>B8 (CFL1 3'UTR) 
GCCCCTTCTGGCCCCCTGCCTGGAGCATCTGGCAGCCCCACACCTGCCCTTGGGGGTTG
CAGGCTGCCCCCTTCCTGCCAGACCGGAGGGGCTGGGGGGATCCCAGCAGGGGGAGGG
CAATCCCTTCACCCCAGTTGCCAAACAGACCCCCCACCCCCTGGATTTTCCTTCTCCCTC
CATCCCTTGACGGTTCTGGCCTTCCCAAACTGCTTTTGATCTTTTGATTCCTCTTGGGCTG
AAGCAGACCAAGTTCCCCCCAGGCACCCCAGTTGTGGGGGAGCCTGTATTTTTTTTAAC
AACATCCCCATTCCCCACCTGGTCCTCCCCCTTCCCATGCTGCCAACTTCTAACCGCAAT
AGTG
SEQ ID NO:8
>B9 (COX411 3'UTR) 
GAGATGCTGGCCTGCGCCTGCACCTGCGCCTGGCTCTGTCACCGCCATGCAACTCCATG
CCTATTTACTGGAAACCTGTTATGCCAAACAGTTGTACCACTGCTAATAAATGACCAGT
TTACCTGAAA
SEQ ID NO:9
>B10 (COX4I1 3'UTR) AGGGACCCACAGGCGCGCCCAGCAGCTCTCGGAAGCGTTGTATCGCATTAAGTTCAAG 
GAGAGCTTTGCTGAGATGAACAGGGGCTCGAACGAGTGGAAGACGGTTGTGGGCGGTG
CCATGTTCTTCATCGGTTTCACCGCGCTCGTTATCATGTGGCAGAAGCACTATGGGCTTA
GCCTCCAAGTGGGACTACGAAAAGAACGAGTGGAAGAAGTGAGAGATGCTGGCCTGCG
CCTGCACCTGCGCCTGGCTCTGTCACCGCCATGCAACTCCATGCCTATTTACTGGAAACC
TGTTATGCCAAACAGTTGTACCACT
SEQ ID NO:10
>B11 (COX4I1 3'UTR) 
AGAGGACCTTCTGCTTTTAGCTTATTTTGTTGCTAACTTTTTACAAACAAAGGGCTAATT
TTAAAATGTCAGTGTTGTCAGTGGTCAGAAACCGTGTGTTTGAGCGGGTGTTGAGTGGC
AGGTGGCTCTGCTGACCTGGTGGCTGGTGTGTCGGGAGGATTTAACCTGTGTGAGGGAT
TGGCCTAGAAACAACCTGTTGAGATAGTCTTGCCCCATAACCTGTCTCACACCGTAGTG
TACGGCCCCCTCCCGCAAAGCTTTGACAAAGAGTGGGTGGCCAAGCAGACCAAGAGGA
TGCTGGAC
SEQ ID NO:11
>B12 (CTSB 3'UTR) 
TCTGCCGTGGGCCTGTCGTGCCAGTCCTGGGGGCGAGATCGGGAGAATCACGTCCTGGA
ACTGCATGTTCTTGCGACTCTTGGGACTTCATCTTAACTTCTCGCTGCCCCAGCCATGTT
TTCAACCATGGCATCCCTCCCCCAATTAGTTCCCTGTCATCCTCGTCAACCTTCTCTGTA
AGTGCCTGGTAAGCTTGCCCTTGCTTAAGAACTCAAAACATAGCTGTGCTCTA
SEQ ID NO:12
>B13 (FAM166A 3'UTR) 
AAATGCTTTAATGGTGGTGTCTGTACAGCATGGATGTGGACAGGGAAAACAGCAAGTG
CACACAGTGGGACAGGCCACAGGACAGGCTGGGAGTGAATAAAGAGTTCACACTGCT
SEQ ID NO:13
>B14 (NDUFB9 3'UTR) 
TATCTAAGTGTATCAATTTAACATTTATAATCACCTTACAAGTAAGTTAAAGTGCATTCC
CTGGTCGTTTTGGCTTTGTCTTTGATTTTGCTTCAGATCTCCTTCTCTTTAGTCCTTTGTCT
GACTTTTTTGCCTTCTTGCTATACTTTCCCTCAACCTGCCTCTCCACTATTTTCTTAGGCA
TTTTTCAGTCATG
SEQ ID NO:14
>A1 (ACTG1 5'UTR)
   CTCTCGCACTCTGTTCTTCCGCCGCTCCGCCGTCGCGTTTCTCTGCCGGTCGCA SEQ ID NO:15
>A2 (ATP6V0B 5'UTR) 
GGGCGGGCGGACAGACTGCGGGACGGACGGTGGACGCTGGGACGCGTTTGTAGCTCCG
GCCCCGCCGTTCCGACCCCCGCCGCCGTCGCCGCCATGACGGGGCTAGCACTGCTCTAC
TCCGGGGTCTTCGTGGCCTTCTGGGCCTGCGCGCTGGCCGTGGGTTCCTGACGGAGACT
TCGCCCTTC
SEQ ID NO:16
>A3 (ATP6V0B 5'UTR) 
AGACTGCGGACGGACGGTGGACGCTGGACGCGTTTGTAGCTCCGGCCCCGCCGTTCC
GACCCCCGCCGCCGTCGCCGCC
SEQ ID NO:17
>A4 (ATP6V0E1 5'UTR)
   GGGGTAGGGGTTGGCGCTCAGGCGGCGACC SEQ ID NO:18
>A5 (ATP6V0E1 5'UTR) 
GTCACGCGGTCAGCTATTGACACTTCCTGGTGGGATCCGAGTGAGGCGACGGGGTAGG
GGTTGGCGCTCAGGCGGCGACC
SEQ ID NO:19
>A6 **(CFL1** 5'UTR) 
GGCCGGCGGGAAGACTCCGTTACCCAGCGAGCGAGGCGGCGGCGCAGGGCCAGCGGA
CTCCATTTCCCGTCGGCTCGCGGTGGGAGCGCCGGAAGCCCGCCCCACCCCTCATTGTG
CGGCTCCTACTAAACGGAAGGGGCCGGGAGAGGCCGCGTTCAGTCGGGTCCCGGCAGC
GGCTGCAGCGCTCTCGTCTTCTGCGGCTCTCGGTGCCCTCTCCTTTTCGTTTCCGGAAAC
SEQ ID NO:20
>A7 (CFL1 5'UTR) 
CTCCTGGCTCGGGCACCCCGAAGCACGACGCAGAGTAGGAAGAGTTTAAAGACCTCGA
GGCCTCTGGGCACTTGAGTTTGGCCTCCGGTGTGGCTGTCTCTGATGGTGTCATCAAGGT
GTTCAACGAC
SEQ ID NO:21
>A8 (CFL1 5'UTR) 
ACGTGGCCGGCTTCTTCTGCAGTTCCGGGGAGTTTGGGGGACCAGATTTACCTTGGATT
GCCCCTCCCTCTCCTGGCTCGGGCACCCCGAAGCACGACGCAGAGCCTCCGGTGTGGCT
GTCTCTGATGGTGTCATCAAGGTGTTCAACGAC
SEQ ID NO:22
>A9 (COX4I1 5'UTR) 
GCGGCCTTGCTCTCTTCCGGTCGCGGGACACCGGGTGTAGAGGGCGGTCGCGGCGGGC
AGTGGCGGCAGA
SEQ ID NO:23
>A10 (COX4I1 5'UTR)
   GGGACACCGGGTGTAGAGGGCGGTCGCGGCGGGCAGTGGCGGCAGA SEQ ID NO:24
>A11 (COX4I1 5'UTR) 
CGCGGCCTTGCTCTCTTCCGGTCGCGGGACACCGGGTGTAGAGGGCGGTCGCGGCGGGC
AGTGGCGGCAGA
SEQ ID NO:25
>A12 (CTSB 5'UTR) 
GGCCGGGGCTGGCCCAGGCTACGGCGGCTGCAGGGCTCCGGCAACCGCTCCGGCAACG
CCAACCGCTCCGCTGCGCGCAGGCTGGGCTGCAGGCTCTCGGCTGCAGCGCTGGGTGGA
TCTAGGATCCGGCTTCCAAC
SEQ ID NO:26
>A13 (FAM166A 5'UTR)
   AAGGGAGCTGGATGCCGGGAGGGACTGGAGCCAGCAAGGCCAGAGTGAAAGCAAA SEQ ID NO:27
>A14 (NDUFB9 5'UTR)
   GCAGCAGGCGTGCAGTTTCCCGGCTCTCCGCGCGGCCGGGGAAGGTCAGCGCCGTA SEQ ID NO:28
>A15 (CHCHD10 5'UTR) 
GGCATTTGTCCCCGCGACAGCACCGCTGCCGCCGTCTCTAAGGTCGCCCGGGTCCCACC
GCCGCCACC
SEQ ID NO:29
>A16 (CHCHD10 5'UTR)
   CCGCTGCCGCCGTCTCTAAGGTCGCCCGGGTCCCACCGCCGCCACC SEQ ID NO:30
>A17 (SLC38A2 5'UTR)
   ATGTCTTTTTTGTGTGTTTGTTTTCATGGTATTCCTATGAA SEQ ID NO:31
>A18 (NDUFA11 5'UTR)
   GCTTCCCGAGCTGGCGGGGTCCGTGGTGCGGGATCGAGATTGCGGGCT SEQ ID NO:32
>A19 (NDUFV3 5'UTR) 
GGCGGCTGTTCAGGCGCGGGTGCGCGCGCAGCTGCTGTGGCCCTGCTTGGTGCGCCCGC
TGTCACCGCC
SEQ ID NO:33
>A20 (PRDX5 5'UTR) 
CGCGCCTGCGCAGTGGAGGCGGCCCAGGCCCGCCTTCCGCAGGGTGTCGCCGCTGTGCC
GCTAGCGGTGCCCCGCCTGCTGCGGTGGCACCAGCCAGGAGGCGGAGTGGAAGTGGCC
GTGGGGCGGGT
SEQ ID NO:34
>A21 (GUK1 5'UTR)
   CAGCAGATGGGGACTAGAGGCCGCACTGCTATCCACAGCCTCTCTTCTCACCCCCAGGC SEQ ID NO:35
>A22 (GUK1 5'UTR) 
GCTGGCCGGGCTGGCTGCGGCCGCCCTGGGCCGGGCCCCACCGGACGCCTCTCTTCTCA
CCCCCAGGC
SEQ ID NO:36
>A23 (GUK1 5'UTR) 
AGAGGTGGCCCCGGATGCTGCGGCGCCCGCTGGCCGGGCTGGCTGCGGCCGCCCTGGG
CCGGGCCCCACCGGACGCCTCTCTTCTCACCCCCAGGC
SEQ ID NO:37
>A24 (IAH1 5'UTR)
   TGGCTGGCGGCCCCGCCCCGCCCCGCCCGGCTGCTCC SEQ ID NO:38
>A25 (ABHD16A 5'UTR) 
GGAGGGCGGGGCCGGCAGGGGGACCTGCTGCTGGAAGAGCAGCGGCCCGAGCCGGGG
CC
SEQ ID NO:39
>A26 (SLC25A39 5'UTR) 
TTTCGGCGTCGGCCTAGGTGCGCTGCGAGCGCGCGCGGACCGCGCACAGGCGGCGGAG
CCGGTATGGGCCCGCCTGACCCTGGGCGCCGCGCCGCACGAGCACCAGCCTAGAGCCA
GGACTGAAGCTTCAAG
SEQ ID NO:40
>A27 (ATPIF1 5'UTR)
   CGAGAGACTGCTTGCTGCGGCAGAGACGCCAGAGGTGCAGCTCCAGCAGCA SEQ ID NO:41
>A28 (ANAPC11 5'UTR) 
CGGAGTTTCGTCATGTTGGCCAGGCCCATTTGAGATCTTTGAAGATATCCTCAACGTGA
GGCTCTGCTGCC
SEQ ID NO:42
>A29 (ANAPC11 5'UTR)
   GGGCGCGGCTTCGGCGGGCGGCAGCCGCTGCAGACGAGCTGCGGGCTCTGCTGCC SEQ ID NO:43
>A30 (CCDC12 5'UTR)
   GCCTGCGCGATGCAAGACGGGAGAAAAGGAGGGGCGTACGCGGGCAAG SEQ ID NO:44
>A31 (MRPL14 5'UTR) 
ATCGCGTCCGCCGGGCTGGGCCTGGCGCGCAGGCGCTAGGAAGAGGCCGCGTGGGGCG
AAGGCGGCGCTTGGCTGGTGGGGCCCGCGGCGGGATTTTCCCGGGCGGCGAGAGCGGA
TCTATCTTGGGATCCC
SEQ ID NO:45
>A32 (APOA1BP 5'UTR)
   GCCGGGGGCGCGCGCTCTGCGAGCTGG SEQ ID NO:46
>A33 (APOA1BP 5'UTR)
   GCCGGGGCCGGGCCGGGCCGGGGGCGCGCGCTCTGCGAGCTGG SEQ ID NO:47
> Mod. 5'UTR
   GGGAAATAAGAGAGAAAAGAAGAGTAAGAAGAAATATAAGAGCCACC SEQ ID NO:48
> α-globin 3'UTR 
TGATAATAGGCTGGAGCCTCGGTGGCCATGCTTCTTGCCCCTTGGGCC
TCCCCCCAGCCCCTCCTCCCCTTCCTGCACCCGTACCCCCGTGGTCTT
TGAATAAAGTCTGAGTGGGCGGC
SEQ ID NO:49
> α-1 globin 5'UTR
   AGACGAACUAGUAUUCUUCUGGUCCCCACAGACUCAGAGAGAACCCGCCACC SEQ ID NO:50
> haplogroup U8b1b1 mitochondrion 3'UTR 
CTCGAGCTGGTACTGCATGCACGCAATGCTAGCTGCCCCTTTCCCGTCCTGGGTACCCC
GAGTCTCCCCCGACCTCGGGTCCCAGGTATGCTCCCACCTCCACCTGCCCCACTCACCA
CCTCTGCTAGTTCCAGACACCTGCTATACTAATCGCTAGCCGCGTCGCTCCCCAGGGTTG
GTCAATTTCGTGCCAGCCACACCGAGACCTGGTCCAGAGCCCAAGCACGCCACACCCCC
ACGGGAAACAGCAGTGATTAACCTTTAGCAATAAACGAAAGTTTAACTAACAGCAATG
CA GCTCAAAACGCTTAGCCTAG
SEQ ID NO:51
> A30L70 PolyA 
AAAAAAAAAAAAAAAAAAAAAAAAAAAAAAAGCATATGACTAAAAAAAAAAAAAA
AAAAAAAAAAAAAAAAAAAAAAAAAAAAAAAAAAAAAAAAAAAAAAAAAAAAAAAA
SEQ ID NO:52
> 5'UTR-Fluc-α globin 3'UTR-120A (Mod.-120A) 
GGGAAATAAGAGAGAAAAGAAGAGTAAGAAGAAATATAAGAGCCACCATGGAAGACG
CCAAAAACATAAAGAAAGGCCCGGCGCCATTCTATCCGCTGGAAGATGGAACCGCTGG
AGAGCAACTGCATAAGGCTATGAAGAGATACGCCCTGGTTCCTGGAACAATTGCTTTTA
CAGATGCACATATCGAGGTGGACATCACTTACGCTGAGTACTTCGAAATGTCCGTTCGG
TTGGCAGAAGCTATGAAACGATATGGGCTGAATACAAATCACAGAATCGTCGTATGCA
GTGAAAACTCTCTTCAATTCTTTATGCCGGTGTTGGGCGCGTTATTTATCGGAGTTGCAG
TTGCGCCCGCGAACGACATTTATAATGAACGTGAATTGCTCAACAGTATGGGCATTTCG
CAGCCTACCGTGGTGTTCGTTTCCAAAAAGGGGTTGCAAAAAATTTTGAACGTGCAAAA
 
AAAGCTCCCAATCATCCAAAAAATTATTATCATGGATTCTAAAACGGATTACCAGGGAT
TTCAGTCGATGTACACGTTCGTCACATCTCATCTACCTCCCGGTTTTAATGAATACGATT
TTGTGCCAGAGTCCTTCGATAGGGACAAGACAATTGCACTGATCATGAACTCCTCTGGA
TCTACTGGTCTGCCTAAAGGTGTCGCTCTGCCTCATAGAACTGCCTGCGTGAGATTCTCG
CATGCCAGAGATCCTATTTTTGGCAATCAAATCATTCCGGATACTGCGATTTTAAGTGTT
GTTCCATTCCATCACGGTTTTGGAATGTTTACTACACTCGGATATTTGATATGTGGATTT
CGAGTCGTCTTAATGTATAGATTTGAAGAAGAGCTGTTTCTGAGGAGCCTTCAGGATTA
CAAGATTCAAAGTGCGCTGCTGGTGCCAACCCTATTCTCCTTCTTCGCCAAAAGCACTCT
GATTGACAAATACGATTTATCTAATTTACACGAAATTGCTTCTGGTGGCGCTCCCCTCTC
TAAGGAAGTCGGGGAAGCGGTTGCCAAGAGGTTCCATCTGCCAGGTATCAGGCAAGGA
TATGGGCTCACTGAGACTACATCAGCTATTCTGATTACACCCGAGGGGGATGATAAACC
GGGCGCGGTCGGTAAAGTTGTTCCATTTTTTGAAGCGAAGGTTGTGGATCTGGATACCG
GGAAAACGCTGGGCGTTAATCAAAGAGGCGAACTGTGTGTGAGAGGTCCTATGATTAT
GTCCGGTTATGTAAACAATCCGGAAGCGACCAACGCCTTGATTGACAAGGATGGATGG
CTACATTCTGGAGACATAGCTTACTGGGACGAAGACGAACACTTCTTCATCGTTGACCG
CCTGAAGTCTCTGATTAAGTACAAAGGCTATCAGGTGGCTCCCGCTGAATTGGAATCCA
TCTTGCTCCAACACCCCAACATCTTCGACGCAGGTGTCGCAGGTCTTCCCGACGATGAC
GCCGGTGAACTTCCCGCCGCCGTTGTTGTTTTGGAGCACGGAAAGACGATGACGGAAA
AAGAGATCGTGGATTACGTCGCCAGTCAAGTAACAACCGCGAAAAAGTTGCGCGGAGG
AGTTGTGTTTGTGGACGAAGTACCGAAAGGTCTTACCGGAAAACTCGACGCAAGAAAA
ATCAGAGAGATCCTCATAAAGGCCAAGAAGGGCGGAAAGATCGCCGTGGATTACAAGG
ATGACGACGATAAGTAAGGGCCCACCGGTTGATAATAGGCTGGAGCCTCGGTGGCCAT
GCTTCTTGCCCCTTGGGCCTCCCCCCAGCCCCTCCTCCCCTTCCTGCACCCGTACCCCCG
TGGTCTTTGAATAAAGTCTGAGTGGGCGGCCTCGAGCCGCGGAAAAAAAAAAAAAAA
AAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAATTATTGTTT
AAACATTTAAATTCTAGA
SEQ ID NO:53
> 5'UTR-Fluc-α globin 3'UTR-A30L70 (Mod.-A30L70) 
GGGAAATAAGAGAGAAAAGAAGAGTAAGAAGAAATATAAGAGCCACCATGGAAGACG
CCAAAAACATAAAGAAAGGCCCGGCGCCATTCTATCCGCTGGAAGATGGAACCGCTGG
AGAGCAACTGCATAAGGCTATGAAGAGATACGCCCTGGTTCCTGGAACAATTGCTTTTA
CAGATGCACATATCGAGGTGGACATCACTTACGCTGAGTACTTCGAAATGTCCGTTCGG
TTGGCAGAAGCTATGAAACGATATGGGCTGAATACAAATCACAGAATCGTCGTATGCA
GTGAAAACTCTCTTCAATTCTTTATGCCGGTGTTGGGCGCGTTATTTATCGGAGTTGCAG
TTGCGCCCGCGAACGACATTTATAATGAACGTGAATTGCTCAACAGTATGGGCATTTCG
CAGCCTACCGTGGTGTTCGTTTCCAAAAAGGGGTTGCAAAAAATTTTGAACGTGCAAAA
AAAGCTCCCAATCATCCAAAAAATTATTATCATGGATTCTAAAACGGATTACCAGGGAT
TTCAGTCGATGTACACGTTCGTCACATCTCATCTACCTCCCGGTTTTAATGAATACGATT
TTGTGCCAGAGTCCTTCGATAGGGACAAGACAATTGCACTGATCATGAACTCCTCTGGA
TCTACTGGTCTGCCTAAAGGTGTCGCTCTGCCTCATAGAACTGCCTGCGTGAGATTCTCG
CATGCCAGAGATCCTATTTTTGGCAATCAAATCATTCCGGATACTGCGATTTTAAGTGTT
GTTCCATTCCATCACGGTTTTGGAATGTTTACTACACTCGGATATTTGATATGTGGATTT
CGAGTCGTCTTAATGTATAGATTTGAAGAAGAGCTGTTTCTGAGGAGCCTTCAGGATTA
CAAGATTCAAAGTGCGCTGCTGGTGCCAACCCTATTCTCCTTCTTCGCCAAAAGCACTCT
GATTGACAAATACGATTTATCTAATTTACACGAAATTGCTTCTGGTGGCGCTCCCCTCTC
TAAGGAAGTCGGGGAAGCGGTTGCCAAGAGGTTCCATCTGCCAGGTATCAGGCAAGGA
TATGGGCTCACTGAGACTACATCAGCTATTCTGATTACACCCGAGGGGGATGATAAACC
GGGCGCGGTCGGTAAAGTTGTTCCATTTTTTGAAGCGAAGGTTGTGGATCTGGATACCG
GGAAAACGCTGGGCGTTAATCAAAGAGGCGAACTGTGTGTGAGAGGTCCTATGATTAT
GTCCGGTTATGTAAACAATCCGGAAGCGACCAACGCCTTGATTGACAAGGATGGATGG
CTACATTCTGGAGACATAGCTTACTGGGACGAAGACGAACACTTCTTCATCGTTGACCG
CCTGAAGTCTCTGATTAAGTACAAAGGCTATCAGGTGGCTCCCGCTGAATTGGAATCCA
TCTTGCTCCAACACCCCAACATCTTCGACGCAGGTGTCGCAGGTCTTCCCGACGATGAC
GCCGGTGAACTTCCCGCCGCCGTTGTTGTTTTGGAGCACGGAAAGACGATGACGGAAA
AAGAGATCGTGGATTACGTCGCCAGTCAAGTAACAACCGCGAAAAAGTTGCGCGGAGG
AGTTGTGTTTGTGGACGAAGTACCGAAAGGTCTTACCGGAAAACTCGACGCAAGAAAA
 
ATCAGAGAGATCCTCATAAAGGCCAAGAAGGGCGGAAAGATCGCCGTGGATTACAAGG
ATGACGACGATAAGTAAGGGCCCACCGGTTGATAATAGGCTGGAGCCTCGGTGGCCAT
GCTTCTTGCCCCTTGGGCCTCCCCCCAGCCCCTCCTCCCCTTCCTGCACCCGTACCCCCG
TGGTCTTTGAATAAAGTCTGAGTGGGCGGCCTCGAGCCGCGGAAAAAAAAAAAAAAAA
AAAAAAAAAAAAAAGCATATGACTAAAAAAAAAAAAAAAAAAAAAAAAAAAAAAAAA
AAAAAAAAAAAAAAAAAAAAAAAAAAAAAAAAAAAAAAAAAATTATTGTTTAAACATTTAA
ATTCTAGA
SEQ ID NO:54
> α-1-globin 5'UTR-Fluc- haplogroup U8b1b1 mitochondrion 3'UTR-A30L70 (BioN.) 
AGACGAACUAGUAUUCUUCUGGUCCCCACAGACUCAGAGAGAACCCGCCACCATGGA
AGACGCCAAAAACATAAAGAAAGGCCCGGCGCCATTCTATCCGCTGGAAGATGGAACC
GCTGGAGAGCAACTGCATAAGGCTATGAAGAGATACGCCCTGGTTCCTGGAACAATTG
CTTTTACAGATGCACATATCGAGGTGGACATCACTTACGCTGAGTACTTCGAAATGTCC
GTTCGGTTGGCAGAAGCTATGAAACGATATGGGCTGAATACAAATCACAGAATCGTCG
TATGCAGTGAAAACTCTCTTCAATTCTTTATGCCGGTGTTGGGCGCGTTATTTATCGGAG
TTGCAGTTGCGCCCGCGAACGACATTTATAATGAACGTGAATTGCTCAACAGTATGGGC
ATTTCGCAGCCTACCGTGGTGTTCGTTTCCAAAAAGGGGTTGCAAAAAATTTTGAACGT
GCAAAAAAAGCTCCCAATCATCCAAAAAATTATTATCATGGATTCTAAAACGGATTACC
AGGGATTTCAGTCGATGTACACGTTCGTCACATCTCATCTACCTCCCGGTTTTAATGAAT
ACGATTTTGTGCCAGAGTCCTTCGATAGGGACAAGACAATTGCACTGATCATGAACTCC
TCTGGATCTACTGGTCTGCCTAAAGGTGTCGCTCTGCCTCATAGAACTGCCTGCGTGAG
ATTCTCGCATGCCAGAGATCCTATTTTTGGCAATCAAATCATTCCGGATACTGCGATTTT
AAGTGTTGTTCCATTCCATCACGGTTTTGGAATGTTTACTACACTCGGATATTTGATATG
TGGATTTCGAGTCGTCTTAATGTATAGATTTGAAGAAGAGCTGTTTCTGAGGAGCCTTC
AGGATTACAAGATTCAAAGTGCGCTGCTGGTGCCAACCCTATTCTCCTTCTTCGCCAAA
AGCACTCTGATTGACAAATACGATTTATCTAATTTACACGAAATTGCTTCTGGTGGCGCT
CCCCTCTCTAAGGAAGTCGGGGAAGCGGTTGCCAAGAGGTTCCATCTGCCAGGTATCAG
GCAAGGATATGGGCTCACTGAGACTACATCAGCTATTCTGATTACACCCGAGGGGGATG
ATAAACCGGGCGCGGTCGGTAAAGTTGTTCCATTTTTTGAAGCGAAGGTTGTGGATCTG
GATACCGGGAAAACGCTGGGCGTTAATCAAAGAGGCGAACTGTGTGTGAGAGGTCCTA
TGATTATGTCCGGTTATGTAAACAATCCGGAAGCGACCAACGCCTTGATTGACAAGGAT
GGATGGCTACATTCTGGAGACATAGCTTACTGGGACGAAGACGAACACTTCTTCATCGT
TGACCGCCTGAAGTCTCTGATTAAGTACAAAGGCTATCAGGTGGCTCCCGCTGAATTGG
AATCCATCTTGCTCCAACACCCCAACATCTTCGACGCAGGTGTCGCAGGTCTTCCCGAC
GATGACGCCGGTGAACTTCCCGCCGCCGTTGTTGTTTTGGAGCACGGAAAGACGATGAC
GGAAAAAGAGATCGTGGATTACGTCGCCAGTCAAGTAACAACCGCGAAAAAGTTGCGC
GGAGGAGTTGTGTTTGTGGACGAAGTACCGAAAGGTCTTACCGGAAAACTCGACGCAA
GAAAAATCAGAGAGATCCTCATAAAGGCCAAGAAGGGCGGAAAGATCGCCGTGGATTA
CAAGGATGACGACGATAAGTAAGGGCCCACCGGCTCGAGCTGGTACTGCATGCACGCA
ATGCTAGCTGCCCCTTTCCCGTCCTGGGTACCCCGAGTCTCCCCCGACCTCGGGTCCCAG
GTATGCTCCCACCTCCACCTGCCCCACTCACCACCTCTGCTAGTTCCAGACACCTGCTAT
ACTAATCGCTAGCCGCGTCGCTCCCCAGGGTTGGTCAATTTCGTGCCAGCCACACCGAG
ACCTGGTCCAGAGCCCAAGCACGCCACACCCCCACGGGAAACAGCAGTGATTAACCTT
TAGCAATAAACGAAAGTTTAACTAACAGCAATGCAGCTCAAAACGCTTAGCCTAGCTC
GAGCCGCGGAAAAAAAAAAAAAAAAAAAAAAAAAAAAAAGCATATGACTAAAAAAA
AAAAAAAAAAAAAAAAAAAAAAAAAAAAAAAAAAAAAAAAAAAAAAAAAAAAAAAAAAAA
AAAAAAATTATTGTTTAAACATTTAAATTCTAGA
SEQ ID NO:55
>5'UTR-Fluc- ACTG1 3'UTR-120A (B1) 
GGGAAATAAGAGAGAAAAGAAGAGTAAGAAGAAATATAAGAGCCACCATGGAAGACG
CCAAAAACATAAAGAAAGGCCCGGCGCCATTCTATCCGCTGGAAGATGGAACCGCTGG
AGAGCAACTGCATAAGGCTATGAAGAGATACGCCCTGGTTCCTGGAACAATTGCTTTTA
CAGATGCACATATCGAGGTGGACATCACTTACGCTGAGTACTTCGAAATGTCCGTTCGG
TTGGCAGAAGCTATGAAACGATATGGGCTGAATACAAATCACAGAATCGTCGTATGCA
GTGAAAACTCTCTTCAATTCTTTATGCCGGTGTTGGGCGCGTTATTTATCGGAGTTGCAG
TTGCGCCCGCGAACGACATTTATAATGAACGTGAATTGCTCAACAGTATGGGCATTTCG
 
CAGCCTACCGTGGTGTTCGTTTCCAAAAAGGGGTTGCAAAAAATTTTGAACGTGCAAAA
AAAGCTCCCAATCATCCAAAAAATTATTATCATGGATTCTAAAACGGATTACCAGGGAT
TTCAGTCGATGTACACGTTCGTCACATCTCATCTACCTCCCGGTTTTAATGAATACGATT
TTGTGCCAGAGTCCTTCGATAGGGACAAGACAATTGCACTGATCATGAACTCCTCTGGA
TCTACTGGTCTGCCTAAAGGTGTCGCTCTGCCTCATAGAACTGCCTGCGTGAGATTCTCG
CATGCCAGAGATCCTATTTTTGGCAATCAAATCATTCCGGATACTGCGATTTTAAGTGTT
GTTCCATTCCATCACGGTTTTGGAATGTTTACTACACTCGGATATTTGATATGTGGATTT
CGAGTCGTCTTAATGTATAGATTTGAAGAAGAGCTGTTTCTGAGGAGCCTTCAGGATTA
CAAGATTCAAAGTGCGCTGCTGGTGCCAACCCTATTCTCCTTCTTCGCCAAAAGCACTCT
GATTGACAAATACGATTTATCTAATTTACACGAAATTGCTTCTGGTGGCGCTCCCCTCTC
TAAGGAAGTCGGGGAAGCGGTTGCCAAGAGGTTCCATCTGCCAGGTATCAGGCAAGGA
TATGGGCTCACTGAGACTACATCAGCTATTCTGATTACACCCGAGGGGGATGATAAACC
GGGCGCGGTCGGTAAAGTTGTTCCATTTTTTGAAGCGAAGGTTGTGGATCTGGATACCG
GGAAAACGCTGGGCGTTAATCAAAGAGGCGAACTGTGTGTGAGAGGTCCTATGATTAT
GTCCGGTTATGTAAACAATCCGGAAGCGACCAACGCCTTGATTGACAAGGATGGATGG
CTACATTCTGGAGACATAGCTTACTGGGACGAAGACGAACACTTCTTCATCGTTGACCG
CCTGAAGTCTCTGATTAAGTACAAAGGCTATCAGGTGGCTCCCGCTGAATTGGAATCCA
TCTTGCTCCAACACCCCAACATCTTCGACGCAGGTGTCGCAGGTCTTCCCGACGATGAC
GCCGGTGAACTTCCCGCCGCCGTTGTTGTTTTGGAGCACGGAAAGACGATGACGGAAA
AAGAGATCGTGGATTACGTCGCCAGTCAAGTAACAACCGCGAAAAAGTTGCGCGGAGG
AGTTGTGTTTGTGGACGAAGTACCGAAAGGTCTTACCGGAAAACTCGACGCAAGAAAA
ATCAGAGAGATCCTCATAAAGGCCAAGAAGGGCGGAAAGATCGCCGTGGATTACAAGG
ATGACGACGATAAGTAAGGGCCCACCGGCCGACTACCTCATGAAGATCCTCACTGAGC
GAGGCTACAGCTTCACCACCACGGCCGAGCGGGAAATCGTGCGCGACATCAAGGAGAA
GCTGTGCTACGTCGCCCTGGACTTCGAGCAGGAGATGGCCACCGCCGCATCCTCCTCTT
CTCTGGAGAAGAGCTACGAGCTGCCCGATGGCCAGGTCATCACCATTGGCAATGAGCG
GTTCCGGTGTCCGGAGGCGCTGTTCCAGCCTTCCTTCCTGGGTATGGAATCTTGCGGCAT
CCACGAGACCACCTTCAACTCCATCATGAAGTGTGACGTGGACATCCGCAAAGACCTGT
ACGCCAACACGGTGCTGTCGGGCGGCACCACCATGTACCCGGGCATTGCCGACAGGAT
GCAGAAGGAGATCACCGCCCTGGCGCCCAGCACCATGAAGATCAAGATCATCGCACCC
CCAGAGCGCAAGTACTCGGTGTGGATCGGTGGCTCCATCCTGGCCTCACTGTCCACCTT
CCAGCAGATGTGGATTAGCTCGAGCCGCGGAAAAAAAAAAAAAAAAAAAAAAAAAAAA
AAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAATTATTGTTTAAACATTTAAA
TTCTAGA
SEQ ID NO:56
> ACTG1 5'UTR-Fluc-α globin 3'UTR-120A (A1) 
CTCTCGCACTCTGTTCTTCCGCCGCTCCGCCGTCGCGTTTCTCTGCCGGTCGCAGCCACC
ATGGAAGACGCCAAAAACATAAAGAAAGGCCCGGCGCCATTCTATCCGCTGGAAGATG
GAACCGCTGGAGAGCAACTGCATAAGGCTATGAAGAGATACGCCCTGGTTCCTGGAAC
AATTGCTTTTACAGATGCACATATCGAGGTGGACATCACTTACGCTGAGTACTTCGAAA
TGTCCGTTCGGTTGGCAGAAGCTATGAAACGATATGGGCTGAATACAAATCACAGAATC
GTCGTATGCAGTGAAAACTCTCTTCAATTCTTTATGCCGGTGTTGGGCGCGTTATTTATC
GGAGTTGCAGTTGCGCCCGCGAACGACATTTATAATGAACGTGAATTGCTCAACAGTAT
GGGCATTTCGCAGCCTACCGTGGTGTTCGTTTCCAAAAAGGGGTTGCAAAAAATTTTGA
ACGTGCAAAAAAAGCTCCCAATCATCCAAAAAATTATTATCATGGATTCTAAAACGGAT
TACCAGGGATTTCAGTCGATGTACACGTTCGTCACATCTCATCTACCTCCCGGTTTTAAT
GAATACGATTTTGTGCCAGAGTCCTTCGATAGGGACAAGACAATTGCACTGATCATGAA
CTCCTCTGGATCTACTGGTCTGCCTAAAGGTGTCGCTCTGCCTCATAGAACTGCCTGCGT
GAGATTCTCGCATGCCAGAGATCCTATTTTTGGCAATCAAATCATTCCGGATACTGCGA
TTTTAAGTGTTGTTCCATTCCATCACGGTTTTGGAATGTTTACTACACTCGGATATTTGAT
ATGTGGATTTCGAGTCGTCTTAATGTATAGATTTGAAGAAGAGCTGTTTCTGAGGAGCC
TTCAGGATTACAAGATTCAAAGTGCGCTGCTGGTGCCAACCCTATTCTCCTTCTTCGCCA
AAAGCACTCTGATTGACAAATACGATTTATCTAATTTACACGAAATTGCTTCTGGTGGC
GCTCCCCTCTCTAAGGAAGTCGGGGAAGCGGTTGCCAAGAGGTTCCATCTGCCAGGTAT
CAGGCAAGGATATGGGCTCACTGAGACTACATCAGCTATTCTGATTACACCCGAGGGG
GATGATAAACCGGGCGCGGTCGGTAAAGTTGTTCCATTTTTTGAAGCGAAGGTTGTGGA
 
TCTGGATACCGGGAAAACGCTGGGCGTTAATCAAAGAGGCGAACTGTGTGTGAGAGGT
CCTATGATTATGTCCGGTTATGTAAACAATCCGGAAGCGACCAACGCCTTGATTGACAA
GGATGGATGGCTACATTCTGGAGACATAGCTTACTGGGACGAAGACGAACACTTCTTCA
TCGTTGACCGCCTGAAGTCTCTGATTAAGTACAAAGGCTATCAGGTGGCTCCCGCTGAA
TTGGAATCCATCTTGCTCCAACACCCCAACATCTTCGACGCAGGTGTCGCAGGTCTTCCC
GACGATGACGCCGGTGAACTTCCCGCCGCCGTTGTTGTTTTGGAGCACGGAAAGACGAT
GACGGAAAAAGAGATCGTGGATTACGTCGCCAGTCAAGTAACAACCGCGAAAAAGTTG
CGCGGAGGAGTTGTGTTTGTGGACGAAGTACCGAAAGGTCTTACCGGAAAACTCGACG
CAAGAAAAATCAGAGAGATCCTCATAAAGGCCAAGAAGGGCGGAAAGATCGCCGTGG
ATTACAAGGATGACGACGATAAGTAAGGGCCCACCGGTTGATAATAGGCTGGAGCCTC
GGTGGCCATGCTTCTTGCCCCTTGGGCCTCCCCCCAGCCCCTCCTCCCCTTCCTGCACCC
GTACCCCCGTGGTCTTTGAATAAAGTCTGAGTGGGCGGCCTCGAGCCGCGGAAAAAA
AAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAA
ATTATTGTTTAAACATTTAAATTCTAGA
SEQ ID NO:57
> B1 mRNA sequence (ACTG1 3'UTR) 
CCGACUACCUCAUGAAGAUCCUCACUGAGCGAGGCUACAGCUUCACCACCACGGCCG
AGCGGGAAAUCGUGCGCGACAUCAAGGAGAAGCUGUGCUACGUCGCCCUGGACUUCG
AGCAGGAGAUGGCCACCGCCGCAUCCUCCUCUUCUCUGGAGAAGAGCUACGAGCUGC
CCGAUGGCCAGGUCAUCACCAUUGGCAAUGAGCGGUUCCGGUGUCCGGAGGCGCUGU
UCCAGCCUUCCUUCCUGGGUAUGGAAUCUUGCGGCAUCCACGAGACCACCUUCAACU
CCAUCAUGAAGUGUGACGUGGACAUCCGCAAAGACCUGUACGCCAACACGGUGCUGU
CGGGCGGCACCACCAUGUACCCGGGCAUUGCCGACAGGAUGCAGAAGGAGAUCACCG
CCCUGGCGCCCAGCACCAUGAAGAUCAAGAUCAUCGCACCCCCAGAGCGCAAGUACU
CGGUGUGGAUCGGUGGCUCCAUCCUGGCCUCACUGUCCACCUUCCAGCAGAUGUGGA
UUAG
SEQ ID NO:58
> B2 mRNA sequence (ATP6V0B 3'UTR) 
AUGAUAUGUGUGGGUGGGGCCGUGCCUCACUUUUAUUUAUUGCUGGUUUUCCUGGG
ACAGCUGGAGCUGUGUCCCUUAGCCUUUCAGAGGCUUGGUGUUCAGGGCCCUCCCUG
CACUCCCCUCUUGCUGCGUGUUGAUUUGGAGGCACUGCAGUCCAGGCCGAGUCCUCA
GUGCGGGGAGCAGGCUGCUGCUGCUGACUCUGUGCAGCUGCGCACCUGUGUCCCCCA
CCUCCACCCUCAACCCAUCUUCCUAGUGUUUGUGAAAUAAACUUGGUAUUUGUCU
SEQ ID NO:59
> B3 mRNA sequence (ATP6V0B 3'UTR) 
GUUGAAGAACUUCUCUCUACCGAGCAGCCCGUAACCCCCUUUUUCUCCCUCACUGCU
GCAGGAGUCUGCUACACCAUUUUUGAUUUGGGCUUCCGCUUUGAUGUGGCAUGGUU
CCUGACGGAGACUUCGCCCUUCAUGUGGUCCAACCUGGGCAUUGGCCUAGCUAUCUC
CCUGUCUGUGGUUGGGGCAGCCUGGGGCAUCUAUAUUACCGGCUCCUCCAUCAUUGG
UGGAGGAGUGAAGGCCCCCAGGAUCAAGACCAAGAACCUGGUCAGCAUCAUCUUCUG
UGAGGCUGUGGCCAUCUACGGCAU
SEQ ID NO:60
> B4 mRNA sequence (ATP6V0E1 3'UTR) 
GGAAGAAGACAUGCUCUACAGUGCUCAGUCUUUGAGGUGACUAUGCUUGUGACCUU
UCUUAUCAGUAUGGUCAGGCAGUCAGAGUUUGCCUUGACUUUUUCUCACAUUUUAU
GGCCAUUUUAACACGUGGCAAGGGCAAGCUGCUGAAUGCAAACACGAUCUGAAGAGC
AGAAAUAAAAUUGGAACUUAAGCCUUAGCUUACCUAAAUCUCAUUAAGAUUCUUUU
U
SEQ ID NO:61
> B5 mRNA sequence (ATP6V0E1 3'UTR) 
AUGCAAAUCACCUCCAAACCAGACCACUUUCUUGACUUGCCUGUUUGGCCAUUA
GCUGCCUUAAACGUUAACAGCACAUUUGAAUGCCUUAUUCUACAAUGCAGCGUGUUU
 
UCCUUUGCCUUUUUUGCACUUUGGUGAAUUACGUGCCUCCAUAACCUGAACUGUGCC
GACUCCACAAAACGAUUAUGUACUCUUCUGAGAUAGAAGAUGCUGUUCUUCUGAGA
GAUACGUUACUCUCUCCUUGGAAUCUGUGGAUUUGAAGAUGGCUCCUGCCUUCUCAC
GUGGGAAUCAGUGAAGUGUUUAGAAACUGCUGCAAGACAAACAAGACUCCAGUGGG
GUGGUCAGUAGGAGAGCACGUUCAGAGGGAAGAGCCAUCUCAACAGAAUCGCACCAA
ACUAUACUUUCAGGAUGAAUUUCUUCUUUCUGCCAUCUUUUGGAAUAAAUAUUUUC
CUCCUUUC
SEQ ID NO:62
> B6 mRNA sequence (CFL1 3'UTR) 
GCCCCUUCUGGCCCCCUGCCUGGAGCAUCUGGCAGCCCCACACCUGCCCUUGGGGGU
UGCAGGCUGCCCCCUUCCUGCCAGACCGGAGGGGCUGGGGGGAUCCCAGCAGGGGGA
GGGCAAUCCCUUCACCCCAGUUGCCAAACAGACCCCCCACCCCCUGGAUUUUCCUUCU
CCCUCCAUCCCUUGACGGUUCUGGCCUUCCCAAACUGCUUUUGAUCUUUUGAUUCCU
CUUGGGCUGAAGCAGACCAAGUUCCCCCCAGGCACCCCAGUUGUGGGGGAGCCUGUA
UUUUUUUUAACAACAUCCCCAUUCCCCACCUGGUCCUCCCCCUUCCCAUGCUGCCAAC
UUCUAACCGCAAUAGUGACUCUGUGCUUGUCUGUUUAGUUCUGUGUAUAAAUGGAA
UGUUGUGGAGAUGACCCCUCCCUGUGCCGGCUGGUUCCUCUCCCUUUUCCCCUGGUC
ACGGCUACUCAUGGAAGCAGGACCAGUAAGGGACCUUCGAUU
SEQ ID NO:63
> B7 mRNA sequence (CFL1 3'UTR) 
GCCCCUUCUGGCCCCCUGCCUGGAGCAUCUGGCAGCCCCACACCUGCCCUUGGGGGU
UGCAGGCUGCCCCCUUCCUGCCAGACCGGAGGGGCUGGGGGGAUCCCAGCAGGGGGA
GGGCAAUCCCUUCACCCCAGUUGCCAAACAGACCCCCCACCCCCUGGAUUUUCCUUCU
CCCUCCAUCCCUUGACGGUUCUGGCCUUCCCAAACUGCUUUUGAUCUUUUGAUUCCU
CUUGGGCUGAAGCAGACCAAGUUCCCCCCAGGCACCCCAGUUGUGGGGGAGCC
SEQ ID NO:64
> B8 mRNA sequence (CFL1 3'UTR) 
GCCCCUUCUGGCCCCCUGCCUGGAGCAUCUGGCAGCCCCACACCUGCCCUUGGGGGU
UGCAGGCUGCCCCCUUCCUGCCAGACCGGAGGGGCUGGGGGGAUCCCAGCAGGGGGA
GGGCAAUCCCUUCACCCCAGUUGCCAAACAGACCCCCCACCCCCUGGAUUUUCCUUCU
CCCUCCAUCCCUUGACGGUUCUGGCCUUCCCAAACUGCUUUUGAUCUUUUGAUUCCU
CUUGGGCUGAAGCAGACCAAGUUCCCCCCAGGCACCCCAGUUGUGGGGGAGCCUGUA
UUUUUUUUAACAACAUCCCCAUUCCCCACCUGGUCCUCCCCCUUCCCAUGCUGCCAAC
UUCUAACCGCAAUAGUG
SEQ ID NO:65
> B9 mRNA sequence (COX4I1 3'UTR) 
GAGAUGCUGGCCUGCGCCUGCACCUGCGCCUGGCUCUGUCACCGCCAUGCAACUCCA
UGCCUAUUUACUGGAAACCUGUUAUGCCAAACAGUUGUACCACUGCUAAUAAAUGAC
CAGUUUACCUGAAA
SEQ ID NO:66
> B10 mRNA sequence (COX4I1 3'UTR) 
AGGGACCCACAGGCGCGCCCAGCAGCUCUCGGAAGCGUUGUAUCGCAUUAAGUUCAA
GGAGAGCUUUGCUGAGAUGAACAGGGGCUCGAACGAGUGGAAGACGGUUGUGGGCG
GUGCCAUGUUCUUCAUCGGUUUCACCGCGCUCGUUAUCAUGUGGCAGAAGCACUAUG
GGCUUAGCCUCCAAGUGGGACUACGAAAAGAACGAGUGGAAGAAGUGAGAGAUGCU
GGCCUGCGCCUGCACCUGCGCCUGGCUCUGUCACCGCCAUGCAACUCCAUGCCUAUU
UACUGGAAACCUGUUAUGCCAAACAGUUGUACCACU
SEQ ID NO:67
> **B11** mRNA sequence (COX4I1 3'UTR) 
AGAGGACCUUCUGCUUUUAGCUUAUUUUGUUGCUAACUUUUUACAAACAAAGGGCU
AAUUUUAAAAUGUCAGUGUUGUCAGUGGUCAGAAACCGUGUGUUGAGCGGUGUU
 
GAGUGGCAGGUGGCUCUGCUGACCUGGUGGCUGGUGUGUCGGGAGGAUUUAACCUG
UGUGAGGGAUUGGCCUAGAAACAACCUGUUGAGAUAGUCUUGCCCCAUAACCUGUCU
CACACCGUAGUGUACGGCCCCCUCCCGCAAAGCUUUGACAAAGAGUGGGUGGCCAAG
CAGACCAAGAGGAUGCUGGAC
SEQ ID NO:68
> B12 mRNA sequence (CTSB 3'UTR) 
UCUGCCGUGGGCCUGUCGUGCCAGUCCUGGGGGCGAGAUCGGGAGAAUCACGUCCUG
GAACUGCAUGUUCUUGCGACUCUUGGGACUUCAUCUUAACUUCUCGCUGCCCCAGCC
AUGUUUUCAACCAUGGCAUCCCUCCCCCAAUUAGUUCCCUGUCAUCCUCGUCAACCU
UCUCUGUAAGUGCCUGGUAAGCUUGCCCUUGCUUAAGAACUCAAAACAUAGCUGUGC
UCUA
SEQ ID NO:69
> B13 mRNA sequence (FAM166A 3'UTR) 
AAAUGCUUUAAUGGUGGUGUCUGUACAGCAUGGAUGUGGACAGGGAAAACAGCAAG
UGCACACAGUGGGACAGGCCACAGGACAGGCUGGGAGUGAAUAAAGAGUUCACACU
GCU
SEQ ID NO:70
> B14 mRNA sequence (NDUFB9 3'UTR) 
UAUCUAAGUGUAUCAAUUUAACAUUUAUAAUCACCUUACAAGUAAGUUAAAGUGCA
UUCCCUGGUCGUUUUGGCUUUGUCUUUGAUUUUGCUUCAGAUCUCCUUCUCUUUAGU
CCUUUGUCUGACUUUUUUGCCUUCUUGCUAUACUUUCCCUCAACCUGCCUCUCCACU
AUUUUCUUAGGCAUUUUUCAGUCAUG
SEQ ID NO:71
> A1 mRNA sequence (ACTG1 5'UTR)
   CUCUCGCACUCUGUUCUUCCGCCGCUCCGCCGUCGCGUUUCUCUGCCGGUCGCA SEQ ID NO:72
> A2 mRNA sequence (ATP6V0B 5'UTR) 
GGGCGGGCGGACAGACUGCGGGACGGACGGUGGACGCUGGGACGCGUUUGUAGCUCC
GGCCCCGCCGUUCCGACCCCCGCCGCCGUCGCCGCCAUGACGGGGCUAGCACUGCUCU
ACUCCGGGGUCUUCGUGGCCUUCUGGGCCUGCGCGCUGGCCGUGGGUUCCUGACGGA
GACUUCGCCCUUC
SEQ ID NO:73
> A3 mRNA sequence (ATP6V0B 5'UTR) 
AGACUGCGGACGGACGGUGGACGCUGGACGCGUUUGUAGCUCCGGCCCCGCCGUU
CCGACCCCCGCCGCCGUCGCCGCC
SEQ ID NO:74
> A4 mRNA sequence (ATP6V0E1 5'UTR)
   GGGGUAGGGGUUGGCGCUCAGGCGGCGACC SEQ ID NO:75
> A5 mRNA sequence (ATP6V0E1 5'UTR) 
GUCACGCGGUCAGCUAUUGACACUUCCUGGUGGGAUCCGAGUGAGGCGACGGGGUAG
GGGUUGGCGCUCAGGCGGCGACC
SEQ ID NO:76
> A6 mRNA sequence (CFL1 5'UTR) 
GGCCGGCGGAAGACUCCGUUACCCAGCGAGCGAGGCGGCGCAGGGCCAGCGGA
CUCCAUUUCCCGUCGGCUCGCGGUGGGAGCGCCGGAAGCCCGCCCCACCCCUCAUUG
UGCGGCUCCUACUAAACGGAAGGGGCCGGGAGAGGCCGCGUUCAGUCGGGUCCCGGC
AGCGGCUGCAGCGCUCUCGUCUUCUGCGGCUCUCGGUGCCCUCUCCUUUUCGUUCC
 
GGAAAC
SEQ ID NO:77
> A7 mRNA sequence (CFL1 5'UTR) 
CUCCUGGCUCGGGCACCCCGAAGCACGACGCAGAGUAGGAAGAGUUUAAAGACCUCG
AGGCCUCUGGGCACUUGAGUUUGGCCUCCGGUGUGGCUGUCUCUGAUGGUGUCAUCA
AGGUGUUCAACGAC
SEQ ID NO:78
> A8 mRNA sequence (CFL1 5'UTR) 
ACGUGGCCGGCUUCUUCUGCAGUUCCGGGGAGUUGGGGACCAGAUUUACCUUGGA
UUGCCCCUCCCUCUCCUGGCUCGGGCACCCCGAAGCACGACGCAGAGCCUCCGGUGU
GGCUGUCUCUGAUGGUGUCAUCAAGGUGUUCAACGAC
SEQ ID NO:79
> A9 mRNA sequence (COX4I1 5'UTR) 
GCGGCCUUGCUCUCUUCCGGUCGCGGGACACCGGGUGUAGAGGGCGGUCGCGGCGGG
CAGUGGCGGCAGA
SEQ ID NO:80
> A10 mRNA sequence (COX4I1 5'UTR)
   GGGACACCGGGUGUAGAGGGCGGUCGCGGCGGGCAGUGGCGGCAGA SEQ ID NO:81
> A11 mRNA sequence (COX4I1 5'UTR) 
CGCGGCCUUGCUCUCUUCCGGUCGCGGGACACCGGGUGUAGAGGGCGGUCGCGGCGG
GCAGUGGCGGCAGA
SEQ ID NO:82
> A12 mRNA sequence (CUSB 5'UTR) 
GGCCGGGGCUGGCCCAGGCUACGGCGGCUGCAGGGCUCCGGCAACCGCUCCGGCAAC
GCCAACCGCUCCGCUGCGCGCAGGCUGGGCUGCAGGCUCUCGGCUGCAGCGCUGGGU
GGAUCUAGGAUCCGGCUUCCAAC
SEQ ID NO:83
> A13 mRNA sequence (FAM166A 5'UTR)
   AAGGGAGCUGGAUGCCGGGAGGGACUGGAGCCAGCAAGGCCAGAGUGAAAGCAAA SEQ ID NO:84
> A14 mRNA sequence (NDUFB9 5'UTR)
   GCAGCAGGCGUGCAGUUUCCCGGCUCUCCGCGCGGCCGGGGAAGGUCAGCGCCGUA SEQ ID NO:85
> A15 mRNA sequence (CHCHD10 5'UTR) 
GGCAUUUGUCCCGCGACAGCACCGCUGCCGCCGUCUCUAAGGUCGCCCGGUCCCA
CCGCCGCCACC
SEQ ID NO:86
> A16 mRNA sequence (CHCHD10 5'UTR)
   CCGCUGCCGCCGUCUCUAAGGUCGCCCGGGUCCCACCGCCGCCACC SEQ ID NO:87
> A17 mRNA sequence (SLC38A2 5'UTR)
   AUGUCUUUUUUGUGUGUUUGUUUUCAUGGUAUUCCUAUGAA SEQ ID NO:88
> A18 mRNA sequence (NDUFA11 5'UTR)
   GCUUCCCGAGCUGGCGGGGUCCGUGGUGCGGGAUCGAGAUUGCGGGCU SEQ ID NO:89
> A19 mRNA sequence (NDUFV3 5'UTR) 
GGCGGCUGUUCAGGCGCGGGUGCGCGCGCAGCUGCUGUGGCCCUGCUUGGUGCGCCC
GCUGUCACCGCC
SEQ ID NO:90
> A20 mRNA sequence (PRDX5 5'UTR) 
CGCGCCUGCGCAGUGGAGGCGGCCCAGGCCCGCCUUCCGCAGGGUGUCGCCGCUGUG
CCGCUAGCGGUGCCCCGCCUGCUGCGGUGGCACCAGCCAGGAGGCGGAGUGGAAGUG
GCCGUGGGGCGGGU
SEQ ID NO:91
> A21 mRNA sequence (GUK1 5'UTR) 
CAGCAGAUGGGGACUAGAGGCCGCACUGCUAUCCACAGCCUCUCUUCUCACCCCCAG
GC
SEQ ID NO:92
> A22 mRNA sequence (GUK1 5'UTR) 
GCUGGCCGGGCUGGCUGCGGCCGCCCUGGGCCGGGCCCCACCGGACGCCUCUCUUCUC
ACCCCCAGGC
SEQ ID NO:93
> A23 mRNA sequence (GUK1 5'UTR) 
AGAGGUGGCCCCGGAUGCUGCGGCGCCCGCUGGCCGGGCUGGCUGCGGCCGCCCUGG
GCCGGGCCCCACCGGACGCCUCUCUUCUCACCCCCAGGC
SEQ ID NO:94
> A24 mRNA sequence (IAH1 5'UTR)
   UGGCUGGCGGCCCCGCCCCGCCCCGCCCGGCUGCUCC SEQ ID NO:95
> A25 mRNA sequence (ABHD 16A 5'UTR) 
GGAGGGCGGGGCCGGCAGGGGGACCUGCUGCUGGAAGAGCAGCGGCCCGAGCCGGGG
CC
SEQ ID NO:96
> A26 mRNA sequence (SLC25A39 5'UTR) 
UUUCGGCGUCGGCCUAGGUGCGCUGCGAGCGCGCGGACCGCACAGGCGGCGGA
GCCGGUAUGGGCCCGCCUGACCCUGGGCGCCGCGCCGCACGAGCACCAGCCUAGAGC
CAGGACUGAAGCUUCAAG
SEQ ID NO:97
> A27 mRNA sequence (AUPIF1 5'UTR)
   CGAGAGACUGCUUGCUGCGGCAGAGACGCCAGAGGUGCAGCUCCAGCAGCA SEQ ID NO:98
> A28 mRNA sequence (ANAPC11 5'UTR) 
CGGAGUUUCGUCAUGUUGGCCAGGCCCAUUUGAGAUCUUUGAAGAUAUCCUCAACGU
GAGGCUCUGCUGCC
SEQ ID NO:99
> A29 mRNA sequence (ANAPC11 5'UTR)
   GGGCGCGGCUUCGGCGGGCGGCAGCCGCUGCAGACGAGCUGCGGGCUCUGCUGCC SEQ ID NO:100
> A30 mRNA sequence (CCDC12 5'UTR)
   GCCUGCGCGAUGCAAGACGGGAGAAAAGGAGGGGCGUACGCGGGCAAG SEQ ID NO:101
> A31 mRNA sequence (MRPL14 5'UTR) 
AUCGCGUCCGCCGGGCUGGGCCUGGCGCGCAGGCGCUAGGAAGAGGCCGCGUGGGGC
GAAGGCGGCGCUUGGCUGGUGGGGCCCGCGGCGGGAUUUUCCCGGGCGGCGAGAGCG
GAUCUAUCUUGGGAUCCC
SEQ ID NO:102
> A32 mRNA sequence (APOA1BP 5'UTR)
   GCCGGGGGCGCGCGCUCUGCGAGCUGG SEQ ID NO:103
> A33 mRNA sequence (APOA1BP 5'UTR)
   GCCGGGGCCGGGCCGGGCCGGGGGCGCGCGCUCUGCGAGCUGG SEQ ID NO:104
> Mod. 5'UTR mRNA sequence
   GGGAAAUAAGAGAGAAAAGAAGAGUAAGAAGAAAUAUAAGAGCCACC SEQ ID NO:105
> α-globin 3'UTR mRNA sequence 
UGAUAAUAGGCUGGAGCCUCGGUGGCCAUGCUUCUUGCCCCUUGGGCCUCCCCCCAG
CCCCUCCUCCCCUUCCUGCACCCGUACCCCCGUGGUCUUUGAAUAAAGUCUGAGUGG
GCGGC
SEQ ID NO:106
> α-1 globin 5'UTR mRNA sequence
   AGACGAACUAGUAUUCUUCUGGUCCCCACAGACUCAGAGAGAACCCGCCACC SEQ ID NO:107
> haplogroup U8b1b1 mitochondrion 3'UTR mRNA sequence 
CUCGAGCUGGUACUGCAUGCACGCAAUGCUAGCUGCCCCUUUCCCGUCCUGGGUACC
CCGAGUCUCCCCCGACCUCGGGUCCCAGGUAUGCUCCCACCUCCACCUGCCCCACUCA
CCACCUCUGCUAGUUCCAGACACCUGCUAUACUAAUCGCUAGCCGCGUCGCUCCCCA
GGGUUGGUCAAUUUCGUGCCAGCCACACCGAGACCUGGUCCAGAGCCCAAGCACGCC
ACACCCCCACGGGAAACAGCAGUGAUUAACCUUUAGCAAUAAACGAAAGUUUAACUA
ACAGCAAUGCA GCUCAAAACGCUUAGCCUAG
SEQ ID NO:108
> hHGF amino acid sequence 
MWVTKLLPALLLQHVLLHLLLLPIAIPYAEGQRKRRNTIHEFKKSAKTTLIKIDPALKIKTKK
VNTADQCANRCTRNKGLPFTCKAFVFDKARKQCLWFPFNSMSSGVKKEFGHEFDLYENKD
YIRNCIIGKGRSYKGTVSITKSGIKCQPWSSMIPHEHSFLPSSYRGKDLQENYCRNPRGEEGG
PWCFTSNPEVRYEVCDIPQCSEVECMTCNGESYRGLMDHTESGKICQRWDHQTPHRHKFLP
ERYPDKGFDDNYCRNPDGQPRPWCYTLDPHTRWEYCAIKTCADNTMNDTDVPLETTECIQ
GQGEGYRGTVNTIWNGIPCQRWDSQYPHEHDMTPENFKCKDLRENYCRNPDGSESPWCFT
TDPNIRVGYCSQIPNCDMSHGQDCYRGNGKNYMGNLSQTRSGLTCSMWDKNMEDLHRHIF
WEPDASKLNENYCRNPDDDAHGPWCYTGNPLIPWDYCPISRCEGDTTPTIVNLDHPVISCA
KTKQLRVVNGIPTRTNIGWMVSLRYRNKHICGGSLIKESWVLTARQCFPSRDLKDYEAWLGI
HDVHGRGDEKCKQVLNVSQLVYGPEGSDLVLMKLARPAVLDDFVSTIDLPNYGCTIPEKTS
CSVYGWGYTGLINYDGLLRVAHLYIMGNEKCSQHHRGKVTLNESEICAGAEKIGSGPCEGD
YGGPLVCEQHKMRMVLGVIVPGRGCAIPNRPGIFVRVAYYAKWIHKIILTYKVPQS
SEQ ID NO:109
> hHGF nucleotide sequence 
ATGTGGGTGACCAAACTCCTGCCAGCCCTGCTGCTGCAGCATGTCCTCCTGCATCTCCTC
CTGCTCCCCATCGCCATCCCCTATGCAGAGGGACAAAGGAAAAGAAGAAATACAATTCAT
GAATTCAAAAAATCAGCAAAGACTACCCTAATCAAAATAGATCCAGCACTGAAGATAAA
AACCAAAAAAGTGAATACTGCAGACCAATGTGCTAATAGATGTACTAGGAATAAAGGACT
TCCATTCACTTGCAAGGCTTTTGTTTTTGATAAAGCAAGAAAACAATGCCTCTGGTTCCC
CTTCAATAGCATGTCAAGTGGAGTGAAAAAAGAATTTGGCCATGAATTTGACCTCTATGA
AAACAAAGACTACATTAGAAACTGCATCATTGGTAAAGGACGCAGCTACAAGGGAACAG
TATCTATCACTAAGAGTGGCATCAAATGTCAGCCCTGGAGTTCCATGATACCACACGAAC
ACAGCTTTTTGCCTTCGAGCTATCGGGGTAAAGACCTACAGGAAAACTACTGTCGAAATC
CTCGAGGGGAAGAAGGGGGACCCTGGTGTTTCACAAGCAATCCAGAGGTACGCTACGA
AGTCTGTGACATTCCTCAGTGTTCAGAAGTTGAATGCATGACCTGCAATGGGGAGAGTTA
TCGAGGTCTCATGGATCATACAGAATCAGGCAAGATTTGTCAGCGCTGGGATCATCAGAC
ACCACACCGGCACAAATTCTTGCCTGAAAGATATCCCGACAAGGGCTTTGATGATAATTA
TTGCCGCAATCCCGATGGCCAGCCGAGGCCATGGTGCTATACTCTTGACCCTCACACCCG
CTGGGAGTACTGTGCAATTAAAACATGCGCTGACAATACTATGAATGACACTGATGTTCC
TTTGGAAACAACTGAATGCATCCAAGGTCAAGGAGAAGGCTACAGGGGCACTGTCAATA
CCATTTGGAATGGAATTCCATGTCAGCGTTGGGATTCTCAGTATCCTCACGAGCATGACAT
GACTCCTGAAAATTTCAAGTGCAAGGACCTACGAGAAAATTACTGCCGAAATCCAGATG
GGTCTGAATCACCCTGGTGTTTTACCACTGATCCAAACATCCGAGTTGGCTACTGCTCCC
AAATTCCAAACTGTGATATGTCACATGGACAAGATTGTTATCGTGGGAATGGCAAAAATT
ATATGGGCAACTTATCCCAAACAAGATCTGGACTAACATGTTCAATGTGGGACAAGAACA
TGGAAGACTTACATCGTCATATCTTCTGGGAACCAGATGCAAGTAAGCTGAATGAGAATT
ACTGCCGAAATCCAGATGATGATGCTCATGGACCCTGGTGCTACACGGGAAATCCACTCA
TTCCTTGGGATTATTGCCCTATTTCTCGTTGTGAAGGTGATACCACACCTACAATAGTCAAT
TTAGACCATCCCGTAATATCTTGTGCCAAAACGAAACAATTGCGAGTTGTAAATGGGATT
CCAACACGAACAAACATAGGATGGATGGTTAGTTTGAGATACAGAAATAAACATATCTGC
GGAGGATCATTGATAAAGGAGAGTTGGGTTCTTACTGCACGACAGTGTTTCCCTTCTCGA
GACTTGAAAGATTATGAAGCTTGGCTTGGAATTCATGATGTCCACGGAAGAGGAGATGA
GAAATGCAAACAGGTTCTCAATGTTTCCCAGCTGGTATATGGCCCTGAAGGATCAGATCT
GGTTTTAATGAAGCTTGCCAGGCCTGCTGTCCTGGATGATTTTGTTAGTACGATTGATTTA
CCTAATTATGGATGCACAATTCCTGAAAAGACCAGTTGCAGTGTTTATGGCTGGGGCTAC
ACTGGATTGATCAACTATGATGGCCTATTACGAGTGGCACATCTCTATATAATGGGAAATG
AGAAATGCAGCCAGCATCATCGAGGGAAGGTGACTCTGAATGAGTCTGAAATATGTGCT
GGGGCTGAAAAGATTGGATCAGGACCATGTGAGGGGGATTATGGTGGCCCACTTGTTTGT
GAGCAACATAAAATGAGAATGGTTCTTGGTGTCATTGTTCCTGGTCGTGGATGTGCCATT
CCAAATCGTCCTGGTATTTTTGTCCGAGTAGCATATTATGCAAAATGGATACACAAAATTAT
TTTAACATATAAGGTACCACAGTCATAG
SEQ ID NO:110
> hHGF codon-optimized sequence 1 (hHGF-OS1) 
ATGTGGGTGACCAAACTGCTCCCCGCTCTGCTGCTGCAGCATGTGCTGCTCCACCTGCTG
CTGCTGCCAATCGCCATCCCCTACGCTGAAGGACAGCGGAAGCGCAGAAACACAATTCA
CGAATTCAAGAAGTCCGCCAAAACAACACTGATCAAGATCGACCCTGCCCTGAAAATCA
AAACCAAGAAGGTGAACACTGCCGATCAGTGCGCCAACAGATGCACAAGAAACAAAGG
CCTGCCCTTCACATGCAAGGCCTTTGTGTTCGACAAGGCCAGAAAGCAGTGCCTGTGGT
TCCCTTTCAACAGCATGAGCTCTGGCGTGAAGAAAGAGTTCGGCCACGAATTCGACCTG
TACGAGAACAAGGATTACATCAGAAATTGTATCATCGGCAAGGGCAGAAGCTACAAAGG
CACAGTGTCCATCACCAAAAGCGGCATTAAGTGTCAGCCTTGGAGCAGCATGATCCCTCA
CGAGCACAGCTTCCTGCCTAGCTCTTACAGAGGCAAGGACCTGCAGGAGAATTACTGCC
GGAACCCTAGAGGCGAGGAAGGCGGCCCTTGGTGCTTTACCAGCAACCCTGAGGTGCG
GTACGAGGTGTGTGACATCCCCCAGTGCAGCGAAGTGGAATGCATGACCTGTAATGGCG
AGAGCTACAGAGGCCTCATGGACCATACAGAGAGCGGAAAGATTTGTCAAAGATGGGAT
CACCAGACACCTCACAGACACAAATTCCTGCCCGAAAGATATCCTGACAAGGGCTTCGA
CGACAACTACTGCAGAAATCCTGATGGCCAACCCAGACCTTGGTGCTACACCCTGGATCC
TCATACCAGATGGGAGTACTGTGCCATCAAGACCTGCGCTGATAATACCATGAACGACAC
CGATGTGCCTCTGGAAACAACCGAGTGCATCCAGGGACAGGGCGAAGGCTACCGGGGC
ACCGTGAACACCATCTGGAACGGCATCCCCTGCCAGAGATGGGACAGCCAGTACCCCCA
CGAACACGATATGACCCCTGAGAACTTCAAGTGCAAGGACCTGAGAGAAAATTACTGCA
 
GAAACCCAGACGGAAGCGAGTCTCCTTGGTGTTTTACCACCGACCCCAACATCCGGGTG
GGTTACTGCAGCCAAATCCCCAATTGCGACATGAGCCACGGCCAGGACTGCTACCGGGG
AAACGGCAAAAACTACATGGGCAATCTGAGCCAGACCCGGAGCGGTCTGACCTGCTCCA
TGTGGGATAAGAACATGGAAGATCTGCACCGGCACATCTTCTGGGAGCCTGACGCTTCTA
AGCTGAACGAGAACTACTGTAGGAACCCAGACGACGATGCCCACGGTCCCTGGTGCTAC
ACCGGCAACCCGCTGATCCCTTGGGACTATTGTCCTATCAGCCGCTGCGAGGGCGATACA
ACACCTACCATCGTGAACCTGGACCACCCTGTGATCTCTTGCGCCAAGACCAAGCAGCT
GAGAGTGGTGAACGGCATCCCAACACGGACCAACATCGGATGGATGGTCTCCCTGAGAT
ACCGGAACAAGCACATCTGCGGCGGCTCTCTGATCAAGGAATCTTGGGTCCTGACCGCC
AGACAGTGCTTCCCCAGCCGGGACCTGAAGGACTATGAGGCCTGGCTGGGAATCCACGA
CGTGCACGGCAGAGGCGACGAGAAGTGCAAGCAAGTGCTGAACGTGTCCCAGCTGGTG
TACGGCCCTGAGGGATCTGATCTGGTCCTGATGAAACTGGCCAGACCCGCCGTGCTGGA
CGACTTTGTGTCCACCATCGACCTGCCTAACTACGGCTGCACCATCCCTGAAAAGACCAG
CTGCAGCGTGTACGGCTGGGGCTATACAGGCCTGATCAACTACGACGGTCTGCTGCGGGT
TGCTCACCTGTACATCATGGGCAACGAGAAGTGCAGCCAGCACCACAGAGGAAAGGTTA
CACTTAACGAGAGCGAGATCTGCGCCGGCGCCGAGAAAATCGGCTCCGGCCCTTGTGAA
GGCGACTACGGAGGCCCTCTGGTGTGCGAACAGCACAAGATGAGAATGGTGCTGGGCGT
GATCGTGCCCGGAAGAGGCTGTGCAATCCCTAATAGGCCCGGCATTTTCGTGCGGGTGGC
CTACTACGCCAAGTGGATCCACAAGATCATCCTGACGTACAAGGTGCCTCAGAGCTGA
SEQ ID NO:111
> hHGF codon-optimized sequence 2 (hHGF-OS2) 
ATGTGGGTCACCAAACTGCTGCCCGCCCTGCTGCTGCAGCACGTGCTCCTGCACCTGCT
GCTGCTGCCTATCGCCATCCCTTACGCCGAAGGCCAGAGAAAGCGGAGAAATACCATTCA
CGAATTTAAGAAGAGCGCCAAGACCACACTGATCAAGATCGACCCTGCCCTGAAAATCA
AGACCAAGAAAGTGAACACAGCCGACCAATGCGCTAATAGATGCACAAGAAACAAAGG
CCTGCCTTTCACCTGCAAGGCCTTCGTGTTCGATAAGGCCAGAAAGCAGTGCCTGTGGTT
CCCCTTCAACAGCATGAGCAGCGGCGTGAAGAAAGAATTCGGTCACGAGTTCGACCTGT
ACGAGAATAAGGACTACATTAGAAACTGCATCATCGGCAAGGGCCGGAGCTACAAGGGC
ACCGTGAGCATCACCAAGTCTGGCATCAAGTGCCAGCCATGGAGTTCCATGATCCCTCAT
GAGCACAGCTTCCTGCCAAGCTCTTACCGGGGAAAGGATCTGCAGGAGAACTATTGTAG
AAATCCCAGAGGCGAGGAAGGAGGACCCTGGTGTTTTACCAGCAACCCAGAGGTGCGG
TACGAGGTGTGCGACATACCCCAGTGCAGCGAGGTGGAATGCATGACATGCAACGGCGA
GAGCTACCGGGGCCTGATGGACCACACAGAGAGCGGCAAAATCTGCCAAAGATGGGATC
ACCAGACCCCTCATAGGCACAAGTTCCTGCCCGAGAGATACCCTGACAAGGGCTTCGAC
GACAATTACTGCCGGAACCCTGATGGACAGCCTAGACCTTGGTGCTACACCCTGGACCCT
CACACCAGATGGGAATACTGCGCCATCAAGACATGTGCCGATAACACCATGAACGACAC
CGATGTGCCACTCGAGACAACTGAGTGTATCCAGGGCCAGGGCGAGGGCTACAGGGGC
ACCGTGAACACCATCTGGAACGGCATCCCTTGTCAGCGGTGGGACAGCCAATACCCCCA
CGAACATGATATGACCCCAGAAAACTTCAAGTGCAAAGATCTGCGGGAAAACTACTGTA
GAAACCCTGACGGCTCCGAGAGCCCTTGGTGCTTCACCACCGACCCTAACATCAGGGTG
GGCTACTGTAGCCAGATCCCCAACTGCGACATGAGCCACGGTCAGGACTGCTACAGAGG
AAACGGCAAGAATTACATGGGCAACCTGAGCCAGACCAGAAGCGGCCTGACCTGCAGC
ATGTGGGACAAAAACATGGAAGATCTGCACCGGCACATCTTTTGGGAGCCTGACGCCAG
CAAGCTGAATGAGAACTACTGTCGGAACCCTGACGACGATGCTCACGGCCCTTGGTGCT
ACACAGGCAACCCTCTGATCCCTTGGGACTATTGTCCTATTAGCAGGTGTGAAGGCGACA
CGACCCCTACAATCGTGAACCTGGATCACCCAGTGATCAGCTGTGCTAAAACAAAGCAA
CTGAGAGTGGTGAATGGTATCCCGACCCGGACCAACATCGGATGGATGGTCAGCCTGCG
GTACAGAAACAAGCACATCTGCGGCGGTAGCCTGATCAAGGAATCCTGGGTGTTGACAG
CCAGACAGTGTTTTCCCAGCAGAGATCTGAAGGACTACGAGGCCTGGCTGGGCATCCAC
GACGTGCACGGCAGAGGCGACGAGAAGTGCAAGCAGGTTCTGAACGTGTCCCAGCTGG
TGTACGGCCCCGAGGGCTCTGATCTGGTTCTGATGAAGCTTGCTAGACCCGCTGTGCTGG
ATGACTTCGTGTCCACCATCGACCTGCCAAATTACGGATGTACAATTCCTGAAAAGACCT
CCTGTTCTGTGTACGGCTGGGGCTACACCGGACTCATCAACTATGACGGCCTGCTGAGAG
TGGCCCACCTGTATATCATGGGCAACGAGAAGTGCAGCCAGCACCATAGAGGCAAGGTG
ACCCTGAACGAGTCTGAAATCTGCGCCGGAGCCGAGAAAATCGGATCTGGCCCCTGCGA
AGGAGATTACGGCGGCCCTCTGGTCTGCGAGCAGCACAAGATGAGAATGGTGCTGGGCG
TGATCGTGCCCGGCAGAGGCTGCGCCATCCCTAACCGGCCTGGAATCTTCGTGCGCGTGG
 
CCTATTACGCCAAATGGATCCACAAAATCATCCTGACATACAAGGTCCCACAGTCTTGA
SEQ ID NO:112
> hHGF codon-optimized sequence 3 (hHGF-OS3) 
ATGTGGGTGACCAAGCTGCTGCCCGCCCTGCTCCTGCAGCACGTGCTGCTGCACCTGCT
CCTGCTGCCCATCGCCATCCCCTACGCCGAGGGGCAGAGAAAGAGAAGAAACACCATCC
ACGAGTTCAAGAAGAGCGCCAAGACCACCCTGATCAAGATCGACCCCGCCCTGAAGATC
AAGACCAAGAAGGTGAACACCGCCGATCAGTGCGCCAACAGATGCACAAGAAACAAGG
GCCTGCCCTTCACCTGCAAGGCCTTCGTGTTCGACAAGGCTAGAAAGCAGTGCCTGTGG
TTCCCCTTCAACAGCATGAGCAGCGGCGTGAAGAAGGAGTTCGGCCACGAGTTCGACCT
GTACGAGAACAAGGACTACATCAGAAACTGCATCATCGGCAAGGGCAGAAGCTACAAG
GGCACCGTGAGCATCACCAAGAGCGGCATCAAGTGTCAGCCCTGGAGCAGCATGATCCC
CCATGAGCACAGCTTCCTGCCTAGCAGCTACAGAGGGAAGGACCTGCAAGAGAACTACT
GCAGAAACCCTAGAGGCGAGGAGGGCGGCCCTTGGTGTTTCACAAGCAATCCCGAGGT
GAGATACGAGGTGTGCGACATCCCTCAGTGCAGCGAGGTGGAGTGCATGACCTGCAACG
GCGAAAGCTATAGAGGCCTGATGGACCACACCGAGAGCGGCAAGATTTGTCAGCGGTGG
GATCACCAAACCCCCCACAGACACAAGTTCCTGCCCGAGAGATACCCCGACAAGGGCTT
CGACGACAATTACTGTAGAAACCCCGACGGGCAGCCTAGACCCTGGTGTTACACCCTGG
ACCCCCACACAAGATGGGAGTACTGCGCCATCAAGACCTGCGCCGACAACACCATGAAC
GACACCGACGTGCCCCTGGAGACCACCGAGTGCATCCAAGGCCAAGGCGAGGGCTACA
GAGGCACCGTGAACACCATCTGGAACGGCATTCCCTGTCAGAGATGGGACAGCCAATAC
CCCCATGAACACGATATGACCCCCGAGAACTTCAAGTGCAAGGACCTGAGAGAGAACTA
CTGCCGGAACCCCGATGGCAGCGAATCCCCTTGGTGCTTCACCACCGACCCCAACATCA
GAGTGGGCTACTGCAGCCAAATCCCCAACTGCGACATGAGCCACGGCCAAGACTGTTAC
AGAGGGAACGGCAAGAACTACATGGGCAACCTGAGCCAAACAAGAAGCGGCCTGACCT
GCAGCATGTGGGACAAGAACATGGAGGACCTGCACAGACACATCTTCTGGGAGCCCGA
CGCTAGCAAGCTGAACGAGAACTACTGTCGGAACCCCGACGACGATGCCCACGGCCCCT
GGTGCTACACCGGCAACCCCCTGATCCCCTGGGACTACTGCCCCATCAGCAGATGCGAG
GGCGACACCACCCCCACCATCGTGAACCTGGACCACCCCGTGATCAGCTGTGCCAAGAC
CAAGCAACTGAGAGTGGTGAACGGCATCCCCACAAGAACCAACATTGGCTGGATGGTG
AGCCTGAGATACAGAAACAAGCACATCTGCGGCGGCAGCCTGATCAAGGAGAGCTGGG
TGCTGACCGCTAGACAGTGCTTCCCTAGCAGAGACCTGAAGGACTACGAGGCCTGGCTG
GGCATCCACGACGTGCACGGCAGAGGCGACGAGAAGTGTAAACAAGTGCTGAACGTGA
GCCAACTGGTGTACGGCCCCGAGGGCAGCGACCTGGTGCTGATGAAGCTGGCTAGACCC
GCCGTGCTGGACGACTTCGTGAGCACCATCGACCTGCCCAACTACGGCTGCACCATCCC
CGAGAAGACAAGCTGCAGCGTGTACGGCTGGGGCTACACCGGCCTGATCAACTACGACG
GCCTGCTGAGAGTGGCCCACCTGTACATCATGGGCAACGAGAAGTGCAGCCAACACCAC
CGGGGCAAAGTGACCCTGAACGAGAGCGAGATCTGCGCCGGGGCTGAGAAAATCGGCA
GCGGCCCCTGCGAGGGGGACTACGGGGGCCCCCTGGTGTGCGAGCAGCACAAGATGAG
AATGGTGCTGGGCGTGATCGTGCCCGGCAGAGGCTGCGCCATCCCCAACAGACCCGGCA
TCTTCGTGAGAGTGGCCTACTACGCCAAGTGGATCCACAAGATCATCCTGACCTACAAGG
TGCCTCAGAGCTGA
SEQ ID NO:113
> 5'UTR-hHGF-α globin 3'UTR-A30L70 (Mod.-hHGF-OS2) 
GGGAAATAAGAGAGAAAAGAAGAGTAAGAAGAAATATAAGAGCCACCGCTAGCCTTA
AGGCCACCATGTGGGTCACCAAACTGCTGCCCGCCCTGCTGCTGCAGCACGTGCTCCTG
CACCTGCTGCTGCTGCCTATCGCCATCCCTTACGCCGAAGGCCAGAGAAAGCGGAGAA
ATACCATTCACGAATTTAAGAAGAGCGCCAAGACCACACTGATCAAGATCGACCCTGC
CCTGAAAATCAAGACCAAGAAAGTGAACACAGCCGACCAATGCGCTAATAGATGCACA
AGAAACAAAGGCCTGCCTTTCACCTGCAAGGCCTTCGTGTTCGATAAGGCCAGAAAGC
AGTGCCTGTGGTTCCCCTTCAACAGCATGAGCAGCGGCGTGAAGAAAGAATTCGGTCAC
GAGTTCGACCTGTACGAGAATAAGGACTACATTAGAAACTGCATCATCGGCAAGGGCC
GGAGCTACAAGGGCACCGTGAGCATCACCAAGTCTGGCATCAAGTGCCAGCCATGGAG
TTCCATGATCCCTCATGAGCACAGCTTCCTGCCAAGCTCTTACCGGGGAAAGGATCTGC
AGGAGAACTATTGTAGAAATCCCAGAGGCGAGGAAGGAGGACCCTGGTGTTTTACCAG
CAACCCAGAGGTGCGGTACGAGGTGTGCGACATACCCCAGTGCAGCGAGGTGGAATGC
ATGACATGCAACGGCGAGAGCTACCGGGGCCTGATGGACCACACAGAGAGCGGCAAA
 
ATCTGCCAAAGATGGGATCACCAGACCCCTCATAGGCACAAGTTCCTGCCCGAGAGAT
ACCCTGACAAGGGCTTCGACGACAATTACTGCCGGAACCCTGATGGACAGCCTAGACCT
TGGTGCTACACCCTGGACCCTCACACCAGATGGGAATACTGCGCCATCAAGACATGTGC
CGATAACACCATGAACGACACCGATGTGCCACTCGAGACAACTGAGTGTATCCAGGGC
CAGGGCGAGGGCTACAGGGGCACCGTGAACACCATCTGGAACGGCATCCCTTGTCAGC
GGTGGGACAGCCAATACCCCCACGAACATGATATGACCCCAGAAAACTTCAAGTGCAA
AGATCTGCGGGAAAACTACTGTAGAAACCCTGACGGCTCCGAGAGCCCTTGGTGCTTCA
CCACCGACCCTAACATCAGGGTGGGCTACTGTAGCCAGATCCCCAACTGCGACATGAGC
CACGGTCAGGACTGCTACAGAGGAAACGGCAAGAATTACATGGGCAACCTGAGCCAGA
CCAGAAGCGGCCTGACCTGCAGCATGTGGGACAAAAACATGGAAGATCTGCACCGGCA
CATCTTTTGGGAGCCTGACGCCAGCAAGCTGAATGAGAACTACTGTCGGAACCCTGACG
ACGATGCTCACGGCCCTTGGTGCTACACAGGCAACCCTCTGATCCCTTGGGACTATTGT
CCTATTAGCAGGTGTGAAGGCGACACGACCCCTACAATCGTGAACCTGGATCACCCAGT
GATCAGCTGTGCTAAAACAAAGCAACTGAGAGTGGTGAATGGTATCCCGACCCGGACC
AACATCGGATGGATGGTCAGCCTGCGGTACAGAAACAAGCACATCTGCGGCGGTAGCC
TGATCAAGGAATCCTGGGTGTTGACAGCCAGACAGTGTTTTCCCAGCAGAGATCTGAAG
GACTACGAGGCCTGGCTGGGCATCCACGACGTGCACGGCAGAGGCGACGAGAAGTGCA
AGCAGGTTCTGAACGTGTCCCAGCTGGTGTACGGCCCCGAGGGCTCTGATCTGGTTCTG
ATGAAGCTTGCTAGACCCGCTGTGCTGGATGACTTCGTGTCCACCATCGACCTGCCAAA
TTACGGATGTACAATTCCTGAAAAGACCTCCTGTTCTGTGTACGGCTGGGGCTACACCG
GACTCATCAACTATGACGGCCTGCTGAGAGTGGCCCACCTGTATATCATGGGCAACGAG
AAGTGCAGCCAGCACCATAGAGGCAAGGTGACCCTGAACGAGTCTGAAATCTGCGCCG
GAGCCGAGAAAATCGGATCTGGCCCCTGCGAAGGAGATTACGGCGGCCCTCTGGTCTG
CGAGCAGCACAAGATGAGAATGGTGCTGGGCGTGATCGTGCCCGGCAGAGGCTGCGCC
ATCCCTAACCGGCCTGGAATCTTCGTGCGCGTGGCCTATTACGCCAAATGGATCCACAA
AATCATCCTGACATACAAGGTCCCACAGTCTTGATAATAGGCTGGAGCCTCGGTGGCCA
TGCTTCTTGCCCCTTGGGCCTCCCCCCAGCCCCTCCTCCCCTTCCTGCACCCGTACCCCC
GTGGTCTTTGAATAAAGTCTGAGTGGGCGGCCTCGAGCCGCGGAAAAAAAAAAAAAA
AAAAAAAAAAAAAAAAGCATATGACTAAAAAAAAAAAAAAAAAAAAAAAAAAAAAA
AAAAAAAAAAAAAAAAAAAAAAAAAAAAAAAAAAAAAAAAGTTTAAACATTTAAATTC
TAGA
SEQ ID NO:114
>ACTGl 5'UTR-hHGF-CTSB 3'UTR-A30L70 A1B12-hHGF-OS2 
CTCTCGCACTCTGTTCTTCCGCCGCTCCGCCGTCGCGTTTCTCTGCCGGTCGCAGCTAGC
GCCACCATGTGGGTCACCAAACTGCTGCCCGCCCTGCTGCTGCAGCACGTGCTCCTGCA
CCTGCTGCTGCTGCCTATCGCCATCCCTTACGCCGAAGGCCAGAGAAAGCGGAGAAATA
CCATTCACGAATTTAAGAAGAGCGCCAAGACCACACTGATCAAGATCGACCCTGCCCTG
AAAATCAAGACCAAGAAAGTGAACACAGCCGACCAATGCGCTAATAGATGCACAAGA
AACAAAGGCCTGCCTTTCACCTGCAAGGCCTTCGTGTTCGATAAGGCCAGAAAGCAGTG
CCTGTGGTTCCCCTTCAACAGCATGAGCAGCGGCGTGAAGAAAGAATTCGGTCACGAGT
TCGACCTGTACGAGAATAAGGACTACATTAGAAACTGCATCATCGGCAAGGGCCGGAG
CTACAAGGGCACCGTGAGCATCACCAAGTCTGGCATCAAGTGCCAGCCATGGAGTTCC
ATGATCCCTCATGAGCACAGCTTCCTGCCAAGCTCTTACCGGGGAAAGGATCTGCAGGA
GAACTATTGTAGAAATCCCAGAGGCGAGGAAGGAGGACCCTGGTGTTTTACCAGCAAC
CCAGAGGTGCGGTACGAGGTGTGCGACATACCCCAGTGCAGCGAGGTGGAATGCATGA
CATGCAACGGCGAGAGCTACCGGGGCCTGATGGACCACACAGAGAGCGGCAAAATCTG
CCAAAGATGGGATCACCAGACCCCTCATAGGCACAAGTTCCTGCCCGAGAGATACCCT
GACAAGGGCTTCGACGACAATTACTGCCGGAACCCTGATGGACAGCCTAGACCTTGGT
GCTACACCCTGGACCCTCACACCAGATGGGAATACTGCGCCATCAAGACATGTGCCGAT
AACACCATGAACGACACCGATGTGCCACTCGAGACAACTGAGTGTATCCAGGGCCAGG
GCGAGGGCTACAGGGGCACCGTGAACACCATCTGGAACGGCATCCCTTGTCAGCGGTG
GGACAGCCAATACCCCCACGAACATGATATGACCCCAGAAAACTTCAAGTGCAAAGAT
CTGCGGGAAAACTACTGTAGAAACCCTGACGGCTCCGAGAGCCCTTGGTGCTTCACCAC
CGACCCTAACATCAGGGTGGGCTACTGTAGCCAGATCCCCAACTGCGACATGAGCCAC
GGTCAGGACTGCTACAGAGGAAACGGCAAGAATTACATGGGCAACCTGAGCCAGACCA
GAAGCGGCCTGACCTGCAGCATGTGGGACAAAAACATGGAAGATCTGCACCGGCACAT
CTTTTGGGAGCCTGACGCCAGCAAGCTGAATGAGAACTACTGTCGGAACCCTGACGAC
 
GATGCTCACGGCCCTTGGTGCTACACAGGCAACCCTCTGATCCCTTGGGACTATTGTCCT
ATTAGCAGGTGTGAAGGCGACACGACCCCTACAATCGTGAACCTGGATCACCCAGTGA
TCAGCTGTGCTAAAACAAAGCAACTGAGAGTGGTGAATGGTATCCCGACCCGGACCAA
CATCGGATGGATGGTCAGCCTGCGGTACAGAAACAAGCACATCTGCGGCGGTAGCCTG
ATCAAGGAATCCTGGGTGTTGACAGCCAGACAGTGTTTTCCCAGCAGAGATCTGAAGG
ACTACGAGGCCTGGCTGGGCATCCACGACGTGCACGGCAGAGGCGACGAGAAGTGCAA
GCAGGTTCTGAACGTGTCCCAGCTGGTGTACGGCCCCGAGGGCTCTGATCTGGTTCTGA
TGAAGCTTGCTAGACCCGCTGTGCTGGATGACTTCGTGTCCACCATCGACCTGCCAAAT
TACGGATGTACAATTCCTGAAAAGACCTCCTGTTCTGTGTACGGCTGGGGCTACACCGG
ACTCATCAACTATGACGGCCTGCTGAGAGTGGCCCACCTGTATATCATGGGCAACGAGA
AGTGCAGCCAGCACCATAGAGGCAAGGTGACCCTGAACGAGTCTGAAATCTGCGCCGG
AGCCGAGAAAATCGGATCTGGCCCCTGCGAAGGAGATTACGGCGGCCCTCTGGTCTGC
GAGCAGCACAAGATGAGAATGGTGCTGGGCGTGATCGTGCCCGGCAGAGGCTGCGCCA
TCCCTAACCGGCCTGGAATCTTCGTGCGCGTGGCCTATTACGCCAAATGGATCCACAAA
ATCATCCTGACATACAAGGTCCCACAGTCTTGATAAGGGCCCACCGGTTCTGCCGTGGG
CCTGTCGTGCCAGTCCTGGGGGCGAGATCGGGAGAATCACGTCCTGGAACTGCATGTTC
TTGCGACTCTTGGGACTTCATCTTAACTTCTCGCTGCCCCAGCCATGTTTTCAACCATGG
CATCCCTCCCCCAATTAGTTCCCTGTCATCCTCGTCAACCTTCTCTGTAAGTGCCTGGTA
AGCTTGCCCTTGCTTAAGAACTCAAAACATAGCTGTGCTCTACCGCGGAAAAAAAAAA
AAAAAAAAAAAAAAAAAAAAGCATATGACTAAAAAAAAAAAAAAAAAAAAAAAAAAA
AAAAAAAAAAAAAAAAAAAAAAAAAAAAAAAAAAAAAAAAAAAAAAGTTTAAACATTTA
AATTCTAGA
SEQ ID NO:115
>CHCHD10 5'UTR-hHGF-CTSB 3'UTR-A30L70 (A15B12-hHGF-OS2) 
GGCATTTGTCCCCGCGACAGCACCGCTGCCGCCGTCTCTAAGGTCGCCCGGGTCCCACC
GCCGCCACCGCTAGCGCCACCATGTGGGTCACCAAACTGCTGCCCGCCCTGCTGCTGCA
GCACGTGCTCCTGCACCTGCTGCTGCTGCCTATCGCCATCCCTTACGCCGAAGGCCAGA
GAAAGCGGAGAAATACCATTCACGAATTTAAGAAGAGCGCCAAGACCACACTGATCAA
GATCGACCCTGCCCTGAAAATCAAGACCAAGAAAGTGAACACAGCCGACCAATGCGCT
AATAGATGCACAAGAAACAAAGGCCTGCCTTTCACCTGCAAGGCCTTCGTGTTCGATAA
GGCCAGAAAGCAGTGCCTGTGGTTCCCCTTCAACAGCATGAGCAGCGGCGTGAAGAAA
GAATTCGGTCACGAGTTCGACCTGTACGAGAATAAGGACTACATTAGAAACTGCATCAT
CGGCAAGGGCCGGAGCTACAAGGGCACCGTGAGCATCACCAAGTCTGGCATCAAGTGC
CAGCCATGGAGTTCCATGATCCCTCATGAGCACAGCTTCCTGCCAAGCTCTTACCGGGG
AAAGGATCTGCAGGAGAACTATTGTAGAAATCCCAGAGGCGAGGAAGGAGGACCCTGG
TGTTTTACCAGCAACCCAGAGGTGCGGTACGAGGTGTGCGACATACCCCAGTGCAGCG
AGGTGGAATGCATGACATGCAACGGCGAGAGCTACCGGGGCCTGATGGACCACACAGA
GAGCGGCAAAATCTGCCAAAGATGGGATCACCAGACCCCTCATAGGCACAAGTTCCTG
CCCGAGAGATACCCTGACAAGGGCTTCGACGACAATTACTGCCGGAACCCTGATGGAC
AGCCTAGACCTTGGTGCTACACCCTGGACCCTCACACCAGATGGGAATACTGCGCCATC
AAGACATGTGCCGATAACACCATGAACGACACCGATGTGCCACTCGAGACAACTGAGT
GTATCCAGGGCCAGGGCGAGGGCTACAGGGGCACCGTGAACACCATCTGGAACGGCAT
CCCTTGTCAGCGGTGGGACAGCCAATACCCCCACGAACATGATATGACCCCAGAAAAC
TTCAAGTGCAAAGATCTGCGGGAAAACTACTGTAGAAACCCTGACGGCTCCGAGAGCC
CTTGGTGCTTCACCACCGACCCTAACATCAGGGTGGGCTACTGTAGCCAGATCCCCAAC
TGCGACATGAGCCACGGTCAGGACTGCTACAGAGGAAACGGCAAGAATTACATGGGCA
ACCTGAGCCAGACCAGAAGCGGCCTGACCTGCAGCATGTGGGACAAAAACATGGAAGA
TCTGCACCGGCACATCTTTTGGGAGCCTGACGCCAGCAAGCTGAATGAGAACTACTGTC
GGAACCCTGACGACGATGCTCACGGCCCTTGGTGCTACACAGGCAACCCTCTGATCCCT
TGGGACTATTGTCCTATTAGCAGGTGTGAAGGCGACACGACCCCTACAATCGTGAACCT
GGATCACCCAGTGATCAGCTGTGCTAAAACAAAGCAACTGAGAGTGGTGAATGGTATC
CCGACCCGGACCAACATCGGATGGATGGTCAGCCTGCGGTACAGAAACAAGCACATCT
GCGGCGGTAGCCTGATCAAGGAATCCTGGGTGTTGACAGCCAGACAGTGTTTTCCCAGC
AGAGATCTGAAGGACTACGAGGCCTGGCTGGGCATCCACGACGTGCACGGCAGAGGCG
ACGAGAAGTGCAAGCAGGTTCTGAACGTGTCCCAGCTGGTGTACGGCCCCGAGGGCTC
TGATCTGGTTCTGATGAAGCTTGCTAGACCCGCTGTGCTGGATGACTTCGTGTCCACCAT
CGACCTGCCAAATTACGGATGTACAATTCCTGAAAAGACCTCCTGTTCTGTGTACGGCT
 
GGGGCTACACCGGACTCATCAACTATGACGGCCTGCTGAGAGTGGCCCACCTGTATATC
ATGGGCAACGAGAAGTGCAGCCAGCACCATAGAGGCAAGGTGACCCTGAACGAGTCTG
AAATCTGCGCCGGAGCCGAGAAAATCGGATCTGGCCCCTGCGAAGGAGATTACGGCGG
CCCTCTGGTCTGCGAGCAGCACAAGATGAGAATGGTGCTGGGCGTGATCGTGCCCGGC
AGAGGCTGCGCCATCCCTAACCGGCCTGGAATCTTCGTGCGCGTGGCCTATTACGCCAA
ATGGATCCACAAAATCATCCTGACATACAAGGTCCCACAGTCTTGATAAGGGCCCACCG
GTTCTGCCGTGGGCCTGTCGTGCCAGTCCTGGGGGCGAGATCGGGAGAATCACGTCCTG
GAACTGCATGTTCTTGCGACTCTTGGGACTTCATCTTAACTTCTCGCTGCCCCAGCCATG
TTTTCAACCATGGCATCCCTCCCCCAATTAGTTCCCTGTCATCCTCGTCAACCTTCTCTGT
AAGTGCCTGGTAAGCTTGCCCTTGCTTAAGAACTCAAAACATAGCTGTGCTCTACCGCG
GAAAAAAAAAAAAAAAAAAAAAAAAAAAAAAAAAGCATATGACTAAAAAAAAAAAAAAAA
AAAAAAAAAAAAAAAAAAAAAAAAAAAAAAAAAAAAAAAAAAAAAAAAAAAAAAAAAAAG
TTTAAACATTTAAATTCTAGA
SEQ ID NO:116
> PD-1 antibody heavy chain amino acid sequence 
MEWSWVFLFFLSVTTGVHSEVQLVESGGGLVQPGGSLRLSCAASGFTFSSYMMSWVRQAP
GKGLEWVATISGGGANTYYPDSVKGRFTISRDNAKNSLYLQMNSLRAEDTAVYYCARQLY
YFDYWGQGTTVTVSSASTKGPSVFPLAPCSRSTSESTAALGCLVKDYFPEPVTVSWNSGAL
TSGVHTFPAVLQSSGLYSLSSVVTVPSSSLGTKTYTCNVDHKPSNTKVDKRVESKYGPPCPP
CPAPEFLGGPSVFLFPPKPKDTLMISRTPEVTCVVVDVSQEDPEVQFNWYVDGVEVHNAKT
KPREEQFNSTYRVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK
SEQ ID NO:117
> PD-1 antibody light chain amino acid sequence 
MSVPTQVLGLLLLWLTDARCDIQMTQSPSSLSASVGDRVTITCLASQTIGTWLTWYQQKPG
KAPKLLIYTATSLADGVPSRFSGSGSGTDFTLTISSLQPEDFATYYCQQVYSIPWTFGGGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO:118
> PD-1 antibody heavy chain DNA sequence 
ATGGAATGGTCCTGGGTGTTCCTGTTCTTCCTGTCCGTGACCACCGGCGTGCACTCCGAA
GTGCAGCTGGTGGAATCTGGCGGCGGACTGGTGCAGCCTGGCGGATCTCTGAGACTGTC
TTGTGCCGCCTCCGGCTTCACCTTCTCCAGCTACATGATGAGCTGGGTGCGACAGGCCC
CTGGCAAGGGACTGGAATGGGTGGCCACAATCTCTGGCGGAGGCGCCAACACCTACTA
CCCCGACTCTGTGAAGGGCCGGTTCACCATCTCTCGGGACAACGCCAAGAACTCCCTGT
ACCTGCAGATGAACAGCCTGCGGGCCGAGGACACCGCCGTGTACTACTGTGCCCGGCA
GCTGTACTACTTCGACTACTGGGGCCAGGGCACCACCGTGACCGTGTCCTCTGCTTCTA
CCAAGGGCCCCTCCGTGTTCCCTCTGGCTCCTTGCTCCAGATCCACCTCCGAGTCCACCG
CTGCTCTGGGCTGCCTCGTGAAGGACTACTTCCCCGAGCCTGTGACAGTGTCCTGGAAC
TCTGGCGCCCTGACCTCTGGCGTGCACACCTTTCCAGCAGTGCTGCAGTCCTCCGGCCTG
TACTCCCTGTCCTCCGTCGTGACAGTGCCCTCCAGCTCTCTGGGCACCAAGACCTACACC
TGTAACGTGGACCACAAGCCCTCCAACACCAAGGTGGACAAGAGAGTGGAATCTAAGT
ACGGCCCTCCCTGCCCCCCTTGTCCTGCCCCTGAATTTCTGGGCGGACCCTCTGTGTTTC
TGTTCCCCCCAAAGCCCAAGGACACCCTGATGATCTCCCGGACCCCCGAAGTGACCTGC
GTGGTGGTGGATGTGTCCCAGGAAGATCCCGAGGTGCAGTTCAATTGGTACGTGGACG
GCGTGGAAGTGCACAATGCCAAGACCAAGCCTAGAGAGGAACAGTTCAACTCCACCTA
CCGGGTGGTGTCCGTGCTGACCGTGCTGCATCAGGACTGGCTGAACGGCAAAGAGTAC
AAGTGCAAGGTGTCCAACAAGGGCCTGCCCAGCTCCATCGAAAAGACCATCTCCAAGG
CCAAGGGCCAGCCCCGGGAACCCCAGGTGTACACACTGCCTCCAAGCCAGGAAGAGAT
GACCAAGAACCAGGTGTCCCTGACCTGCCTCGTGAAAGGCTTCTACCCCTCCGATATCG
CCGTGGAATGGGAGTCCAACGGCCAGCCTGAGAACAACTACAAGACCACCCCCCCTGT
GCTGGACTCCGACGGCTCCTTCTTTCTGTACTCTCGCCTGACCGTGGACAAGTCCCGGTG
GCAGGAAGGCAACGTGTTCTCCTGCAGCGTGATGCACGAGGCCCTGCACAACCACTAC
ACCCAGAAGTCCCTGTCCCTGTCTCTGGGAAAG
 SEQ ID NO:119
> **PD-1** antibody light chain DNA sequence 
ATGTCCGTGCCTACCCAGGTGCTGGGACTGCTGCTGCTGTGGCTGACCGACGCCAGATG
CGACATCCAGATGACCCAGTCCCCCTCCAGCCTGTCTGCCTCTGTGGGCGACAGAGTGA
CCATCACCTGTCTGGCCTCCCAGACCATCGGCACCTGGCTGACCTGGTATCAGCAGAAG
CCTGGCAAGGCCCCCAAGCTGCTGATCTACACCGCCACCAGTCTGGCCGATGGCGTGCC
CTCTAGATTCTCCGGCTCTGGCTCTGGCACCGACTTTACCCTGACCATCAGCTCCCTGCA
GCCCGAGGACTTCGCCACCTACTACTGCCAGCAGGTGTACTCCATCCCCTGGACCTTTG
GCGGAGGCACCAAGGTGGAAATCAAGCGGACCGTGGCCGCTCCCTCCGTGTTCATCTTC
CCACCTTCCGACGAGCAGCTGAAGTCCGGAACCGCCAGTGTCGTGTGCCTGCTGAACAA
CTTCTACCCCCGCGAGGCCAAGGTGCAGTGGAAGGTGGACAACGCCCTGCAGTCCGGC
AACTCCCAGGAATCCGTGACCGAGCAGGACTCCAAGGACAGCACCTACTCCCTGTCCTC
CACCCTGACCCTGTCCAAGGCCGACTACGAGAAGCACAAGGTGTACGCCTGCGAAGTG
ACCCACCAGGGCCTGTCTAGCCCCGTGACCAAGTCTTTCAACCGGGGCGAGTGC
SEQ ID NO:120
> **PD-1** antibody heavy chain mRNA sequence 
AUGGAAUGGUCCUGGGUGUUCCUGUUCUUCCUGUCCGUGACCACCGGCGUGCACUCC
GAAGUGCAGCUGGUGGAAUCUGGCGGCGGACUGGUGCAGCCUGGCGGAUCUCUGAG
ACUGUCUUGUGCCGCCUCCGGCUUCACCUUCUCCAGCUACAUGAUGAGCUGGGUGCG
ACAGGCCCCUGGCAAGGGACUGGAAUGGGUGGCCACAAUCUCUGGCGGAGGCGCCAA
CACCUACUACCCCGACUCUGUGAAGGGCCGGUUCACCAUCUCUCGGGACAACGCCAA
GAACUCCCUGUACCUGCAGAUGAACAGCCUGCGGGCCGAGGACACCGCCGUGUACUA
CUGUGCCCGGCAGCUGUACUACUUCGACUACUGGGGCCAGGGCACCACCGUGACCGU
GUCCUCUGCUUCUACCAAGGGCCCCUCCGUGUUCCCUCUGGCUCCUUGCUCCAGAUC
CACCUCCGAGUCCACCGCUGCUCUGGGCUGCCUCGUGAAGGACUACUUCCCCGAGCC
UGUGACAGUGUCCUGGAACUCUGGCGCCCUGACCUCUGGCGUGCACACCUUUCCAGC
AGUGCUGCAGUCCUCCGGCCUGUACUCCCUGUCCUCCGUCGUGACAGUGCCCUCCAG
CUCUCUGGGCACCAAGACCUACACCUGUAACGUGGACCACAAGCCCUCCAACACCAA
GGUGGACAAGAGAGUGGAAUCUAAGUACGGCCCUCCCUGCCCCCCUUGUCCUGCCCC
UGAAUUUCUGGGCGGACCCUCUGUGUUUCUGUUCCCCCCAAAGCCCAAGGACACCCU
GAUGAUCUCCCGGACCCCCGAAGUGACCUGCGUGGUGGUGGAUGUGUCCCAGGAAGA
UCCCGAGGUGCAGUUCAAUUGGUACGUGGACGGCGUGGAAGUGCACAAUGCCAAGAC
CAAGCCUAGAGAGGAACAGUUCAACUCCACCUACCGGGUGGUGUCCGUGCUGACCGU
GCUGCAUCAGGACUGGCUGAACGGCAAAGAGUACAAGUGCAAGGUGUCCAACAAGG
GCCUGCCCAGCUCCAUCGAAAAGACCAUCUCCAAGGCCAAGGGCCAGCCCCGGGAAC
CCCAGGUGUACACACUGCCUCCAAGCCAGGAAGAGAUGACCAAGAACCAGGUGUCCC
UGACCUGCCUCGUGAAAGGCUUCUACCCCUCCGAUAUCGCCGUGGAAUGGGAGUCCA
ACGGCCAGCCUGAGAACAACUACAAGACCACCCCCCCUGUGCUGGACUCCGACGGCU
CCUUCUUUCUGUACUCUCGCCUGACCGUGGACAAGUCCCGGUGGCAGGAAGGCAACG
UGUUCUCCUGCAGCGUGAUGCACGAGGCCCUGCACAACCACUACACCCAGAAGUCCC
UGUCCCUGUCUCUGGGAAAG
SEQ ID NO:121
> **PD-1** antibody light chain mRNA sequence 
AUGUCCGUGCCUACCCAGGUGCUGGGACUGCUGCUGCUGUGGCUGACCGACGCCAGA
UGCGACAUCCAGAUGACCCAGUCCCCCUCCAGCCUGUCUGCCUCUGUGGGCGACAGA
GUGACCAUCACCUGUCUGGCCUCCCAGACCAUCGGCACCUGGCUGACCUGGUAUCAG
CAGAAGCCUGGCAAGGCCCCCAAGCUGCUGAUCUACACCGCCACCAGUCUGGCCGAU
GGCGUGCCCUCUAGAUUCUCCGGCUCUGGCUCUGGCACCGACUUUACCCUGACCAUC
AGCUCCCUGCAGCCCGAGGACUUCGCCACCUACUACUGCCAGCAGGUGUACUCCAUC
CCCUGGACCUUUGGCGGAGGCACCAAGGUGGAAAUCAAGCGGACCGUGGCCGCUCCC
UCCGUGUUCAUCUUCCCACCUUCCGACGAGCAGCUGAAGUCCGGAACCGCCAGUGUC
GUGUGCCUGCUGAACAACUUCUACCCCCGCGAGGCCAAGGUGCAGUGGAAGGUGGAC
AACGCCCUGCAGUCCGGCAACUCCCAGGAAUCCGUGACCGAGCAGGACUCCAAGGAC
AGCACCUACUCCCUGUCCUCCACCCUGACCCUGUCCAAGGCCGACUACGAGAAGCAC
AAGGUGUACGCCUGCGAAGUGACCCACCAGGGCCUGUCUAGCCCCGUGACCAAGUCU
 
UUCAACCGGGGCGAGUGC
SEQ ID NO:122
> 5'UTR-anti PD-1-α globin 3'UTR-A30L70 (Mod.-anti PD-1) 
GGGAAATAAGAGAGAAAAGAAGAGTAAGAAGAAATATAAGAGCCACCATGGAATGGT
CCTGGGTGTTCCTGTTCTTCCTGTCCGTGACCACCGGCGTGCACTCCGAAGTGCAGCTGG
TGGAATCTGGCGGCGGACTGGTGCAGCCTGGCGGATCTCTGAGACTGTCTTGTGCCGCC
TCCGGCTTCACCTTCTCCAGCTACATGATGAGCTGGGTGCGACAGGCCCCTGGCAAGGG
ACTGGAATGGGTGGCCACAATCTCTGGCGGAGGCGCCAACACCTACTACCCCGACTCTG
TGAAGGGCCGGTTCACCATCTCTCGGGACAACGCCAAGAACTCCCTGTACCTGCAGATG
AACAGCCTGCGGGCCGAGGACACCGCCGTGTACTACTGTGCCCGGCAGCTGTACTACTT
CGACTACTGGGGCCAGGGCACCACCGTGACCGTGTCCTCTGCTTCTACCAAGGGCCCCT
CCGTGTTCCCTCTGGCTCCTTGCTCCAGATCCACCTCCGAGTCCACCGCTGCTCTGGGCT
GCCTCGTGAAGGACTACTTCCCCGAGCCTGTGACAGTGTCCTGGAACTCTGGCGCCCTG
ACCTCTGGCGTGCACACCTTTCCAGCAGTGCTGCAGTCCTCCGGCCTGTACTCCCTGTCC
TCCGTCGTGACAGTGCCCTCCAGCTCTCTGGGCACCAAGACCTACACCTGTAACGTGGA
CCACAAGCCCTCCAACACCAAGGTGGACAAGAGAGTGGAATCTAAGTACGGCCCTCCC
TGCCCCCCTTGTCCTGCCCCTGAATTTCTGGGCGGACCCTCTGTGTTTCTGTTCCCCCCA
AAGCCCAAGGACACCCTGATGATCTCCCGGACCCCCGAAGTGACCTGCGTGGTGGTGG
ATGTGTCCCAGGAAGATCCCGAGGTGCAGTTCAATTGGTACGTGGACGGCGTGGAAGT
GCACAATGCCAAGACCAAGCCTAGAGAGGAACAGTTCAACTCCACCTACCGGGTGGTG
TCCGTGCTGACCGTGCTGCATCAGGACTGGCTGAACGGCAAAGAGTACAAGTGCAAGG
TGTCCAACAAGGGCCTGCCCAGCTCCATCGAAAAGACCATCTCCAAGGCCAAGGGCCA
GCCCCGGGAACCCCAGGTGTACACACTGCCTCCAAGCCAGGAAGAGATGACCAAGAAC
CAGGTGTCCCTGACCTGCCTCGTGAAAGGCTTCTACCCCTCCGATATCGCCGTGGAATG
GGAGTCCAACGGCCAGCCTGAGAACAACTACAAGACCACCCCCCCTGTGCTGGACTCC
GACGGCTCCTTCTTTCTGTACTCTCGCCTGACCGTGGACAAGTCCCGGTGGCAGGAAGG
CAACGTGTTCTCCTGCAGCGTGATGCACGAGGCCCTGCACAACCACTACACCCAGAAGT
CCCTGTCCCTGTCTCTGGGAAAGGCCACCAACTTCTCCCTGCTGAAGCAGGCCGGCGAC
GTGGAGGAGAACCCCGGCCCCATGTCCGTGCCTACCCAGGTGCTGGGACTGCTGCTGCT
GTGGCTGACCGACGCCAGATGCGACATCCAGATGACCCAGTCCCCCTCCAGCCTGTCTG
CCTCTGTGGGCGACAGAGTGACCATCACCTGTCTGGCCTCCCAGACCATCGGCACCTGG
CTGACCTGGTATCAGCAGAAGCCTGGCAAGGCCCCCAAGCTGCTGATCTACACCGCCAC
CAGTCTGGCCGATGGCGTGCCCTCTAGATTCTCCGGCTCTGGCTCTGGCACCGACTTTAC
CCTGACCATCAGCTCCCTGCAGCCCGAGGACTTCGCCACCTACTACTGCCAGCAGGTGT
ACTCCATCCCCTGGACCTTTGGCGGAGGCACCAAGGTGGAAATCAAGCGGACCGTGGC
CGCTCCCTCCGTGTTCATCTTCCCACCTTCCGACGAGCAGCTGAAGTCCGGAACCGCCA
GTGTCGTGTGCCTGCTGAACAACTTCTACCCCCGCGAGGCCAAGGTGCAGTGGAAGGTG
GACAACGCCCTGCAGTCCGGCAACTCCCAGGAATCCGTGACCGAGCAGGACTCCAAGG
ACAGCACCTACTCCCTGTCCTCCACCCTGACCCTGTCCAAGGCCGACTACGAGAAGCAC
AAGGTGTACGCCTGCGAAGTGACCCACCAGGGCCTGTCTAGCCCCGTGACCAAGTCTTT
CAACCGGGGCGAGTGCTGATAAGGGCCCACCGGTTGATAATAGGCTGGAGCCTCGGTG
GCCATGCTTCTTGCCCCTTGGGCCTCCCCCCAGCCCCTCCTCCCCTTCCTGCACCCGTAC
CCCCGTGGTCTTTGAATAAAGTCTGAGTGGGCGGCCTCGAGCCGCGGAAAAAAAAAAA
AAAAAAAAAAAAAAAAAAAGCATATGACTAAAAAAAAAAAAAAAAAAAAAAAAAAAA
AAAAAAAAAAAAAAAAAAAAAAAAAAAAAAAAAAAAAAAAAAAAAAATTATTGTTTAAACA
TTTAAATTCTAGA
SEQ ID NO:123
>ACTG1 5'UTR- anti PD-1-CTSB 3'UTR-A30L70 (A1B12- anti PD-1) 
CTCTCGCACTCTGTTCTTCCGCCGCTCCGCCGTCGCGTTTCTCTGCCGGTCGCACCACCA
TGGAATGGTCCTGGGTGTTCCTGTTCTTCCTGTCCGTGACCACCGGCGTGCACTCCGAAG
TGCAGCTGGTGGAATCTGGCGGCGGACTGGTGCAGCCTGGCGGATCTCTGAGACTGTCT
TGTGCCGCCTCCGGCTTCACCTTCTCCAGCTACATGATGAGCTGGGTGCGACAGGCCCC
TGGCAAGGGACTGGAATGGGTGGCCACAATCTCTGGCGGAGGCGCCAACACCTACTAC
CCCGACTCTGTGAAGGGCCGGTTCACCATCTCTCGGGACAACGCCAAGAACTCCCTGTA
CCTGCAGATGAACAGCCTGCGGGCCGAGGACACCGCCGTGTACTACTGTGCCCGGCAG
CTGTACTACTTCGACTACTGGGGCCAGGGCACCACCGTGACCGTGTCCTCTGCTTCTACC
 
AAGGGCCCCTCCGTGTTCCCTCTGGCTCCTTGCTCCAGATCCACCTCCGAGTCCACCGCT
GCTCTGGGCTGCCTCGTGAAGGACTACTTCCCCGAGCCTGTGACAGTGTCCTGGAACTC
TGGCGCCCTGACCTCTGGCGTGCACACCTTTCCAGCAGTGCTGCAGTCCTCCGGCCTGT
ACTCCCTGTCCTCCGTCGTGACAGTGCCCTCCAGCTCTCTGGGCACCAAGACCTACACCT
GTAACGTGGACCACAAGCCCTCCAACACCAAGGTGGACAAGAGAGTGGAATCTAAGTA
CGGCCCTCCCTGCCCCCCTTGTCCTGCCCCTGAATTTCTGGGCGGACCCTCTGTGTTTCT
GTTCCCCCCAAAGCCCAAGGACACCCTGATGATCTCCCGGACCCCCGAAGTGACCTGCG
TGGTGGTGGATGTGTCCCAGGAAGATCCCGAGGTGCAGTTCAATTGGTACGTGGACGGC
GTGGAAGTGCACAATGCCAAGACCAAGCCTAGAGAGGAACAGTTCAACTCCACCTACC
GGGTGGTGTCCGTGCTGACCGTGCTGCATCAGGACTGGCTGAACGGCAAAGAGTACAA
GTGCAAGGTGTCCAACAAGGGCCTGCCCAGCTCCATCGAAAAGACCATCTCCAAGGCC
AAGGGCCAGCCCCGGGAACCCCAGGTGTACACACTGCCTCCAAGCCAGGAAGAGATGA
CCAAGAACCAGGTGTCCCTGACCTGCCTCGTGAAAGGCTTCTACCCCTCCGATATCGCC
GTGGAATGGGAGTCCAACGGCCAGCCTGAGAACAACTACAAGACCACCCCCCCTGTGC
TGGACTCCGACGGCTCCTTCTTTCTGTACTCTCGCCTGACCGTGGACAAGTCCCGGTGGC
AGGAAGGCAACGTGTTCTCCTGCAGCGTGATGCACGAGGCCCTGCACAACCACTACAC
CCAGAAGTCCCTGTCCCTGTCTCTGGGAAAGGCCACCAACTTCTCCCTGCTGAAGCAGG
CCGGCGACGTGGAGGAGAACCCCGGCCCCATGTCCGTGCCTACCCAGGTGCTGGGACT
GCTGCTGCTGTGGCTGACCGACGCCAGATGCGACATCCAGATGACCCAGTCCCCCTCCA
GCCTGTCTGCCTCTGTGGGCGACAGAGTGACCATCACCTGTCTGGCCTCCCAGACCATC
GGCACCTGGCTGACCTGGTATCAGCAGAAGCCTGGCAAGGCCCCCAAGCTGCTGATCTA
CACCGCCACCAGTCTGGCCGATGGCGTGCCCTCTAGATTCTCCGGCTCTGGCTCTGGCA
CCGACTTTACCCTGACCATCAGCTCCCTGCAGCCCGAGGACTTCGCCACCTACTACTGC
CAGCAGGTGTACTCCATCCCCTGGACCTTTGGCGGAGGCACCAAGGTGGAAATCAAGC
GGACCGTGGCCGCTCCCTCCGTGTTCATCTTCCCACCTTCCGACGAGCAGCTGAAGTCC
GGAACCGCCAGTGTCGTGTGCCTGCTGAACAACTTCTACCCCCGCGAGGCCAAGGTGCA
GTGGAAGGTGGACAACGCCCTGCAGTCCGGCAACTCCCAGGAATCCGTGACCGAGCAG
GACTCCAAGGACAGCACCTACTCCCTGTCCTCCACCCTGACCCTGTCCAAGGCCGACTA
CGAGAAGCACAAGGTGTACGCCTGCGAAGTGACCCACCAGGGCCTGTCTAGCCCCGTG
ACCAAGTCTTTCAACCGGGGCGAGTGCTGATAAGGGCCCACCGGTCTGCCGTGGGCCTG
TCGTGCCAGTCCTGGGGGCGAGATCGGGAGAATCACGTCCTGGAACTGCATGTTCTTGC
GACTCTTGGGACTTCATCTTAACTTCTCGCTGCCCCAGCCATGTTTTCAACCATGGCATC
CCTCCCCCAATTAGTTCCCTGTCATCCTCGTCAACCTTCTCTGTAAGTGCCTGGTAAGCT
TGCCCTTGCTTAAGAACTCAAAACATAGCTGTGCTCTACTCGAGCCGCGGAAAAAAAAA
AAAAAAAAAAAAAAAAAAAAAGCATATGACTAAAAAAAAAAAAAAAAAAAAAAAAA
AAAAAAAAAAAAAAAAAAAAAAAAAAAAAAAAAAAAAAAAAAAAATTATTGTTTAAA
CATTTAAATTCTAGA
SEQ ID NO:124
>CHCHD10 5'UTR- anti PD-1-CTSB 3'UTR-A30L70 (A15B12- anti PD-1) 
GGCATTTGTCCCCGCGACAGCACCGCTGCCGCCGTCTCTAAGGTCGCCCGGGTCCCACC
GCCGCCACCATGGAATGGTCCTGGGTGTTCCTGTTCTTCCTGTCCGTGACCACCGGCGTG
CACTCCGAAGTGCAGCTGGTGGAATCTGGCGGCGGACTGGTGCAGCCTGGCGGATCTCT
GAGACTGTCTTGTGCCGCCTCCGGCTTCACCTTCTCCAGCTACATGATGAGCTGGGTGC
GACAGGCCCCTGGCAAGGGACTGGAATGGGTGGCCACAATCTCTGGCGGAGGCGCCAA
CACCTACTACCCCGACTCTGTGAAGGGCCGGTTCACCATCTCTCGGGACAACGCCAAGA
ACTCCCTGTACCTGCAGATGAACAGCCTGCGGGCCGAGGACACCGCCGTGTACTACTGT
GCCCGGCAGCTGTACTACTTCGACTACTGGGGCCAGGGCACCACCGTGACCGTGTCCTC
TGCTTCTACCAAGGGCCCCTCCGTGTTCCCTCTGGCTCCTTGCTCCAGATCCACCTCCGA
GTCCACCGCTGCTCTGGGCTGCCTCGTGAAGGACTACTTCCCCGAGCCTGTGACAGTGT
CCTGGAACTCTGGCGCCCTGACCTCTGGCGTGCACACCTTTCCAGCAGTGCTGCAGTCC
TCCGGCCTGTACTCCCTGTCCTCCGTCGTGACAGTGCCCTCCAGCTCTCTGGGCACCAAG
ACCTACACCTGTAACGTGGACCACAAGCCCTCCAACACCAAGGTGGACAAGAGAGTGG
AATCTAAGTACGGCCCTCCCTGCCCCCCTTGTCCTGCCCCTGAATTTCTGGGCGGACCCT
CTGTGTTTCTGTTCCCCCCAAAGCCCAAGGACACCCTGATGATCTCCCGGACCCCCGAA
GTGACCTGCGTGGTGGTGGATGTGTCCCAGGAAGATCCCGAGGTGCAGTTCAATTGGTA
CGTGGACGGCGTGGAAGTGCACAATGCCAAGACCAAGCCTAGAGAGGAACAGTTCAAC
TCCACCTACCGGGTGGTGTCCGTGCTGACCGTGCTGCATCAGGACTGGCTGAACGGCAA
 
AGAGTACAAGTGCAAGGTGTCCAACAAGGGCCTGCCCAGCTCCATCGAAAAGACCATC
TCCAAGGCCAAGGGCCAGCCCCGGGAACCCCAGGTGTACACACTGCCTCCAAGCCAGG
AAGAGATGACCAAGAACCAGGTGTCCCTGACCTGCCTCGTGAAAGGCTTCTACCCCTCC
GATATCGCCGTGGAATGGGAGTCCAACGGCCAGCCTGAGAACAACTACAAGACCACCC
CCCCTGTGCTGGACTCCGACGGCTCCTTCTTTCTGTACTCTCGCCTGACCGTGGACAAGT
CCCGGTGGCAGGAAGGCAACGTGTTCTCCTGCAGCGTGATGCACGAGGCCCTGCACAA
CCACTACACCCAGAAGTCCCTGTCCCTGTCTCTGGGAAAGGCCACCAACTTCTCCCTGC
TGAAGCAGGCCGGCGACGTGGAGGAGAACCCCGGCCCCATGTCCGTGCCTACCCAGGT
GCTGGGACTGCTGCTGCTGTGGCTGACCGACGCCAGATGCGACATCCAGATGACCCAGT
CCCCCTCCAGCCTGTCTGCCTCTGTGGGCGACAGAGTGACCATCACCTGTCTGGCCTCCC
AGACCATCGGCACCTGGCTGACCTGGTATCAGCAGAAGCCTGGCAAGGCCCCCAAGCT
GCTGATCTACACCGCCACCAGTCTGGCCGATGGCGTGCCCTCTAGATTCTCCGGCTCTG
GCTCTGGCACCGACTTTACCCTGACCATCAGCTCCCTGCAGCCCGAGGACTTCGCCACC
TACTACTGCCAGCAGGTGTACTCCATCCCCTGGACCTTTGGCGGAGGCACCAAGGTGGA
AATCAAGCGGACCGTGGCCGCTCCCTCCGTGTTCATCTTCCCACCTTCCGACGAGCAGC
TGAAGTCCGGAACCGCCAGTGTCGTGTGCCTGCTGAACAACTTCTACCCCCGCGAGGCC
AAGGTGCAGTGGAAGGTGGACAACGCCCTGCAGTCCGGCAACTCCCAGGAATCCGTGA
CCGAGCAGGACTCCAAGGACAGCACCTACTCCCTGTCCTCCACCCTGACCCTGTCCAAG
GCCGACTACGAGAAGCACAAGGTGTACGCCTGCGAAGTGACCCACCAGGGCCTGTCTA
GCCCCGTGACCAAGTCTTTCAACCGGGGCGAGTGCTGATAAGGGCCCACCGGTCTGCCG
TGGGCCTGTCGTGCCAGTCCTGGGGGCGAGATCGGGAGAATCACGTCCTGGAACTGCAT
GTTCTTGCGACTCTTGGGACTTCATCTTAACTTCTCGCTGCCCCAGCCATGTTTTCAACC
ATGGCATCCCTCCCCCAATTAGTTCCCTGTCATCCTCGTCAACCTTCTCTGTAAGTGCCT
GGTAAGCTTGCCCTTGCTTAAGAACTCAAAACATAGCTGTGCTCTACTCGAGCCGCGGA
AAAAAAAAAAAAAAAAAAAAAAAAAAAAAAAAAGCATATGACTAAAAAAAAAAAAAAAAAA
AAAAAAAAAAAAAAAAAAAAAAAAAAAAAAAAAAAAAAAAAAAAAAAAAAAAAAAAATTA
TTGTTTAAACATTTAAATTCTAGA
SEQ ID NO:125
> Firefly luciferase nucleotide sequence (Firefly Luciferase, Fluc) 
ATGGAAGACGCCAAAAACATAAAGAAAGGCCCGGCGCCATTCTATCCGCTGGAAGATG
GAACCGCTGGAGAGCAACTGCATAAGGCTATGAAGAGATACGCCCTGGTTCCTGGAAC
AATTGCTTTTACAGATGCACATATCGAGGTGGACATCACTTACGCTGAGTACTTCGAAA
TGTCCGTTCGGTTGGCAGAAGCTATGAAACGATATGGGCTGAATACAAATCACAGAATC
GTCGTATGCAGTGAAAACTCTCTTCAATTCTTTATGCCGGTGTTGGGCGCGTTATTTATC
GGAGTTGCAGTTGCGCCCGCGAACGACATTTATAATGAACGTGAATTGCTCAACAGTAT
GGGCATTTCGCAGCCTACCGTGGTGTTCGTTTCCAAAAAGGGGTTGCAAAAAATTTTGA
ACGTGCAAAAAAAGCTCCCAATCATCCAAAAAATTATTATCATGGATTCTAAAACGGAT
TACCAGGGATTTCAGTCGATGTACACGTTCGTCACATCTCATCTACCTCCCGGTTTTAAT
GAATACGATTTTGTGCCAGAGTCCTTCGATAGGGACAAGACAATTGCACTGATCATGAA
CTCCTCTGGATCTACTGGTCTGCCTAAAGGTGTCGCTCTGCCTCATAGAACTGCCTGCGT
GAGATTCTCGCATGCCAGAGATCCTATTTTTGGCAATCAAATCATTCCGGATACTGCGA
TTTTAAGTGTTGTTCCATTCCATCACGGTTTTGGAATGTTTACTACACTCGGATATTTGAT
ATGTGGATTTCGAGTCGTCTTAATGTATAGATTTGAAGAAGAGCTGTTTCTGAGGAGCC
TTCAGGATTACAAGATTCAAAGTGCGCTGCTGGTGCCAACCCTATTCTCCTTCTTCGCCA
AAAGCACTCTGATTGACAAATACGATTTATCTAATTTACACGAAATTGCTTCTGGTGGC
GCTCCCCTCTCTAAGGAAGTCGGGGAAGCGGTTGCCAAGAGGTTCCATCTGCCAGGTAT
CAGGCAAGGATATGGGCTCACTGAGACTACATCAGCTATTCTGATTACACCCGAGGGG
GATGATAAACCGGGCGCGGTCGGTAAAGTTGTTCCATTTTTTGAAGCGAAGGTTGTGGA
TCTGGATACCGGGAAAACGCTGGGCGTTAATCAAAGAGGCGAACTGTGTGTGAGAGGT
CCTATGATTATGTCCGGTTATGTAAACAATCCGGAAGCGACCAACGCCTTGATTGACAA
GGATGGATGGCTACATTCTGGAGACATAGCTTACTGGGACGAAGACGAACACTTCTTCA
TCGTTGACCGCCTGAAGTCTCTGATTAAGTACAAAGGCTATCAGGTGGCTCCCGCTGAA
TTGGAATCCATCTTGCTCCAACACCCCAACATCTTCGACGCAGGTGTCGCAGGTCTTCCC
GACGATGACGCCGGTGAACTTCCCGCCGCCGTTGTTGTTTTGGAGCACGGAAAGACGAT
GACGGAAAAAGAGATCGTGGATTACGTCGCCAGTCAAGTAACAACCGCGAAAAAGTTG
CGCGGAGGAGTTGTGTTTGTGGACGAAGTACCGAAAGGTCTTACCGGAAAACTCGACG
CAAGAAAAATCAGAGAGATCCTCATAAAGGCCAAGAAGGGCGGAAAGATCGCCGTGT
 
AA
SEQ ID NO:126
> **HGF-OS2** expression vector CHCHD10 5'UTR-hHGF-CTSB 3'UTR-A30L70 (A16B12-HGF-OS2) 
CCGCTGCCGCCGTCTCTAAGGTCGCCCGGGTCCCACCGCCGCCACCGCTAGCGCCACCA
TGTGGGTCACCAAACTGCTGCCCGCCCTGCTGCTGCAGCACGTGCTCCTGCACCTGCTG
CTGCTGCCTATCGCCATCCCTTACGCCGAAGGCCAGAGAAAGCGGAGAAATACCATTCA
CGAATTTAAGAAGAGCGCCAAGACCACACTGATCAAGATCGACCCTGCCCTGAAAATC
AAGACCAAGAAAGTGAACACAGCCGACCAATGCGCTAATAGATGCACAAGAAACAAA
GGCCTGCCTTTCACCTGCAAGGCCTTCGTGTTCGATAAGGCCAGAAAGCAGTGCCTGTG
GTTCCCCTTCAACAGCATGAGCAGCGGCGTGAAGAAAGAATTCGGTCACGAGTTCGAC
CTGTACGAGAATAAGGACTACATTAGAAACTGCATCATCGGCAAGGGCCGGAGCTACA
AGGGCACCGTGAGCATCACCAAGTCTGGCATCAAGTGCCAGCCATGGAGTTCCATGATC
CCTCATGAGCACAGCTTCCTGCCAAGCTCTTACCGGGGAAAGGATCTGCAGGAGAACTA
TTGTAGAAATCCCAGAGGCGAGGAAGGAGGACCCTGGTGTTTTACCAGCAACCCAGAG
GTGCGGTACGAGGTGTGCGACATACCCCAGTGCAGCGAGGTGGAATGCATGACATGCA
ACGGCGAGAGCTACCGGGGCCTGATGGACCACACAGAGAGCGGCAAAATCTGCCAAAG
ATGGGATCACCAGACCCCTCATAGGCACAAGTTCCTGCCCGAGAGATACCCTGACAAG
GGCTTCGACGACAATTACTGCCGGAACCCTGATGGACAGCCTAGACCTTGGTGCTACAC
CCTGGACCCTCACACCAGATGGGAATACTGCGCCATCAAGACATGTGCCGATAACACC
ATGAACGACACCGATGTGCCACTCGAGACAACTGAGTGTATCCAGGGCCAGGGCGAGG
GCTACAGGGGCACCGTGAACACCATCTGGAACGGCATCCCTTGTCAGCGGTGGGACAG
CCAATACCCCCACGAACATGATATGACCCCAGAAAACTTCAAGTGCAAAGATCTGCGG
GAAAACTACTGTAGAAACCCTGACGGCTCCGAGAGCCCTTGGTGCTTCACCACCGACCC
TAACATCAGGGTGGGCTACTGTAGCCAGATCCCCAACTGCGACATGAGCCACGGTCAG
GACTGCTACAGAGGAAACGGCAAGAATTACATGGGCAACCTGAGCCAGACCAGAAGCG
GCCTGACCTGCAGCATGTGGGACAAAAACATGGAAGATCTGCACCGGCACATCTTTTGG
GAGCCTGACGCCAGCAAGCTGAATGAGAACTACTGTCGGAACCCTGACGACGATGCTC
ACGGCCCTTGGTGCTACACAGGCAACCCTCTGATCCCTTGGGACTATTGTCCTATTAGC
AGGTGTGAAGGCGACACGACCCCTACAATCGTGAACCTGGATCACCCAGTGATCAGCT
GTGCTAAAACAAAGCAACTGAGAGTGGTGAATGGTATCCCGACCCGGACCAACATCGG
ATGGATGGTCAGCCTGCGGTACAGAAACAAGCACATCTGCGGCGGTAGCCTGATCAAG
GAATCCTGGGTGTTGACAGCCAGACAGTGTTTTCCCAGCAGAGATCTGAAGGACTACGA
GGCCTGGCTGGGCATCCACGACGTGCACGGCAGAGGCGACGAGAAGTGCAAGCAGGTT
CTGAACGTGTCCCAGCTGGTGTACGGCCCCGAGGGCTCTGATCTGGTTCTGATGAAGCT
TGCTAGACCCGCTGTGCTGGATGACTTCGTGTCCACCATCGACCTGCCAAATTACGGAT
GTACAATTCCTGAAAAGACCTCCTGTTCTGTGTACGGCTGGGGCTACACCGGACTCATC
AACTATGACGGCCTGCTGAGAGTGGCCCACCTGTATATCATGGGCAACGAGAAGTGCA
GCCAGCACCATAGAGGCAAGGTGACCCTGAACGAGTCTGAAATCTGCGCCGGAGCCGA
GAAAATCGGATCTGGCCCCTGCGAAGGAGATTACGGCGGCCCTCTGGTCTGCGAGCAG
CACAAGATGAGAATGGTGCTGGGCGTGATCGTGCCCGGCAGAGGCTGCGCCATCCCTA
ACCGGCCTGGAATCTTCGTGCGCGTGGCCTATTACGCCAAATGGATCCACAAAATCATC
CTGACATACAAGGTCCCACAGTCTTGATAAGGGCCCACCGGTTCTGCCGTGGGCCTGTC
GTGCCAGTCCTGGGGGCGAGATCGGGAGAATCACGTCCTGGAACTGCATGTTCTTGCGA
CTCTTGGGACTTCATCTTAACTTCTCGCTGCCCCAGCCATGTTTTCAACCATGGCATCCC
TCCCCCAATTAGTTCCCTGTCATCCTCGTCAACCTTCTCTGTAAGTGCCTGGTAAGCTTG
CCCTTGCTTAAGAACTCAAAACATAGCTGTGCTCTACCGCGGAAAAAAAAAAAAAAAA
AAAAAAAAAAAAAAGCATATGACTAAAAAAAAAAAAAAAAAAAAAAAAAAAAAAAAA
AAAAAAAAAAAAAAAAAAAAAAAAAAAAAAAAAAAAAAAAGTTTAAACATTTAAATTCT
AGA
SEQ ID NO:127
> **hHGF** protein mRNA sequence 
AUGUGGGUGACCAAACUCCUGCCAGCCCUGCUGCUGCAGCAUGUCCUCCUGCAUCUC
CUCCUGCUCCCCAUCGCCAUCCCCUAUGCAGAGGGACAAAGGAAAAGAAGAAAUACA
AUUCAUGAAUUCAAAAAAUCAGCAAAGACUACCCUAAUCAAAAUAGAUCCAGCACUG
AAGAUAAAAACCAAAAAAGUGAAUACUGCAGACCAAUGUGCUAAUAGAUGUACUAG
GAAUAAAGGACUUCCAUUCACUUGCAAGGCUUUUGUUUUUGAUAAAGCAAGAAAAC
 
AAUGCCUCUGGUUCCCCUUCAAUAGCAUGUCAAGUGGAGUGAAAAAAGAAUUUGGC
CAUGAAUUUGACCUCUAUGAAAACAAAGACUACAUUAGAAACUGCAUCAUUGGUAA
AGGACGCAGCUACAAGGGAACAGUAUCUAUCACUAAGAGUGGCAUCAAAUGUCAGCC
CUGGAGUUCCAUGAUACCACACGAACACAGCUUUUUGCCUUCGAGCUAUCGGGGUAA
AGACCUACAGGAAAACUACUGUCGAAAUCCUCGAGGGGAAGAAGGGGGACCCUGGU
GUUUCACAAGCAAUCCAGAGGUACGCUACGAAGUCUGUGACAUUCCUCAGUGUUCAG
AAGUUGAAUGCAUGACCUGCAAUGGGGAGAGUUAUCGAGGUCUCAUGGAUCAUACA
GAAUCAGGCAAGAUUUGUCAGCGCUGGGAUCAUCAGACACCACACCGGCACAAAUUC
UUGCCUGAAAGAUAUCCCGACAAGGGCUUUGAUGAUAAUUAUUGCCGCAAUCCCGAU
GGCCAGCCGAGGCCAUGGUGCUAUACUCUUGACCCUCACACCCGCUGGGAGUACUGU
GCAAUUAAAACAUGCGCUGACAAUACUAUGAAUGACACUGAUGUUCCUUUGGAAAC
AACUGAAUGCAUCCAAGGUCAAGGAGAAGGCUACAGGGGCACUGUCAAUACCAUUU
GGAAUGGAAUUCCAUGUCAGCGUUGGGAUUCUCAGUAUCCUCACGAGCAUGACAUG
ACUCCUGAAAAUUUCAAGUGCAAGGACCUACGAGAAAAUUACUGCCGAAAUCCAGAU
GGGUCUGAAUCACCCUGGUGUUUUACCACUGAUCCAAACAUCCGAGUUGGCUACUGC
UCCCAAAUUCCAAACUGUGAUAUGUCACAUGGACAAGAUUGUUAUCGUGGGAAUGG
CAAAAAUUAUAUGGGCAACUUAUCCCAAACAAGAUCUGGACUAACAUGUUCAAUGU
GGGACAAGAACAUGGAAGACUUACAUCGUCAUAUCUUCUGGGAACCAGAUGCAAGU
AAGCUGAAUGAGAAUUACUGCCGAAAUCCAGAUGAUGAUGCUCAUGGACCCUGGUG
CUACACGGGAAAUCCACUCAUUCCUUGGGAUUAUUGCCCUAUUUCUCGUUGUGAAGG
UGAUACCACACCUACAAUAGUCAAUUUAGACCAUCCCGUAAUAUCUUGUGCCAAAAC
GAAACAAUUGCGAGUUGUAAAUGGGAUUCCAACACGAACAAACAUAGGAUGGAUGG
UUAGUUUGAGAUACAGAAAUAAACAUAUCUGCGGAGGAUCAUUGAUAAAGGAGAGU
UGGGUUCUUACUGCACGACAGUGUUUCCCUUCUCGAGACUUGAAAGAUUAUGAAGC
UUGGCUUGGAAUUCAUGAUGUCCACGGAAGAGGAGAUGAGAAAUGCAAACAGGUUC
UCAAUGUUUCCCAGCUGGUAUAUGGCCCUGAAGGAUCAGAUCUGGUUUUAAUGAAG
CUUGCCAGGCCUGCUGUCCUGGAUGAUUUUGUUAGUACGAUUGAUUUACCUAAUUA
UGGAUGCACAAUUCCUGAAAAGACCAGUUGCAGUGUUUAUGGCUGGGGCUACACUG
GAUUGAUCAACUAUGAUGGCCUAUUACGAGUGGCACAUCUCUAUAUAAUGGGAAAU
GAGAAAUGCAGCCAGCAUCAUCGAGGGAAGGUGACUCUGAAUGAGUCUGAAAUAUG
UGCUGGGGCUGAAAAGAUUGGAUCAGGACCAUGUGAGGGGGAUUAUGGUGGCCCAC
UUGUUUGUGAGCAACAUAAAAUGAGAAUGGUUCUUGGUGUCAUUGUUCCUGGUCGU
GGAUGUGCCAUUCCAAAUCGUCCUGGUAUUUUUGUCCGAGUAGCAUAUUAUGCAAA
AUGGAUACACAAAAUUAUUUUAACAUAUAAGGUACCACAGUCAUAG
SEQ ID NO:128
> hHGF codon-optimized mRNA sequence 1 (hHGF-OS1) 
AUGUGGGUGACCAAACUGCUCCCCGCUCUGCUGCUGCAGCAUGUGCUGCUCCACCUG
CUGCUGCUGCCAAUCGCCAUCCCCUACGCUGAAGGACAGCGGAAGCGCAGAAACACA
AUUCACGAAUUCAAGAAGUCCGCCAAAACAACACUGAUCAAGAUCGACCCUGCCCUG
AAAAUCAAAACCAAGAAGGUGAACACUGCCGAUCAGUGCGCCAACAGAUGCACAAGA
AACAAAGGCCUGCCCUUCACAUGCAAGGCCUUUGUGUUCGACAAGGCCAGAAAGCAG
UGCCUGUGGUUCCCUUUCAACAGCAUGAGCUCUGGCGUGAAGAAAGAGUUCGGCCAC
GAAUUCGACCUGUACGAGAACAAGGAUUACAUCAGAAAUUGUAUCAUCGGCAAGGG
CAGAAGCUACAAAGGCACAGUGUCCAUCACCAAAAGCGGCAUUAAGUGUCAGCCUUG
GAGCAGCAUGAUCCCUCACGAGCACAGCUUCCUGCCUAGCUCUUACAGAGGCAAGGA
CCUGCAGGAGAAUUACUGCCGGAACCCUAGAGGCGAGGAAGGCGGCCCUUGGUGCUU
UACCAGCAACCCUGAGGUGCGGUACGAGGUGUGUGACAUCCCCCAGUGCAGCGAAGU
GGAAUGCAUGACCUGUAAUGGCGAGAGCUACAGAGGCCUCAUGGACCAUACAGAGA
GCGGAAAGAUUUGUCAAAGAUGGGAUCACCAGACACCUCACAGACACAAAUUCCUGC
CCGAAAGAUAUCCUGACAAGGGCUUCGACGACAACUACUGCAGAAAUCCUGAUGGCC
AACCCAGACCUUGGUGCUACACCCUGGAUCCUCAUACCAGAUGGGAGUACUGUGCCA
UCAAGACCUGCGCUGAUAAUACCAUGAACGACACCGAUGUGCCUCUGGAAACAACCG
AGUGCAUCCAGGGACAGGGCGAAGGCUACCGGGGCACCGUGAACACCAUCUGGAACG
GCAUCCCCUGCCAGAGAUGGGACAGCCAGUACCCCCACGAACACGAUAUGACCCCUG
AGAACUUCAAGUGCAAGGACCUGAGAGAAAAUUACUGCAGAAACCCAGACGGAAGC
GAGUCUCCUUGGUGUUUUACCACCGACCCCAACAUCCGGGUGGGUUACUGCAGCCAA
AUCCCCAAUUGCGACAUGAGCCACGGCCAGGACUGCUACCGGGGAAACGGCAAAAAC
 
UACAUGGGCAAUCUGAGCCAGACCCGGAGCGGUCUGACCUGCUCCAUGUGGGAUAAG
AACAUGGAAGAUCUGCACCGGCACAUCUUCUGGGAGCCUGACGCUUCUAAGCUGAAC
GAGAACUACUGUAGGAACCCAGACGACGAUGCCCACGGUCCCUGGUGCUACACCGGC
AACCCGCUGAUCCCUUGGGACUAUUGUCCUAUCAGCCGCUGCGAGGGCGAUACAACA
CCUACCAUCGUGAACCUGGACCACCCUGUGAUCUCUUGCGCCAAGACCAAGCAGCUG
AGAGUGGUGAACGGCAUCCCAACACGGACCAACAUCGGAUGGAUGGUCUCCCUGAGA
UACCGGAACAAGCACAUCUGCGGCGGCUCUCUGAUCAAGGAAUCUUGGGUCCUGACC
GCCAGACAGUGCUUCCCCAGCCGGGACCUGAAGGACUAUGAGGCCUGGCUGGGAAUC
CACGACGUGCACGGCAGAGGCGACGAGAAGUGCAAGCAAGUGCUGAACGUGUCCCAG
CUGGUGUACGGCCCUGAGGGAUCUGAUCUGGUCCUGAUGAAACUGGCCAGACCCGCC
GUGCUGGACGACUUUGUGUCCACCAUCGACCUGCCUAACUACGGCUGCACCAUCCCU
GAAAAGACCAGCUGCAGCGUGUACGGCUGGGGCUAUACAGGCCUGAUCAACUACGAC
GGUCUGCUGCGGGUUGCUCACCUGUACAUCAUGGGCAACGAGAAGUGCAGCCAGCAC
CACAGAGGAAAGGUUACACUUAACGAGAGCGAGAUCUGCGCCGGCGCCGAGAAAAUC
GGCUCCGGCCCUUGUGAAGGCGACUACGGAGGCCCUCUGGUGUGCGAACAGCACAAG
AUGAGAAUGGUGCUGGGCGUGAUCGUGCCCGGAAGAGGCUGUGCAAUCCCUAAUAG
GCCCGGCAUUUUCGUGCGGGUGGCCUACUACGCCAAGUGGAUCCACAAGAUCAUCCU
GACGUACAAGGUGCCUCAGAGCUGA
SEQ ID NO:129
> hHGF codon-optimized mRNA sequence 2 (hHGF-OS2) 
AUGUGGGUCACCAAACUGCUGCCCGCCCUGCUGCUGCAGCACGUGCUCCUGCACCUG
CUGCUGCUGCCUAUCGCCAUCCCUUACGCCGAAGGCCAGAGAAAGCGGAGAAAUACC
AUUCACGAAUUUAAGAAGAGCGCCAAGACCACACUGAUCAAGAUCGACCCUGCCCUG
AAAAUCAAGACCAAGAAAGUGAACACAGCCGACCAAUGCGCUAAUAGAUGCACAAGA
AACAAAGGCCUGCCUUUCACCUGCAAGGCCUUCGUGUUCGAUAAGGCCAGAAAGCAG
UGCCUGUGGUUCCCCUUCAACAGCAUGAGCAGCGGCGUGAAGAAAGAAUUCGGUCAC
GAGUUCGACCUGUACGAGAAUAAGGACUACAUUAGAAACUGCAUCAUCGGCAAGGG
CCGGAGCUACAAGGGCACCGUGAGCAUCACCAAGUCUGGCAUCAAGUGCCAGCCAUG
GAGUUCCAUGAUCCCUCAUGAGCACAGCUUCCUGCCAAGCUCUUACCGGGGAAAGGA
UCUGCAGGAGAACUAUUGUAGAAAUCCCAGAGGCGAGGAAGGAGGACCCUGGUGUU
UUACCAGCAACCCAGAGGUGCGGUACGAGGUGUGCGACAUACCCCAGUGCAGCGAGG
UGGAAUGCAUGACAUGCAACGGCGAGAGCUACCGGGGCCUGAUGGACCACACAGAGA
GCGGCAAAAUCUGCCAAAGAUGGGAUCACCAGACCCCUCAUAGGCACAAGUUCCUGC
CCGAGAGAUACCCUGACAAGGGCUUCGACGACAAUUACUGCCGGAACCCUGAUGGAC
AGCCUAGACCUUGGUGCUACACCCUGGACCCUCACACCAGAUGGGAAUACUGCGCCA
UCAAGACAUGUGCCGAUAACACCAUGAACGACACCGAUGUGCCACUCGAGACAACUG
AGUGUAUCCAGGGCCAGGGCGAGGGCUACAGGGGCACCGUGAACACCAUCUGGAACG
GCAUCCCUUGUCAGCGGUGGGACAGCCAAUACCCCCACGAACAUGAUAUGACCCCAG
AAAACUUCAAGUGCAAAGAUCUGCGGGAAAACUACUGUAGAAACCCUGACGGCUCCG
AGAGCCCUUGGUGCUUCACCACCGACCCUAACAUCAGGGUGGGCUACUGUAGCCAGA
UCCCCAACUGCGACAUGAGCCACGGUCAGGACUGCUACAGAGGAAACGGCAAGAAUU
ACAUGGGCAACCUGAGCCAGACCAGAAGCGGCCUGACCUGCAGCAUGUGGGACAAAA
ACAUGGAAGAUCUGCACCGGCACAUCUUUUGGGAGCCUGACGCCAGCAAGCUGAAUG
AGAACUACUGUCGGAACCCUGACGACGAUGCUCACGGCCCUUGGUGCUACACAGGCA
ACCCUCUGAUCCCUUGGGACUAUUGUCCUAUUAGCAGGUGUGAAGGCGACACGACCC
CUACAAUCGUGAACCUGGAUCACCCAGUGAUCAGCUGUGCUAAAACAAAGCAACUGA
GAGUGGUGAAUGGUAUCCCGACCCGGACCAACAUCGGAUGGAUGGUCAGCCUGCGGU
ACAGAAACAAGCACAUCUGCGGCGGUAGCCUGAUCAAGGAAUCCUGGGUGUUGACAG
CCAGACAGUGUUUUCCCAGCAGAGAUCUGAAGGACUACGAGGCCUGGCUGGGCAUCC
ACGACGUGCACGGCAGAGGCGACGAGAAGUGCAAGCAGGUUCUGAACGUGUCCCAGC
UGGUGUACGGCCCCGAGGGCUCUGAUCUGGUUCUGAUGAAGCUUGCUAGACCCGCUG
UGCUGGAUGACUUCGUGUCCACCAUCGACCUGCCAAAUUACGGAUGUACAAUUCCUG
AAAAGACCUCCUGUUCUGUGUACGGCUGGGGCUACACCGGACUCAUCAACUAUGACG
GCCUGCUGAGAGUGGCCCACCUGUAUAUCAUGGGCAACGAGAAGUGCAGCCAGCACC
AUAGAGGCAAGGUGACCCUGAACGAGUCUGAAAUCUGCGCCGGAGCCGAGAAAAUCG
GAUCUGGCCCCUGCGAAGGAGAUUACGGCGGCCCUCUGGUCUGCGAGCAGCACAAGA
UGAGAAUGGUGCUGGGCGUGAUCGUGCCCGGCAGAGGCUGCGCCAUCCCUAACCGGC
 
CUGGAAUCUUCGUGCGCGUGGCCUAUUACGCCAAAUGGAUCCACAAAAUCAUCCUGA
CAUACAAGGUCCCACAGUCUUGA
SEQ ID NO:130
> hHGF codon-optimized mRNA sequence 3 (hHGF-OS3) 
AUGUGGGUGACCAAGCUGCUGCCCGCCCUGCUCCUGCAGCACGUGCUGCUGCACCUG
CUCCUGCUGCCCAUCGCCAUCCCCUACGCCGAGGGGCAGAGAAAGAGAAGAAACACC
AUCCACGAGUUCAAGAAGAGCGCCAAGACCACCCUGAUCAAGAUCGACCCCGCCCUG
AAGAUCAAGACCAAGAAGGUGAACACCGCCGAUCAGUGCGCCAACAGAUGCACAAGA
AACAAGGGCCUGCCCUUCACCUGCAAGGCCUUCGUGUUCGACAAGGCUAGAAAGCAG
UGCCUGUGGUUCCCCUUCAACAGCAUGAGCAGCGGCGUGAAGAAGGAGUUCGGCCAC
GAGUUCGACCUGUACGAGAACAAGGACUACAUCAGAAACUGCAUCAUCGGCAAGGGC
AGAAGCUACAAGGGCACCGUGAGCAUCACCAAGAGCGGCAUCAAGUGUCAGCCCUGG
AGCAGCAUGAUCCCCCAUGAGCACAGCUUCCUGCCUAGCAGCUACAGAGGGAAGGAC
CUGCAAGAGAACUACUGCAGAAACCCUAGAGGCGAGGAGGGCGGCCCUUGGUGUUUC
ACAAGCAAUCCCGAGGUGAGAUACGAGGUGUGCGACAUCCCUCAGUGCAGCGAGGUG
GAGUGCAUGACCUGCAACGGCGAAAGCUAUAGAGGCCUGAUGGACCACACCGAGAGC
GGCAAGAUUUGUCAGCGGUGGGAUCACCAAACCCCCCACAGACACAAGUUCCUGCCC
GAGAGAUACCCCGACAAGGGCUUCGACGACAAUUACUGUAGAAACCCCGACGGGCAG
CCUAGACCCUGGUGUUACACCCUGGACCCCCACACAAGAUGGGAGUACUGCGCCAUC
AAGACCUGCGCCGACAACACCAUGAACGACACCGACGUGCCCCUGGAGACCACCGAG
UGCAUCCAAGGCCAAGGCGAGGGCUACAGAGGCACCGUGAACACCAUCUGGAACGGC
AUUCCCUGUCAGAGAUGGGACAGCCAAUACCCCCAUGAACACGAUAUGACCCCCGAG
AACUUCAAGUGCAAGGACCUGAGAGAGAACUACUGCCGGAACCCCGAUGGCAGCGAA
UCCCCUUGGUGCUUCACCACCGACCCCAACAUCAGAGUGGGCUACUGCAGCCAAAUC
CCCAACUGCGACAUGAGCCACGGCCAAGACUGUUACAGAGGGAACGGCAAGAACUAC
AUGGGCAACCUGAGCCAAACAAGAAGCGGCCUGACCUGCAGCAUGUGGGACAAGAAC
AUGGAGGACCUGCACAGACACAUCUUCUGGGAGCCCGACGCUAGCAAGCUGAACGAG
AACUACUGUCGGAACCCCGACGACGAUGCCCACGGCCCCUGGUGCUACACCGGCAAC
CCCCUGAUCCCCUGGGACUACUGCCCCAUCAGCAGAUGCGAGGGCGACACCACCCCCA
CCAUCGUGAACCUGGACCACCCCGUGAUCAGCUGUGCCAAGACCAAGCAACUGAGAG
UGGUGAACGGCAUCCCCACAAGAACCAACAUUGGCUGGAUGGUGAGCCUGAGAUACA
GAAACAAGCACAUCUGCGGCGGCAGCCUGAUCAAGGAGAGCUGGGUGCUGACCGCUA
GACAGUGCUUCCCUAGCAGAGACCUGAAGGACUACGAGGCCUGGCUGGGCAUCCACG
ACGUGCACGGCAGAGGCGACGAGAAGUGUAAACAAGUGCUGAACGUGAGCCAACUG
GUGUACGGCCCCGAGGGCAGCGACCUGGUGCUGAUGAAGCUGGCUAGACCCGCCGUG
CUGGACGACUUCGUGAGCACCAUCGACCUGCCCAACUACGGCUGCACCAUCCCCGAG
AAGACAAGCUGCAGCGUGUACGGCUGGGGCUACACCGGCCUGAUCAACUACGACGGC
CUGCUGAGAGUGGCCCACCUGUACAUCAUGGGCAACGAGAAGUGCAGCCAACACCAC
CGGGGCAAAGUGACCCUGAACGAGAGCGAGAUCUGCGCCGGGGCUGAGAAAAUCGGC
AGCGGCCCCUGCGAGGGGGACUACGGGGGCCCCCUGGUGUGCGAGCAGCACAAGAUG
AGAAUGGUGCUGGGCGUGAUCGUGCCCGGCAGAGGCUGCGCCAUCCCCAACAGACCC
GGCAUCUUCGUGAGAGUGGCCUACUACGCCAAGUGGAUCCACAAGAUCAUCCUGACC
UACAAGGUGCCUCAGAGCUGA
SEQ ID NO:131
>CHCHD10 5'UTR- hHGF-CTSB 3'UTR-A120

AGGGAAGCUUCCGCUGCCGCCGUCUCUAAGGUCGCCCGGGUCCCACCGCCGCCACCGC
CGCCACCAUGUGGGUCACCAAACUGCUGCCCGCCCUGCUGCUGCAGCACGUGCUCCU
GCACCUGCUGCUGCUGCCUAUCGCCAUCCCUUACGCCGAAGGCCAGAGAAAGCGGAG
AAAUACCAUUCACGAAUUUAAGAAGAGCGCCAAGACCACACUGAUCAAGAUCGACCC
UGCCCUGAAAAUCAAGACCAAGAAAGUGAACACAGCCGACCAAUGCGCUAAUAGAUG
CACAAGAAACAAAGGCCUGCCUUUCACCUGCAAGGCCUUCGUGUUCGAUAAGGCCAG
AAAGCAGUGCCUGUGGUUCCCCUUCAACAGCAUGAGCAGCGGCGUGAAGAAAGAAUU
CGGUCACGAGUUCGACCUGUACGAGAAUAAGGACUACAUUAGAAACUGCAUCAUCGG
CAAGGGCCGGAGCUACAAGGGCACCGUGAGCAUCACCAAGUCUGGCAUCAAGUGCCA
GCCAUGGAGUUCCAUGAUCCCUCAUGAGCACAGCUUCCUGCCAAGCUCUUACCGGGG
AAAGGAUCUGCAGGAGAACUAUUGUAGAAAUCCCAGAGGCGAGGAAGGAGGACCCU

GGUGUUUUACCAGCAACCCAGAGGUGCGGUACGAGGUGUGCGACAUACCCCAGUGCA
GCGAGGUGGAAUGCAUGACAUGCAACGGCGAGAGCUACCGGGGCCUGAUGGACCACA
CAGAGAGCGGCAAAAUCUGCCAAAGAUGGGAUCACCAGACCCCUCAUAGGCACAAGU
UCCUGCCCGAGAGAUACCCUGACAAGGGCUUCGACGACAAUUACUGCCGGAACCCUG
AUGGACAGCCUAGACCUUGGUGCUACACCCUGGACCCUCACACCAGAUGGGAAUACU
GCGCCAUCAAGACAUGUGCCGAUAACACCAUGAACGACACCGAUGUGCCACUCGAGA
CAACUGAGUGUAUCCAGGGCCAGGGCGAGGGCUACAGGGGCACCGUGAACACCAUCU
GGAACGGCAUCCCUUGUCAGCGGUGGGACAGCCAAUACCCCCACGAACAUGAUAUGA
CCCCAGAAAACUUCAAGUGCAAAGAUCUGCGGGAAAACUACUGUAGAAACCCUGACG
GCUCCGAGAGCCCUUGGUGCUUCACCACCGACCCUAACAUCAGGGUGGGCUACUGUA
GCCAGAUCCCCAACUGCGACAUGAGCCACGGUCAGGACUGCUACAGAGGAAACGGCA
AGAAUUACAUGGGCAACCUGAGCCAGACCAGAAGCGGCCUGACCUGCAGCAUGUGGG
ACAAAAACAUGGAAGAUCUGCACCGGCACAUCUUUGGAGCCUGACGCCAGCAAGC
UGAAUGAGAACUACUGUCGGAACCCUGACGACGAUGCUCACGGCCCUUGGUGCUACA
CAGGCAACCCUCUGAUCCCUUGGGACUAUUGUCCUAUUAGCAGGUGUGAAGGCGACA
CGACCCCUACAAUCGUGAACCUGGAUCACCCAGUGAUCAGCUGUGCUAAAACAAAGC
AACUGAGAGUGGUGAAUGGUAUCCCGACCCGGACCAACAUCGGAUGGAUGGUCAGCC
UGCGGUACAGAAACAAGCACAUCUGCGGCGGUAGCCUGAUCAAGGAAUCCUGGGUGU
UGACAGCCAGACAGUGUUUUCCCAGCAGAGAUCUGAAGGACUACGAGGCCUGGCUGG
GCAUCCACGACGUGCACGGCAGAGGCGACGAGAAGUGCAAGCAGGUUCUGAACGUGU
CCCAGCUGGUGUACGGCCCCGAGGGCUCUGAUCUGGUUCUGAUGAAGCUUGCUAGAC
CCGCUGUGCUGGAUGACUUCGUGUCCACCAUCGACCUGCCAAAUUACGGAUGUACAA
UUCCUGAAAAGACCUCCUGUUCUGUGUACGGCUGGGGCUACACCGGACUCAUCAACU
AUGACGGCCUGCUGAGAGUGGCCCACCUGUAUAUCAUGGGCAACGAGAAGUGCAGCC
AGCACCAUAGAGGCAAGGUGACCCUGAACGAGUCUGAAAUCUGCGCCGGAGCCGAGA
AAAUCGGAUCUGGCCCCUGCGAAGGAGAUUACGGCGGCCCUCUGGUCUGCGAGCAGC
ACAAGAUGAGAAUGGUGCUGGGCGUGAUCGUGCCCGGCAGAGGCUGCGCCAUCCCUA
ACCGGCCUGGAAUCUUCGUGCGCGUGGCCUAUUACGCCAAAUGGAUCCACAAAAUCA
UCCUGACAUACAAGGUCCCACAGUCUUGAACCGGUUCUGCCGUGGGCCUGUCGUGCC
AGUCCUGGGGGCGAGAUCGGGAGAAUCACGUCCUGGAACUGCAUGUUCUUGCGACUC
UUGGGACUUCAUCUUAACUUCUCGCUGCCCCAGCCAUGUUUUCAACCAUGGCAUCCC
UCCCCCAAUUAGUUCCCUGUCAUCCUCGUCAACCUUCUCUGUAAGUGCCUGGUAAGC
UUGCCCUUGCUUAAGAACUCAAAACAUAGCUGUGCUCUACCGCGGAAAAAAAAAAAA
AAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAUUAU
UUCUAG
SEQ ID NO:132

## Claims

1. A lipid nanoparticle, comprising a nucleic acid construct and a lipid phase,
the nucleic acid construct comprises:
(a) an open reading frame (ORF), and
(b) an untranslated region element (UTR), wherein the UTR is derived from a UTR of the gene ACTG1, ATP6V0B, ATP6V0E1, CFL1, COX4I1, CTSB, FAM166A, NDUFB9, CHCHD10, SLC38A2, NDUFA11, NDUFV3, PRDX5, GUK1, IAH1, ABHD16A, SLC25A39, ATPIF1, ANAPC11, CCDC12, MRPL14, or APOA1BP;
the lipid phase comprises a cationic lipid of Formula I,

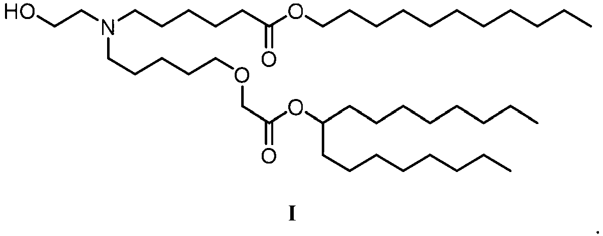

2. The lipid nanoparticle according to claim 1, wherein the cationic lipid comprises from 20 mol% to 70 mol% of the total lipids present in the lipid nanoparticle; preferably, the cationic lipid comprises from 30 mol% to 70 mol% of the total lipids present in the lipid nanoparticle; and more preferably, the cationic lipid comprises from 40 mol% to 70 mol% of the total lipids present in the lipid nanoparticle.

3. The lipid nanoparticle according to claim 1, wherein the lipid nanoparticle comprises at least one non-cationic lipid, and preferably the non-cationic lipid is selected from a mixture of a phospholipid and a cholesterol or its derivative.

4. The lipid nanoparticle according to claim 3, wherein the non-cationic lipid comprises from 20 mol% to 70 mol% of the total lipids present in the lipid nanoparticle; preferably, the non-cationic lipid comprises 30 mol% to 60 mol% of the total lipids present in the lipid nanoparticle; and more preferably, the non-cationic lipid comprises from 20 mol% to 50 mol% of the total lipids present in the lipid nanoparticle.

5. The lipid nanoparticle according to claim 3, wherein the phospholipid comprise from 5 mol% to 30 mol% of the total lipids present in the lipid nanoparticle.

6. The lipid nanoparticle according to claim 3, wherein the cholesterol or its derivative comprises from 20 mol% to 60 mol% of the total lipids present in the lipid nanoparticle; preferably, the cholesterol or its derivative comprises from 30 mol% to 60 mol% of the total lipids present in the lipid nanoparticle; and more preferably, the cholesterol or its derivative comprises from 20 mol% to 45 mol% of the total lipids present in the lipid nanoparticle.

7. The lipid nanoparticle according to claim 1, wherein the lipid nanoparticle further comprises at least one conjugated lipid; and preferably, the conjugated lipid in the lyophilized composition comprises from 0.5 mol% to 5 mol% of the total lipids present in the lipid nanoparticle.

8. The lipid nanoparticle according to claim 1, comprising:
a) a nucleic acid construct;
b) a cationic lipid, wherein the cationic lipid comprises a compound of Formula I or a pharmaceutically acceptable salt thereof, and the cationic lipid comprises from 10 mol% to 75 mol% of the total lipids present in the lipid nanoparticle, 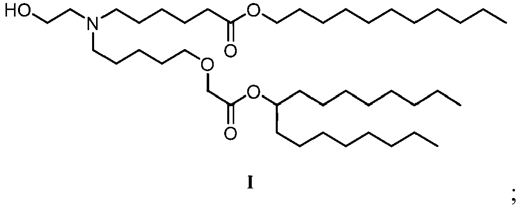
c) a non-cationic lipid, wherein the non-cationic lipid is selected from a phospholipid and a cholesterol or its derivative, wherein the phospholipid comprises from 5 mol% to 40 mol% of the total lipids present in the lipid nanoparticle, and the cholesterol or its derivative comprises from 15 mol% to 60 mol% of the total lipids present in the lipid nanoparticle;
and d) a conjugated lipid, wherein the conjugate comprises from 0.5 mol% to 2 mol% of the total lipids present in the lipid nanoparticle.

9. A lyophilized composition, comprising the lipid nanoparticle according to any one of claims 1 to 8.

10. The lyophilized composition according to claim 9, comprising the lipid nanoparticle according to any one of claims 1 to 8 and sodium chloride, preferably the weight ratio of sodium chloride to the cationic lipid is 1:10 to 10:1; more preferably, the weight ratio of sodium chloride to the cationic lipid is 1:5 to 10:1; more preferably, the weight ratio of sodium chloride to the cationic lipid is 1:5 to 5:1; more preferably, the weight ratio of sodium chloride to the cationic lipid is 1:3 to 5:1; more preferably, the weight ratio of sodium chloride to the cationic lipid is 1:2 to 5:1; and more preferably, the weight ratio of sodium chloride to the cationic lipid is 1:2 to 4:1.

11. The lyophilized composition according to any one of claims 9 to 10, comprising at least one lyoprotectant, preferably the lyoprotectant is one or more selected from sucrose, trehalose, mannitol, and maltose; and preferably the lyoprotectant is selected from sucrose, and a combination of sucrose and trehalose.

12. A lyophilized preparation, obtained by freeze-drying the lipid nanoparticle according to any one of claims 1 to 8 or the lyophilized composition according to any one of claims 9 to 11; preferably, in the lyophilized preparation, the weight ratio of sodium chloride to the cationic lipid is 1:10 to 10:1; more preferably, the weight ratio of sodium chloride to the cationic lipid is 1:5 to 10:1; more preferably, the weight ratio of sodium chloride to the cationic lipid is 1:5 to 5:1; more preferably, the weight ratio of sodium chloride to the cationic lipid is 1:3 to 5:1; more preferably, the weight ratio of sodium chloride to the cationic lipid is 1:2 to 5:1; and more preferably, the weight ratio of sodium chloride to the cationic lipid is 1:2 to 4:1.

13. A reconstituted solution, obtained by reconstituting the lyophilized preparation according to claim 12 with a reconstitution solvent; preferably, the reconstitution solvent is water; and more preferably, the reconstitution solvent is water for injection.

14. A product, comprising:
1) the lyophilized composition according to any one of claims 9 to 11 or the lyophilized preparation according to claim 12, and
2) a reconstitution solvent;
preferably, the product comprises the lyophilized composition and the reconstitution solvent in separate packages; preferably, the reconstitution solvent is water; and more preferably, the reconstitution solvent is water for injection.

15. A spray preparation, comprising the lyophilized composition according to any one of claims 9 to 11, the lyophilized preparation according to claim 12, or the reconstituted solution according to claim 13.

16. A method for preparing a reconstituted solution, comprising preparing the lyophilized composition according to any one of claims 9 to 11, and subjecting the lyophilized composition to a freeze-drying treatment to obtain a lyophilized preparation; and
reconstituting the lyophilized preparation to obtain the reconstituted solution;
preferably, the reconstitution solvent is water; and more preferably, the reconstitution solvent is water for injection.

17. A method for preparing a lyophilized preparation, comprising preparing the lyophilized composition according to any one of claims 9 to 11, and subjecting the lyophilized composition to a freeze-drying treatment to obtain the lyophilized preparation.

18. The lipid nanoparticle according to any one of claims 1 to 8, wherein the nucleic acid construct comprises:
(a) an open reading frame (ORF), and
(b) an untranslated region element (UTR), wherein the UTR comprises the following sequence:
a sequence set forth in any one of SEQ ID NO: 12, 26 or a sequence having an identity of at least 90% therewith,
a sequence set forth in any one of SEQ ID NO: 1, 15 or a sequence having an identity of at least 90% therewith,
a sequence set forth in any one of SEQ ID NO: 2, 3, 16, 17 or a sequence having an identity of at least 90% therewith,
a sequence set forth in any one of SEQ ID NO: 4-5, 18-19 or a sequence having an identity of at least 90% therewith,
a sequence set forth in any one of SEQ ID NO: 6-8, 20-22 or a sequence having an identity of at least 90% therewith,
a sequence set forth in any one of SEQ ID NO: 9-11, 23-25 or a sequence having an identity of at least 90% therewith,
a sequence set forth in any one of SEQ ID NO: 13, 27 or a sequence having an identity of at least 90% therewith,
a sequence set forth in any one of SEQ ID NO: 14, 28 or a sequence having an identity of at least 90% therewith,
a sequence set forth in any one of SEQ ID NO: 29, 30 or a sequence having an identity of at least 90% therewith,
a sequence set forth in any one of SEQ ID NOs: 35-37 or a sequence having an identity of at least 90% therewith,
a sequence set forth in any one of SEQ ID NO: 42, 43 or a sequence having an identity of at least 90% therewith,
a sequence set forth in any one of SEQ ID NO: 46, 47 or a sequence having an identity of at least 90% therewith,
a sequence set forth in any one of SEQ ID NO: 31-34, 38-41, 44-45 or a sequence having an identity of at least 90% therewith,
a sequence set forth in any one of SEQ ID NO: 69, 83 or a sequence having an identity of at least 90% therewith,
a sequence set forth in any one of SEQ ID NO: 58, 72 or a sequence having an identity of at least 90% therewith,
a sequence set forth in any one of SEQ ID NO: 59-60, 73-74 or a sequence having an identity of at least 90% therewith,
a sequence set forth in any one of SEQ ID NO: 61-62, 75-76 or a sequence having an identity of at least 90% therewith,
a sequence set forth in any one of SEQ ID NO: 63-65, 77-79 or a sequence having an identity of at least 90% therewith,
a sequence set forth in any one of SEQ ID NO: 66-68, 80-82 or a sequence having an identity of at least 90% therewith,
a sequence set forth in any one of SEQ ID NO: 70, 84 or a sequence having an identity of at least 90% therewith,
a sequence set forth in any one of SEQ ID NO: 71, 85 or a sequence having an identity of at least 90% therewith,
a sequence set forth in any one of SEQ ID NO: 86, 87 or a sequence having an identity of at least 90% therewith,
a sequence set forth in any one of SEQ ID NO: 92-94 or a sequence having an identity of at least 90% therewith,
a sequence set forth in any one of SEQ ID NO: 99, 100 or a sequence having an identity of at least 90% therewith,
a sequence set forth in any one of SEQ ID NO: 103, 104 or a sequence having an identity of at least 90% therewith, or
a sequence set forth in any one of SEQ ID NO: 88-91, 95-98, 101-102 or a sequence having an identity of at least 90% therewith.

19. The lipid nanoparticle according to claim 18, wherein (b) comprises a combination of a 3' untranslated region element (3'UTR) and a 5' untranslated region element (5'UTR) selected from any one of the following:
1) the 3'UTR comprises a sequence set forth in SEQ ID NO: 1 or a sequence having an identity of at least 90% therewith, and the 5'UTR comprises a sequence set forth in any one of SEQ ID NO: 15-47 or a sequence having an identity of at least 90% with any thereof;
2) the 3'UTR comprises a sequence set forth in SEQ ID NO: 2 or a sequence having an identity of at least 90% therewith, and the 5'UTR comprises a sequence set forth in any one of SEQ ID NO: 15-47 or a sequence having an identity of at least 90% with any thereof;
3) the 3'UTR comprises a sequence set forth in SEQ ID NO: 3 or a sequence having an identity of at least 90% therewith, and the 5'UTR comprises a sequence set forth in any one of SEQ ID NO: 15-47 or a sequence having an identity of at least 90% with any thereof;
4) the 3'UTR comprises a sequence set forth in SEQ ID NO: 4 or a sequence having an identity of at least 90% therewith, and the 5'UTR comprises a sequence set forth in any one of SEQ ID NO: 15-47 or a sequence having an identity of at least 90% with any thereof;
5) the 3'UTR comprises a sequence set forth in SEQ ID NO: 5 or a sequence having an identity of at least 90% therewith, and the 5'UTR comprises a sequence set forth in any one of SEQ ID NO: 15-47 or a sequence having an identity of at least 90% with any thereof;
6) the 3'UTR comprises a sequence set forth in SEQ ID NO: 6 or a sequence having an identity of at least 90% therewith, and the 5'UTR comprises a sequence set forth in any one of SEQ ID NO: 15-47 or a sequence having an identity of at least 90% with any thereof;
7) the 3'UTR comprises a sequence set forth in SEQ ID NO: 7 or a sequence having an identity of at least 90% therewith, and the 5'UTR comprises a sequence set forth in any one of SEQ ID NO: 15-47 or a sequence having an identity of at least 90% with any thereof;
8) the 3'UTR comprises a sequence set forth in SEQ ID NO: 8 or a sequence having an identity of at least 90% therewith, and the 5'UTR comprises a sequence set forth in any one of SEQ ID NO: 15-47 or a sequence having an identity of at least 90% with any thereof;
9) the 3'UTR comprises a sequence set forth in SEQ ID NO: 9 or a sequence having an identity of at least 90% therewith, and the 5'UTR comprises a sequence set forth in any one of SEQ ID NO: 15-47 or a sequence having an identity of at least 90% with any thereof;
10) the 3'UTR comprises a sequence set forth in SEQ ID NO: 10 or a sequence having an identity of at least 90% therewith, and the 5'UTR comprises a sequence set forth in any one of SEQ ID NO: 15-47 or a sequence having an identity of at least 90% with any thereof;
11) the 3'UTR comprises a sequence set forth in SEQ ID NO: 11 or a sequence having an identity of at least 90% therewith, and the 5'UTR comprises a sequence set forth in any one of SEQ ID NO: 15-47 or a sequence having an identity of at least 90% with any thereof;
12) the 3'UTR comprises a sequence set forth in SEQ ID NO: 12 or a sequence having an identity of at least 90% therewith, and the 5'UTR comprises a sequence set forth in any one of SEQ ID NO: 15-47 or a sequence having an identity of at least 90% with any thereof;
13) the 3'UTR comprises a sequence set forth in SEQ ID NO: 13 or a sequence having an identity of at least 90% therewith, and the 5'UTR comprises a sequence set forth in any one of SEQ ID NO: 15-47 or a sequence having an identity of at least 90% with any thereof;
14) the 3'UTR comprises a sequence set forth in SEQ ID NO: 14 or a sequence having an identity of at least 90% therewith, and the 5'UTR comprises a sequence set forth in any one of SEQ ID NO: 15-47 or a sequence having an identity of at least 90% with any thereof;
15) the 3'UTR comprises a sequence set forth in any one of SEQ ID NO: 1-14 or a sequence having an identity of at least 90% with any thereof, and the 5'UTR comprises a sequence set forth in SEQ ID NO: 15 or a sequence having an identity of at least 90% therewith;
16) the 3'UTR comprises a sequence set forth in any one of SEQ ID NO: 1-14 or a sequence having an identity of at least 90% with any thereof, and the 5'UTR comprises a sequence set forth in SEQ ID NO: 16 or a sequence having an identity of at least 90% therewith;
17) the 3'UTR comprises a sequence set forth in any one of SEQ ID NO: 1-14 or a sequence having an identity of at least 90% with any thereof, and the 5'UTR comprises a sequence set forth in SEQ ID NO: 17 or a sequence having an identity of at least 90% therewith;
18) the 3'UTR comprises a sequence set forth in any one of SEQ ID NO: 1-14 or a sequence having an identity of at least 90% with any thereof, and the 5'UTR comprises a sequence set forth in SEQ ID NO: 18 or a sequence having an identity of at least 90% therewith;
19) the 3'UTR comprises a sequence set forth in any one of SEQ ID NO: 1-14 or a sequence having an identity of at least 90% with any thereof, and the 5'UTR comprises a sequence set forth in SEQ ID NO: 19 or a sequence having an identity of at least 90% therewith;
20) the 3'UTR comprises a sequence set forth in any one of SEQ ID NO: 1-14 or a sequence having an identity of at least 90% with any thereof, and the 5'UTR comprises a sequence set forth in SEQ ID NO: 20 or a sequence having an identity of at least 90% therewith;
21) the 3'UTR comprises a sequence set forth in any one of SEQ ID NO: 1-14 or a sequence having an identity of at least 90% with any thereof, and the 5'UTR comprises a sequence set forth in SEQ ID NO: 21 or a sequence having an identity of at least 90% therewith;
22) the 3'UTR comprises a sequence set forth in any one of SEQ ID NO: 1-14 or a sequence having an identity of at least 90% with any thereof, and the 5'UTR comprises a sequence set forth in SEQ ID NO: 22 or a sequence having an identity of at least 90% therewith;
23) the 3'UTR comprises a sequence set forth in any one of SEQ ID NO: 1-14 or a sequence having an identity of at least 90% with any thereof, and the 5'UTR comprises a sequence set forth in SEQ ID NO: 23 or a sequence having an identity of at least 90% therewith;
24) the 3'UTR comprises a sequence set forth in any one of SEQ ID NO: 1-14 or a sequence having an identity of at least 90% with any thereof, and the 5'UTR comprises a sequence set forth in SEQ ID NO: 24 or a sequence having an identity of at least 90% therewith;
25) the 3'UTR comprises a sequence set forth in any one of SEQ ID NO: 1-14 or a sequence having an identity of at least 90% with any thereof, and the 5'UTR comprises a sequence set forth in SEQ ID NO: 25 or a sequence having an identity of at least 90% therewith;
26) the 3'UTR comprises a sequence set forth in any one of SEQ ID NO: 1-14 or a sequence having an identity of at least 90% with any thereof, and the 5'UTR comprises a sequence set forth in SEQ ID NO: 26 or a sequence having an identity of at least 90% therewith;
27) the 3'UTR comprises a sequence set forth in any one of SEQ ID NO: 1-14 or a sequence having an identity of at least 90% with any thereof, and the 5'UTR comprises a sequence set forth in SEQ ID NO: 27 or a sequence having an identity of at least 90% therewith;
28) the 3'UTR comprises a sequence set forth in any one of SEQ ID NO: 1-14 or a sequence having an identity of at least 90% with any thereof, and the 5'UTR comprises a sequence set forth in SEQ ID NO: 28 or a sequence having an identity of at least 90% therewith;
29) the 3'UTR comprises a sequence set forth in any one of SEQ ID NO: 1-14 or a sequence having an identity of at least 90% with any thereof, and the 5'UTR comprises a sequence set forth in SEQ ID NO: 29 or a sequence having an identity of at least 90% therewith;
30) the 3'UTR comprises a sequence set forth in any one of SEQ ID NO: 1-14 or a sequence having an identity of at least 90% with any thereof, and the 5'UTR comprises a sequence set forth in SEQ ID NO: 30 or a sequence having an identity of at least 90% therewith;
31) the 3'UTR comprises a sequence set forth in any one of SEQ ID NO: 1-14 or a sequence having an identity of at least 90% with any thereof, and the 5'UTR comprises a sequence set forth in SEQ ID NO: 31 or a sequence having an identity of at least 90% therewith;
32) the 3'UTR comprises a sequence set forth in any one of SEQ ID NO: 1-14 or a sequence having an identity of at least 90% with any thereof, and the 5'UTR comprises a sequence set forth in SEQ ID NO: 32 or a sequence having an identity of at least 90% therewith;
33) the 3'UTR comprises a sequence set forth in any one of SEQ ID NO: 1-14 or a sequence having an identity of at least 90% with any thereof, and the 5'UTR comprises a sequence set forth in SEQ ID NO: 33 or a sequence having an identity of at least 90% therewith;
34) the 3'UTR comprises a sequence set forth in any one of SEQ ID NO: 1-14 or a sequence having an identity of at least 90% with any thereof, and the 5'UTR comprises a sequence set forth in SEQ ID NO: 34 or a sequence having an identity of at least 90%, 95% therewith;
35) the 3'UTR comprises a sequence set forth in any one of SEQ ID NO: 1-14 or a sequence having an identity of at least 90% with any thereof, and the 5'UTR comprises a sequence set forth in SEQ ID NO: 35 or a sequence having an identity of at least 90% therewith;
36) the 3'UTR comprises a sequence set forth in any one of SEQ ID NO: 1-14 or a sequence having an identity of at least 90% with any thereof, and the 5'UTR comprises a sequence set forth in SEQ ID NO: 36 or a sequence having an identity of at least 90% therewith;
37) the 3'UTR comprises a sequence set forth in any one of SEQ ID NO: 1-14 or a sequence having an identity of at least 90% with any thereof, and the 5'UTR comprises a sequence set forth in SEQ ID NO: 37 or a sequence having an identity of at least 90% therewith;
38) the 3'UTR comprises a sequence set forth in any one of SEQ ID NO: 1-14 or a sequence having an identity of at least 90% with any thereof, and the 5'UTR comprises a sequence set forth in SEQ ID NO: 38 or a sequence having an identity of at least 90% therewith;
39) the 3'UTR comprises a sequence set forth in any one of SEQ ID NO: 1-14 or a sequence having an identity of at least 90% with any thereof, and the 5'UTR comprises a sequence set forth in SEQ ID NO: 39 or a sequence having an identity of at least 90% therewith;
40) the 3'UTR comprises a sequence set forth in any one of SEQ ID NO: 1-14 or a sequence having an identity of at least 90% with any thereof, and the 5'UTR comprises a sequence set forth in SEQ ID NO: 40 or a sequence having an identity of at least 90% therewith;
41) the 3'UTR comprises a sequence set forth in any one of SEQ ID NO: 1-14 or a sequence having an identity of at least 90% with any thereof, and the 5'UTR comprises a sequence set forth in SEQ ID NO: 41 or a sequence having an identity of at least 90% therewith;
42) the 3'UTR comprises a sequence set forth in any one of SEQ ID NO: 1-14 or a sequence having an identity of at least 90% with any thereof, and the 5'UTR comprises a sequence set forth in SEQ ID NO: 42 or a sequence having an identity of at least 90% therewith;
43) the 3'UTR comprises a sequence set forth in any one of SEQ ID NO: 1-14 or a sequence having an identity of at least 90% with any thereof, and the 5'UTR comprises a sequence set forth in SEQ ID NO: 43 or a sequence having an identity of at least 90% therewith;
44) the 3'UTR comprises a sequence set forth in any one of SEQ ID NO: 1-14 or a sequence having an identity of at least 90% with any thereof, and the 5'UTR comprises a sequence set forth in SEQ ID NO: 44 or a sequence having an identity of at least 90% therewith;
45) the 3'UTR comprises a sequence set forth in any one of SEQ ID NO: 1-14 or a sequence having an identity of at least 90% with any thereof, and the 5'UTR comprises a sequence set forth in SEQ ID NO: 45 or a sequence having an identity of at least 90% with any thereof;
46) the 3'UTR comprises a sequence set forth in any one of SEQ ID NO: 1-14 or a sequence having an identity of at least 90% with any thereof, and the 5'UTR comprises a sequence set forth in SEQ ID NO: 46 or a sequence having an identity of at least 90% with any thereof; or
47) the 3'UTR comprises a sequence set forth in any one of SEQ ID NO: 1-14 or a sequence having an identity of at least 90% with any thereof, and the 5'UTR comprises a sequence set forth in SEQ ID NO: 47 or a sequence having an identity of at least 90% with any thereof;
48) the 3'UTR comprises a sequence set forth in SEQ ID NO: 58 or a sequence having an identity of at least 90% therewith, and the 5'UTR comprises a sequence set forth in SEQ ID NO: 72-104 or a sequence having an identity of at least 90% with any thereof;
49) the 3'UTR comprises a sequence set forth in SEQ ID NO: 59 or a sequence having an identity of at least 90% therewith, and the 5'UTR comprises a sequence set forth in SEQ ID NO: 72-104 or a sequence having an identity of at least 90% with any thereof;
50) the 3'UTR comprises a sequence set forth in SEQ ID NO: 60 or a sequence having an identity of at least 90% therewith, and the 5'UTR comprises a sequence set forth in SEQ ID NO: 72-104 or a sequence having an identity of at least 90% with any thereof;
51) the 3'UTR comprises a sequence set forth in SEQ ID NO: 61 or a sequence having an identity of at least 90% therewith, and the 5'UTR comprises a sequence set forth in SEQ ID NO: 72-104 or a sequence having an identity of at least 90% with any thereof;
52) the 3'UTR comprises a sequence set forth in SEQ ID NO: 62 or a sequence having an identity of at least 90% therewith, and the 5'UTR comprises a sequence set forth in SEQ ID NO: 72-104 or a sequence having an identity of at least 90% with any thereof;
53) the 3'UTR comprises a sequence set forth in SEQ ID NO: 63 or a sequence having an identity of at least 90% therewith, and the 5'UTR comprises a sequence set forth in SEQ ID NO: 72-104 or a sequence having an identity of at least 90% with any thereof;
54) the 3'UTR comprises a sequence set forth in SEQ ID NO: 64 or a sequence having an identity of at least 90% therewith, and the 5'UTR comprises a sequence set forth in SEQ ID NO: 72-104 or a sequence having an identity of at least 90% with any thereof;
55) the 3'UTR comprises a sequence set forth in SEQ ID NO: 65 or a sequence having an identity of at least 90% therewith, and the 5'UTR comprises a sequence set forth in SEQ ID NO: 72-104 or a sequence having an identity of at least 90% with any thereof;
56) the 3'UTR comprises a sequence set forth in SEQ ID NO: 66 or a sequence having an identity of at least 90% therewith, and the 5'UTR comprises a sequence set forth in SEQ ID NO: 72-104 or a sequence having an identity of at least 90% with any thereof;
57) the 3'UTR comprises a sequence set forth in SEQ ID NO: 67 or a sequence having an identity of at least 90% therewith, and the 5'UTR comprises a sequence set forth in any one of SEQ ID NO: 72-104 or a sequence having an identity of at least 90% with any thereof;
58) the 3'UTR comprises a sequence set forth in SEQ ID NO: 68 or a sequence having an identity of at least 90% therewith, and the 5'UTR comprises a sequence set forth in SEQ ID NO: 72-104 or a sequence having an identity of at least 90% with any thereof;
59) the 3'UTR comprises a sequence set forth in SEQ ID NO: 69 or a sequence having an identity of at least 90% therewith, and the 5'UTR comprises a sequence set forth in any one of SEQ ID NO: 72-104 or a sequence having an identity of at least 90% with any thereof;
60) the 3'UTR comprises a sequence set forth in SEQ ID NO: 70 or a sequence having an identity of at least 90% therewith, and the 5'UTR comprises a sequence set forth in any one of SEQ ID NO: 72-104 or a sequence having an identity of at least 90% with any thereof;
61) the 3'UTR comprises a sequence set forth in SEQ ID NO: 71 or a sequence having an identity of at least 90% therewith, and the 5'UTR comprises a sequence set forth in any one of SEQ ID NO: 72-104 or a sequence having an identity of at least 90% with any thereof;
62) the 3'UTR comprises a sequence set forth in any one of SEQ ID NO: 58-71 or a sequence having an identity of at least 90% therewith, and the 5'UTR comprises a sequence set forth in SEQ ID NO: 72 or a sequence having an identity of at least 90% therewith;
63) the 3'UTR comprises a sequence set forth in any one of SEQ ID NO: 58-71 or a sequence having an identity of at least 90% with any thereof, and the 5'UTR comprises a sequence set forth in SEQ ID NO: 73 or a sequence having an identity of at least 90% therewith;
64) the 3'UTR comprises a sequence set forth in any one of SEQ ID NO: 58-71 or a sequence having an identity of at least 90% with any thereof, and the 5'UTR comprises a sequence set forth in SEQ ID NO: 74 or a sequence having an identity of at least 90% therewith;
65) the 3'UTR comprises a sequence set forth in any one of SEQ ID NO: 58-71 or a sequence having an identity of at least 90% with any thereof, and the 5'UTR comprises a sequence set forth in SEQ ID NO: 75 or a sequence having an identity of at least 90% therewith;
66) the 3'UTR comprises a sequence set forth in any one of SEQ ID NO: 58-71 or a sequence having an identity of at least 90% with any thereof, and the 5'UTR comprises a sequence set forth in SEQ ID NO: 76 or a sequence having an identity of at least 90% therewith;
67) the 3'UTR comprises a sequence set forth in any one of SEQ ID NO: 58-71 or a sequence having an identity of at least 90% with any thereof, and the 5'UTR comprises a sequence set forth in SEQ ID NO: 77 or a sequence having an identity of at least 90% therewith;
68) the 3'UTR comprises a sequence set forth in any one of SEQ ID NO: 58-71 or a sequence having an identity of at least 90% with any thereof, and the 5'UTR comprises a sequence set forth in SEQ ID NO: 78 or a sequence having an identity of at least 90% therewith;
69) the 3'UTR comprises a sequence set forth in any one of SEQ ID NO: 58-71 or a sequence having an identity of at least 90% with any thereof, and the 5'UTR comprises a sequence set forth in SEQ ID NO: 79 or a sequence having an identity of at least 90% therewith;
70) the 3'UTR comprises a sequence set forth in any one of SEQ ID NO: 58-71 or a sequence having an identity of at least 90% with any thereof, and the 5'UTR comprises a sequence set forth in SEQ ID NO: 80 or a sequence having an identity of at least 90% therewith;
71) the 3'UTR comprises a sequence set forth in any one of SEQ ID NO: 58-71 or a sequence having an identity of at least 90% with any thereof, and the 5'UTR comprises a sequence set forth in SEQ ID NO: 81 or a sequence having an identity of at least 90% therewith;
72) the 3'UTR comprises a sequence set forth in any one of SEQ ID NO: 58-71 or a sequence having an identity of at least 90% with any thereof, and the 5'UTR comprises a sequence set forth in SEQ ID NO: 82 or a sequence having an identity of at least 90% therewith;
73) the 3'UTR comprises a sequence set forth in any one of SEQ ID NO: 58-71 or a sequence having an identity of at least 90% with any thereof, and the 5'UTR comprises a sequence set forth in SEQ ID NO: 83 or a sequence having an identity of at least 90% therewith;
74) the 3'UTR comprises a sequence set forth in any one of SEQ ID NO: 58-71 or a sequence having an identity of at least 90% with any thereof, and the 5'UTR comprises a sequence set forth in SEQ ID NO: 84 or a sequence having an identity of at least 90% therewith;
75) the 3'UTR comprises a sequence set forth in any one of SEQ ID NO: 58-71 or a sequence having an identity of at least 90% with any thereof, and the 5'UTR comprises a sequence set forth in SEQ ID NO: 85 or a sequence having an identity of at least 90% therewith;
76) the 3'UTR comprises a sequence set forth in any one of SEQ ID NO: 58-71 or a sequence having an identity of at least 90% with any thereof, and the 5'UTR comprises a sequence set forth in SEQ ID NO: 86 or a sequence having an identity of at least 90% therewith;
77) the 3'UTR comprises a sequence set forth in any one of SEQ ID NO: 58-71 or a sequence having an identity of at least 90% with any thereof, and the 5'UTR comprises a sequence set forth in SEQ ID NO: 87 or a sequence having an identity of at least 90% therewith;
78) the 3'UTR comprises a sequence set forth in any one of SEQ ID NO: 58-71 or a sequence having an identity of at least 90% with any thereof, and the 5'UTR comprises a sequence set forth in SEQ ID NO: 88 or a set forth having an identity of at least 90% therewith;
79) the 3'UTR comprises a sequence set forth in any one of SEQ ID NO: 58-71 or a sequence having an identity of at least 90% with any thereof, and the 5'UTR comprises a sequence set forth in SEQ ID NO: 89 or a sequence having an identity of at least 90% therewith;
80) the 3'UTR comprises a sequence set forth in any one of SEQ ID NO: 58-71 or a sequence having an identity of at least 90% with any thereof, and the 5'UTR comprises a sequence set forth in SEQ ID NO: 90 or a sequence having an identity of at least 90% therewith;
81) the 3'UTR comprises a sequence set forth in any one of SEQ ID NO: 58-71 or a sequence having an identity of at least 90% with any thereof, and the 5'UTR comprises a sequence set forth in SEQ ID NO: 91 or a sequence having an identity of at least 90%, 95% with any thereof;
82) the 3'UTR comprises a sequence set forth in any one of SEQ ID NO: 58-71 or a sequence having an identity of at least 90% with any thereof, and the 5'UTR comprises a sequence set forth in SEQ ID NO: 92 or a sequence having an identity of at least 90% therewith;
83) the 3'UTR comprises a sequence set forth in any one of SEQ ID NO: 58-71 or a sequence having an identity of at least 90% with any thereof, and the 5'UTR comprises a sequence set forth in SEQ ID NO: 93 or a sequence having an identity of at least 90% therewith;
84) the 3'UTR comprises a sequence set forth in any one of SEQ ID NO: 58-71 or a sequence having an identity of at least 90% with any thereof, and the 5'UTR comprises a sequence set forth in SEQ ID NO: 94 or a sequence having an identity of at least 90% therewith;
85) the 3'UTR comprises a sequence set forth in any one of SEQ ID NO: 58-71 or a sequence having an identity of at least 90% with any thereof, and the 5'UTR comprises a sequence set forth in SEQ ID NO: 95 or a sequence having an identity of at least 90% therewith;
86) the 3'UTR comprises a sequence set forth in any one of SEQ ID NO: 58-71 or a sequence having an identity of at least 90% with any thereof, and the 5'UTR comprises a sequence set forth in SEQ ID NO: 96 or a sequence having an identity of at least 90% therewith;
87) the 3'UTR comprises a sequence set forth in any one of SEQ ID NO: 58-71 or a sequence having an identity of at least 90% with any thereof, and the 5'UTR comprises a sequence set forth in SEQ ID NO: 97 or a sequence having an identity of at least 90% therewith;
88) the 3'UTR comprises a sequence set forth in any one of SEQ ID NO: 58-71 or a sequence having an identity of at least 90% with any thereof, and the 5'UTR comprises a sequence set forth in SEQ ID NO: 98 or a sequence having an identity of at least 90% therewith;
89) the 3'UTR comprises a sequence set forth in any one of SEQ ID NO: 58-71 or a sequence having an identity of at least 90% with any thereof, and the 5'UTR comprises a sequence set forth in SEQ ID NO: 99 or a sequence having an identity of at least 90% therewith;
90) the 3'UTR comprises a sequence set forth in any one of SEQ ID NO: 58-71 or a sequence having an identity of at least 90% with any thereof, and the 5'UTR comprises a sequence set forth in SEQ ID NO: 100 or a sequence having an identity of at least 90% therewith;
91) the 3'UTR comprises a sequence set forth in any one of SEQ ID NO: 58-71 or a sequence having an identity of at least 90% with any thereof, and the 5'UTR comprises a sequence set forth in SEQ ID NO: 101 or a sequence having an identity of at least 90% therewith;
92) the 3'UTR comprises a sequence set forth in any one of SEQ ID NO: 58-71 or a sequence having an identity of at least 90% with any thereof, and the 5'UTR comprises a sequence set forth in SEQ ID NO: 102 or a sequence having an identity of at least 90% therewith;
93) the 3'UTR comprises a sequence set forth in any one of SEQ ID NO: 58-71 or a sequence having an identity of at least 90% with any thereof, and the 5'UTR comprises a sequence set forth in SEQ ID NO: 103 or a sequence having an identity of at least 90% therewith; or
94) the 3'UTR comprises a sequence set forth in any one of SEQ ID NO: 58-71 or a sequence having an identity of at least 90% with any thereof, and the 5'UTR comprises a sequence set forth in SEQ ID NO: 104 or a sequence having an identity of at least 90% therewith.

20. The lipid nanoparticle according to any one of claims 1 to 8 and 18 to 19, wherein,
the 3'UTR comprises a sequence set forth in any one of SEQ ID NO: 12-14 or a sequence having an identity of at least 90% with any thereof, and the 5'UTR comprises a sequence set forth in any one of SEQ ID NO: 15, 29, 30, 32 or a sequence having an identity of at least 90% with any thereof; or
the 3'UTR comprises a sequence set forth in any one of SEQ ID NO: 69-71 or a sequence having an identity of at least 90% therewith, and the 5'UTR comprises a sequence set forth in any one of SEQ ID NO: 72, 86, 87, and 89 or a sequence having an identity of at least 90% with any thereof;
preferably,
the 3'UTR comprises a sequence set forth in SEQ ID NO: 12 or a sequence having an identity of at least 90% therewith, and the 5'UTR comprises a sequence set forth in any one of SEQ ID NO: 15, 29, 30, 32 or a sequence having an identity of at least 90% therewith,
the 3'UTR comprises a sequence set forth in SEQ ID NO: 13 or a sequence having an identity of at least 90% therewith, and the 5'UTR comprises a sequence set forth in any one of SEQ ID NO: 15, 29, 30, 32 or a sequence having an identity of at least 90% therewith,
the 3'UTR comprises a sequence set forth in SEQ ID NO: 14 or a sequence having an identity of at least 90% therewith, and the 5'UTR comprises a sequence set forth in any one of SEQ ID NO: 15, 29, 30, 32 or a sequence having an identity of at least 90% therewith,
the 3'UTR comprises a sequence set forth in any one of SEQ ID NO: 12-14 or a sequence having an identity of at least 90% therewith, and the 5'UTR comprises a sequence set forth in SEQ ID NO: 15 or a sequence having an identity of at least 90% therewith,
the 3'UTR comprises a sequence set forth in any one of SEQ ID NO: 12-14 or a sequence having an identity of at least 90% therewith, and the 5'UTR comprises a sequence set forth in SEQ ID NO: 29 or a sequence having an identity of at least 90% therewith,
the 3'UTR comprises a sequence set forth in any one of SEQ ID NO: 12-14 or a sequence having an identity of at least 90% therewith, and the 5'UTR comprises a sequence set forth in SEQ ID NO: 30 or a sequence having an identity of at least 90% therewith,
the 3'UTR comprises a sequence set forth in any one of SEQ ID NO: 12-14 or a sequence having an identity of at least 90% therewith, and the 5'UTR comprises a sequence set forth in SEQ ID NO: 32 or a sequence having an identity of at least 90% therewith,
the 3'UTR comprises a sequence set forth in SEQ ID NO: 69 or a sequence having an identity of at least 90% therewith, and the 5'UTR comprises a sequence set forth in any one of SEQ ID NO: 72, 86, 87, 89 or a sequence having an identity of at least 90% therewith,
the 3'UTR comprises a sequence set forth in SEQ ID NO: 70 or a sequence having an identity of at least 90% therewith, and the 5'UTR comprises a sequence set forth in any one of SEQ ID NO: 72, 86, 87, 89 or a sequence having an identity of at least 90% therewith,
the 3'UTR comprises a sequence set forth in SEQ ID NO: 71 or a sequence having an identity of at least 90% therewith, and the 5'UTR comprises a sequence set forth in any one of SEQ ID NO: 72, 86, 87, 89 or a sequence having an identity of at least 90% therewith,
the 3'UTR comprises a sequence set forth in any one of SEQ ID NO: 69-71 or a sequence having an identity of at least 90% therewith, and the 5'UTR comprises a sequence set forth in SEQ ID NO: 72 or a sequence having an identity of at least 90% therewith,
the 3'UTR comprises a sequence set forth in any one of SEQ ID NO: 69-71 or a sequence having an identity of at least 90% therewith, and the 5'UTR comprises a sequence set forth in SEQ ID NO: 86 or a sequence having an identity of at least 90% therewith,
the 3'UTR comprises a sequence set forth in any one of SEQ ID NO: 69-71 or a sequence having an identity of at least 90% therewith, and the 5'UTR comprises a sequence set forth in SEQ ID NO: 87 or a sequence having an identity of at least 90% therewith, or
the 3'UTR comprises a sequence set forth in any one of SEQ ID NO: 69-71 or a sequence having an identity of at least 90% therewith, and the 5'UTR comprises a sequence set forth in SEQ ID NO: 89 or a sequence having an identity of at least 90% therewith.

21. The lipid nanoparticle according to any one of claims 1 to 8 and 18 to 20, wherein the nucleic acid construct further comprises:
(c) a polyadenylation (poly-A) tail;
preferably, the poly-A tail is selected from A120, A30L70, HGH polyA, SV40 polyA, BGH polyA, rbGlob polyA, and SV40late polyA;
preferably, the poly-A tail is selected from A120 and A30L70, the A120 comprises 120 adenine nucleotides, and the A30L70 comprises a sequence set forth in SEQ ID NO: 52 or a sequence having an identity of at least 90% therewith.

22. The lipid nanoparticle according to any one of claims 1 to 8 and 18 to 21, wherein the ORF encodes hepatocyte growth factor (HGF), an antibody, or an antigen-binding fragment thereof, and preferably human hepatocyte growth factor (hHGF), an anti-PD-1 antibody, or an antigen-binding fragment thereof.

23. The lipid nanoparticle according to claim 22, wherein the ORF comprises any one selected from the following 1) to 2):
1) a polynucleotide sequence encoding an amino acid sequence set forth in SEQ ID NO: 109;
2) a polynucleotide sequence set forth in any one of SEQ ID NO: 110-113, 128-131 or a polynucleotide sequence having an identity of at least 90% therewith;
or, the ORF comprises any one selected from the following 1) to 4):
1) a polynucleotide sequence encoding a heavy chain amino acid sequence set forth in SEQ ID NO: 117, and/or a polynucleotide sequence encoding a light chain amino acid sequence set forth in SEQ ID NO: 118;
2) a polynucleotide sequence encoding HCDR1, HCDR2, and HCDR3 in the heavy chain amino acid sequence set forth in SEQ ID NO: 117, and a polynucleotide sequence encoding LCDR1, LCDR2, and LCDR3 in the light chain amino acid sequence set forth in SEQ ID NO: 118, wherein the CDRs are defined according to a Kabat, IMGT, Chothia, AbM, or Contact numbering system, and preferably defined according to the Kabat numbering system;
3) a polynucleotide sequence set forth in SEQ ID NO: 119 or a polynucleotide sequence having an identity of at least 90% therewith, and/or a polynucleotide sequence set forth in SEQ ID NO: 120 or a polynucleotide sequence having an identity of at least 90% therewith;
4) a polynucleotide sequence set forth in SEQ ID NO: 121 or a polynucleotide sequence having an identity of at least 90% therewith, and/or a polynucleotide sequence set forth in SEQ ID NO: 122 or a polynucleotide sequence having an identity of at least 90% therewith.

24. The lipid nanoparticle according to any one of claims 22 to 23, comprising a sequence set forth in any one of SEQ ID NO: 115, 116, 127, 132 or a sequence having an identity of at least 90% therewith; or, comprising a sequence set forth in SEQ ID NO: 124 or a sequence having an identity of at least 90% therewith and/or a sequence set forth in SEQ ID NO: 125 or a sequence having an identity of at least 90% therewith.

25. The lipid nanoparticle according to any one of claims 1 to 8 and 18 to 24, wherein the nucleic acid construct is DNA or RNA, preferably RNA, and more preferably mRNA.

26. The lipid nanoparticle according to claim 25, wherein the RNA further comprises: (d) a 5' cap structure (5'Cap);
preferably, the 5'Cap is selected from Cap0, Cap1, Cap2, Cap3, Cap4, ARCA, modified ARCA, inosine, N1-methyl-guanosine, 2'-fluoro-guanosine, 7-deaza-guanosine, 8-oxo-guanosine, 2-amino-guanosine, LNA-guanosine, and 2-azido-guanosine;
more preferably, the 5'Cap is selected from ARCA, 3'-O-Me-m⁷G(5')ppp(5')G, m⁷G(5')ppp(5')(2'OMeA)pU, m⁷Gppp(A2'O-MOE)pG, m⁷G(5')ppp(5')(2'OMeA)pG, m⁷G(5')ppp(5')(2'OMeG)pG, m⁷(3'OMeG)(5')ppp(5')(2'OMeG)pG and m⁷(3'OMeG)(5')ppp(5')(2'OMeA)pG.

27. The lipid nanoparticle according to claim 25, wherein the RNA comprises one or more modifications, preferably, the modification comprises a backbone modification, a glycosyl modification, a base modification, and/or a lipid modification; and more preferably, the base modification is a uracil modification.

28. A method for treating and/or preventing a disease, comprising administering a therapeutically effective amount of the lipid nanoparticle according to any one of claims 1 to 8 and 18 to 27, the lyophilized composition according to any one of claims 9 to 11, the lyophilized preparation according to claim 12, the reconstituted solution according to claim 13, the product according to claim 14, or the spray preparation according to claim 15 to a subject in need thereof;
the disease is selected from ischemic disease, metabolic syndrome, diabetes and complications thereof, restenosis, and a nerve injury;
preferably, the ischemic disease is selected from coronary artery disease (CAD), peripheral artery disease (PAD), myocardial infarction, limb ischemia, thromboangiitis obliterans (TAO), and diabetic artery obliterans (DAO); more preferably, the limb ischemia is Lower limb ischemia, and most preferably, the limb ischemia is critical limb ischemia (CLI);
preferably, the diabetes and complications thereof are selected from diabetic peripheral neuropathy, diabetic foot ulcer (DFU), and diabetic artery obliterans (DAO);
preferably, the restenosis is selected from post-surgical restenosis and post-perfusion restenosis;
preferably, the nerve injury is selected from neurodegenerative disease, traumatic nerve injury, and peripheral neuropathy; more preferably, the neurodegenerative disease is selected from amyotrophic lateral sclerosis (ALS), Parkinson's disease, and dementia, and the peripheral neuropathy is diabetic peripheral neuropathy.
